# EUROPEAN PATENT APPLICATION

(11) **EP 4 595 979 A1**
(43) Date of publication of application: **06.08.2025**
(21) Application number: 23870979.4
(22) Date of filing: 28.09.2023
(51) Int. Cl.: A61K 47/68, A61K 47/65, A61K 47/64, A61K 38/07, C07K 5/02, A61P 35/00

(54) **AURISTATIN DRUG WITH HIGH-STABILITY HYDROPHILIC LINKING UNIT AND CONJUGATE THEREOF**

(30) Priority: 30.09.2022 CN 202211211457; 25.09.2023 CN 202311244532
(71) Applicant: SystImmune, Inc., Redmond WA 98052 (US)
(72) Inventor: ZHU, Yi, Redmond, WA 98052 (US); WAN, Weili, Chengdu, Sichuan 611130 (CN); ZHUO, Shi, Chengdu, Sichuan 611130 (CN); LAI, Weirong, Chengdu, Sichuan 611130 (CN); YANG, Xiujuan, Chengdu, Sichuan 611130 (CN)
(74) Representative: Mathys & Squire
(86) International application number: PCT/CN2023/122381
(87) International publication number: WO 2024/067754

(57) **Abstract**

The application relates to the field of pharmaceutical technology and specifically relates to auristatin-based drugs and conjugates thereof having a highly stable hydrophilic linker unit. In particular, the application relates to a ligand-drug conjugate or pharmaceutically acceptable salts or solvates thereof, and method of preparation and use thereof. Furthermore, the application relates to linker-payloads or isomers, mesomers, racemates, enantiomers or mixtures thereof, or pharmaceutically acceptable salts or solvates thereof, and method of preparation and use thereof.

## Description

### TECHNICAL FIELD

The application relates to the field of pharmaceutical technology and in particular to ligand-drug couplers, linker-payloads, methods of preparation thereof and uses thereof.

### BACKGROUND TECHNOLOGY

Antibody-Drug Conjugates (ADCs) are a new type of targeted therapeutic drugs that combine the advantages of high selectivity of antibodies and high activity of cytotoxic drugs. They have the advantages of "high efficiency and low toxicity" and have become a research hotspot for targeted tumor therapy. In recent years, ADCs have developed rapidly and have developed to the third generation. Currently, 13 ADCs have been approved for marketing, including Mylotarg, Adcetris, Kadcyla, Besponsa, Lumoxiti, Polivy, Enhertu, Padcev, Trodelvy, Blenrep, Zynlonta, Edixi and Tivdak, and more than 200 ADCs have entered clinical trials. ADCs are playing an increasingly important role in the field of targeted tumor therapy.

Antibody-drug conjugates (ADCs) consist of three parts: antibodies (mAbs) with high specificity and affinity, highly stable linkers, and highly efficient small molecule cytotoxic drugs (payload/warhead). The antibody part of ADCs is most commonly IgG1, and the warhead part is generally a cytotoxic drug that acts on microtubules, DNA or RNA, such as maytansines, auristatins, calicheamicin, camptothecins, pyrrolobenzodiazepines and amanita phalloidins. There are two main types of linkers: one is a cleavable linker, and the other is a non-cleavable linker.

Auristatins are a class of tubulin inhibitors that can block tubulin from binding to GTP and block the binding of microtubules to vinblastine binding sites, thereby inducing cell apoptosis and inhibiting tumor growth. Currently, MMAE (US6884869) and MMAF (US7498298) are the most used ADC warheads, both of which are pentapeptides derived from the modification of Dolastatin 10. Both MMAE and MMAF show good anti-tumor activity, but due to factors such as lack of selectivity and low therapeutic index, they cannot be used clinically as a single drug. However, the high cytotoxicity of MMAE and MMAF makes them ideal warheads for ADC. MMAE/MMAF has been widely used in the field of ADC. Currently, there are 5 ADC drugs with MMAE as their warhead and 1 ADC drug with MMAF as its warhead on the market, namely Adcetris, Polivy, Padcev, Aidixi, Tivdak and Blenrep. However, these ADCs with auristatins as toxins have some shortcomings. For example, ADCs with MMAE as toxins have adverse reactions such as neurotoxicity (peripheral neuropathy) and hematotoxicity (thrombocytopenia and neutropenia), and ADCs with MMAF as toxins have eye toxicity, etc., and their safety needs to be improved. In addition, most of the linkers used in these ADCs are VClinkers, and the highest DAR is 4. This is because VClinkers are highly hydrophobic. However, high DAR easily leads to precipitation and aggregation of ADCs, so it is impossible to increase their DAR and enhance the efficacy.

Furthermore, most of the current ADCs using MMAE as a warhead introduce linkers from the secondary amine at the N-terminus of MMAE. There are few examples of introducing linkers from the hydroxyl group of MMAE, which have only been reported by Seagen, the Technical University of Denmark, Mersana, and Shanghai New Concept.

In its patent application US2005009751A1, Seagen esterified the hydroxyl group of the MMAE analog Auristatin E and then introduced a linker with a hydrazone bond. This type of linker with an ester bond and a hydrazone bond is unstable in human plasma and will undergo a certain degree of hydrolysis, resulting in some off-target toxicity (see Nat Biotechnol. 2003 Jul;21(7):778-84). In its patent CN105813653B, Seagen introduced a self-eliminating group, methylene alkoxy carbamate (MAC), into the hydroxyl group of MMAE, and then introduced a β-glucuronide linker. Although the ADC with this type of linker has good stability in human plasma, the release of its warhead involves the catalysis of β-glucuronidase and the subsequent self-elimination process, which is relatively complicated.

In its patent application WO2020260597A1, the Technical University of Denmark introduced a linker on the hydroxyl group of the MMAE analog in a similar way to Seagen, and introduced the linker after the esterification reaction, but after the introduction of the linker, its ester bond is difficult to be hydrolyzed by enzymes, and it is not easy to release the warhead. In its patent application CN110234357A, Mersana introduced a linker from the hydroxyl group of the MMAE analog in a similar way to Seagen, introducing an ester bond or carbonate structure, but the ester bond is difficult to be hydrolyzed by enzymes, and the plasma stability of carbonate is poor, which will reduce the efficacy. In its patent application CN106279352A, Shanghai New Concept Company introduced a carbamate on the hydroxyl group of the MMAE analog and then introduced the Mc-Vc-PAB linker. This carbamate has poor stability in plasma and has a potential off-target risk.

Therefore, it is of great clinical significance to develop a new way to introduce a linker to obtain auristatin ADC drugs with higher safety and efficacy.

### SUMMARY

One of the technical problems to be solved by the application is to explore and discover better anti-tumor auristatin ADC drugs, so that they have higher safety and effectiveness and better meet clinical needs.

The application connects auristatin or its derivatives to the enzymatic peptide unit through the aminomethylene structure from the hydroxyl site to form a corresponding linker-payload, which can increase the DAR value of ADC to 8, with better hydrophilicity under high drug loading, better plasma stability under high drug loading value, and more advantages in drug efficacy. Compared with positive drugs (such as ADCs with Vc-MMAE as linker-payload), the same tumor inhibition effect can be achieved with a lower dose, the MTD is improved, and the neurotoxicity and hematotoxicity are reduced. Therefore, the application provides the following applications:
In the first aspect, the application provides a ligand-drug conjugate as shown in general formula I or a pharmaceutically acceptable salt or solvate thereof, 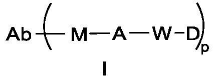 wherein:
Ab is a ligand unit selected from antibodies, antibody fragments, targeting proteins or Fc fusion proteins;
M is a linker unit connected to Ab;
A is selected from a peptide residue consisting of 2 to 7 amino acids, wherein, optionally, each of the amino acids is independently substituted by one or more substituents selected from: deuterium atoms, halogens, hydroxyl groups, cyano groups, amino groups, nitro groups, alkyl groups, substituted alkyl groups, alkoxy groups, cycloalkyl groups, substituted cycloalkyl groups;
W represents an amino methylene oxide structural unit as shown in formula (i): 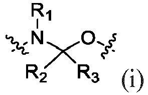 wherein:
   The wavy line on the left represents the connection site between the nitrogen atom and A in formula (i), and the wavy line on the right represents the connection site between the oxygen atom in formula (i) and drug D, and the oxygen atom is a common group between drug D and W;
   R₁, R₂ and R₃ are each independently selected from a hydrogen atom, a deuterium atom, an alkyl group and a substituted alkyl group;
   p is an integer or decimal selected from 1 to 20 (e.g., 1, 2, 3, 4, 5, 6, 7, 8, 9, 10, 11, 12, 13, 14, 15, 16, 17, 18, 19, 20); and
   Drug D is auristatin having a structure shown in formula D, or isomers, meso-, racemic-, enantiomers or mixtures thereof, or a pharmaceutically acceptable salt thereof, 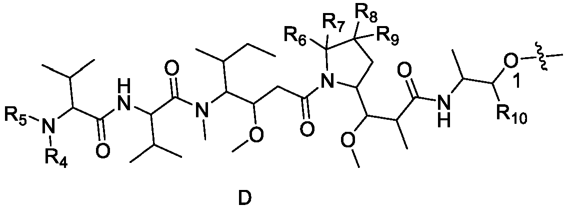 wherein:
      R₄ and R₅ are independently selected from hydrogen atoms, deuterium atoms, alkyl groups and deuterated alkyl groups, or R₄ and R₅ are connected to form the following structure: - (CR₁₁R₁₂)ₙ-B-(CR₁₃R₁₄)ₘ-, wherein R₁₁, R₁₂, R₁₃ and R₁₄ are selected from hydrogen atoms, deuterium atoms, alkyl groups and deuterated alkyl groups; B is selected from O, NR₁₅, CR₁₆R₁₇, wherein R₁₅, R₁₆, R₁₇ are selected from hydrogen atoms, deuterium atoms and alkyl groups; n and m are respectively selected from integers of 0-8 (e.g., 0, 1, 2, 3, 4, 5, 6, 7, 8); the nitrogen atom bonded to R₄ and R₅ forms a ring together with -(CR₁₁R₁₂)ₙ-B-(CR₁₃R₁₄)ₘ-;
      R₆, R₇, R₈, R₉ is independently selected from hydrogen atom, deuterium atom, halogen, azido, alkyl and NR₁₈R₁₉, or any two groups among R₆, R₇, R₈, R₉ together with the atoms to which they are bonded form a cycloalkyl group, and the remaining two groups are independently selected from hydrogen atom, halogen, azido, alkyl and NR₁₈R₁₉, wherein R₁₈ and R₁₉ are selected from hydrogen atom and alkyl;
      R₁₀ is selected from aryl and heteroaryl, and the aryl or heteroaryl is optionally substituted by one or more substituents selected from: hydrogen atom, halogen, alkyl, alkoxy, amino, nitro;
      The wavy line in formula D represents the connection site between oxygen atom 1 and W in the structure of drug D, and the oxygen atom is a common group of drug D and W.

In certain embodiments, R₄ and R₅ are each independently hydrogen, or C₁-C₄alkyl.

In some embodiments, R₄ and R₅ are linked to form the following structure: -(CH₂)₂-B-(CH₂)₂-, B is O, or NH, and to the nitrogen atoms bonded with R₄ and R₅ form a ring together with -(CH₂)₂-B-(CH₂)₂-

In certain embodiments, R₆, R₇, R₈, and R₉ in formula D are each hydrogen.

In certain embodiments, one of R₆, R₇, R₈, and R₉ in formula D is halogen, azide, amino, or the remaining three are each hydrogen.

In certain embodiments, any two groups among R₆, R₇, R₈, R₉ in formula D form a cyclopropyl group together with the atom to which it is bonded, and the remaining two groups are each independently hydrogen.

In certain embodiments, R₁₀ in formula D is a phenyl group, optionally said phenyl is substituted by one or more of said substituents. In certain embodiments, said substituent is amino, or nitro.

In certain embodiments, drug D is, without limitation, selected from the following compounds:

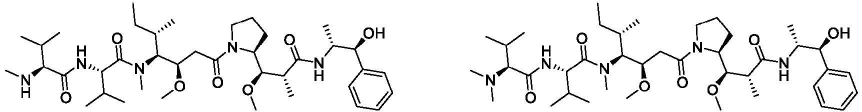

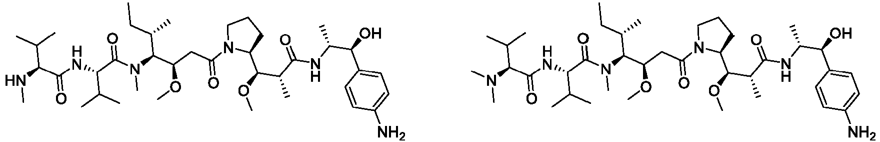

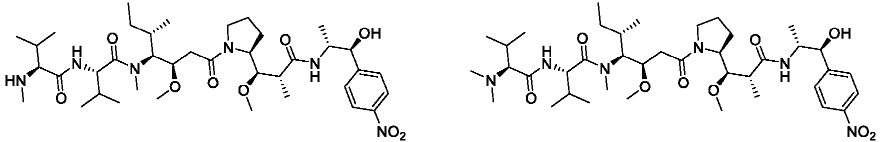

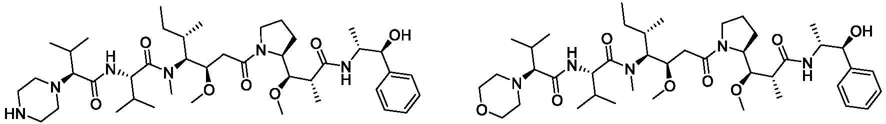

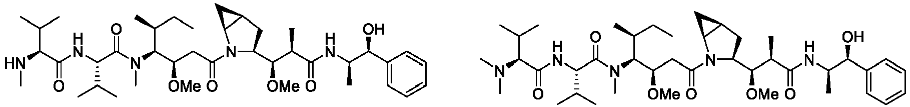

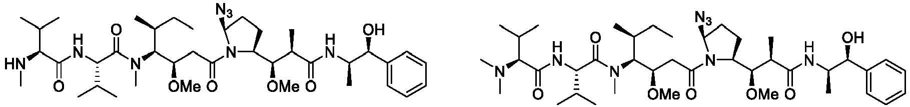

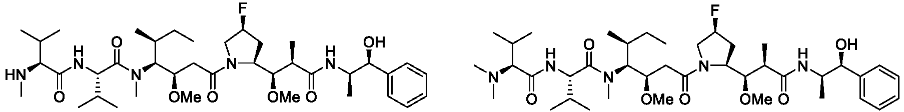

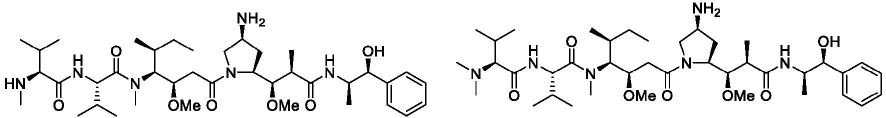

In the above embodiments, drug D is connected to W via a hydroxyl group.

In certain embodiments, the linker unit M comprises maleimide. In these embodiments, the ligand-drug conjugate can be hydrolyzed under hydrolysis conditions, and the hydrolysis site is the maleimide portion of the linker unit. When the ligand contains multiple linkers-drugs, the following situations may occur depending on the degree of hydrolysis:
Maleimide is not hydrolyzed at all, that is, the maleimide is in a closed ring form 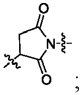 Maleimide is not completely hydrolyzed, that is, part of the maleimide is in a closed ring form, 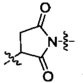 while the other part is in an open ring form 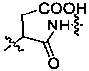 or

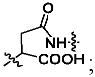

Maleimide is completely hydrolyzed, that is, maleimide is in the open ring form 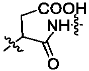 or

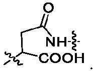

Therefore, when there are multiple maleimide group-containing linking units M in the ADC (i.e., Ab is connected to multiple maleimide group-containing drug-linkers), these maleimide groups can all be in closed-loop form, some in open-loop form, or all in open-loop form.

In the above-mentioned maleimide structural formulas, the left wavy line represents the linkage site to Ab, and the right wavy line represents other structures in M.

In certain embodiments, said ligand-drug conjugate has the structure shown in formula Ia: 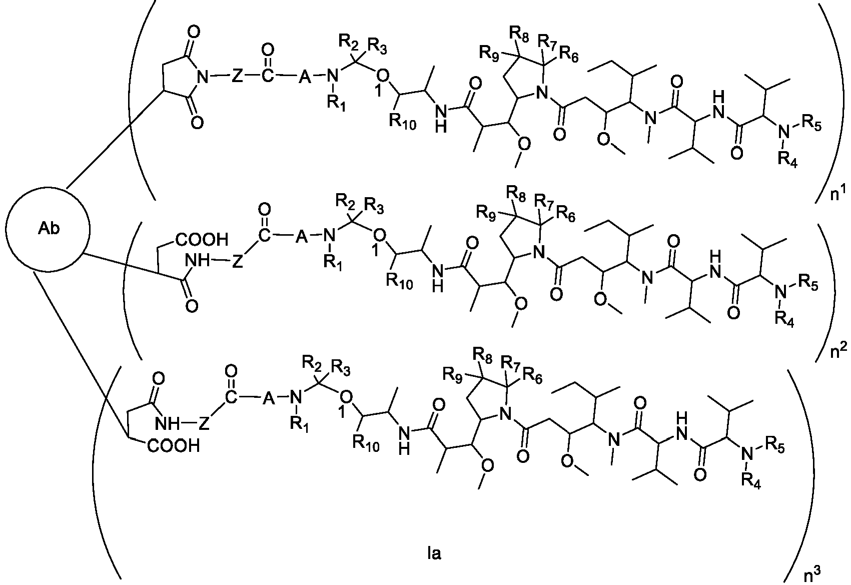 wherein:
Z is selected from -C₁-C₁₀ alkylene-, -C₃-C₈ carbocycle-, -arylene-, -C1-C10 alkylene-arylene-, -arylene-C₁-C₁₀-alkylene-, -C₁-C₁₀ alkylene-(C₃-C₈ carbocycle)-, -(C₃-C₈ carbocycle)-C₁-C₁₀ alkylene-, 3-8 membered heterocycle-, -C₁-C₁₀ alkylene -(3-8 membered heterocycle)-, - (3-8 membered heterocycle)-C₁-C₁₀ alkylene-, -(CH₂CH₂O)ᵣ-, -(CH₂CH₂O)ᵣ-CH₂-, or 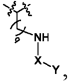 wherein:
X is selected from the group -C₁-C₁₀-C₁-C10 alkylene-, -C₃-C₈ carbocycle-, -arylene-, -C₁-C₁₀ alkylene-arylene-, -arylene-C₁-C₁₀-alkylene-, -C₁-C₁₀ alkylene-(C₃-C₈ carbocycle)-, -(C₃-C₈ carbocycle)-C₁-C₁₀ alkylene-, -3-8 membered heterocycle-, -C₁-C₁₀ alkylene-(3-8 membered heterocycle)-, -(3-8 membered heterocycle)-C₁-C₁₀ alkylene-, -(CH₂CH₂O)ᵣ-, -(CH₂CH₂O)ᵣ-CH₂;
Y is a hydrophilic structure selected from carboxyl, phosphoric acid, polyphosphoric acid, phosphorous acid, sulfonic acid, sulfinic acid or polyethylene glycol (PEG); the heterocycles each independently contain 1 atom selected from N, O, S; the -C₁-C₁₀ alkylene-, -C₃-C₈ carbocycle-, and heterocycle are each independently substituted by one or more substituents selected from deuterium atoms, halogens, hydroxyl groups, cyano groups, nitro groups, amino groups, alkyl groups, heteroalkyl groups, substituted alkyl groups, alkoxy groups, carboxyl groups, or cycloalkyl groups;
the left wavy line in the figure 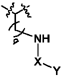 represents the connecting site to the N on the maleimide, and the right wavy line represents the connecting site to the carbonyl group;
r is selected from an integer between 1 and 10 (e.g., 1, 2, 3, 4, 5, 6, 7, 8, 9, 10);
q is selected from an integer between 1 and 8 (e.g., 1, 2, 3, 4, 5, 6, 7, 8);
n¹, n², and n³ are independently chosen from integers or decimals between 0 and 20, n¹, n², and n³ are not simultaneously 0 and n¹ + n² + n³ ≤ 20, e.g., 1 ≤ n¹ + n² + n³ ≤ 2, or 7 ≤ n¹ + n² + n³ ≤ 8.

In some embodiments, n¹, n², n³ are independently selected from 0, 1, 2, 3, 4, 5, 6, 7, 8, 9, 10, 11, 12, 13, 14, 15, 16, 17, 18, 19, 20 (e.g., 0, 0.1, 0.2, 0.3, 0.4, 0.5, 0.6, 0.7, 0.8, 0.9, 1, 1.1, 1.2 1.3, 1.4, 1.5, 1.6, 1.7, 1.8, 1.9, 2, 2.1, 2.2, 2.3, 2.4, 2.5, 2.6, 2.7, 2.8, 2.9, 3 3,.1 3..,2 3,3, 3.4, 3.5, 3.6, 3.7, 3.8, 3.9, 4 4,.1, 4.2, , 4.34.4, 4.5, 4.6 4.7, 4.8, 4.9, 5, 5.1, 5.2, 5.3, 5.4, 5.5, 5.6, 5.7, 5.8 5.,9, 6, 6.1, 6.2, 6.3, 6.4, 6.5, 6.6, 6.7, 6.8, 6.9, 7 7,.1, 7.2, 7.3, 7.4, 7.5, , 7.67.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9 or 10).

In certain embodiments, A in formula I or Ia is selected from a polypeptide residue formed by 2 to 7 amino acids selected from phenylalanine (F), glycine (G), valine (V), lysine (K), alanine (A), citrulline, serine (S), glutamic acid (E) or aspartic acid (D).
In certain embodiments, A is a peptide residue formed by 2-4 amino acids selected from phenylalanine and glycine. In certain embodiments, A is a tetrapeptide residue consisting of glycine-glycine-phenylalanine-glycine.

In certain embodiments, Z is selected from -C₁-C₁₀ alkylene-, such as -C₄-C₆ alkylene-, such as -C₅ alkylene-.

In certain embodiments, Z is 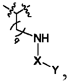 wherein q is selected from an integer between 1-8 (e.g., 1, 2, 3, 4, 5, 6, 7, 8), such as 1.

In certain embodiments, R₁, R₂ and R₃ are each independently selected from a hydrogen atom, a deuterium atom, an alkyl group, a halogenated alkyl group, a deuterated alkyl group and a hydroxyalkyl group.

In some embodiments, R₁, R₂ and R₃ are simultaneously hydrogen atoms or deuterium atoms.

In some embodiments, R₁, R₂ and R₃ are simultaneously hydrogen atoms. In some embodiments, the ligand-drug conjugate has a structure as shown in formula Ib,

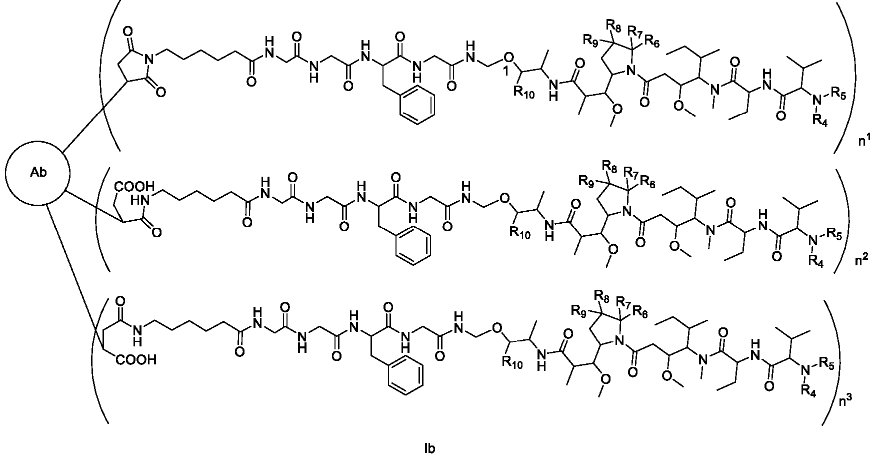

In certain embodiments, said ligand-drug conjugate has a structure as shown in formula Ic,

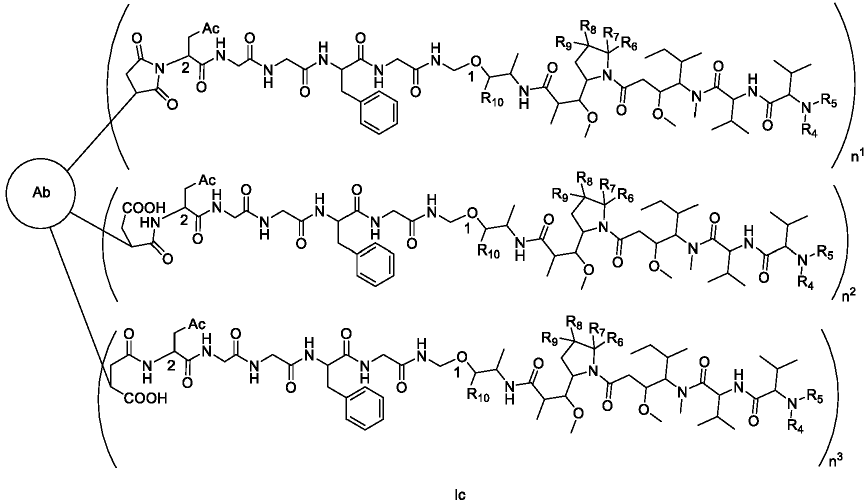

Ac is a hydrophilic structural unit having a structure shown in formula Ic:

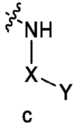

Ac is connected to the marked methylene carbon at position 2 in formula Ie through an amino functional group, and X and Y are as defined above.

In certain embodiments, Ac is selected from glycine, (D/L) alanine, (D/L) leucine, (D/L) isoleucine, (D/L) valine, (D/L) phenylalanine, (D/L) proline, (D/L) tryptophan, (D/L) serine, (D/L) tyrosine, (D/L) cysteine, (D/L) cystine, (D/L) arginine, (D/L) histidine, (D/L) methionine, (D/L) asparagine, (D/L) glutamine, (D/L) threonine, (D/L) aspartic acid, (D/L) glutamic acid, natural or non-natural amino acid derivatives or the following structures,

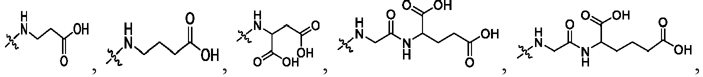

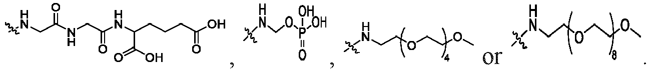

The ligand-drug conjugates of the application can be selected from the following structures without limitation: 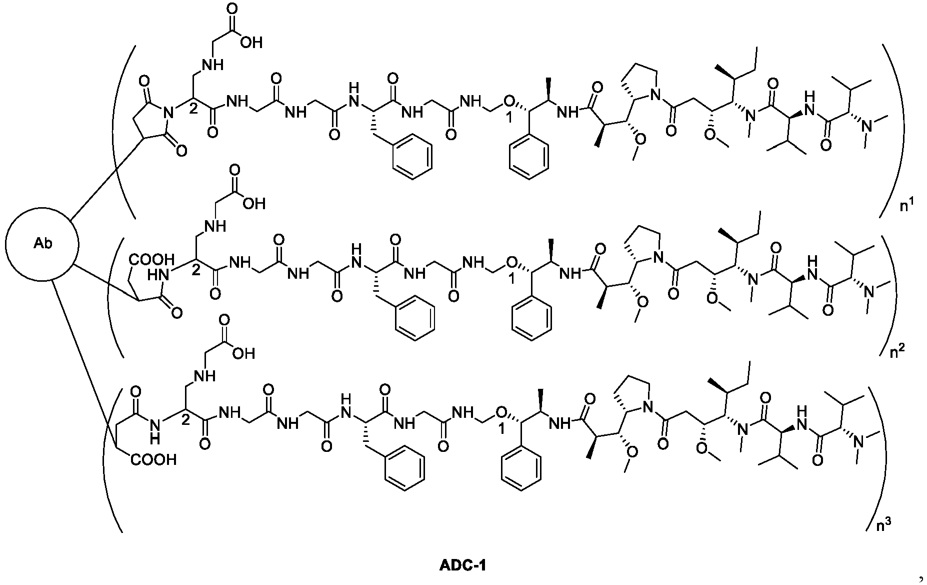 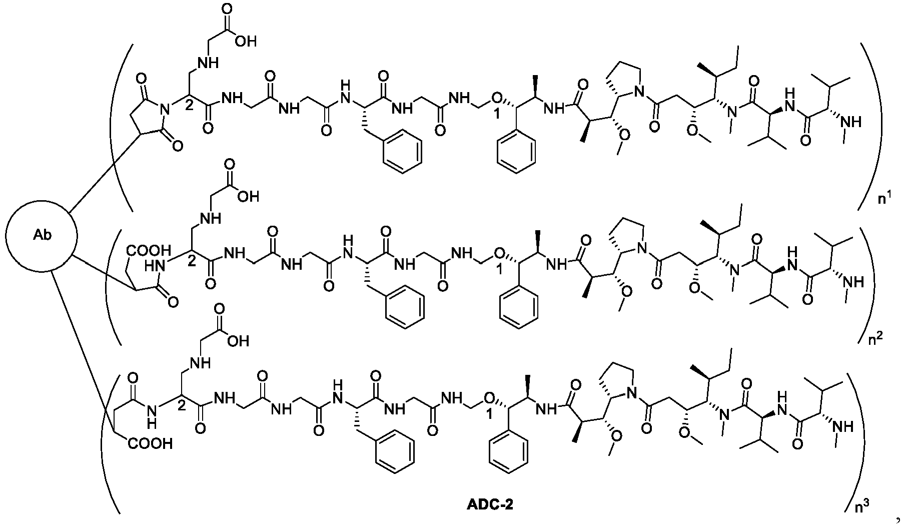 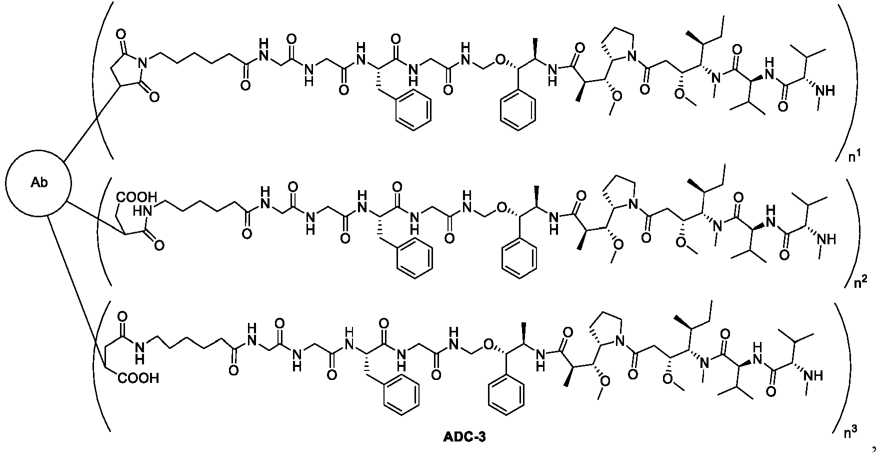 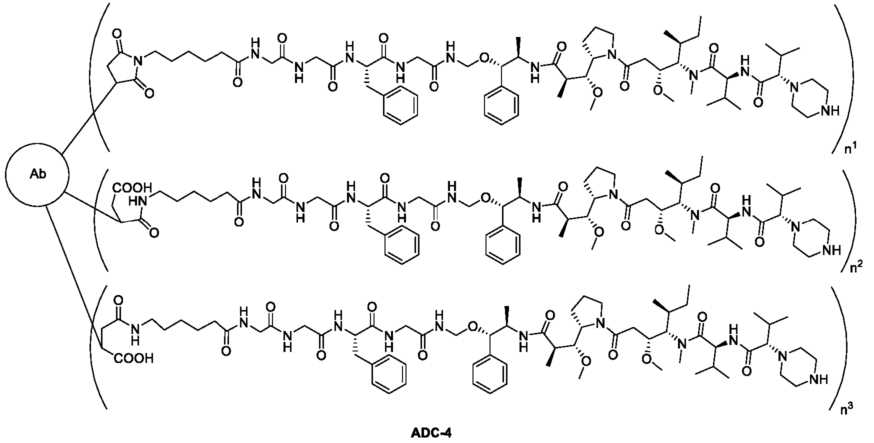 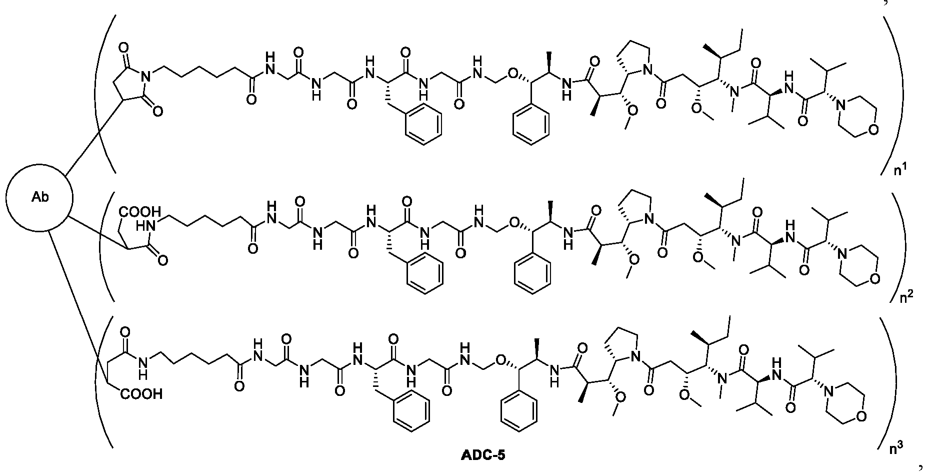 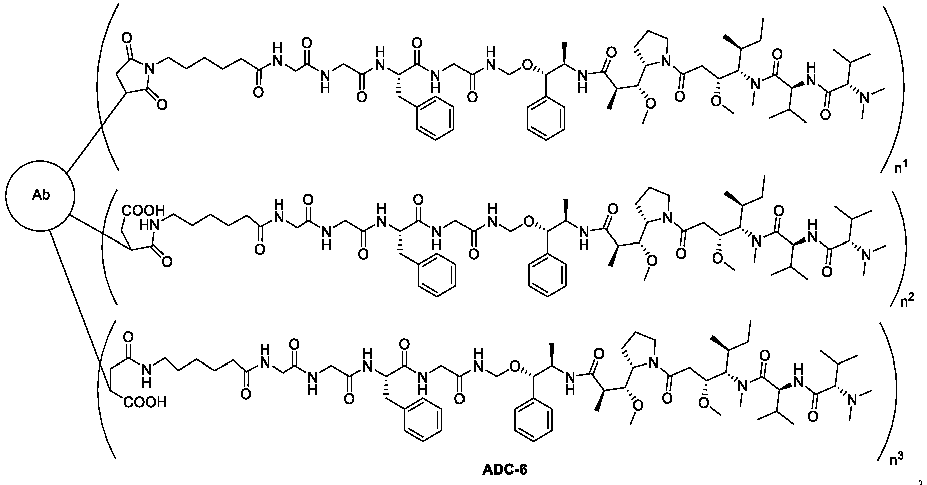 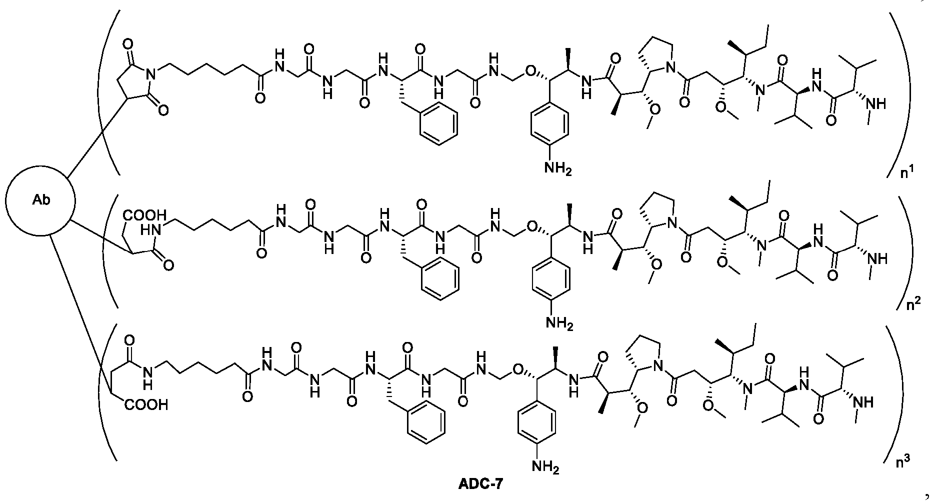 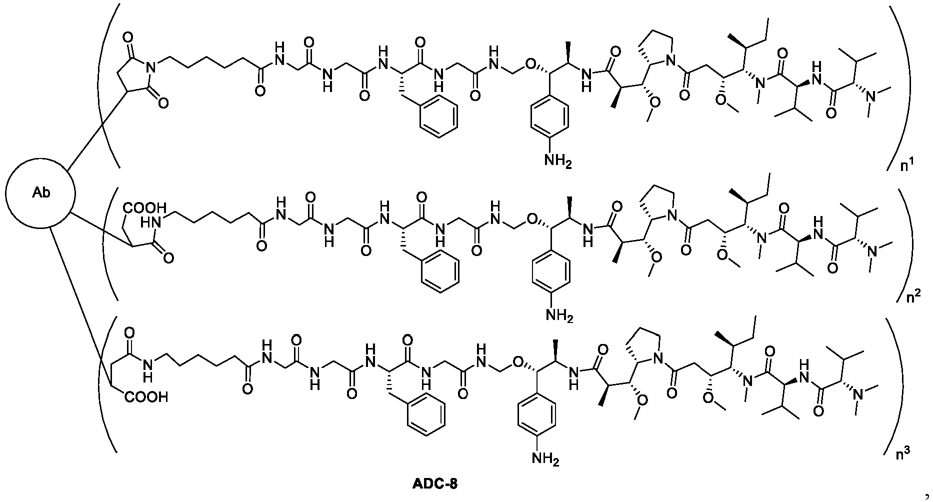 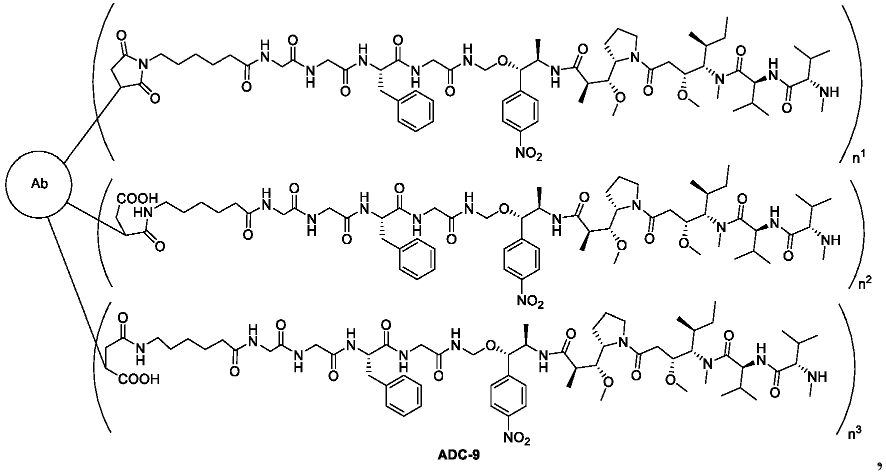 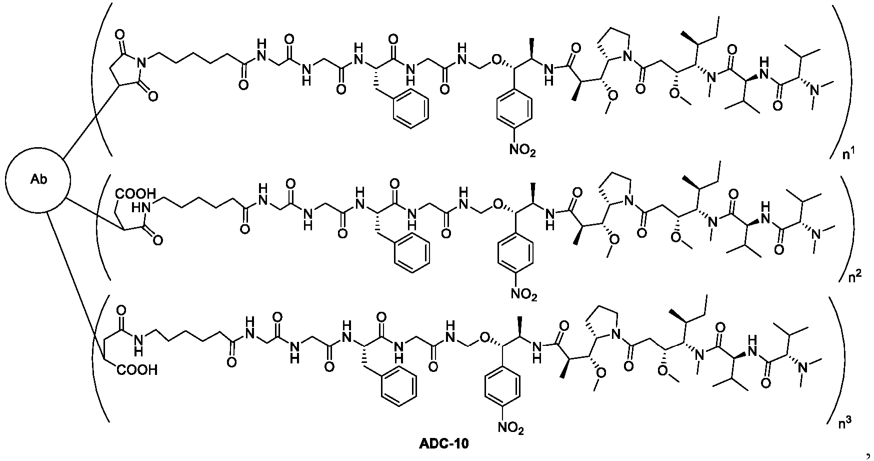 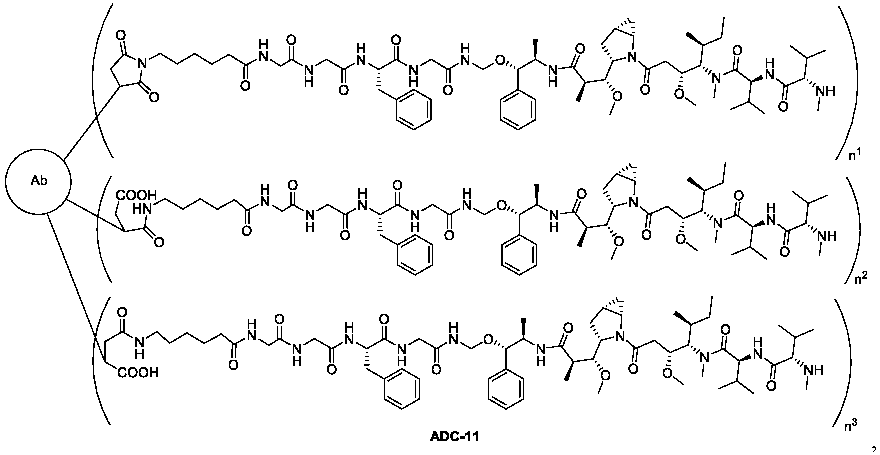 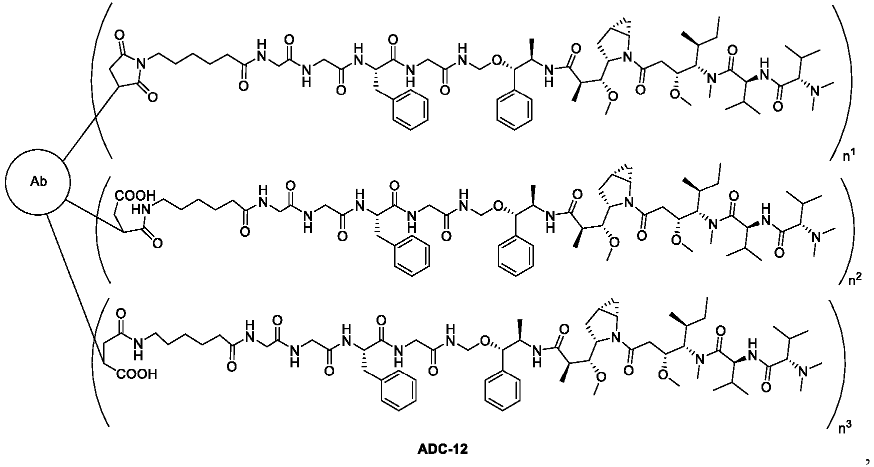 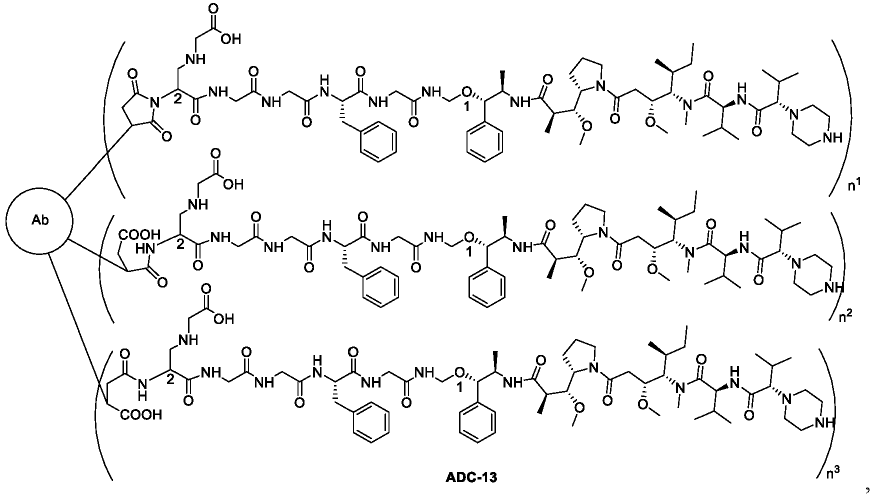 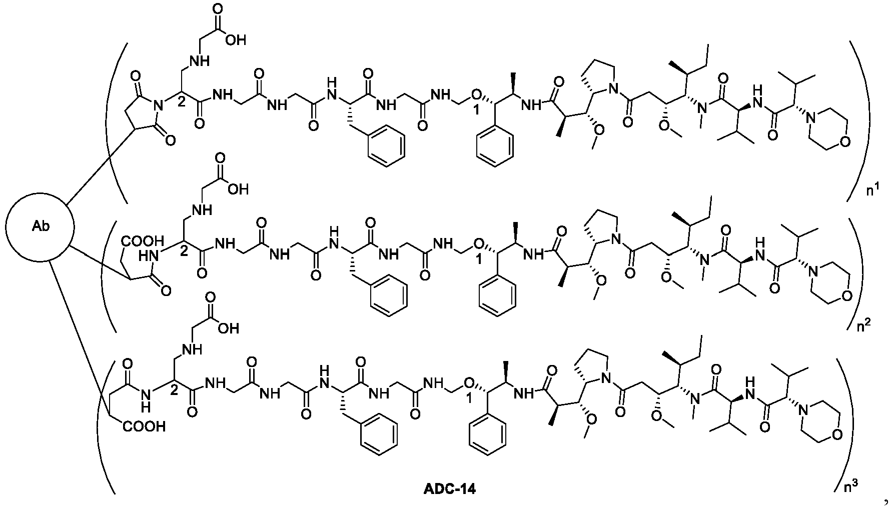 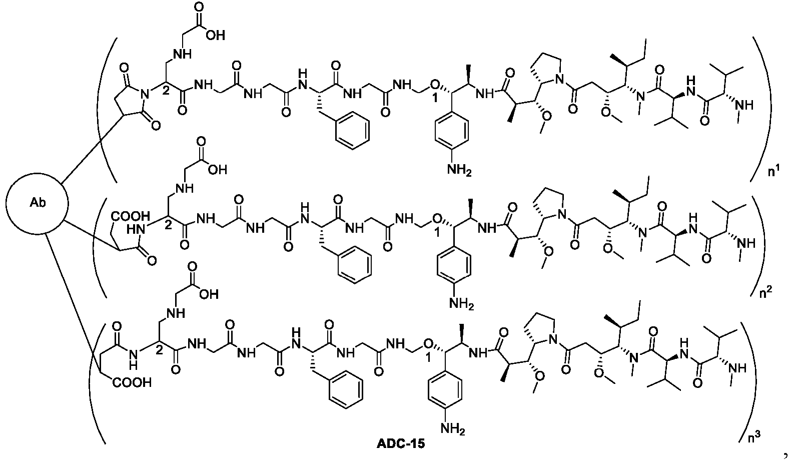 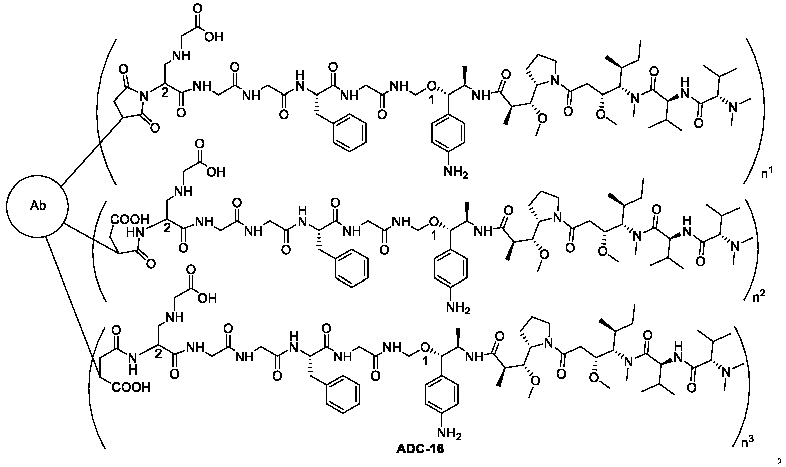 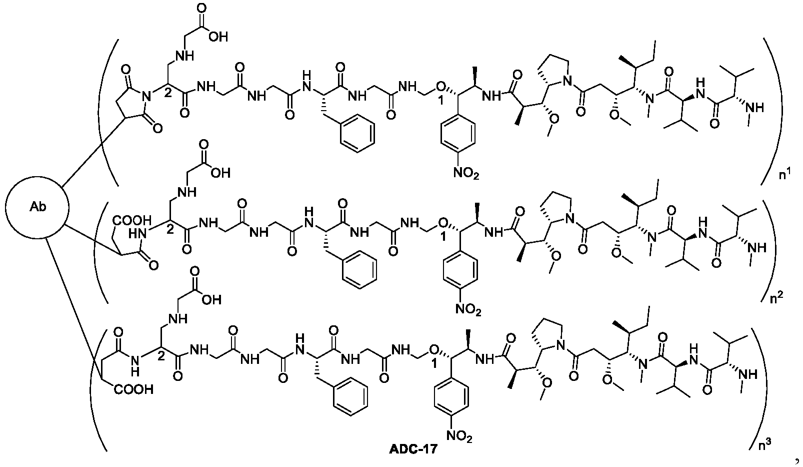 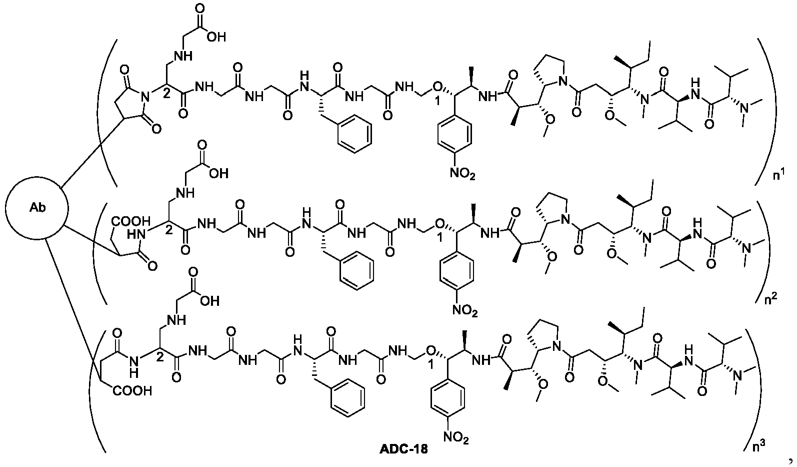 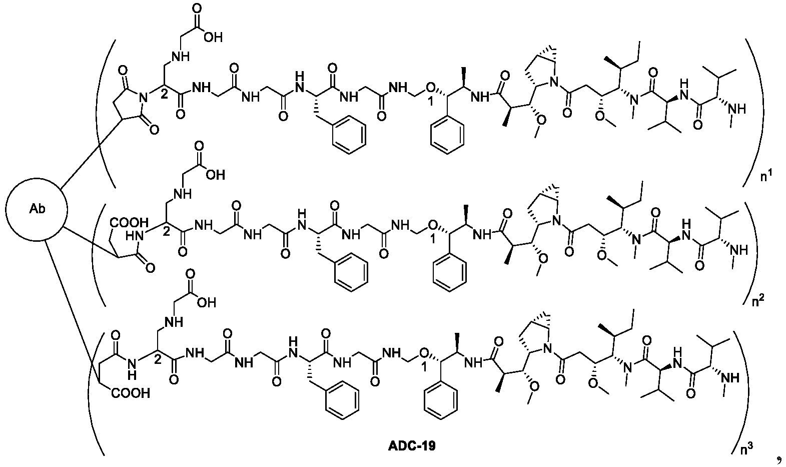 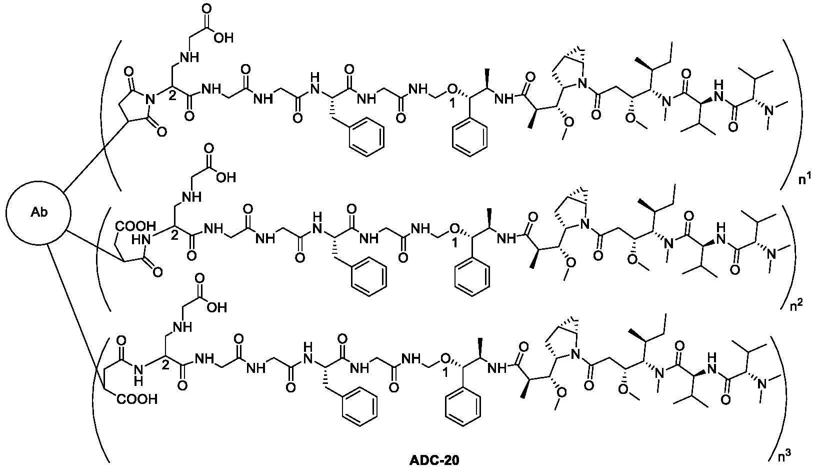 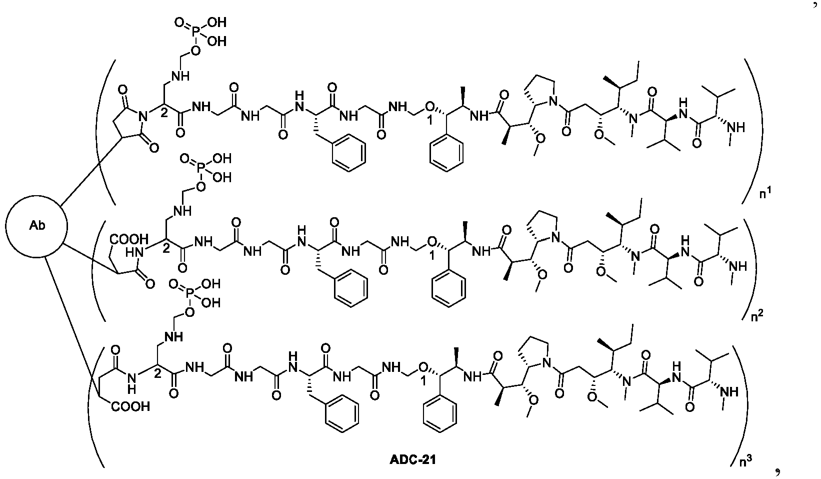 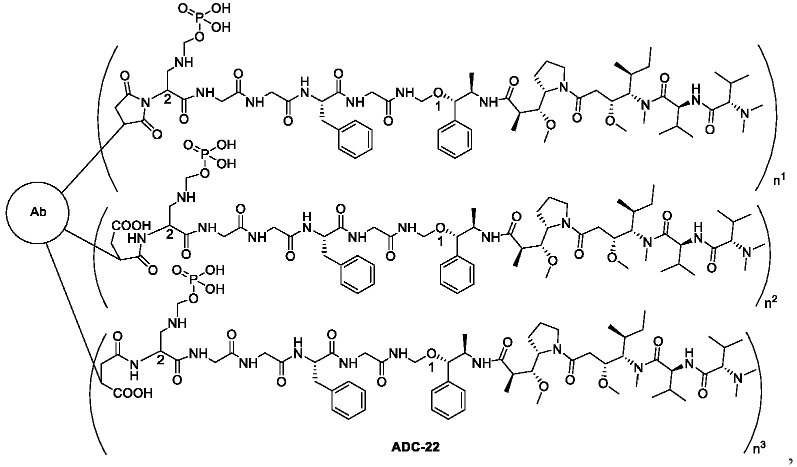 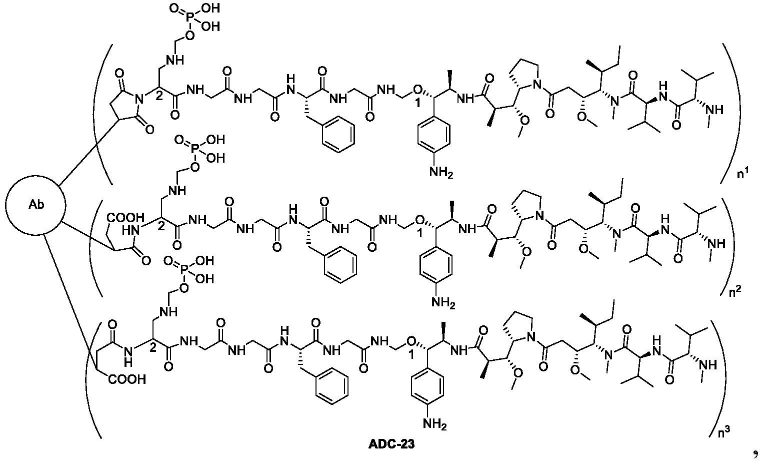 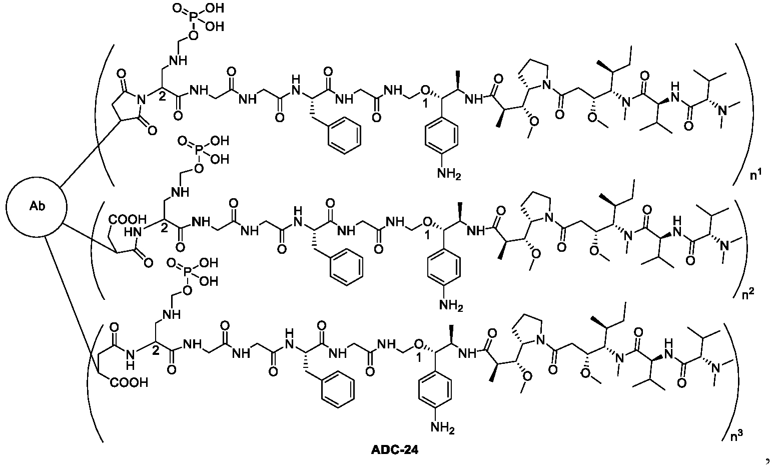 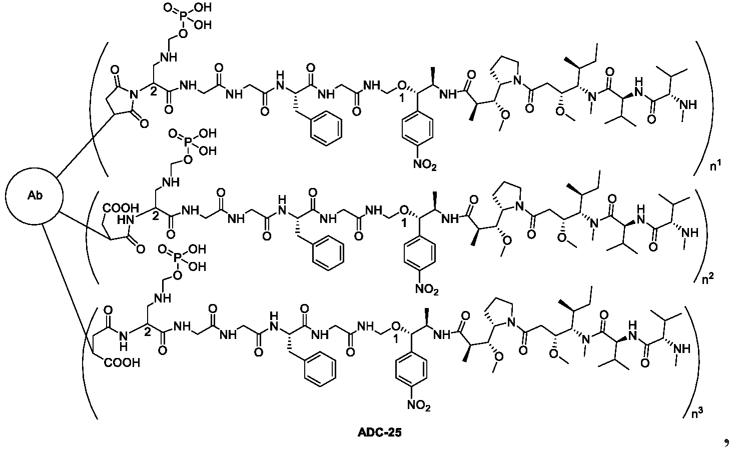 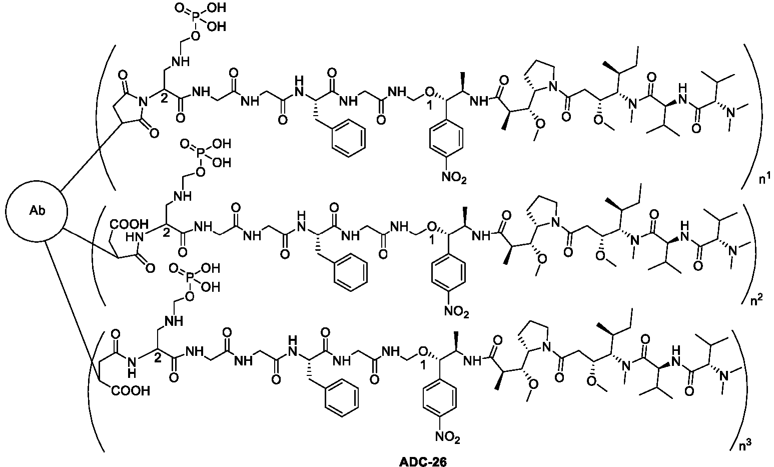 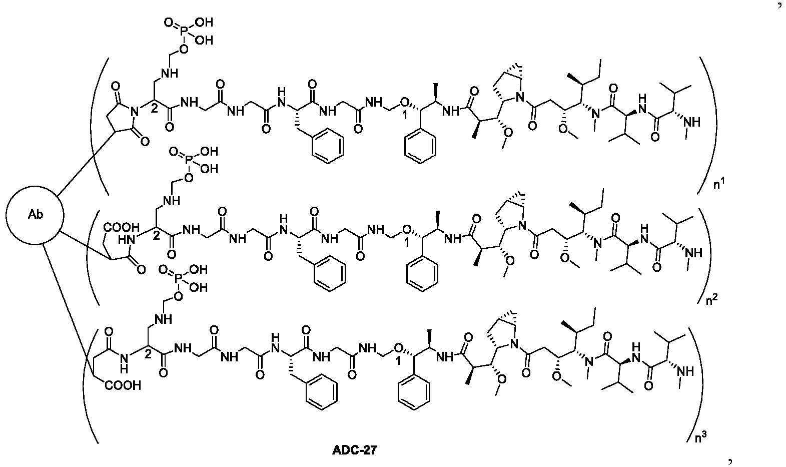 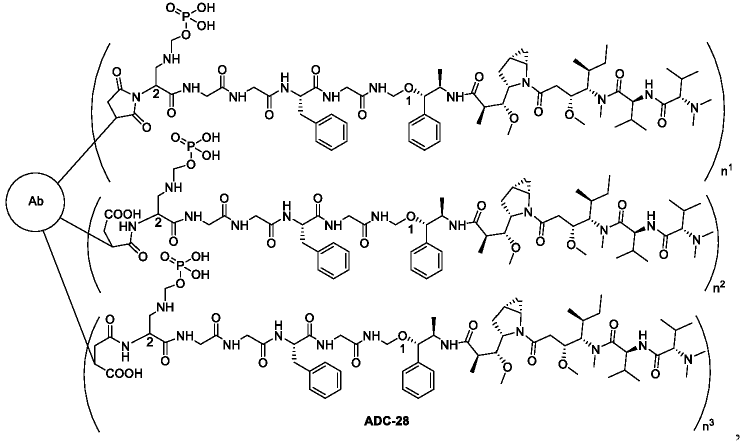 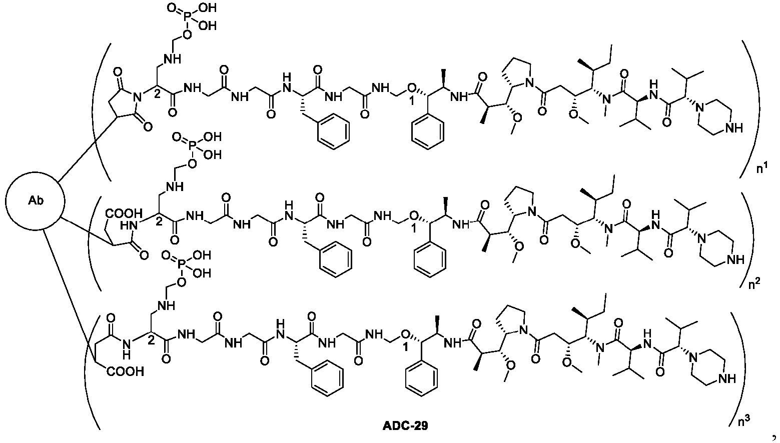 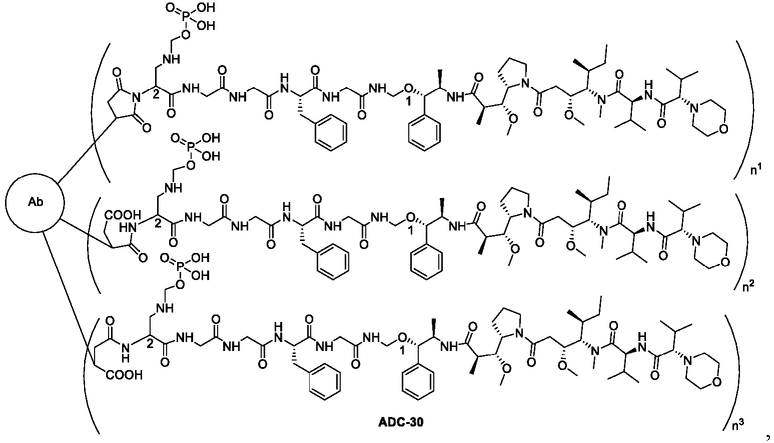 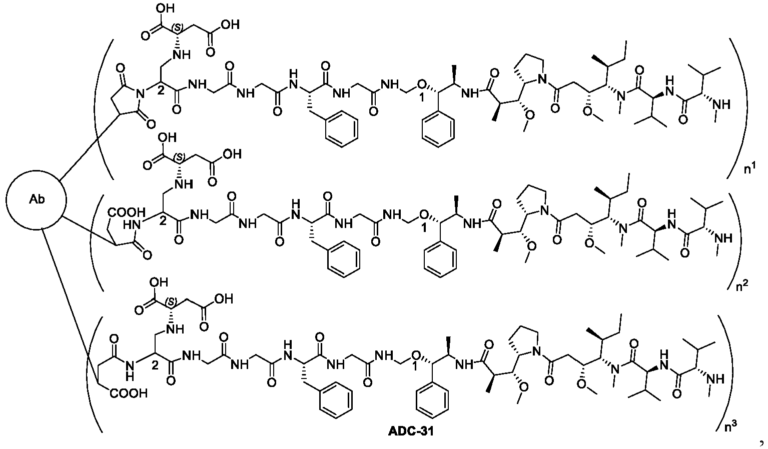 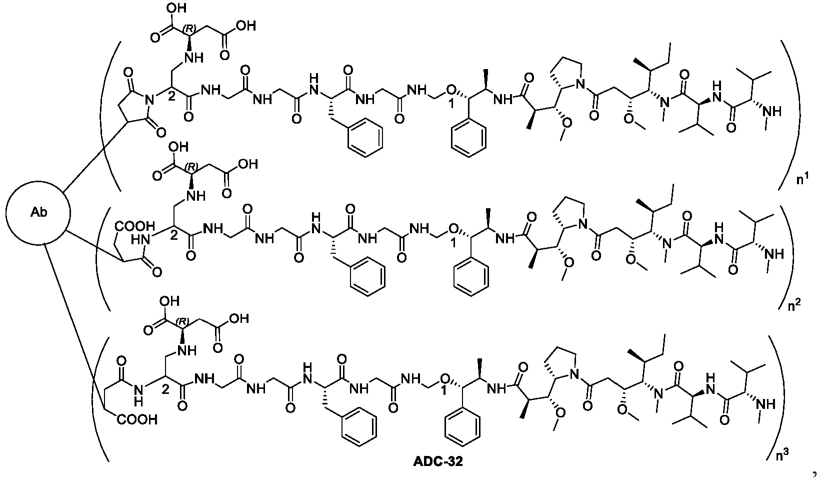 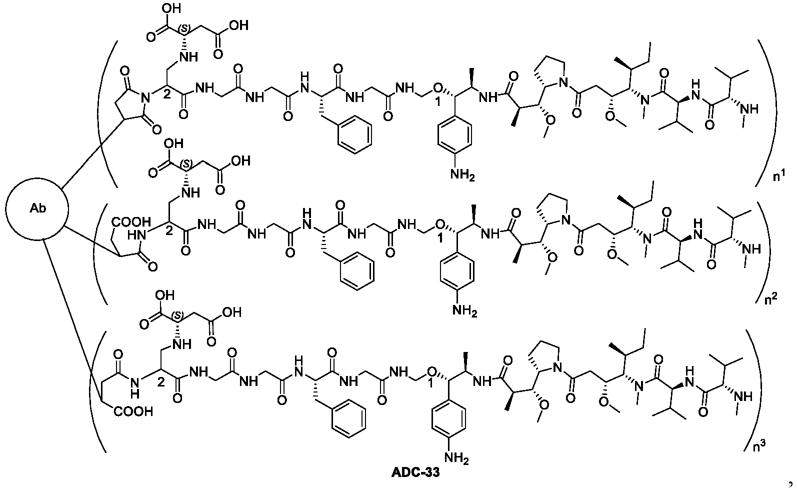 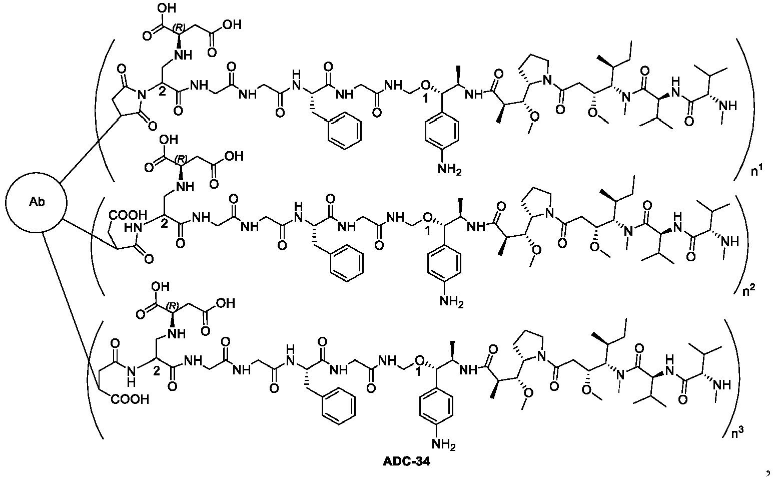 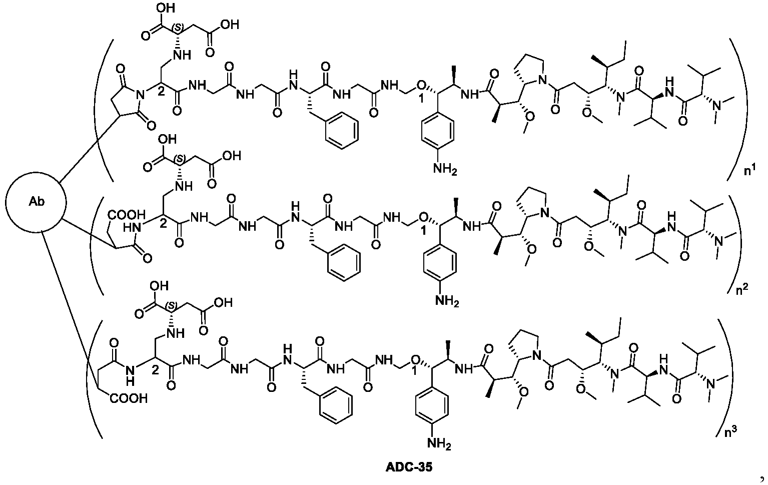 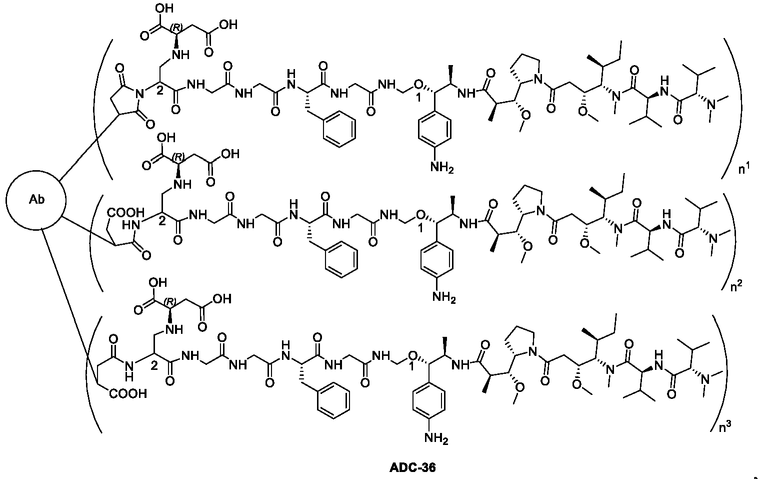 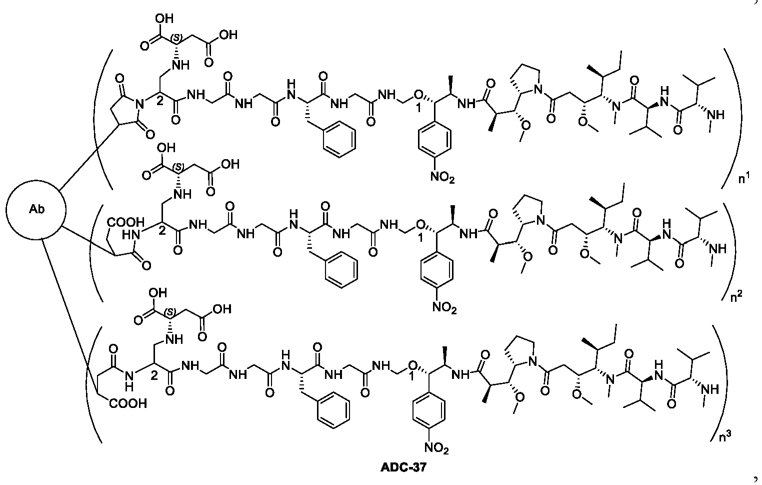 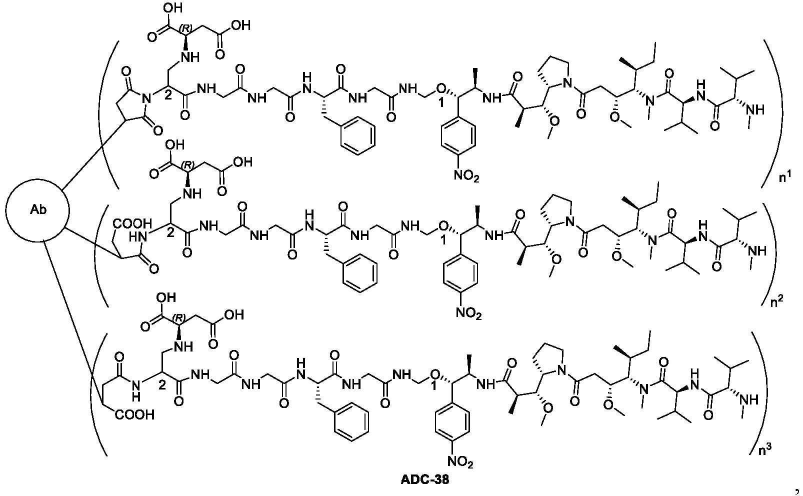 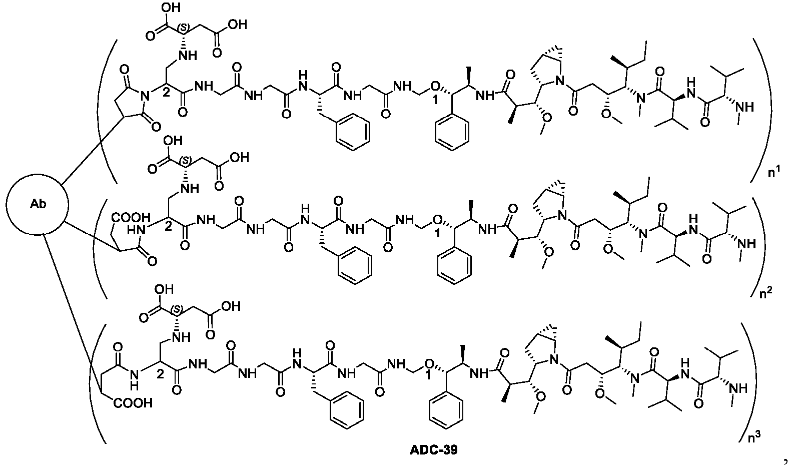 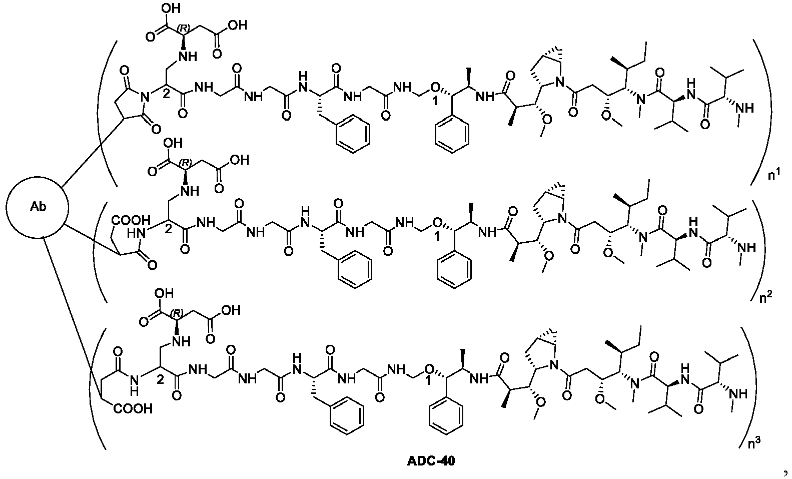 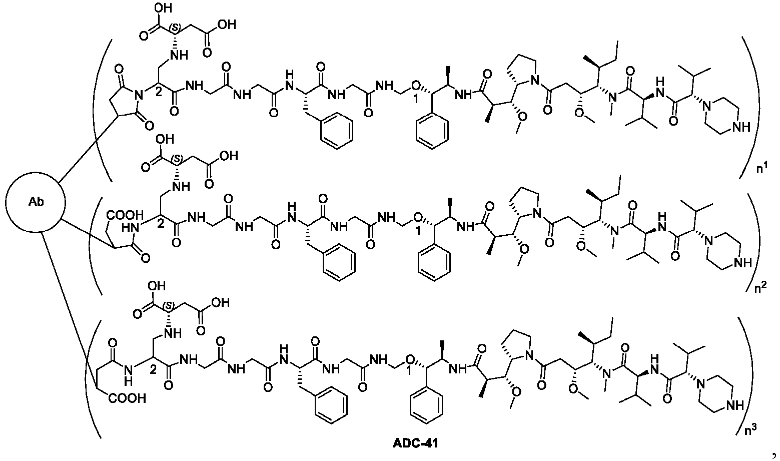 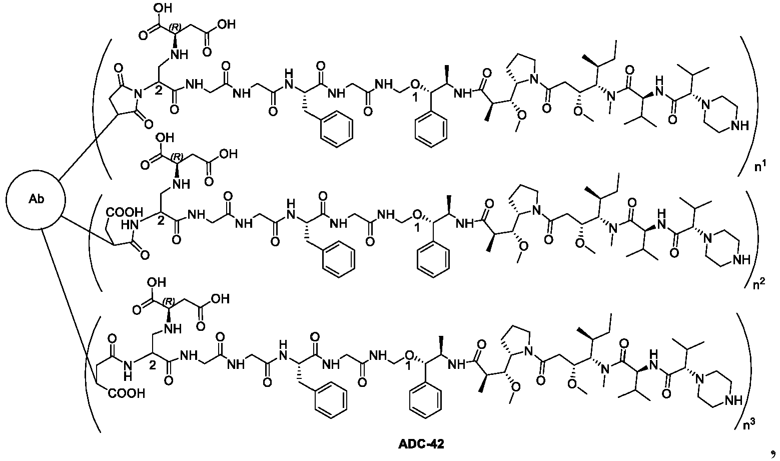 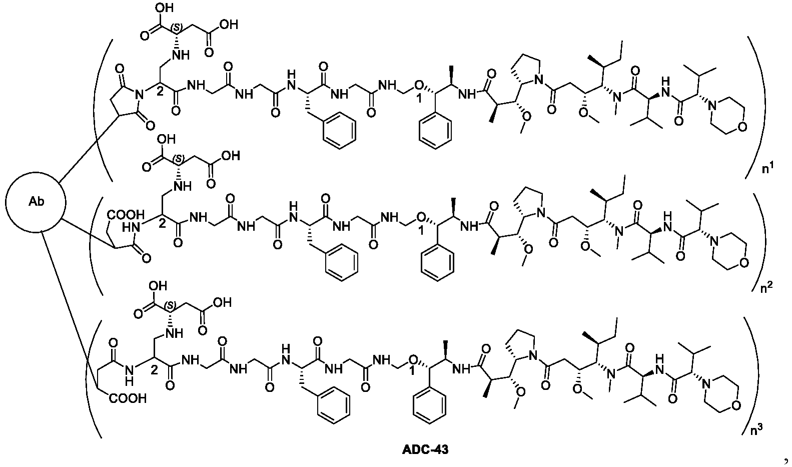 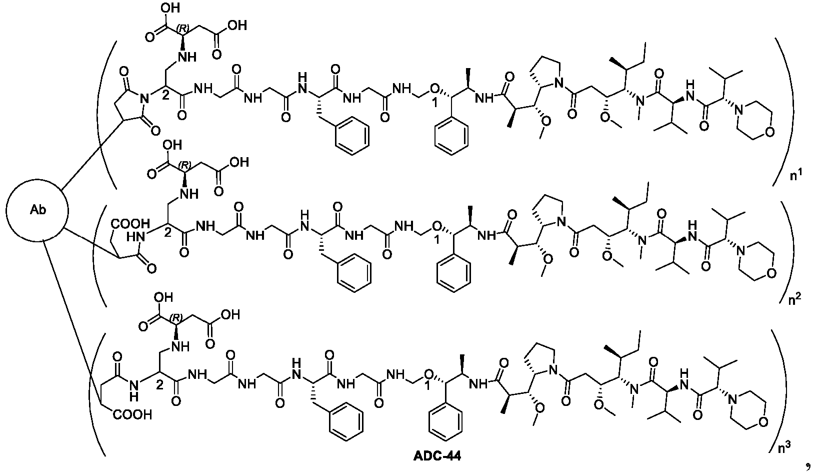 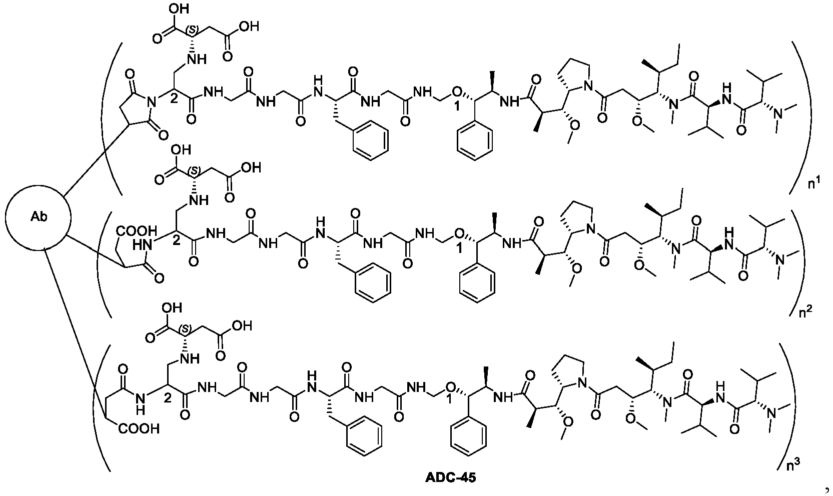 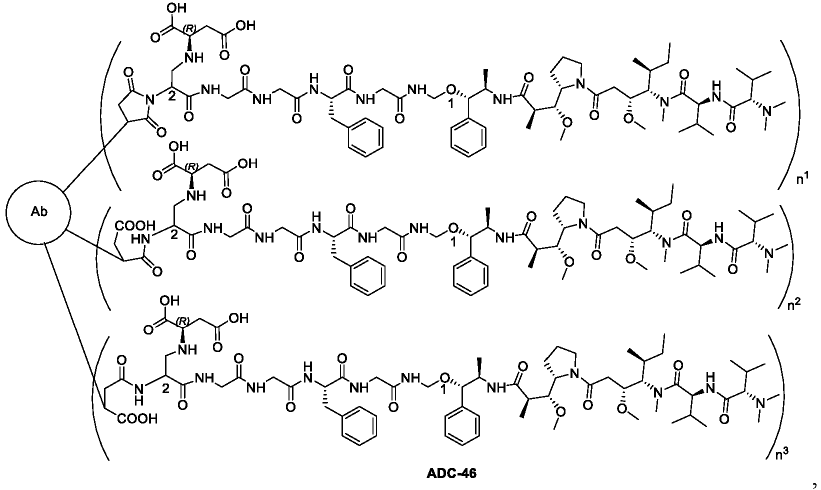 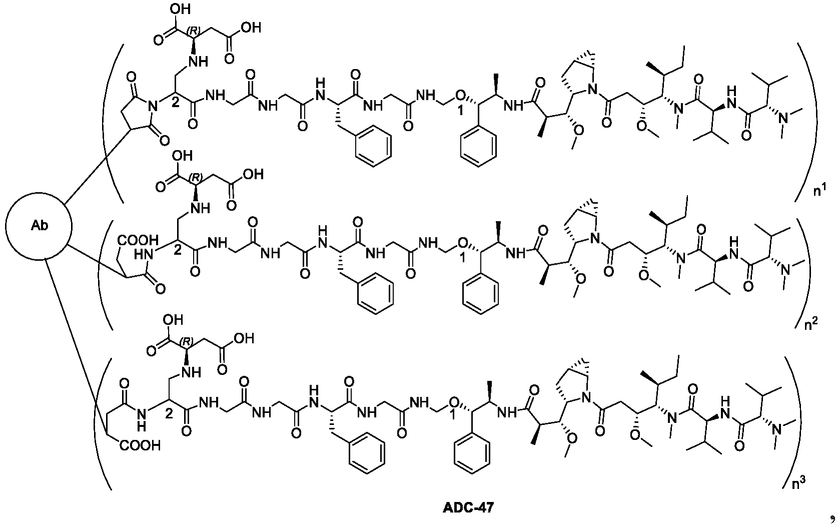 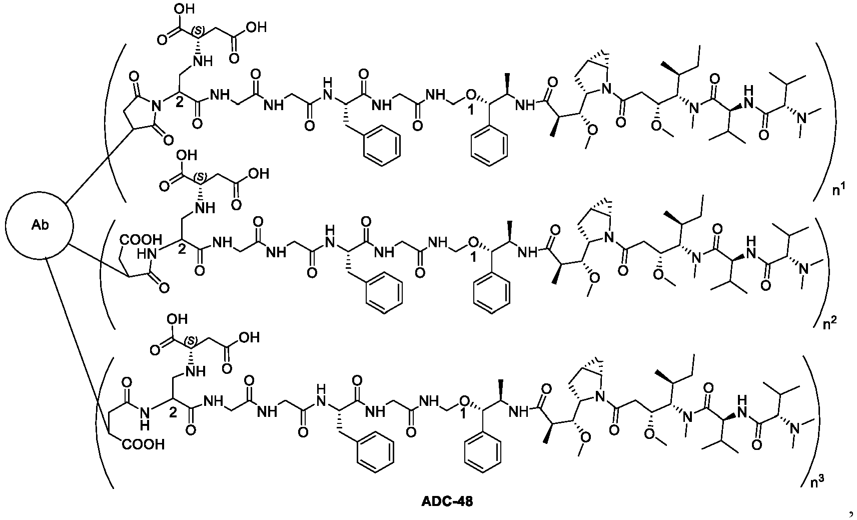 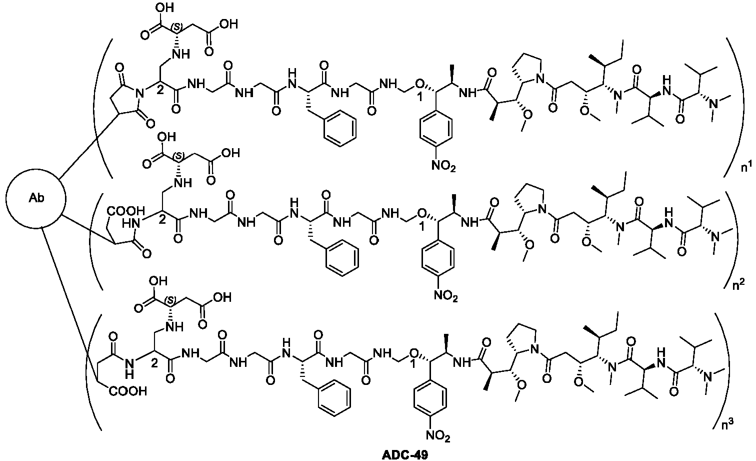 or 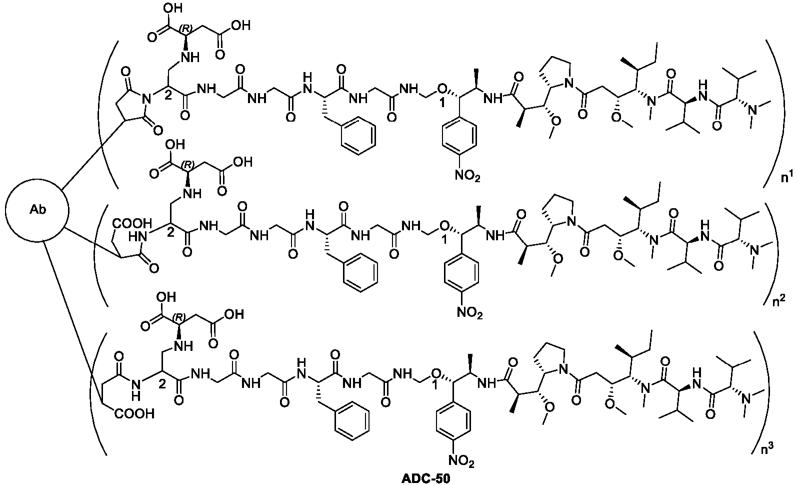 wherein,
the configuration of the chiral carbon at position 2 is R type or S type.

In the second aspect, the application provides a linker-payload as shown in formula II, or a pharmaceutically acceptable salt or solvate thereof,

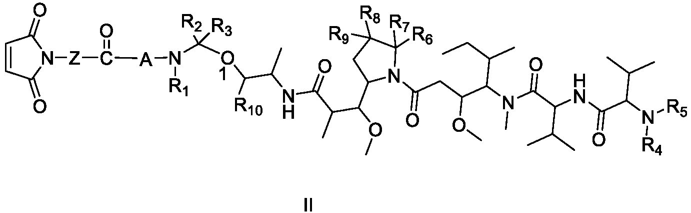

Z, A, R₁, R₂, R₃, R₄, R₅, R₁₁, R₁₂, R₁₃, R₁₄, B, R₁₅, R₁₆, R₁₇, n, m, R₆, R₇, R₈, R₉₍, R₁₈₎, R₁₉, and R₁₀ are as defined in any of the above.

In certain embodiments, said linker-payload has the structure shown in formula IIa,

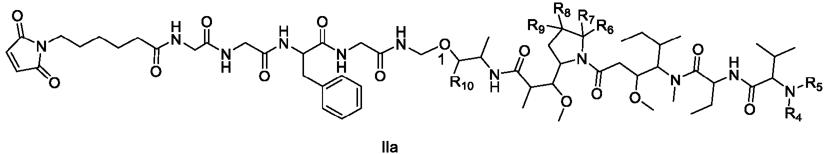

In certain embodiments, said linker-payload has the structure shown in formula IIb, 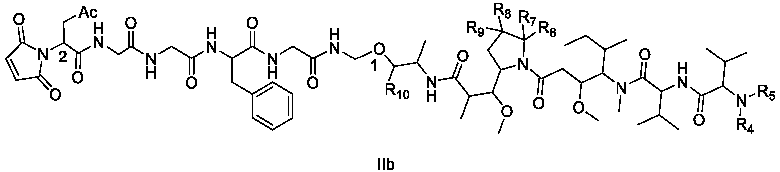 wherein, Ac is a hydrophilic structural unit having a structure shown in formula c: 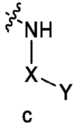 wherein, X and Y are as defined above, and Ac is connected to the 2-position methylene carbon indicated in formula IIb via -NH-.

The linker-payload of the application can be selected from the following structures without limitation: 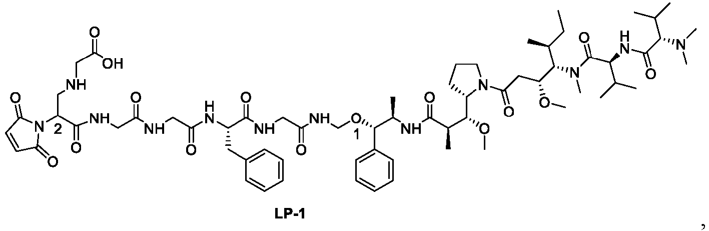 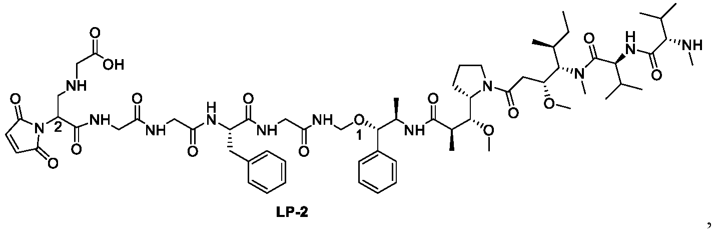 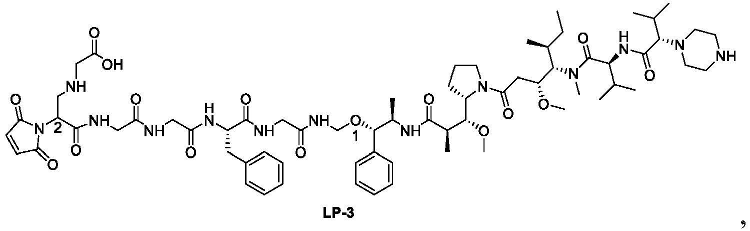 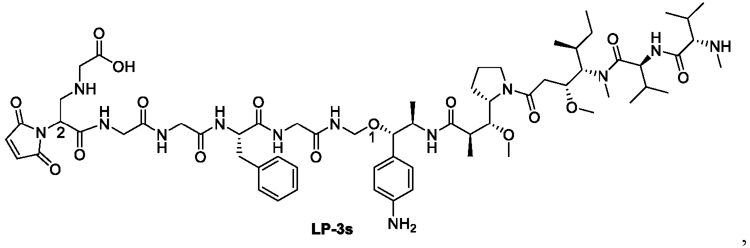 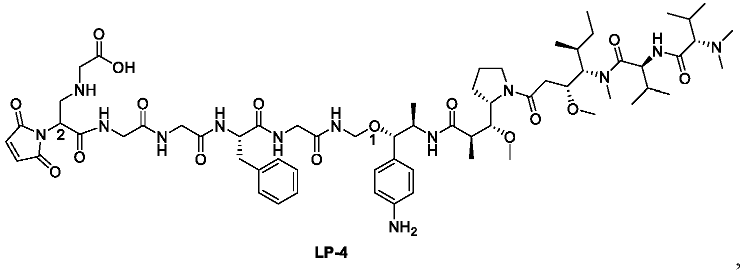 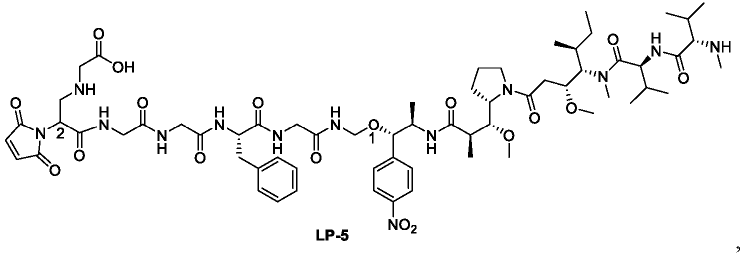 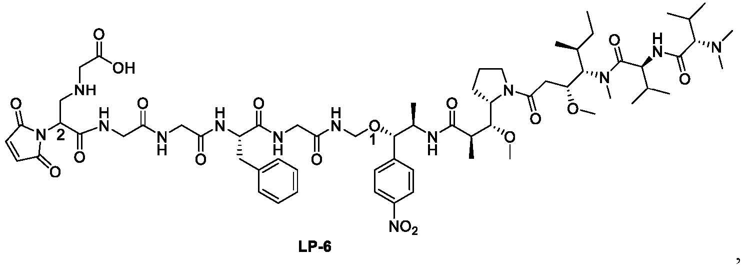 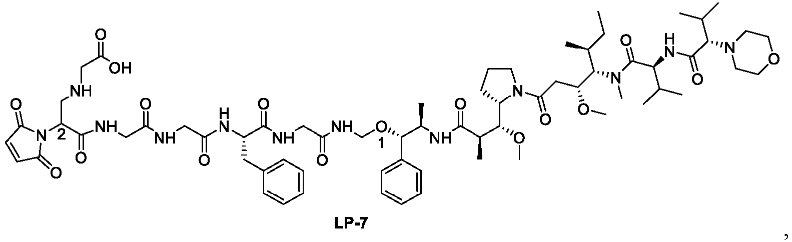 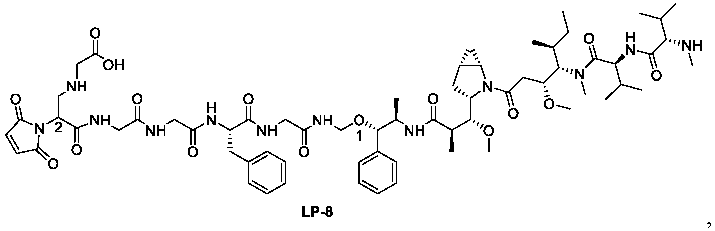 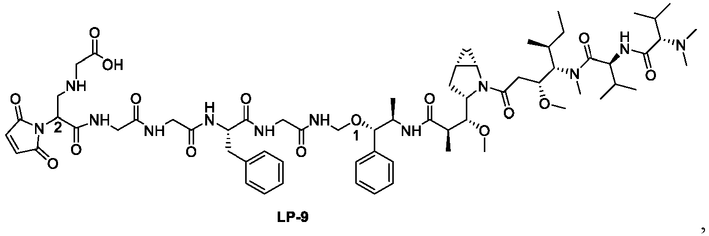 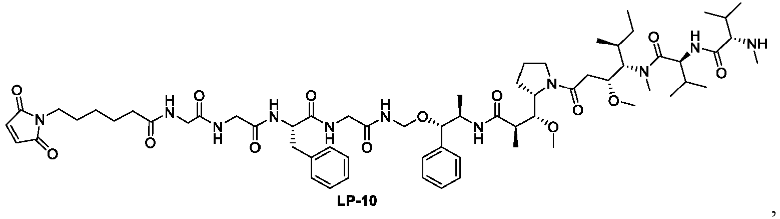 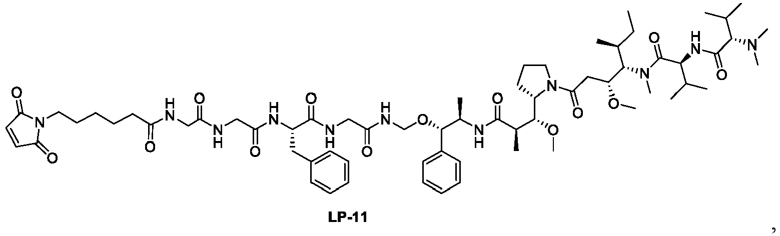 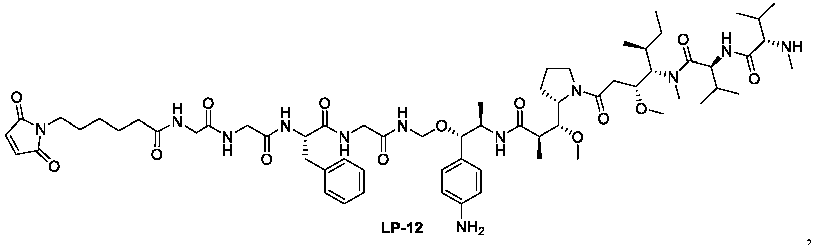 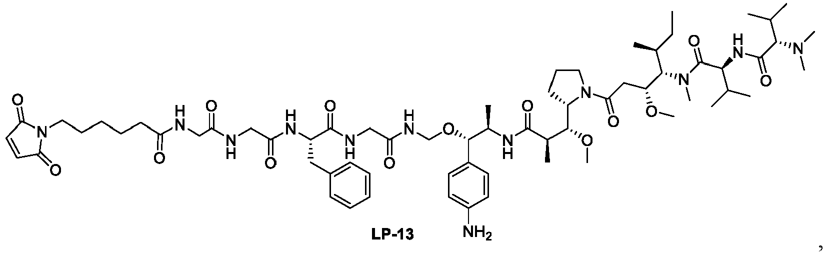 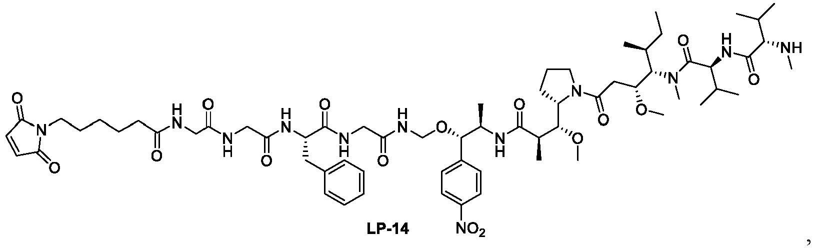 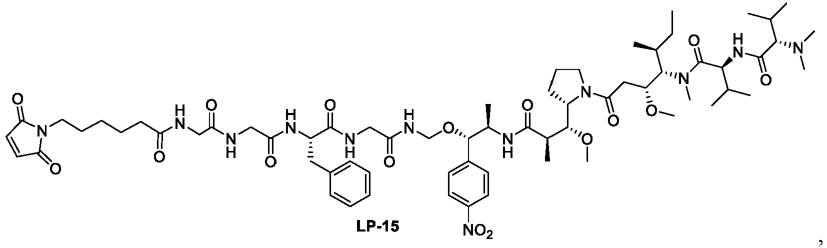 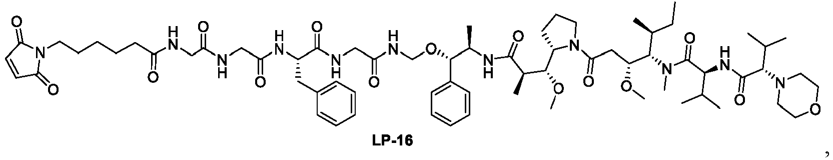 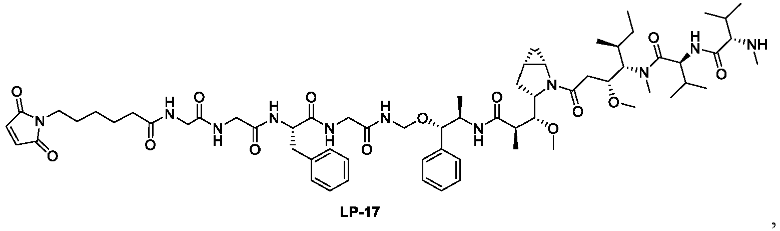 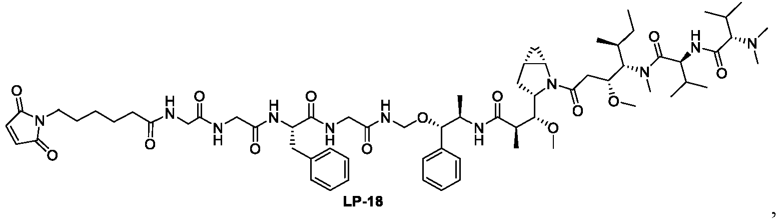 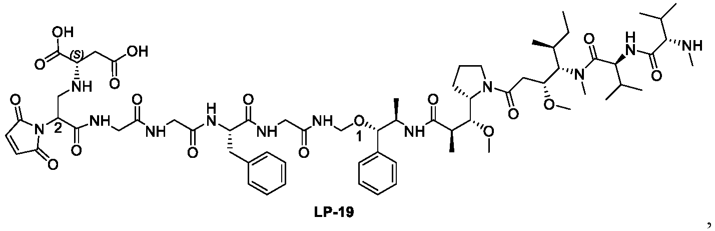 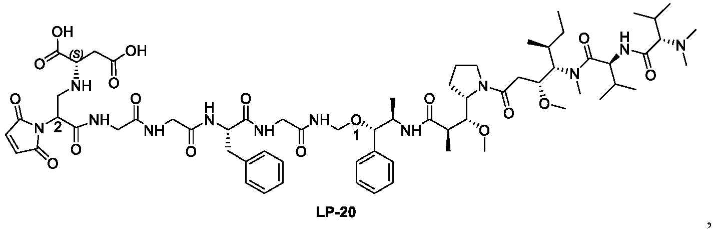 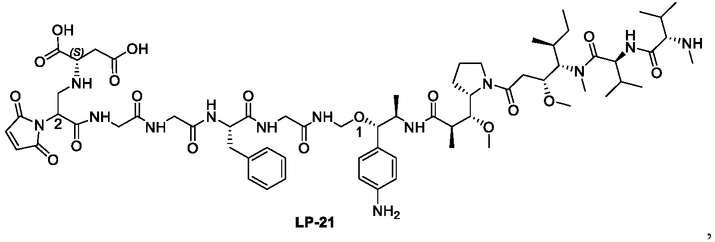 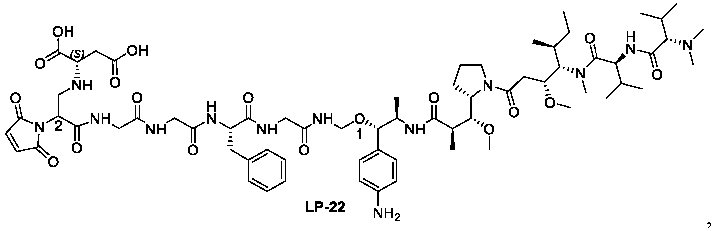 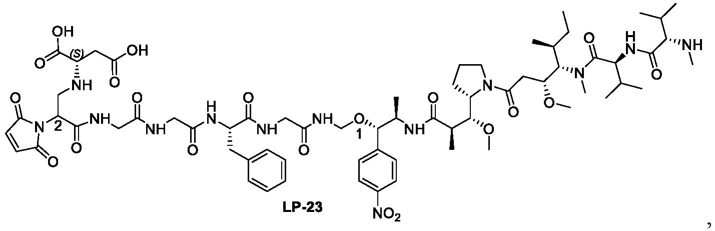 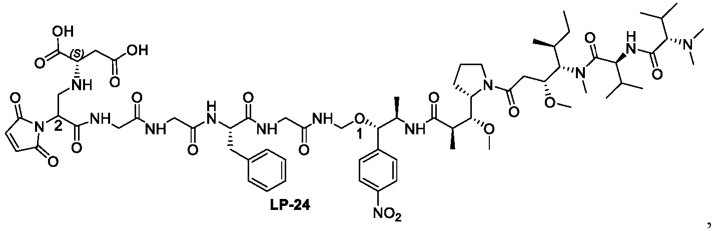 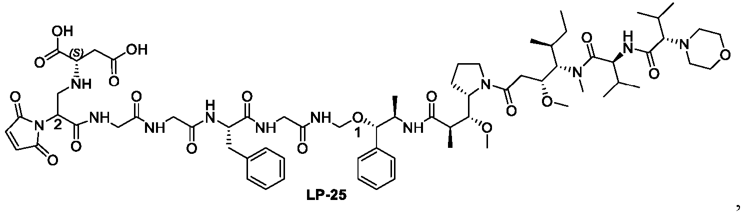 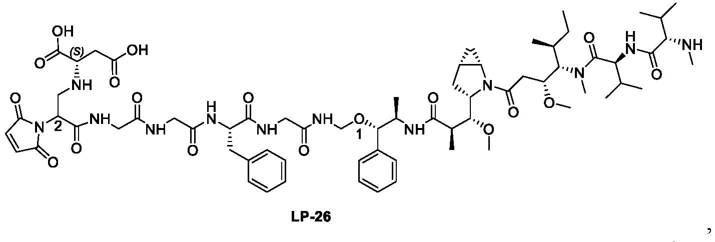 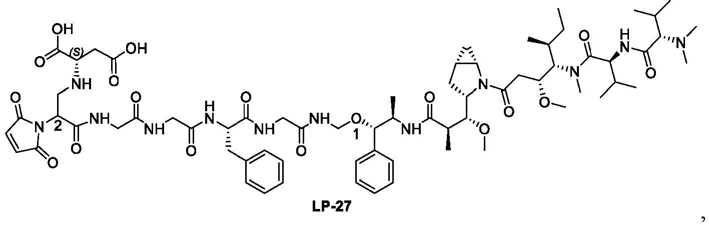 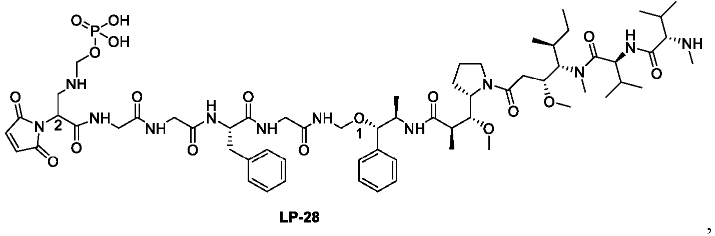 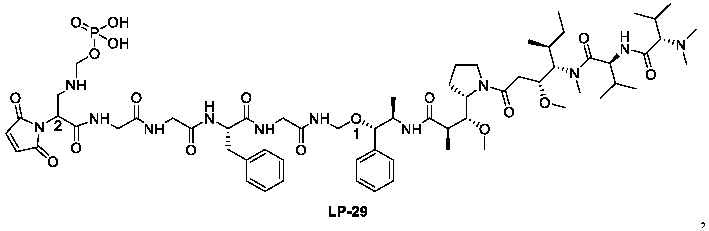 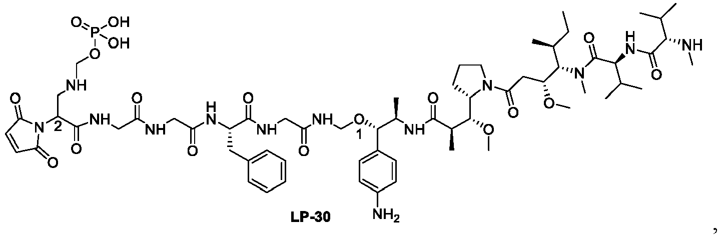 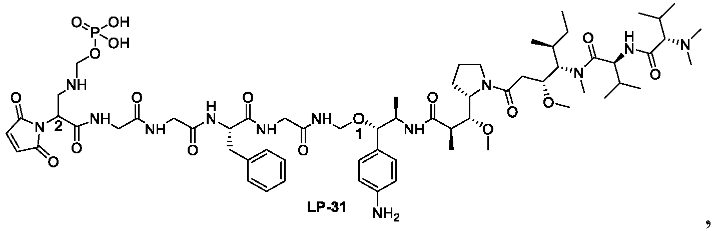 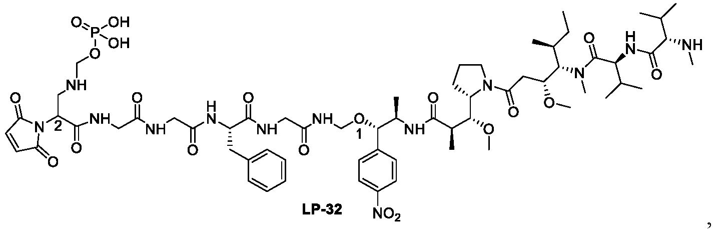 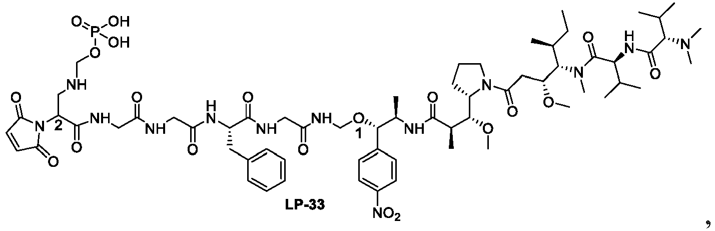 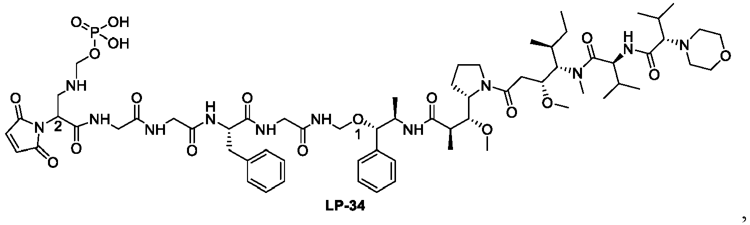 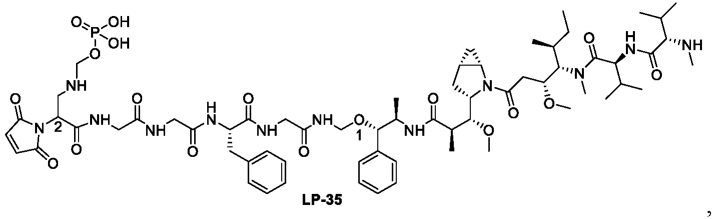 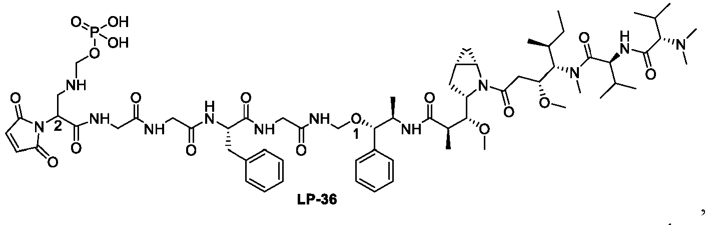 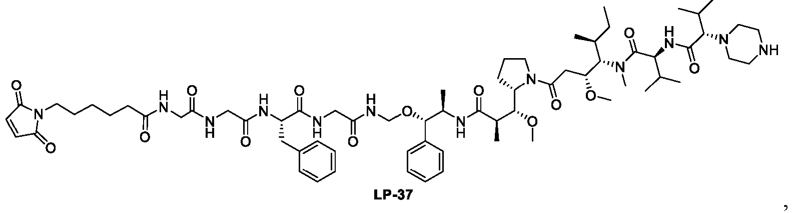 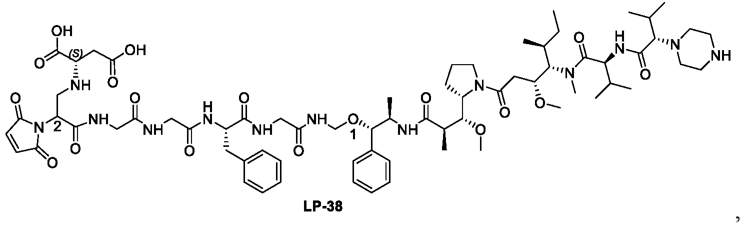 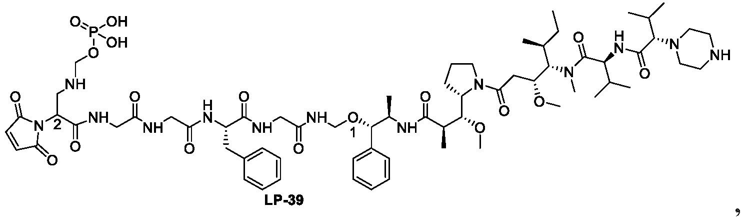 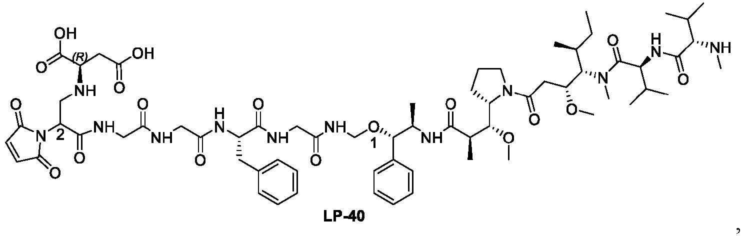 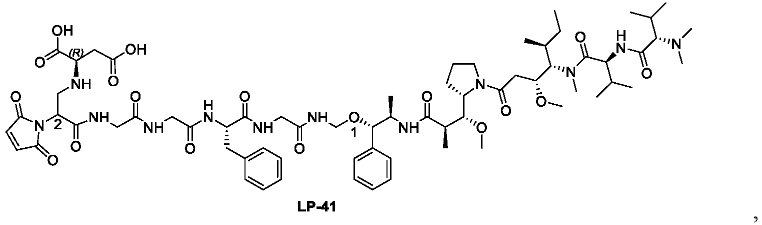 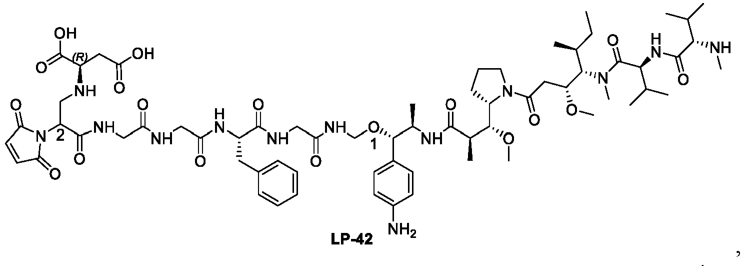 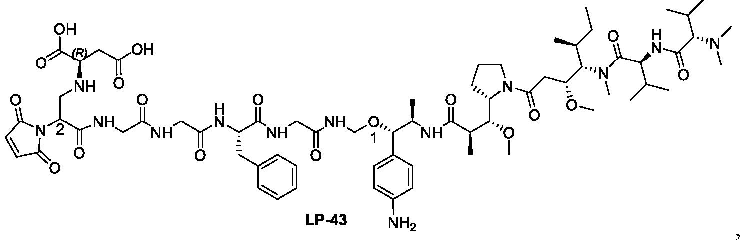 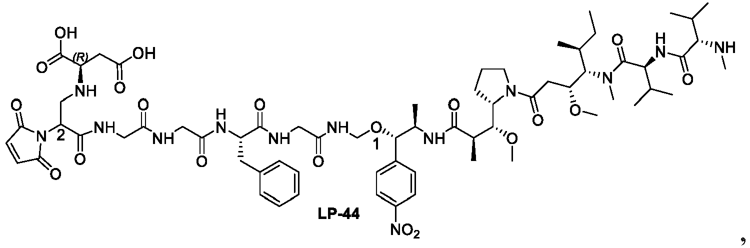 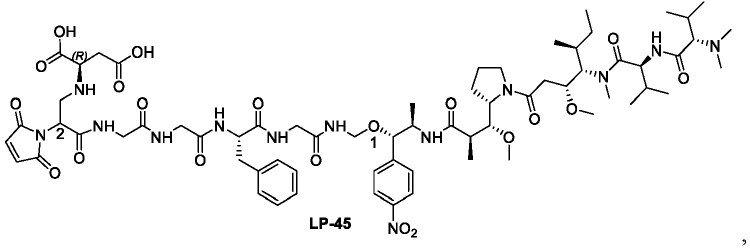 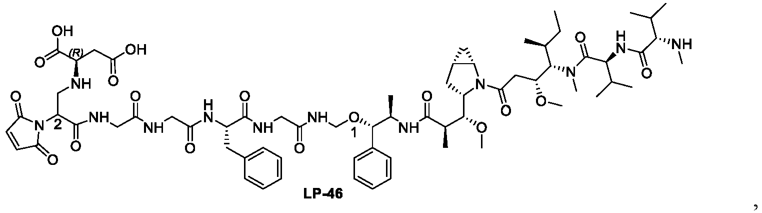 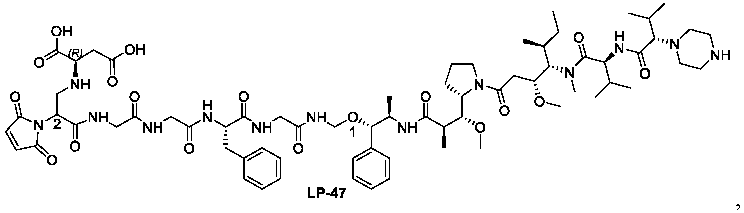 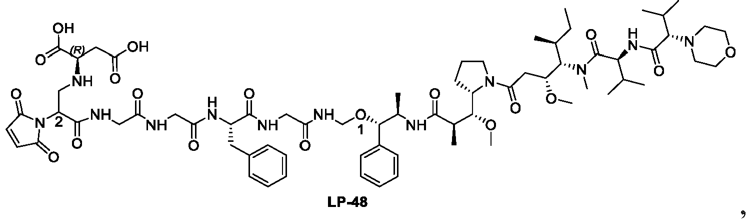 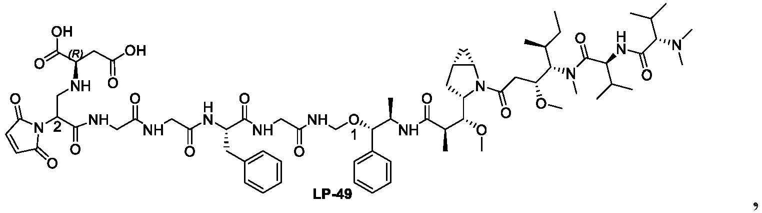 or 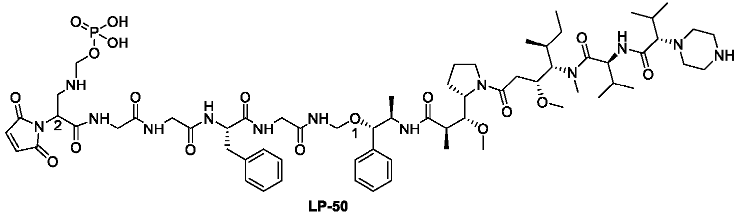 wherein, the configuration of the chiral carbon at position 2 is R type or S type.

In the application, the ligand unit Ab can be selected from antibodies, antibody fragments or proteins, wherein the antibody is selected from murine antibodies, rabbit antibodies, phage display antibodies, yeast display antibodies, chimeric antibodies, humanized antibodies, fully human antibodies, antibody fragments, bispecific antibodies and multispecific antibodies.

In certain embodiments, the antibody is a monoclonal antibody selected from, but not limited to, anti-EGFRvIII antibody, anti-PD-1 antibody, anti-PD-L1 antibody, anti-DLL3 antibody, anti-PSMA antibody, anti-CD70 antibody, anti-MUC16 antibody, anti-ENPP3 antibody, anti-TDGF1 antibody, anti-ETBR antibody, anti-MSLN antibody, anti-TIM-1 antibody, anti-LRRC15 antibody, anti-LIV-1 antibody, anti-CanAg/AFP antibody, anti-Claudin18.2 antibody, anti-Mesothelin antibody, anti-HER2 (ErbB2) antibody, anti-EGFR antibody, anti-cMET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3 (ErbB3) antibody, anti-MUC1/CD227 antibody, anti-AXL antibody, anti-CD166 antibody, anti-B7 H3 (CD276) antibody, anti-PTK7/CCK4 antibody, anti-PRLR antibody, anti-EFNA4 antibody, anti-5T4 antibody, anti-NOTCH3 antibody, anti-Nectin 4 antibody, anti-Trop2 antibody, anti-CD142 antibody, anti-CA6 antibody, anti-GPR20 antibody, anti-CD174 antibody, anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody, anti-CD33 antibody, anti-CD56 antibody, anti-CD74 antibody, anti-CD22 antibody, anti-CD30 antibody, anti-CD37 antibody, anti-CD47 antibody, anti-CD138 antibody, anti-CD352 antibody, anti-CD25 antibody or anti-CD123 antibody.

In certain embodiments, the antibody is an anti-Trop2 antibody. In certain embodiments, the antibody is composed of a light chain and a heavy chain, the light chain comprising CDR-L1, CDR-L2 and CDR-L3, whose amino acid sequences are shown in SEQ ID NO: 1, SEQ ID NO: 2, and SEQ ID NO: 3, respectively. In certain embodiments, the heavy chain comprises CDR-H1, CDR-H2, and CDR-H3, whose amino acid sequences are shown in SEQ ID NO: 4, SEQ ID NO: 5, and SEQ ID NO: 6, respectively.

In certain embodiments, the light chain comprises a light chain variable region having an amino acid sequence of SEQ ID NO: 7. In certain embodiments, the light chain further comprises a light chain constant region having an amino acid sequence of SEQ ID NO: 8. In certain embodiments, the amino acid sequence of the light chain is SEQ ID NO: 9.

In certain embodiments, the light chain comprises a light chain variable region having an amino acid sequence of SEQ ID NO: 10. In certain embodiments, the light chain further comprises a light chain constant region having an amino acid sequence of SEQ ID NO: 11. In certain embodiments, the amino acid sequence of the light chain is SEQ ID NO: 12.

In certain embodiments, the heavy chain comprises a heavy chain variable region having an amino acid sequence of SEQ ID NO: 13. In certain embodiments, the heavy chain further comprises a heavy chain constant region having an amino acid sequence of SEQ ID NO: 14. In certain embodiments, the amino acid sequence of the heavy chain is SEQ ID NO: 15.

In certain embodiments, the antibody is an anti-Trop2 antibody. In certain embodiments, the antibody is composed of a light chain and a heavy chain, the light chain comprising CDR-L1, CDR-L2 and CDR-L3, whose nucleic acid coding sequences are shown in SEQ ID NO: 16, SEQ ID NO: 17, and SEQ ID NO: 18, respectively. In certain embodiments, the heavy chain comprises CDR-H1, CDR-H2, and CDR-H3, whose nucleic acid coding sequences are shown in SEQ ID NO: 19, SEQ ID NO: 20, and SEQ ID NO: 21, respectively.

In certain embodiments, the light chain comprises a light chain variable region whose nucleic acid coding sequence is SEQ ID NO: 22. In certain embodiments, the light chain further comprises a light chain constant region whose nucleic acid coding sequence is SEQ ID NO: 23. In certain embodiments, the nucleic acid coding sequence of the light chain is SEQ ID NO: 24.

In certain embodiments, the light chain comprises a light chain variable region whose nucleic acid coding sequence is SEQ ID NO: 25. In certain embodiments, the light chain further comprises a light chain constant region whose nucleic acid encoding sequence is SEQ ID NO: 26. In certain embodiments, the nucleic acid encoding sequence of the light chain is SEQ ID NO: 27.

In certain embodiments, the heavy chain comprises a heavy chain variable region whose nucleic acid encoding sequence is SEQ ID NO: 28. In certain embodiments, the heavy chain further comprises a heavy chain constant region whose nucleic acid encoding sequence is SEQ ID NO: 29. In certain embodiments, the nucleic acid encoding sequence of the heavy chain is SEQ ID NO: 30.

In certain embodiments, the antibody is an anti-Trop2 antibody. In certain embodiments, the antibody is composed of a light chain and a heavy chain, the light chain comprising CDR-L1, CDR-L2 and CDR-L3, whose amino acid sequences are shown in SEQ ID NO: 31, SEQ ID NO: 32, and SEQ ID NO: 33, respectively. In certain embodiments, the heavy chain comprises CDR-H1, CDR-H2, and CDR-H3, whose amino acid sequences are shown in SEQ ID NO: 34, SEQ ID NO: 35, and SEQ ID NO: 36, respectively.

In certain embodiments, the light chain comprises a light chain variable region having an amino acid sequence of SEQ ID NO: 37. In certain embodiments, the light chain further comprises a light chain constant region having an amino acid sequence of SEQ ID NO: 38. In certain embodiments, the amino acid sequence of the light chain is SEQ ID NO: 39.

In certain embodiments, the heavy chain comprises a heavy chain variable region having an amino acid sequence of SEQ ID NO: 40. In certain embodiments, the heavy chain further comprises a heavy chain constant region having an amino acid sequence of SEQ ID NO: 41. In certain embodiments, the amino acid sequence of the heavy chain is SEQ ID NO: 42.

In certain embodiments, the antibody is an anti-Trop2 antibody. In certain embodiments, the antibody is composed of a light chain and a heavy chain, and the light chain comprises CDR-L1, CDR-L2 and CDR-L3, and the nucleic acid coding sequences thereof are shown in SEQ ID NO: 43, SEQ ID NO: 44, and SEQ ID NO: 45, respectively. In certain embodiments, the heavy chain comprises CDR-H1, CDR-H2, and CDR-H3, and the nucleic acid coding sequences thereof are shown in SEQ ID NO: 46, SEQ ID NO: 47, and SEQ ID NO: 48, respectively.

In certain embodiments, the light chain comprises a light chain variable region having a nucleic acid coding sequence of SEQ ID NO: 49. In certain embodiments, the light chain further comprises a light chain constant region having a nucleic acid coding sequence of SEQ ID NO: 50. In certain embodiments, the nucleic acid coding sequence of the light chain is SEQ ID NO: 51.

In certain embodiments, the heavy chain comprises a heavy chain variable region whose nucleic acid encoding sequence is SEQ ID NO: 52. In certain embodiments, the heavy chain further comprises a heavy chain constant region whose nucleic acid encoding sequence is SEQ ID NO: 53. In certain embodiments, the heavy chain nucleic acid encoding sequence is SEQ ID NO: 54.

The information of the partial sequences involved in the application is provided in Table 1.

**Table 1: Description of sequences (Sequence Listing)**

| SEQ ID NO. | Descriptive | Sequences |
|---|---|---|
| 1 | Amino acid sequence of CDR-L1 from TR000 and TR005 | RASQDINKYLA |
| 2 | Amino acid sequence of CDR-L2 from TR000 and TR005 | STSTLQS |
| 3 | Amino acid sequence of CDR-L3 from TR000 and TR005 | LQYDDLFT |
| 4 | Amino acid sequence of CDR-H1 of TR000 and TR005 | SFDIN |
| 5 | Amino acid sequence of CDR-H2 of TR000 and TR005 | WIFPGDGNTKYSQKFQG |
| 6 | Amino acid sequence of CDR-H3 of TR000 and TR005 | GEALYYFDY |
| 7 | Amino acid sequence of the light chain variable region of TR000 | DIQMTQSPSSLSASVGDRVTITCRASQDINKYLA WYQQKPGKVPKLLIYSTSTLQSGVPSRFSGSGSG TDFTLTISSLQPEDVATYYCLQYDDLFTFGQGTKL EIK |
| 8 | Amino acid sequence of the light chain constant region of TR000 | |
| 9 | Amino acid sequence of the light chain of TR000 | DIQMTQSPSSLSASVGDRVTITCRASQDINKYLA WYQQKPGKVPKLLIYSTSTLQSGVPSRFSGSGSG TDFTLTISSLQPEDVATYYCLQYDDLFTFGQGTKL EIKRTVAAPSVFIFPPSDEQLKSGTASVVCLLNNF YPREAKVQWKVDNALQSGNSQESVTEQDSKDS TYSLSSTLTLSKADYEKHKVYACEVTHQGLSSPV TKSFNRGEC |
| 10 | Amino acid sequence of the light chain variable region of TR005 | DIQMTQSPSSLSASVGDRVTITCRASQDINKYLA WYQQKPGKVPKLLIYSTSTLQSGVPSRFSGSGSG TDFTLTISSLQPEDVATYYCLQYDDLFTFGQGTKL EIK |
| 11 | Amino acid sequence of the light chain constant region of TR005 | RTVAAPSVFIFPPSDEQLKSGTASVVCLLNNFYPR EAKVQWKVDNALQSGNSQESVTEQDSKDSTYS LSSTLTLSKADYEKHKVYACEVTHQGLSSPVCTK SFNRGEC |
| 12 | Amino acid sequence of the light chain of TR005 | DIQMTQSPSSLSASVGDRVTITCRASQDINKYLA WYQQKPGKVPKLLIYSTSTLQSGVPSRFSGSGSG TDFTLTISSLQPEDVATYYCLQYDDLFTFGQGTKL EIKRTVAAPSVFIFPPSDEQLKSGTASVVCLLNNF YPREAKVQWKVDNALQSGNSQESVTEQDSKDS TYSLSSTLTLSKADYEKHKVYACEVTHQGLSSPV CTKSFNRGEC |
| 13 | Amino acid sequences of the heavy chain variable regions of TR000 and TR005 | QVQLVQSGAEVKKPGASVKLSCKASGYTFTSFDI NWVRQAPEQRLEWMGWIFPGDGNTKYSQKFQG RATITRDTSASTAYMELSSLRSEDTAVYYCVRGE ALYYFDYWGQGTLVTVSS |
| 14 | Amino acid sequences of the heavy chain constant regions of TR000 and TR005 | ASTKGPSVFPLAPSSKSTSGGTAALGCLVKDYFP EPVTVSWNSGALTSGVHTFPAVLQSSGLYSLSSV VTVPSSSLGTQTYICNVNHKPSNTKVDKRVEPKS CDKTHTCPPCPAPELLGGPSVFLFPPKPKDTLMIS RTPEVTCVVVDVSHEDPEVKFNWYVDGVEVHN AKTKPREEQYNSTYRVVSVLTVLHQDWLNGKE YKCKVSNKALPAPIEKTISKAKGQPREPQVYTLP PSRDELTKNQVSLTCLVKGFYPSDIAVEWESNGQ PENNYKTTPPVLDSDGSFFLYSKLTVDKSRWQQG NVFSCSVMHEALHNHYTQKSLSLSPG |
| 15 | Amino acid sequences of the heavy chains of TR000 and TR005 | QVQLVQSGAEVKKPGASVKLSCKASGYTFTSFDI NWVRQAPEQRLEWMGWIFPGDGNTKYSQKFQG RATITRDTSASTAYMELSSLRSEDTAVYYCVRGE ALYYFDYWGQGTLVTVSSASTKGPSVFPLAPSSK STSGGTAALGCLVKDYFPEPVTVSWNSGALTSG VHTFPAVLQSSGLYSLSSVVTVPSSSLGTQTYICN VNHKPSNTKVDKRVEPKSCDKTHTCPPCPAPELL GGPSVFLFPPKPKDTLMISRTPEVTCVVVDVSHE DPEVKFNWYVDGVEVHNAKTKPREEQYNSTYR VVSVLTVLHQDWLNGKEYKCKVSNKALPAPIEK TISKAKGQPREPQVYTLPPSRDELTKNQVSLTCLV KGFYPSDIAVEWESNGQPENNYKTTPPVLDSDGS FFLYSKLTVDKSRWQQGNVFSCSVMHEALHNHY TQKSLSLSPG |
| 16 | Nucleic acid coding sequence for CDR-L1 of TR000 and TR005 | AGAGCTTCTCAGGATATCAATAAGTATCTGGCT |
| 17 | Nucleic acid coding sequence for CDR-L2 of TR000 and TR005 | TCTACATCTACCCTGCAGTCT |
| 18 | Nucleic acid coding sequence for CDR-L3 of TR000 and TR005 | CTGCAGTATGATGATCTGTTCACC |
| 19 | Nucleic acid coding sequence for CDR-H1 of TR000 and TR005 | TCCTTCGACATTAAC |
| 20 | Nucleic acid coding sequence for CDR-H2 of TR000 and TR005 | TGGATCTTCCCCGGCGACGGCAACACCAAGTA CTCCCAGAAGTTCCAGGGA |
| 21 | Nucleic acid coding sequence for CDR-H3 of TR000 and TR005 | GGAGAGGCTCTGTACTATTTTGATTAT |
| 22 | Nucleic acid coding sequence of the light chain variable region of TR000 | GATATCCAGATGACCCAGTCTCCATCTAGCCTG TCCGCTTCTGTGGGCGATAGAGTGACCATCACA TGCAGAGCTTCTCAGGATATCAATAAGTATCTG GCTTGGTATCAGCAGAAGCCTGGAAAGGTGCC TAAGCTGCTGATCTACTCTACATCTACCCTGCA GTCTGGAGTGCCTTCTAGATTTTCTGGATCTGG CTCTGGCACCGATTTTACACTGACAATCTCTTC TCTGCAGCCTGAGGATGTGGCTACATATTATTGT CTGCAGTATGATGATCTGTTCACCTTTGGCCAG GGCACCAAGCTGGAGATCAAG |
| 23 | Nucleic acid coding sequence of the light chain constant region of TR000 | CGTACGGTGGCTGCACCATCTGTCTTCATCTTC CCGCCATCTGATGAGCAGTTGAAATCTGGAACT GCCTCTGTTGTGTGCCTGCTGAATAACTTCTAT CCCAGAGAGGCCAAAGTACAGTGGAAGGTGG ATAACGCCCTCCAATCGGGTAACTCCCAGGAG AGTGTCACAGAGCAGGACAGCAAGGACAGCA CCTACAGCCTCAGCAGCACCCTGACGCTGAGC AAAGCAGACTACGAGAAACACAAAGTCTACGC CTGCGAAGTCACCCATCAGGGCCTGAGCTCGC CCGTCACAAAGAGCTTCAACAGGGGAGAGTGT TAG |
| 24 | Nucleic acid coding sequence for the light chain of TR000 | GATATCCAGATGACCCAGTCTCCATCTAGCCTG TCCGCTTCTGTGGGCGATAGAGTGACCATCACA TGCAGAGCTTCTCAGGATATCAATAAGTATCTG GCTTGGTATCAGCAGAAGCCTGGAAAGGTGCC TAAGCTGCTGATCTACTCTACATCTACCCTGCA GTCTGGAGTGCCTTCTAGATTTTCTGGATCTGG CTCTGGCACCGATTTTACACTGACAATCTCTTC TCTGCAGCCTGAGGATGTGGCTACATATTATTGT CTGCAGTATGATGATCTGTTCACCTTTGGCCAG GGCACCAAGCTGGAGATCAAGCGTACGGTGGC TGCACCATCTGTCTTCATCTTCCCGCCATCTGAT GAGCAGTTGAAATCTGGAACTGCCTCTGTTGT GTGCCTGCTGAATAACTTCTATCCCAGAGAGGC CAAAGTACAGTGGAAGGTGGATAACGCCCTCC AATCGGGTAACTCCCAGGAGAGTGTCACAGAG CAGGACAGCAAGGACAGCACCTACAGCCTCAG CAGCACCCTGACGCTGAGCAAAGCAGACTACG AGAAACACAAAGTCTACGCCTGCGAAGTCACC CATCAGGGCCTGAGCTCGCCCGTCACAAAGAG CTTCAACAGGGGAGAGTGTTAG |
| 25 | Nucleic acid coding sequence of the light chain variable region of TR005 | GATATCCAGATGACCCAGTCTCCATCTAGCCTG TCCGCTTCTGTGGGCGATAGAGTGACCATCACA TGCAGAGCTTCTCAGGATATCAATAAGTATCTG GCTTGGTATCAGCAGAAGCCTGGAAAGGTGCC TAAGCTGCTGATCTACTCTACATCTACCCTGCA GTCTGGAGTGCCTTCTAGATTTTCTGGATCTGG CTCTGGCACCGATTTTACACTGACAATCTCTTC TCTGCAGCCTGAGGATGTGGCTACATATTATTGT CTGCAGTATGATGATCTGTTCACCTTTGGCCAG GGCACCAAGCTGGAGATCAAG |
| 26 | Nucleic acid coding sequence for the light chain constant region of TR005 | CGTACGGTGGCTGCACCATCTGTCTTCATCTTC CCGCCATCTGATGAGCAGTTGAAATCTGGAACT GCCTCTGTTGTGTGCCTGCTGAATAACTTCTAT CCCAGAGAGGCCAAAGTACAGTGGAAGGTGG ATAACGCCCTCCAATCGGGTAACTCCCAGGAG AGTGTCACAGAGCAGGACAGCAAGGACAGCA CCTACAGCCTCAGCAGCACCCTGACGCTGAGC AAAGCAGACTACGAGAAACACAAAGTCTACGC CTGCGAAGTCACCCATCAGGGCCTGAGCTCGC CCGTCTGTACAAAGAGCTTCAACAGGGGAGAG TGTTAG |
| 27 | Nucleic acid coding sequence for the light chain of TR005 | GATATCCAGATGACCCAGTCTCCATCTAGCCTG TCCGCTTCTGTGGGCGATAGAGTGACCATCACA TGCAGAGCTTCTCAGGATATCAATAAGTATCTG GCTTGGTATCAGCAGAAGCCTGGAAAGGTGCC TAAGCTGCTGATCTACTCTACATCTACCCTGCA GTCTGGAGTGCCTTCTAGATTTTCTGGATCTGG CTCTGGCACCGATTTTACACTGACAATCTCTTC TCTGCAGCCTGAGGATGTGGCTACATATTATTGT CTGCAGTATGATGATCTGTTCACCTTTGGCCAG GGCACCAAGCTGGAGATCAAGCGTACGGTGGC TGCACCATCTGTCTTCATCTTCCCGCCATCTGAT GAGCAGTTGAAATCTGGAACTGCCTCTGTTGT GTGCCTGCTGAATAACTTCTATCCCAGAGAGGC CAAAGTACAGTGGAAGGTGGATAACGCCCTCC AATCGGGTAACTCCCAGGAGAGTGTCACAGAG CAGGACAGCAAGGACAGCACCTACAGCCTCAG CAGCACCCTGACGCTGAGCAAAGCAGACTACG AGAAACACAAAGTCTACGCCTGCGAAGTCACC CATCAGGGCCTGAGCTCGCCCGTCTGTACAAA GAGCTTCAACAGGGGAGAGTGTTAG |
| 28 | Nucleic acid coding sequence of the heavy chain variable region of TR000 and TR005 | CAGGTGCAGCTGGTGCAGTCCGGCGCCGAGGT GAAGAAGCCCGGCGCCTCCGTGAAGCTGAGCT GTAAGGCCTCCGGCTACACCTTCACCTCCTTCG ACATTAACTGGGTGCGGCAGGCCCCCGAGCAG CGCCTGGAGTGGATGGGCTGGATCTTCCCCGG CGACGGCAACACCAAGTACTCCCAGAAGTTCC AGGGAAGAGCTACCATCACCAGAGATACATCC GCTTCTACAGCTTACATGGAGCTGTCTAGCCTG AGATCTGAGGATACAGCTGTGTATTACTGTGTG AGAGGAGAGGCTCTGTACTATTTTGATTATTGG GGCCAGGGCACCCTGGTGACAGTGTCTTCT |
| 29 | Nucleic acid coding sequences of the heavy chain constant regions of TR000 and TR005 | GCTAGCACCAAGGGCCCATCGGTCTTCCCCCTG GCACCCTCCTCCAAGAGCACCTCTGGGGGCAC AGCGGCCCTGGGCTGCCTGGTCAAGGACTACT TCCCCGAACCGGTGACGGTGTCGTGGAACTCA GGCGCCCTGACCAGCGGCGTGCACACCTTCCC GGCTGTCCTACAGTCCTCAGGACTCTACTCCCT CAGCAGCGTGGTGACCGTGCCCTCCAGCAGCT TGGGCACCCAGACCTACATCTGCAACGTGAAT CACAAGCCCAGCAACACCAAGGTGGACAAGA GAGTTGAGCCCAAATCTTGTGACAAAACTCAC ACATGCCCACCGTGCCCAGCACCTGAACTCCT GGGGGGACCGTCAGTCTTCCTCTTCCCCCCAA AACCCAAGGACACCCTCATGATCTCCCGGACC CCTGAGGTCACATGCGTGGTGGTGGACGTGAG CCACGAAGACCCTGAGGTCAAGTTCAACTGGT ACGTGGACGGCGTGGAGGTGCATAATGCCAAG ACAAAGCCGCGGGAGGAGCAGTACAACAGCA CGTACCGTGTGGTCAGCGTCCTCACCGTCCTGC ACCAGGACTGGCTGAATGGCAAGGAGTACAAG TGCAAGGTCTCCAACAAAGCCCTCCCAGCCCC CATCGAGAAAACCATCTCCAAAGCCAAAGGGC AGCCCCGAGAACCACAGGTGTACACCCTGCCC CCATCCCGGGATGAGCTGACCAAGAACCAGGT CAGCCTGACCTGCCTGGTCAAAGGCTTCTATCC CAGCGACATCGCCGTGGAGTGGGAGAGCAATG GGCAGCCGGAGAACAACTACAAGACCACGCCT CCCGTGCTGGACTCCGACGGCTCCTTCTTCCTC TATAGCAAGCTCACCGTGGACAAGAGCAGGTG GCAGCAGGGGAACGTCTTCTCATGCTCCGTGA TGCATGAGGCTCTGCACAACCACTACACGCAG AAGAGCCTCTCCCTGTCTCCGGGTTAA |
| 30 | Nucleic acid coding sequences for the heavy chains of TR000 and TR005 | CAGGTGCAGCTGGTGCAGTCCGGCGCCGAGGT GAAGAAGCCCGGCGCCTCCGTGAAGCTGAGCT GTAAGGCCTCCGGCTACACCTTCACCTCCTTCG ACATTAACTGGGTGCGGCAGGCCCCCGAGCAG CGCCTGGAGTGGATGGGCTGGATCTTCCCCGG CGACGGCAACACCAAGTACTCCCAGAAGTTCC AGGGAAGAGCTACCATCACCAGAGATACATCC GCTTCTACAGCTTACATGGAGCTGTCTAGCCTG AGATCTGAGGATACAGCTGTGTATTACTGTGTG AGAGGAGAGGCTCTGTACTATTTTGATTATTGG GGCCAGGGCACCCTGGTGACAGTGTCTTCTGC TAGCACCAAGGGCCCATCGGTCTTCCCCCTGGC ACCCTCCTCCAAGAGCACCTCTGGGGGCACAG CGGCCCTGGGCTGCCTGGTCAAGGACTACTTC CCCGAACCGGTGACGGTGTCGTGGAACTCAGG CGCCCTGACCAGCGGCGTGCACACCTTCCCGG CTGTCCTACAGTCCTCAGGACTCTACTCCCTCA GCAGCGTGGTGACCGTGCCCTCCAGCAGCTTG GGCACCCAGACCTACATCTGCAACGTGAATCA CAAGCCCAGCAACACCAAGGTGGACAAGAGA GTTGAGCCCAAATCTTGTGACAAAACTCACAC ATGCCCACCGTGCCCAGCACCTGAACTCCTGG GGGGACCGTCAGTCTTCCTCTTCCCCCCAAAA CCCAAGGACACCCTCATGATCTCCCGGACCCCT GAGGTCACATGCGTGGTGGTGGACGTGAGCCA CGAAGACCCTGAGGTCAAGTTCAACTGGTACG TGGACGGCGTGGAGGTGCATAATGCCAAGACA AAGCCGCGGGAGGAGCAGTACAACAGCACGT ACCGTGTGGTCAGCGTCCTCACCGTCCTGCAC CAGGACTGGCTGAATGGCAAGGAGTACAAGTG CAAGGTCTCCAACAAAGCCCTCCCAGCCCCCA TCGAGAAAACCATCTCCAAAGCCAAAGGGCAG CCCCGAGAACCACAGGTGTACACCCTGCCCCC ATCCCGGGATGAGCTGACCAAGAACCAGGTCA GCCTGACCTGCCTGGTCAAAGGCTTCTATCCCA GCGACATCGCCGTGGAGTGGGAGAGCAATGGG CAGCCGGAGAACAACTACAAGACCACGCCTCC CGTGCTGGACTCCGACGGCTCCTTCTTCCTCTA TAGCAAGCTCACCGTGGACAAGAGCAGGTGGC AGCAGGGGAACGTCTTCTCATGCTCCGTGATG CATGAGGCTCTGCACAACCACTACACGCAGAA GAGCCTCTCCCTGTCTCCGGGTTAA |
| 31 | Amino acid sequence of CDR-L1 of hu7F11 | KASQSVSNDVV |
| 32 | Amino acid sequence of CDR-L2 of hu7F11 | YASNRYT |
| 33 | Amino acid sequence of CDR-L3 of hu7F11 | QQDYSSPWT |
| 34 | Amino acid sequence of CDR-H1 of hu7F11 | DHVIS |
| 35 | Amino acid sequence of CDR-H2 of hu7F11 | QIYPGSDNSYYAQKFQG |
| 36 | Amino acid sequence of CDR-H3 of hu7F11 | EGYGYGKNGVGYAMDY |
| 37 | Amino acid sequence of the light chain variable region of hu7F 11 | DIVMTQSPDSLAVSLGERATINCKASQSVSNDVV WYQQKPGQPPKLLIYYASNRYTGVPDRFSGSGY GTDFTLTISSLQAEDVAVYYCQQDYSSPWTFGGG TKVEIK |
| 38 | Amino acid sequence of the light chain constant region of hu7F 11 | |
| 39 | Amino acid sequence of the light chain of hu7F 11 | DIVMTQSPDSLAVSLGERATINCKASQSVSNDVV WYQQKPGQPPKLLIYYASNRYTGVPDRFSGSGY GTDFTLTISSLQAEDVAVYYCQQDYSSPWTFGGG TKVEIKRTVAAPSVFIFPPSDEQLKSGTASVVCLL NNFYPREAKVQWKVDNALQSGNSQESVTEQDS KDSTYSLSSTLTLSKADYEKHKVYACEVTHQGLS SPVTKSFNRGEC |
| 40 | Amino acid sequence of the heavy chain variable region of hu7F 11 | QVQLVQSGAEVKKPGASVKVSCKASGYTFTDH VISWVRQATGQGLEWMGQIYPGSDNSYYAQKF QGRVTLTADKSINTAYMELSSLRSEDTAVYYCAR EGYGYGKNGVGYAMDYWGQGTLVTVSS |
| 41 | Amino acid sequence of the heavy chain constant region of hu7F 11 | ASTKGPSVFPLAPSSKSTSGGTAALGCLVKDYFP EPVTVSWNSGALTSGVHTFPAVLQSSGLYSLSSV VTVPSSSLGTQTYICNVNHKPSNTKVDKRVEPKS CDKTHTCPPCPAPELLGGPSVFLFPPKPKDTLMIS RTPEVTCVVVDVSHEDPEVKFNWYVDGVEVHN AKTKPREEQYNSTYRVVSVLTVLHQDWLNGKE YKCKVSNKALPAPIEKTISKAKGQPREPQVYTLP PSRDELTKNQVSLTCLVKGFYPSDIAVEWESNGQ PENNYKTTPPVLDSDGSFFLYSKLTVDKSRWQQG NVFSCSVMHEALHNHYTQKSLSLSPG |
| 42 | Amino acid sequence of the heavy chain of hu7F 11 | QVQLVQSGAEVKKPGASVKVSCKASGYTFTDH VISWVRQATGQGLEWMGQIYPGSDNSYYAQKF QGRVTLTADKSINTAYMELSSLRSEDTAVYYCAR EGYGYGKNGVGYAMDYWGQGTLVTVSSASTK GPSVFPLAPSSKSTSGGTAALGCLVKDYFPEPVT VSWNSGALTSGVHTFPAVLQSSGLYSLSSVVTVP SSSLGTQTYICNVNHKPSNTKVDKRVEPKSCDKT HTCPPCPAPELLGGPSVFLFPPKPKDTLMISRTPE VTCVVVDVSHEDPEVKFNWYVDGVEVHNAKT KPREEQYNSTYRVVSVLTVLHQDWLNGKEYKC KVSNKALPAPIEKTISKAKGQPREPQVYTLPPSRD ELTKNQVSLTCLVKGFYPSDIAVEWESNGQPENN YKTTPPVLDSDGSFFLYSKLTVDKSRWQQGNVFS CSVMHEALHNHYTQKSLSLSPG |
| 43 | Nucleic acid coding sequence for CDR-L1 of hu7F11 | AAGGCTTCTCAGTCTGTGTCTAATGATGTGGTG |
| 44 | Nucleic acid coding sequence of CDR-L2 of hu7F 11 | TACGCCTCCAACAGGTACACC |
| 45 | Nucleic acid coding sequence of CDR-L3 of hu7F 11 | CAGCAGGACTACTCCTCCCCCTGGACC |
| 46 | Nucleic acid coding sequence for CDR-H1 of hu7F11 | GACCACGTGATCTCC |
| 47 | Nucleic acid coding sequence of CDR-H2 of hu7F 11 | CAGATCTACCCCGGCTCCGACAACTCCTACTAC GCCCAGAAGTTCCAGGGC |
| 48 | Nucleic acid coding sequence of CDR- H3 of hu7F11 | GAGGGCTACGGCTACGGCAAGAACGGCGTGG GCTACGCCATGGATTAT |
| 49 | Nucleic acid coding sequence of the light chain variable region of hu7F 11 | GATATCGTGATGACACAGTCTCCTGATTCTCTG GCCGTGTCTCTGGGCGAAAGAGCTACAATCAA CTGTAAGGCTTCTCAGTCTGTGTCTAATGATGT GGTGTGGTACCAGCAGAAGCCTGGGCAGCCCC CCAAGCTGCTGATCTACTACGCCTCCAACAGGT ACACCGGCGTGCCCGACAGGTTCTCCGGCTCC GGCTACGGCACCGACTTCACCCTGACCATCTCC TCCCTGCAGGCCGAGGACGTGGCCGTGTACTA CTGCCAGCAGGACTACTCCTCCCCCTGGACCTT CGGCGGCGGCACCAAGGTGGAGATCAAG |
| 50 | Nucleic acid coding sequence for the light chain constant region of hu7F 11 | CGTACGGTGGCTGCACCATCTGTCTTCATCTTC CCGCCATCTGATGAGCAGTTGAAATCTGGAACT GCCTCTGTTGTGTGCCTGCTGAATAACTTCTAT CCCAGAGAGGCCAAAGTACAGTGGAAGGTGG ATAACGCCCTCCAATCGGGTAACTCCCAGGAG AGTGTCACAGAGCAGGACAGCAAGGACAGCA CCTACAGCCTCAGCAGCACCCTGACGCTGAGC AAAGCAGACTACGAGAAACACAAAGTCTACGC CTGCGAAGTCACCCATCAGGGCCTGAGCTCGC CCGTCACAAAGAGCTTCAACAGGGGAGAGTGT TAG |
| 51 | Nucleic acid coding sequence for the light chain of hu7F 11 | GATATCGTGATGACACAGTCTCCTGATTCTCTG GCCGTGTCTCTGGGCGAAAGAGCTACAATCAA CTGTAAGGCTTCTCAGTCTGTGTCTAATGATGT GGTGTGGTACCAGCAGAAGCCTGGGCAGCCCC CCAAGCTGCTGATCTACTACGCCTCCAACAGGT ACACCGGCGTGCCCGACAGGTTCTCCGGCTCC GGCTACGGCACCGACTTCACCCTGACCATCTCC TCCCTGCAGGCCGAGGACGTGGCCGTGTACTA CTGCCAGCAGGACTACTCCTCCCCCTGGACCTT CGGCGGCGGCACCAAGGTGGAGATCAAGCGTA CGGTGGCTGCACCATCTGTCTTCATCTTCCCGC CATCTGATGAGCAGTTGAAATCTGGAACTGCCT CTGTTGTGTGCCTGCTGAATAACTTCTATCCCA GAGAGGCCAAAGTACAGTGGAAGGTGGATAAC GCCCTCCAATCGGGTAACTCCCAGGAGAGTGT CACAGAGCAGGACAGCAAGGACAGCACCTAC AGCCTCAGCAGCACCCTGACGCTGAGCAAAGC AGACTACGAGAAACACAAAGTCTACGCCTGCG AAGTCACCCATCAGGGCCTGAGCTCGCCCGTC ACAAAGAGCTTCAACAGGGGAGAGTGTTAG |
| 52 | Nucleic acid coding sequence of the heavy chain variable region of hu7F 11 | CAGGTGCAGCTGGTGCAGTCTGGGGCCGAGGT GAAAAAGCCAGGCGCTTCTGTGAAGGTGTCTT GCAAGGCCTCCGGCTACACCTTCACCGACCAC GTGATCTCCTGGGTGCGCCAGGCCACCGGCCA GGGCCTGGAGTGGATGGGCCAGATCTACCCCG GCTCCGACAACTCCTACTACGCCCAGAAGTTC CAGGGCAGGGTGACTCTGACCGCCGACAAGTC CATCAACACCGCCTACATGGAGCTGTCCTCCCT GAGGTCCGAGGACACCGCCGTGTACTACTGCG CCAGGGAGGGCTACGGCTACGGCAAGAACGG CGTGGGCTACGCCATGGATTATTGGGGCCAGGG CACCCTGGTGACAGTGTCTTCT |
| 53 | Nucleic acid coding sequence for the heavy chain constant region of hu7F 11 | GCTAGCACCAAGGGCCCATCGGTCTTCCCCCTG GCACCCTCCTCCAAGAGCACCTCTGGGGGCAC AGCGGCCCTGGGCTGCCTGGTCAAGGACTACT TCCCCGAACCGGTGACGGTGTCGTGGAACTCA GGCGCCCTGACCAGCGGCGTGCACACCTTCCC GGCTGTCCTACAGTCCTCAGGACTCTACTCCCT CAGCAGCGTGGTGACCGTGCCCTCCAGCAGCT TGGGCACCCAGACCTACATCTGCAACGTGAAT CACAAGCCCAGCAACACCAAGGTGGACAAGA GAGTTGAGCCCAAATCTTGTGACAAAACTCAC ACATGCCCACCGTGCCCAGCACCTGAACTCCT GGGGGGACCGTCAGTCTTCCTCTTCCCCCCAA AACCCAAGGACACCCTCATGATCTCCCGGACC CCTGAGGTCACATGCGTGGTGGTGGACGTGAG CCACGAAGACCCTGAGGTCAAGTTCAACTGGT ACGTGGACGGCGTGGAGGTGCATAATGCCAAG ACAAAGCCGCGGGAGGAGCAGTACAACAGCA CGTACCGTGTGGTCAGCGTCCTCACCGTCCTGC ACCAGGACTGGCTGAATGGCAAGGAGTACAAG TGCAAGGTCTCCAACAAAGCCCTCCCAGCCCC CATCGAGAAAACCATCTCCAAAGCCAAAGGGC AGCCCCGAGAACCACAGGTGTACACCCTGCCC CCATCCCGGGATGAGCTGACCAAGAACCAGGT CAGCCTGACCTGCCTGGTCAAAGGCTTCTATCC CAGCGACATCGCCGTGGAGTGGGAGAGCAATG GGCAGCCGGAGAACAACTACAAGACCACGCCT CCCGTGCTGGACTCCGACGGCTCCTTCTTCCTC TATAGCAAGCTCACCGTGGACAAGAGCAGGTG GCAGCAGGGGAACGTCTTCTCATGCTCCGTGA TGCATGAGGCTCTGCACAACCACTACACGCAG AAGAGCCTCTCCCTGTCTCCGGGTTAA |
| 54 | Nucleic acid coding sequence for the heavy chain of hu7F 11 | CAGGTGCAGCTGGTGCAGTCTGGGGCCGAGGT GAAAAAGCCAGGCGCTTCTGTGAAGGTGTCTT GCAAGGCCTCCGGCTACACCTTCACCGACCAC GTGATCTCCTGGGTGCGCCAGGCCACCGGCCA GGGCCTGGAGTGGATGGGCCAGATCTACCCCG GCTCCGACAACTCCTACTACGCCCAGAAGTTC CAGGGCAGGGTGACTCTGACCGCCGACAAGTC CATCAACACCGCCTACATGGAGCTGTCCTCCCT GAGGTCCGAGGACACCGCCGTGTACTACTGCG CCAGGGAGGGCTACGGCTACGGCAAGAACGG CGTGGGCTACGCCATGGATTATTGGGGCCAGGG CACCCTGGTGACAGTGTCTTCTGCTAGCACCA AGGGCCCATCGGTCTTCCCCCTGGCACCCTCCT CCAAGAGCACCTCTGGGGGCACAGCGGCCCTG GGCTGCCTGGTCAAGGACTACTTCCCCGAACC GGTGACGGTGTCGTGGAACTCAGGCGCCCTGA CCAGCGGCGTGCACACCTTCCCGGCTGTCCTA CAGTCCTCAGGACTCTACTCCCTCAGCAGCGT GGTGACCGTGCCCTCCAGCAGCTTGGGCACCC AGACCTACATCTGCAACGTGAATCACAAGCCC AGCAACACCAAGGTGGACAAGAGAGTTGAGC CCAAATCTTGTGACAAAACTCACACATGCCCA CCGTGCCCAGCACCTGAACTCCTGGGGGGACC GTCAGTCTTCCTCTTCCCCCCAAAACCCAAGG ACACCCTCATGATCTCCCGGACCCCTGAGGTCA CATGCGTGGTGGTGGACGTGAGCCACGAAGAC CCTGAGGTCAAGTTCAACTGGTACGTGGACGG CGTGGAGGTGCATAATGCCAAGACAAAGCCGC GGGAGGAGCAGTACAACAGCACGTACCGTGTG GTCAGCGTCCTCACCGTCCTGCACCAGGACTG GCTGAATGGCAAGGAGTACAAGTGCAAGGTCT CCAACAAAGCCCTCCCAGCCCCCATCGAGAAA ACCATCTCCAAAGCCAAAGGGCAGCCCCGAGA ACCACAGGTGTACACCCTGCCCCCATCCCGGG ATGAGCTGACCAAGAACCAGGTCAGCCTGACC TGCCTGGTCAAAGGCTTCTATCCCAGCGACATC GCCGTGGAGTGGGAGAGCAATGGGCAGCCGG AGAACAACTACAAGACCACGCCTCCCGTGCTG GACTCCGACGGCTCCTTCTTCCTCTATAGCAAG CTCACCGTGGACAAGAGCAGGTGGCAGCAGG GGAACGTCTTCTCATGCTCCGTGATGCATGAGG CTCTGCACAACCACTACACGCAGAAGAGCCTC TCCCTGTCTCCGGGTTAA |

In a third aspect, the application also provides the use of the ligand-drug conjugate of the application or pharmaceutically acceptable salt or solvate thereof, or the linker-payload of the application or isomer, mesomer, racemate, enantiomer or mixture thereof, or its pharmaceutically acceptable salt or solvate thereof for preparing a drug, the drug is used to treat or prevent tumors.

In certain embodiments, the tumor is selected from solid tumors or non-solid tumors, such as breast cancer, ovarian cancer, cervical cancer, uterine cancer, prostate cancer, kidney cancer, urethral cancer, bladder cancer, liver cancer, gastric cancer, endometrial cancer, salivary gland cancer, esophageal cancer, lung cancer, colon cancer, rectal cancer, colorectal cancer, bone cancer, skin cancer, thyroid cancer, pancreatic cancer, melanoma, glioma, neuroblastoma, glioblastoma multiforme, sarcoma, lymphoma and leukemia.

In a further aspect, the application also provides a pharmaceutical composition comprising an effective amount of the ligand-drug conjugate of the application or a pharmaceutically acceptable salt or solvate thereof, or a linker-payload of the application or an isomer, mesomer, racemate, enantiomer or a mixture thereof, or a pharmaceutically acceptable salt or solvate thereof, and a pharmaceutically acceptable carrier, diluent or excipient.

In a further aspect, the application also provides a method for treating or preventing tumors, comprising administering an effective amount of the ligand-drug conjugate of the application or a pharmaceutically acceptable salt or solvate thereof, or a linker-payload of the application or an isomer, mesomer, racemate, enantiomer or a mixture thereof, or a pharmaceutically acceptable salt or solvate thereof to a subject in need thereof. In certain embodiments, the subject is a mammal, such as a human.

In a further aspect, the application also provides the use of the linker-payload of the application or isomer, mesomer, racemate, enantiomer or mixture thereof, or pharmaceutically acceptable salt or solvate thereof for preparing a ligand-drug conjugate or pharmaceutically acceptable salt or solvate thereof. In certain embodiments, the ligand-drug conjugate is selected from the ligand-drug conjugate of the application.

In a further aspect, the application also provides a method for preparing the ligand-drug conjugate of the application or pharmaceutically acceptable salt or solvate thereof, the method comprising: conjugation a reduced antibody or its antigen-binding fragment with the linker-payload of the application or isomer, mesomer, racemate, enantiomer or mixture thereof, or its pharmaceutically acceptable salt or solvate thereof to obtain the ligand-drug conjugate. The antibody or its antigen-binding fragment can be reduced by a thiol reducing agent (e.g., tris(2-carboxyethyl)phosphine (TCEP)).

### Definition of Terms

Unless otherwise defined, all technical and scientific terms used herein are consistent with the common understanding of one of ordinary skill in the art to which the application belongs. Although any methods and materials similar or equivalent to those described herein may be used to practice or test the application, the application describes preferred methods and materials. In describing and claiming the application, the following terms are used in accordance with the following definitions.

When a trade name is used in the application, the applicant intends to include the formulations of the trade name product, the generic drugs and active pharmaceutical ingredients of the trade name product.

Unless otherwise stated, the terms used in the specification and claims have the following meanings.

The term "ligand" is a targeting agent that specifically binds to a target moiety. The ligand is capable of specifically binding to a cellular component or to other target molecules of interest. The target moiety or target is typically on the surface of a cell. In some aspects, the role of the ligand is to deliver the drug unit to a specific target cell population with which the ligand unit interacts. Ligands include, but are not limited to, proteins, polypeptides, and peptides, as well as non-proteins such as sugars. Suitable ligand units include, for example, antibodies, such as full-length (intact) antibodies and antigen-binding fragments thereof. In the embodiment where the ligand unit is a non-antibody targeting agent, it can be a peptide or polypeptide, or a non-protein molecule. Examples of such targeting agents include interferons, lymphokines, hormones, growth factors and colony stimulating factors, vitamins, nutrient transport molecules, or any other cell binding molecules or substances. In some embodiments, the linker is covalently attached to the sulphur atom of the ligand. In some aspects, the sulphur atom is the sulphur atom of a cysteine residue, which forms the interchain disulfide bond of the antibody. On the other hand, the sulphur atom is the sulphur atom of a cysteine residue that has been introduced into the ligand unit, which forms the interchain disulfide bond of the antibody. On the other hand, the sulphur atom is the sulphur atom of a cysteine residue that is introduced into the ligand unit by, for example, site-directed mutagenesis or chemical reaction.

The term "drug" refers to a cytotoxic drug, that is, a molecule that has a strong ability to destroy the normal growth of tumor or cancer cells. In principle, cytotoxic drugs can kill tumor cells at sufficiently high concentrations, but due to the lack of specificity, while killing tumor or cancer cells, they can also cause apoptosis of normal cells, which can easily lead to serious side effects.

The term "ligand-drug conjugate" refers to a molecule formed by connecting a ligand to a drug through a stable linking unit. In the application, "ligand-drug conjugate" is in one embodiment an antibody-drug conjugate (ADC), which refers to connecting a monoclonal antibody or a functional antibody fragment or a targeted protein to a cytotoxic drug through a stable linking unit.

The term "antibody" or "functional antibody fragment" includes any part of the antibody structure within its scope. This unit can bind, reactively associate or complex with a receptor, antigen or other receptor unit possessed by a targeted cell population. Antibodies can be any protein or protein-like molecule that can bind, complex or react with a part of the cell population to be treated or biomodified.

The antibodies of the application include but are not limited to murine antibodies, chimeric antibodies, humanized antibodies and fully human antibodies, in one embodiment humanized antibodies and fully human antibodies.

The three-letter codes and single-letter codes of amino acids used in the application are as described in J. boil. Chem. 1968, 243, 3558.

The term "natural amino acid" refers to amino acids that can be synthesized by organisms. Natural amino acids are generally L-type, but there are a few exceptions, such as glycine, including natural and biologically synthesized ones.

The term "unnatural amino acid" refers to amino acids that can only be synthesized by artificial methods.

The term "alkyl" refers to a saturated aliphatic hydrocarbon group, which is a straight or branched chain group containing 1 to 20 carbon atoms, in one embodiment an alkyl group containing 1 to 12 carbon atoms, in one embodiment an alkyl group containing 1 to 10 carbon atoms, and in one embodiment an alkyl group containing 1 to 6 or 1 to 4 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2, 2-dimethylpentyl, 3, 3-dimethylpentyl, 2-ethylpentyl, 3-ethylpentyl, n-octyl, 2, 3-dimethylhexyl, 2, 4-dimethylhexyl, 2, 5-dimethylhexyl, 2, 2-dimethylhexyl, 3, 3-dimethylhexyl, 4, 4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2, 2-diethylpentyl, n-decyl, 3, 3-diethylhexyl, 2, 2-diethylhexyl, and various branched chain isomers thereof. More preferred are lower alkyl groups having 1 to 6 (e.g., 1 to 4) carbon atoms, non-limiting examples of which include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl group may be substituted or unsubstituted. When substituted, the substituent may be substituted at any available point of attachment. The substituent is in one embodiment one or more of the following groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkyloxy, heterocycloalkyloxy, cycloalkylthio, heterocycloalkylthio, and oxo.

The term "substituted alkyl" means that the hydrogen in the alkyl group is replaced by a substituent group. Unless otherwise specified in the context, the substituent group of the alkyl group can be one or more groups selected from the following groups: -halogen, -OR', -NR'R", - SR', -SiR'R"R‴, -OC(O)R', -C(O)R', -CO₂R', -CONR'R", -OC(O)NR'R", -NR"C(O)R', - NR'-C(O)NR'R', -R"', -NR"C(O)₂R', -NH-C(NH₂)=NH, -NR'C(NH₂)=NH, -NH-C(NH₂)=NR', - S(O)R', -S(O)₂R', -S(O)₂NR'R", -NR'S(O)₂R", -CN and -NO₂, and the number of substituents is 1 to (2m'+1), wherein m' is the total number of carbon atoms in the group, for example 1, 2, 3, 4, 5 or 6. R', R" and R‴ each represent hydrogen, C₁₋₈ alkyl, aryl, aryl substituted by 1-3 halogens, C₁₋₈ alkyl substituted by 1-3 halogens, C₁₋₈ alkoxy or C₁₋₈ thioalkoxy, or unsubstituted aryl-C₁₋₄ alkyl. When R' and R" are attached to the same nitrogen atom, they can form a 3-, 4-, 5-, 6- or 7-membered ring with the nitrogen atom. For example, -NR'R" includes 1-pyrrolidinyl and 4-morpholinyl.

The term "heteroalkyl" refers to a group formed by replacing one or more carbons on the alkyl group with N, O or S.

The term "cycloalkyl" refers to a saturated or partially unsaturated monocyclic or polycyclic cyclic hydrocarbon group, wherein the ring of the cycloalkyl group contains 3 to 20 carbon atoms, in one embodiment 3 to 12 carbon atoms, in one embodiment 3 to 10 carbon atoms, and in one embodiment 3 to 8 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; polycyclic cycloalkyl groups include spirocyclic, fused and bridged cycloalkyl groups.

The term "alkoxy" refers to -O-(alkyl) and -O-(cycloalkyl), wherein alkyl or cycloalkyl is as defined above. Non-limiting examples of alkoxy groups include: methoxy, ethoxy, propoxy, butoxy, cyclopropyloxy, cyclobutyloxy, cyclopentyloxy, cyclohexyloxy. The alkoxy group may be optionally substituted or unsubstituted, and when substituted, the substituent is in one embodiment one or more of the following groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkyloxy, heterocycloalkyloxy, cycloalkylthio, heterocycloalkylthio.

The term "heterocycle" refers to a saturated or partially unsaturated monocyclic or polycyclic cyclic hydrocarbon containing 3 to 20 ring atoms, one or more (e.g., 1, 2, 3 or 4) of which are heteroatoms selected from nitrogen, oxygen or S(O)ₘ (wherein m is 0, 1 or 2), and the remaining ring atoms are carbon. In one embodiment, it contains 3 to 12 ring atoms, of which 1 to 4 are heteroatoms; in one embodiment, it contains 3 to 10 or 3 to 8 ring atoms. Non-limiting examples of monocyclic heterocyclic groups include pyrrolidinyl, piperidinyl, piperazinyl, morpholinyl, thiomorpholinyl, homopiperazinyl, etc. Polycyclic heterocyclic groups include spirocyclic, fused ring and bridged heterocyclic groups.

The term "aryl" refers to a 6- to 14-membered all-carbon monocyclic or fused polycyclic (i.e., a ring that shares adjacent pairs of carbon atoms) group having a conjugated π electron system, in one embodiment 6-10 members, such as phenyl. The aryl group may be substituted or unsubstituted, and when substituted, the substituent may be one or more of the following groups, selected without limitation from alkyl, alkenyl, alkynyl, alkoxy, alkylthio, alkylamino, halogen, deuterium atom, mercapto, hydroxyl, nitro, cyano, cycloalkyl, heterocycloalkyl, aryl, heteroaryl, cycloalkyloxy, heterocycloalkyloxy, cycloalkylthio or heterocycloalkylthio.

The term "heteroaryl" includes 5-8-membered monocyclic heteroaryl and 8-12-membered fused heteroaryl.

The term "5-8-membered monocyclic heteroaryl" refers to a monocyclic cyclic group with aromaticity containing 5-8 ring atoms (at least one of which is a heteroatom, such as a nitrogen atom, an oxygen atom or a sulfur atom). Optionally, the ring atoms (such as carbon atoms, nitrogen atoms or sulfur atoms) in the ring structure can be oxidized. "5-8-membered monocyclic heteroaryl" includes, for example, "5-7-membered monocyclic heteroaryl", "5-6-membered monocyclic heteroaryl", "5-6-membered monocyclic nitrogen-containing heteroaryl", "6-membered monocyclic nitrogen-containing heteroaryl", etc. The heteroatom in the "nitrogen-containing heteroaryl" contains at least one nitrogen atom, for example, only 1 or 2 nitrogen atoms, or one nitrogen atom and other 1 or 2 heteroatoms (such as oxygen atoms and/or sulfur atoms), or 2 nitrogen atoms and other 1 or 2 heteroatoms (such as oxygen atoms and/or sulfur atoms). Specific examples of "5-8 membered monocyclic heteroaryl" include but are not limited to furanyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, 2-pyridone, 4-pyridone, pyrimidinyl, pyridazinyl, pyrazinyl, 1,2,3-triazinyl, 1,3,5-triazinyl, 1,2,4,5-tetrazinyl, azacycloheptatrienyl, 1,3-diazacycloheptatrienyl, azacyclooctatetraenyl, etc.

The term "8-12 membered fused heteroaryl" refers to an unsaturated aromatic cyclic structure formed by two or more cyclic structures sharing two adjacent atoms, containing 8-12 ring atoms (at least one of which is a heteroatom, such as a nitrogen atom, an oxygen atom or a sulfur atom). Optionally, the ring atoms (such as carbon atoms, nitrogen atoms or sulfur atoms) in the cyclic structure may be oxo-substituted. "8-12 membered fused heteroaryl" includes "8-10 membered fused heteroaryl", "8-9 membered fused heteroaryl" and the like; specific examples include, but are not limited to, pyrrolopyrrole, pyrrolofuran, pyrazolopyrrole, pyrazolothiophene, furathiophene, pyrazolooxazole, benzofuranyl, benzisofuranyl, benzothiophenyl, indolyl, isoindolyl, benzoxazolyl, benzimidazolyl, indazolyl, benzotriazolyl, quinolyl, 2-quinolinone, 4-quinolinone, 1-isoquinolinone, isoquinolyl, acridinyl, phenanthridinyl, benzopyridazinyl, phthalazinyl, quinazolinyl, quinoxalinyl, purinyl, naphthyridinyl and the like.

The term "haloalkyl" refers to an alkyl group substituted with one or more halogens, wherein alkyl is as defined above.

The term "deuteratedalkyl" refers to an alkyl group substituted with one or more deuterium atoms, wherein alkyl is as defined above.

The term "hydroxyl" refers to an -OH group.

The term "halogen" refers to fluorine, chlorine, bromine or iodine.

The term "amino" refers to -NH₂.

The term "nitro" refers to -NO₂.

The term "derivative" refers to a substance having a chemical structure similar to a compound but also containing at least one chemical group not present in the compound and/or lacking at least one chemical group present in the compound. The compound to which the derivative is compared is called the "parent" compound. Typically, a "derivative" can be produced from a parent compound in one or more chemical reaction steps.

The term "pharmaceutically acceptable salt" refers to a pharmaceutically acceptable organic or inorganic salt of a compound (e.g., a drug, a linker-payload, or a ligand-drug conjugate). The compound or conjugate may contain at least one amino or carboxyl group and may thus form an addition salt with the corresponding acid or base. Exemplary salts include, but are not limited to, sulfates, trifluoroacetates, citrates, acetates, oxalates, chlorides, bromides, iodides, nitrates, bisulfates, phosphates, acid phosphates, isonicotinates, lactates, salicylates, acid citrates, tartrates, oleates, tannates, pantothenates, bitartrates, ascorbates, salicylates, formates, benzoates, glutamates, methanesulfonates, ethanesulfonates, benzenesulfonates, p-toluenesulfonates, potassium salts, sodium salts, and the like.

The term "solvate" refers to the linker-payload or ligand-drug conjugate of the application formed with one or more solvent molecules, including but not limited to water, ethanol, acetonitrile, isopropanol, DMSO, ethyl acetate, etc.

The term "pharmaceutical composition" refers to a mixture containing one or more compounds of the application or their physiologically/pharmaceutically acceptable salts or prodrugs and other chemical components, as well as other components such as physiologically/pharmaceutically acceptable carriers and/or excipients. The purpose of the pharmaceutical composition is to promote administration to the organism, facilitate the absorption of the active ingredients and thus exert biological activity.

The term "carrier" refers to a system that can change the way drugs enter the human body and their distribution in the body, control the release rate of drugs, and deliver drugs to the target. Drug carrier release and targeting systems can reduce drug degradation and loss, reduce side effects, and improve bioavailability.

The term "excipient" refers to additives or excipients other than the main drug in a drug preparation. Such as adhesives, fillers, disintegrants, and lubricants in tablets; matrix backup in semi-solid preparations ointments and creams; preservatives, antioxidants, flavoring agents, fragrances, cosolvents, emulsifiers, permeability enhancers, osmotic pressure regulators, colorants, etc. in liquid preparations can all be called excipients.

The term "diluent" or "filler" is mainly used to increase the weight and/or volume of the preparation. The addition of diluent not only ensures a certain volume size but also reduces the dosage deviation of the main ingredients and improves the compression molding of the drug.

### Beneficial effects

The application provides an auristatin-based drug conjugate with a highly stable hydrophilic linking unit, wherein the conjugate introduces a linker by forming an ether bond between an aminomethyl group with a highly stable hydrophilic linking unit and a hydroxyl group, which can carry multiple toxins, has good plasma stability, good water solubility, uniformity and safety, can specifically bind to receptors highly expressed in tumor cells, and release toxins in tumor cells, has good anti-tumor activity, and can be used to prevent or treat diseases such as tumors.

### BRIEF DESCRIPTION OF THE DRAWINGS

Figure 1 shows the LC-MS spectrum of compound LP-1a;
Figure 2 shows the LC-MS spectrum of VcMMAE;
Figure 3 shows the SEC-HPLC detection result of ADC-1-1a;
Figure 4 shows the SEC-HPLC detection result of ADC-2-1a;
Figure 5 shows the SEC-HPLC detection result of ADC-1-2a;
Figure 6 shows the SEC-HPLC detection result of ADC-2-2a;
Figure 7 shows the mass spectrometry detection result of the light chain of the reduced antibody TR005;
Figures 8 and 9 show the mass spectrometry detection results of the light chain of reduced ADC-2-1a.

### EXAMPLES

The application is further described below in conjunction with specific examples. It should be understood that these examples are only used to illustrate the application and are not used to limit the scope of the application. The test methods in the following examples that do not specify specific conditions are usually carried out under conventional conditions or according to the conditions recommended by the manufacturer. Unless otherwise specified, all percentages, proportions, ratios, or parts are by weight. Unless otherwise defined, all professional and scientific terms used in the text have the same meanings as those familiar to those skilled in the art. In addition, any method and material similar or equivalent to the content described can be applied to the method of the application. The preferred implementation methods and materials described in the text are for demonstration purposes only.

The general steps used in the following examples of the application are:

### General Step A

### Preparation of ADC by full reduction

Antibody with a monomer rate greater than 95% after preliminary purification is replaced with phosphate buffer containing EDTA using an ultrafiltration centrifuge tube at a concentration of 10 mg/mL. To a solution of antibody (1 equiv.) is added a solution of TCEP (10 equiv.) and the mixture is incubated at room temperature for 6 hours. Then the interchain disulfide bonds of the antibody are opened, and the number of free thiols is determined by the Ellman method to determine whether all disulfide bonds are opened. Then, a solution of linker-payload (10 equiv.) is added, and the mixture is incubated at room temperature for 6 hours. After the reaction is completed, use an ultrafiltration centrifuge tube with a molecular weight cutoff of 30KDa to exchange the solution into PBS, and remove the unconjugated linker-payload to obtain the conjugated ADC (DAR=8).

### General Step B

### Preparation of ADC by partial reduction

The antibody with a monomer rate exceeding 95% after initial purification, is exchanged into a phosphate buffer containing EDTA at a concentration of 10 mg/mL using an ultrafiltration centrifuge tube. TCEP is added at eight times the molar amount of the antibody, and the reaction is allowed to proceed at room temperature for 3 hours. The solution is then exchanged into a phosphate buffer at pH 6.5 using the ultrafiltration centrifuge tube, followed by the addition of DHAA (dehydroascorbic acid, 8 equiv.) and the reaction is incubalated at room temperature for 3 hours. Subsequently, a solution of linker-payload (5 equiv.) is added, and the mixture is incubated at room temperature for 3 hours. After the reaction is completed, the solution is exchanged into PBS using an ultrafiltration centrifuge tube with a molecular weight cutoff of 30KDa, and the unconjugated linker-payload is removed to obtain the site-specific conjugated ADC (DAR=2).

### General Step C

Detection of DAR by reversed-phase high-performance liquid chromatography (RP-HPLC)

### Preparation of RP-HPLC mobile phase:

RP mobile phase A: 0.1% TFA aqueous solution, RP mobile phase B: 0.1% TFA acetonitrile solution.

Dilute the sample to be tested and the corresponding antibody control to 1mg/mL with sample diluent. Add 2µl DTT stock solution to each 98µl diluted sample. Prepare a blank control with 98u1 sample diluent and 2µl DTT stock solution. Mix the samples and heat them in a 65°C metal bath for 30min. Centrifuge the treated samples at 14000rpm for 5 minutes or filter them with a 0.22µm filter to remove large particles in the sample and place the inner tube in the sample bottle and cover it.

Place the sample bottle with the sample on the sample plate, and set the corresponding position, injection volume, injection needle number and injection method for each sample according to the "UPLC Standard Operating Procedure". The chromatographic column model is Proteomix RP-1000 (4.6*100mm, 5µm), Sepax.

The detection wavelength is 214nm and 280nm. The running method is edited as follows:

| Time min | Flow rate mL/min | Volume percent (RP mobile phase A %) | Volume percent (RP mobile phase B %) |
|---|---|---|---|
| 0 | 0.5 | 75 | 25 |
| 3 | 0.5 | 75 | 25 |
| 28 | 0.5 | 50 | 50 |
| 30 | 0.5 | 5 | 95 |
| 32 | 0.5 | 5 | 95 |
| 33 | 0.5 | 75 | 25 |
| 40 | 0.5 | 75 | 25 |

### General Step D

### SEC detection of antibody monomer rate

Prepare SEC mobile phase 50mM PB+300mM NaCl+200mM Arg, IPA=95:5, adjust pH=6.5 (the meanings of the abbreviations are as follows: PB: phosphate buffer solution, Arg: arginine, IPA: isopropanol). Dilute the sample to a concentration between 1~2mg/ml and then centrifuge the sample at 14000rpm for 5 minutes or filter it with a 0.22µm filter to remove large particles in the sample. Place the sample bottle on the sample plate and set the position, injection volume, number of injection needles and injection method for each sample according to the "UPLC Standard Operating Procedure". The column model is: XBridge BEH SEC, 200Å, 1.7µm, 4.6*150mm, Waters.

Set the detection wavelength to 214nm and 280nm, and edit the running method as follows:

| Time min | Flow rate ml/min | Percentage of mobile phase (SEC %) |
|---|---|---|
| 0 | 0.8 | 100 |
| 12 | 0.8 | 100 |

### General Step E

### Hydrophobic Interaction Chromatography (HIC) Assay

Analysis of ADC was performed using hydrophobic interaction chromatography (HIC). Elution was performed using 0-100% mobile phase B (MPB), where mobile phase A (MPA) consisted of 1.5M ammonium sulfate and 0.025M sodium phosphate, and MPB consisted of 0.025M sodium phosphate, 25% isopropanol. The sample loading was approximately 20µg, and the gradient elution was completed in 15 minutes. The detection wavelength was 280nm, and the more hydrophobic the sample, the later the peak.

### General Step F

### Plasma Stability Study

Take a certain amount of ADC sample and add it to human plasma from which IgG has been removed. Repeat three tubes for each ADC and incubate in a 37°C water bath. After incubation for 0 days, 3 days, 7 days and 21 days, take out the ADC sample, add ProteinA (MabSelectSuReTMLX Lot: #10257475GE) to each tube, take 100µl of PBS washed and shake it in a vertical mixer for 2h adsorption, and after washing and elution steps, obtain the incubated ADC. Perform RP-HPLC detection on the ADC samples incubated for a specific time. Determine the plasma stability of the sample.

### Example 1 Synthesis of Compound 1

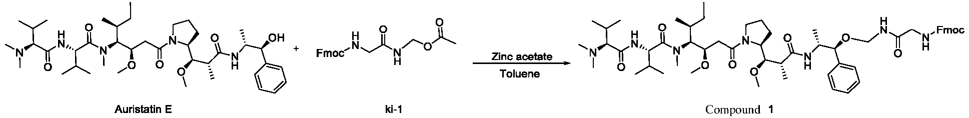

Auristatin E (826 mg, 1.128 mmol, 1.0 eq), ki-1 (831.4 mg, 2.256 mmol, 2.0 eq, the method of synthesis refers to the synthesis of compound 1 in CN111686259A), zinc acetate (414.2 mg, 5.64 mmol, 2.0 eq) and toluene (15 mL) were added to a 50 mL single-neck round-bottom flask in sequence, and nitrogen was replaced 3 times. The reaction was refluxed at 115 °C for 4 h, and the reaction was stopped. The mixture was cooled to room temperature, filtered, and the filtrate was concentrated under reduced pressure. The mixture was purified by reverse phase preparative column and freeze-dried to obtain a white solid (605 mg, 51.55%). LC-MS m/z (ES+): [M+H]⁺: 1041.3.

### Example 2 Synthesis of Compound 3

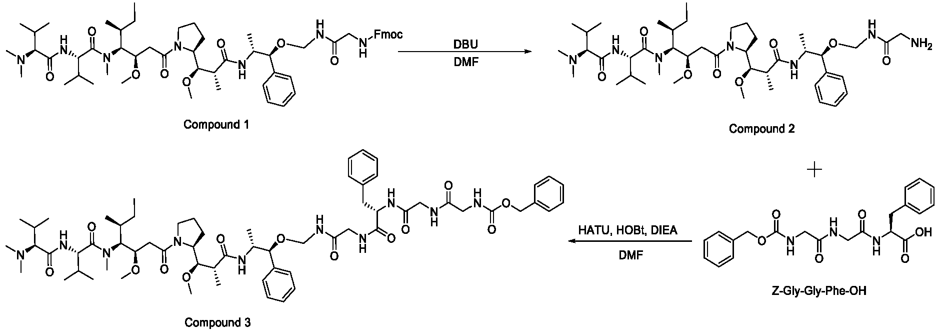

Z-Gly-Gly-Phe-OH (240 mg, 0.581 mmol, 1.0 eq), HATU (264 mg, 0.697 mmol, 1.2 eq), HOBt (94 mg, 0.697 mmol, 1.2 eq), and DMF (2 mL) were added to a 10 mL EP tube in sequence and stirred at room temperature for later use.

Compound 1 (605 mg, 0.581 mmol, 1.0 eq) and DMF (8 mL) were added to another 25 mL single-neck round-bottom flask in sequence. After stirring at room temperature for dissolution, DBU (95.6 µL, 0.64 mmol, 1.1 eq) was added. The reaction was carried out at room temperature for 0.5 h. The raw material disappeared under TLC monitoring to produce compound 2. The above mixture was then added to this flask, and DIEA (96 µL, 0.581 mmol, 1.0 eq) was added. The reaction was carried out at room temperature for 1 h and monitored by HPLC. After the reaction, the product was purified by reverse phase preparative column to obtain a white solid product (487 mg, 69.08%). LC-MS m/z (ES+): [M+H]⁺: 1213.6.

### Example 3 Synthesis of Compound 5

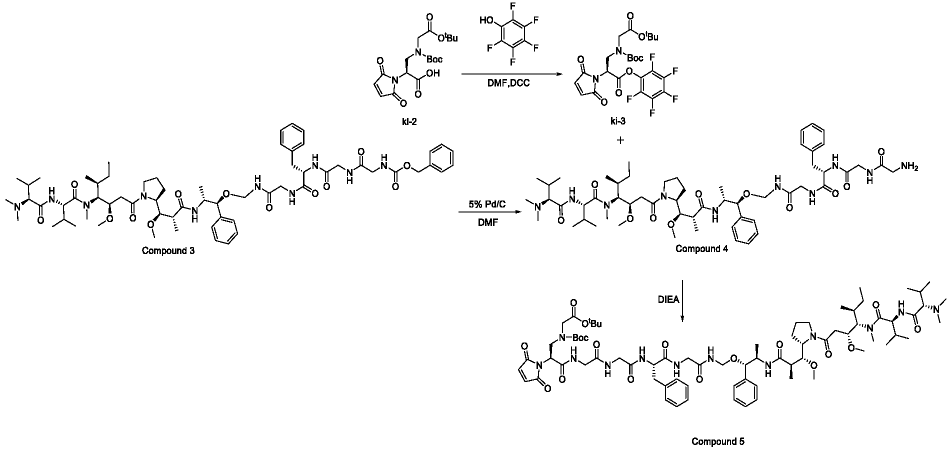

Compound 3 (487 mg, 0.402 mmol, 1.0 eq), 5% Pd/C (48.7 mg) and DMF (5 mL) were added to a 25 mL single-neck round-bottom flask in sequence. After hydrogen replacement 3 times, the mixture was reacted at room temperature for 1 h. The reaction was monitored by HPLC. The raw material disappeared and a new peak was generated, which was compound 4, recorded as reaction solution ①.

In another 25 mL single-neck round-bottom flask, ki-2 (176 mg, 0.442 mmol, 1.1 eq (synthesis method refers to CN108452321A), Pentafluorophenol (81 mg, 0.442 mmol, 1.1 eq), DCC (91 mg, 0.442 mmol, 1.1 eq) and DMF (3 mL) were added. The mixture was reacted at room temperature for 30 min. The reaction was monitored by TLC. The reaction was completed to obtain ki-3, recorded as reaction solution ②.

Then filter the reaction solution ① into a new 25 mL single-neck round-bottom flask, add DIEA (73 µL, 0.442 mmol, 1.1 eq) under ice-water bath, and heat the reaction solution of ② to room temperature for 1 hour after addition. Monitor the reaction by HPLC. Filter the reaction solution, add the filtrate to the reaction solution containing compound 4 under ice-bath stirring, then add, remove the ice bath, react at room temperature for 1 hour, and monitor by HPLC. The reaction solution was directly purified by reverse phase preparation, and the preparation solution was lyophilized to obtain a white solid product (320 mg, 54.53%). LC-MS m/z (ES+): [M/2+H]+: 730.4.

### Example 4 Synthesis of compound LP-1a

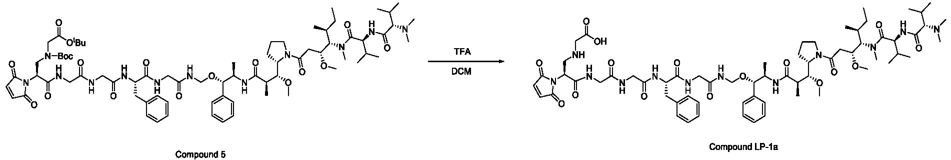

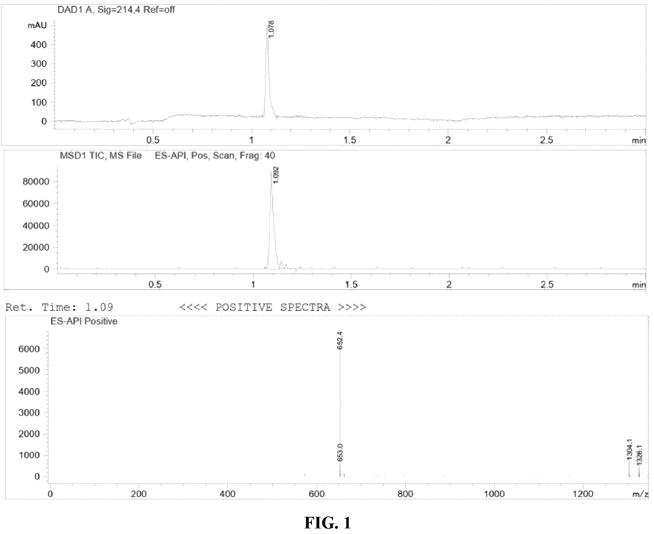

Compound 5 (100 mg, 0.0685 mmol, 1.0 eq) was dissolved in 10 mL dry dichloromethane and 4 mL TFA, and reacted at room temperature for 3 h, monitored by HPLC. After the reaction, the solvent was removed by concentration under reduced pressure, purified by reverse phase preparative column, and the preparative solution was lyophilized to obtain a white solid product (21 mg, 29.78%). LC-MS m/z (ES+): [M/2+H]+: 652.4. The LC-MS spectrum is shown in Figure 1.

### Example 5 Synthesis of Compound 6

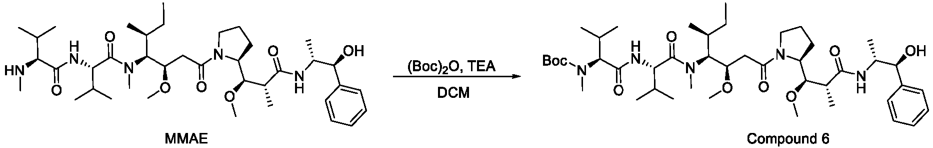

MMAE (2.0 g, 2.79 mmol, 1.0eq), (Boc)2O (1.21g, 5.57 mmol, 2.0eq) and DCM (20 mL) were added to a 100 mL single-neck round-bottom flask, and TEA (563 mg, 5.57 mmol, 2.0eq) was added under an ice-water bath. After the addition, the temperature was raised to room temperature and reacted for 72 h. The raw material MMAE was monitored by TLC and the reaction was complete. Post-treatment: After the reaction solution was concentrated under reduced pressure, it was purified by column chromatography (eluent: DCM/MeOH=20/1) to obtain a white solid (2.28g, 100%). LC-MSm/z (ES+): [M+H]⁺: 818.4.

### Example 6 Synthesis of Compound 7

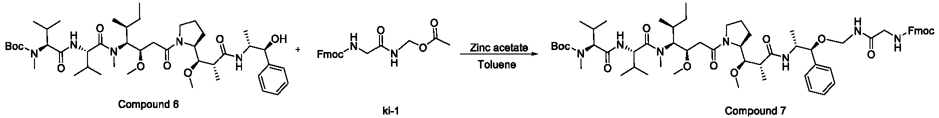

Compound 6 (2.28 g, 2.79 mmol, 1.0eq), ki-1 (2.05 g, 5.57 mmol, 2.0eq), zinc acetate (1.02g, 5.57 mmol, 2.0eq), and toluene (30 mL) were added to a 100 mL single-neck round-bottom flask in sequence. After N₂ replacement 3 times, the temperature was raised to 115°C under N₂ protection for 4 hours, the reaction was stopped, cooled to room temperature, filtered, the filtrate was concentrated under reduced pressure, purified by reverse phase preparative column, and the preparative solution was lyophilized to obtain a white solid product (1.071g, 34%). LC-MSm/z (ES+): [M+H]⁺: 1126.4.

### Example 7 Synthesis of Compound 9

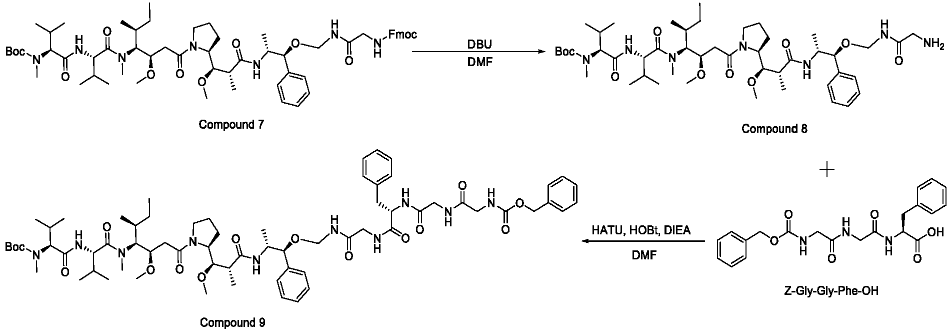

Compound 7 (900 mg, 0.8 mmol, 1.0 eq) and DMF (9 mL) were added to a 50 mL single-necked round-bottom flask. After stirring and dissolving, DBU (134 mg, 0.88 mmol, 1.1 eq) was added dropwise under an ice-water bath. After addition, the temperature was raised to room temperature for reaction for 30 min. The reaction was completed by TLC monitoring and recorded as reaction solution ①;

In another 50 mL single-necked round-bottom flask, Z-Gly-Gly-Phe-OH (364 mg, 0.88 mmol, 1.1 eq), HATU (365 mg, 0.96 mmol, 1.2 eq), HOBt (129.7 mg, 0.96 mmol, 1.2 eq) and DMF (7 mL) were added dropwise under an ice-water bath. After dissolving, reaction solution ① and DIEA (103.4 mg, 0.8 mmol, 1.0 eq) were added dropwise in turn under an ice-water bath. After addition, the temperature was raised to room temperature for reaction for 1 h. The reaction was monitored by HPLC. The reaction solution was purified by reverse phase preparation, and the prepared solution was lyophilized to obtain a white solid (960 mg, 92.4 %). LC-MS m/z (ES+): [M+H]⁺: 1299.6.

### Example 8 Synthesis of Compound 11

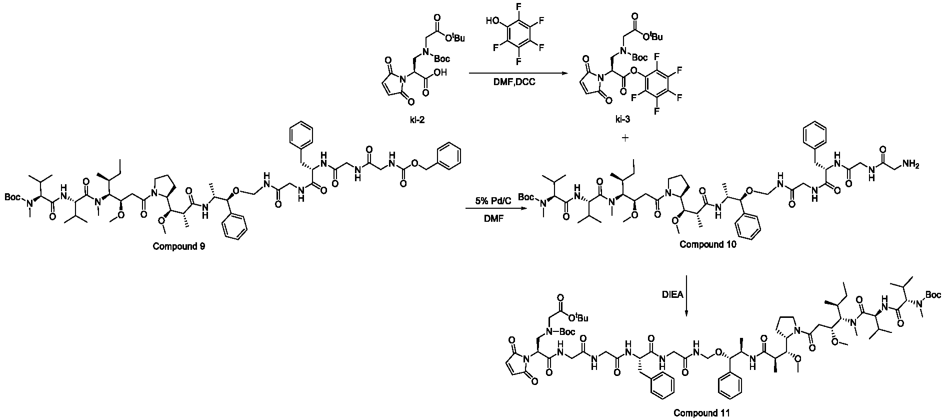

Compound 10 (960 mg, 0.74 mmol, 1.0 eq), 5% Pd/C (960 mg) and DMF (10 mL) were added to a 50 mL single-neck round-bottom flask. After H₂ replacement 3 times, the reaction was carried out at room temperature for 1h. The reaction was monitored by HPLC and recorded as reaction solution ①;

In another 25 mL single-neck round-bottom flask, ki-2 (322.7mg, 0.81 mmol, 1.1 eq), DCC (167 mg, 0.81 mmol, 1.1 eq) and DMF (5 mL) were added to dissolve, and pentafluorophenol (149 mg, 0.81 mmol, 1.1 eq) was added under ice-water bath. After the addition was completed, the temperature was raised to room temperature and reacted for 30min. The reaction was monitored by TLC. After the reaction was completed, ki-3 was obtained, which was recorded as reaction solution ②.

The reaction solution ① was filtered into a 50 mL single-neck round-bottom flask, and DIEA (105 mg, 0.81 mmol, 1.1 eq) was added under an ice-water bath. The reaction solution ② was filtrated and heated to room temperature for 1 h. The reaction was monitored by HPLC. The reaction solution was purified by reverse phase preparation, and the prepared solution was lyophilized to obtain a white solid (895 mg, 78.5%). LC-MSm/z (ES+): [M/2+H]+: 773.4.

### Example 9 Synthesis of compound LP-2a

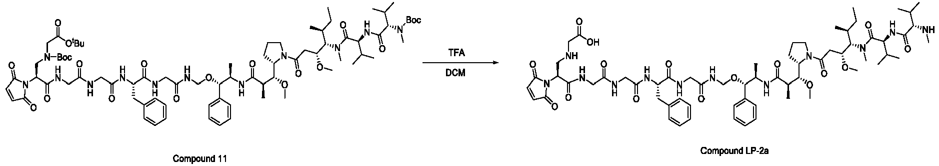

Compound 12 (400 mg, 0.259 mmol, 1.0 eq) was dissolved in 20 mL dry dichloromethane and 8 mL TFA, and reacted at room temperature for 3 h, monitored by HPLC. After the reaction, the solvent was removed by concentration under reduced pressure, and the crude product was purified by reverse phase preparative column, and the preparative solution was lyophilized to obtain a white solid (248 mg, 74%), LC-MS m/z (ES+): [M/2+H]+: 645.3.

### Example 10 Synthesis of VcMMAE

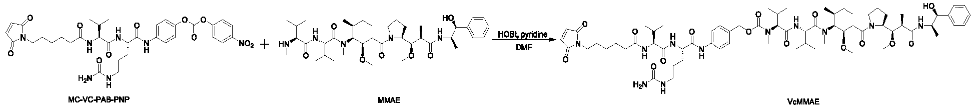

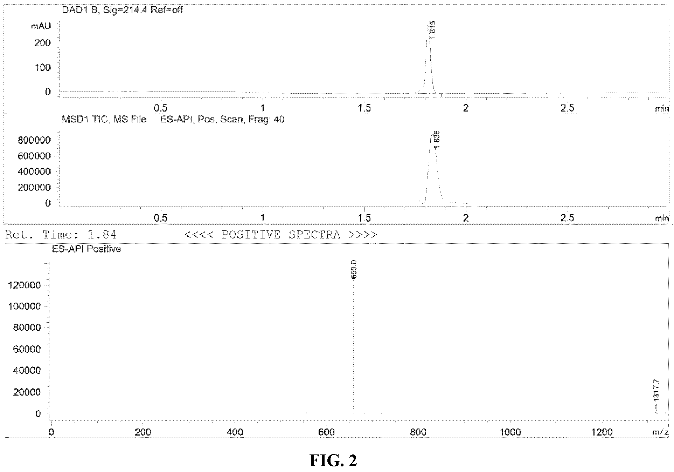

In a 25 mL round-bottom flask, MMAE (120 mg, 0.167 mmol, 1.0 eq) and MC-VC-PAB-PNP (186 mg, 0.25 mmol, 1.5 eq) were added, dissolved in DMF (5 mL), and HOBt (27.1 mg, 0.20 mmol, 1.2 eq) and pyridine (1 mL) were added in sequence, stirred overnight at room temperature, and monitored by HPLC. After the reaction, the crude product was purified by reverse phase preparative column, and the preparative solution was lyophilized to obtain a white solid (158.3 mg, 72%), LC-MSm/z (ES+): [M/2+H]+: 659.0. The LC-MS spectrum is shown in Figure 2.

### Example 11 Synthesis of Compound 13

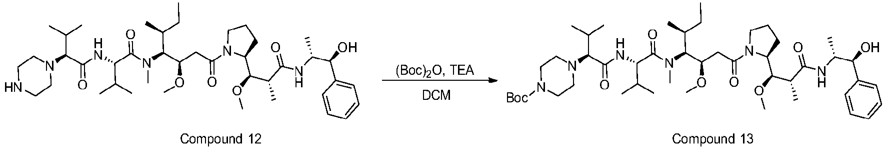

Referring to the synthesis method of Example 5, compound 13 was synthesized using compound 12 (refer to the synthesis of compound 17 in CN106279352 as the raw material. LC-MSm/z (ES+): [M+H]⁺: 873.6

### Example 12 Synthesis of Compound 14

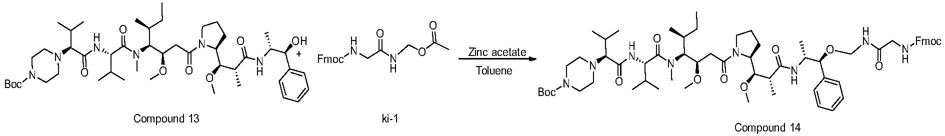

Referring to the synthesis method of Example 6, compound 14 was synthesized using compound 13 and ki-1 as raw materials. LC-MSm/z (ES+): [M+H]⁺: 1180.7.

### Example 13 Synthesis of Compound 16

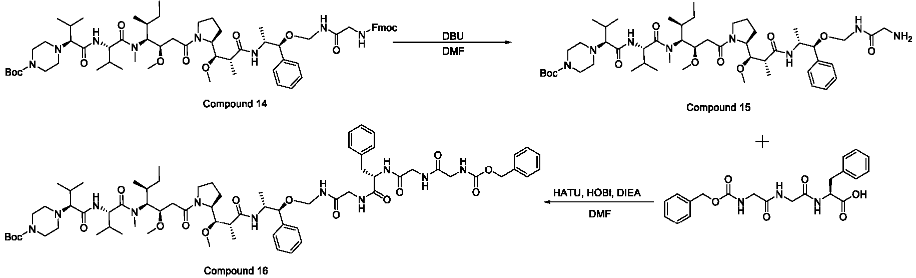

Referring to the synthesis method of Example 7, compound 16 was synthesized using compound 14 as the raw material. LC-MSm/z (ES+): [M+H]⁺: 1353.9.

### Example 14 Synthesis of Compound 18

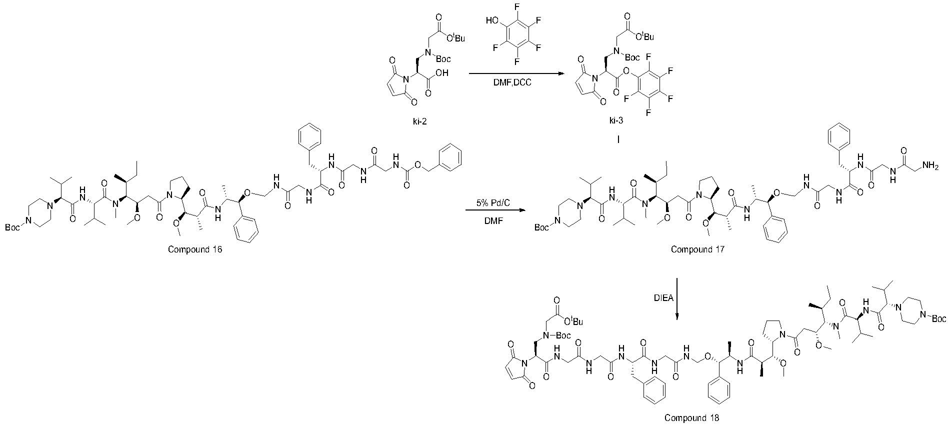

Referring to the synthesis method of Example 8, compound 18 was synthesized using compound 16 as the raw material. LC-MSm/z (ES+): [M+H]⁺: 1600.9.

### Example 15 Synthesis of Compound LP-3a

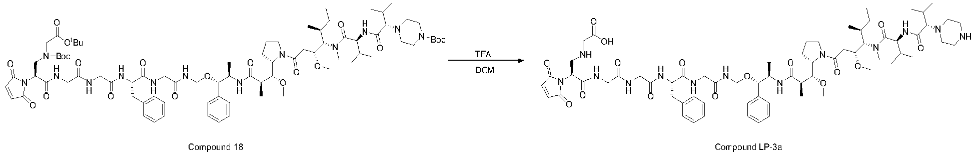

Referring to the synthesis method of Example 9, compound LP-3a was synthesized using compound 18 as the raw material. LC-MS m/z (ES+): [M+H]⁺: 1344.7.

### Example 16 Synthesis of compound 23

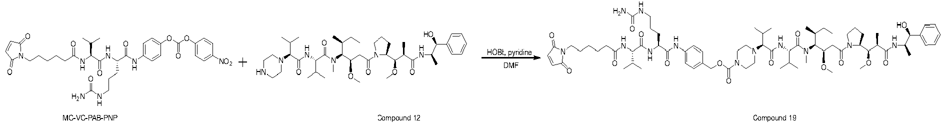

Referring to the synthesis method of Example 10, compound 19 was synthesized using compound 12 and MC-VC-PAB-PNP as raw materials. LC-MS m/z (ES+): [M+H]⁺: 1371.8.

### Example 17 Synthesis of compound 21

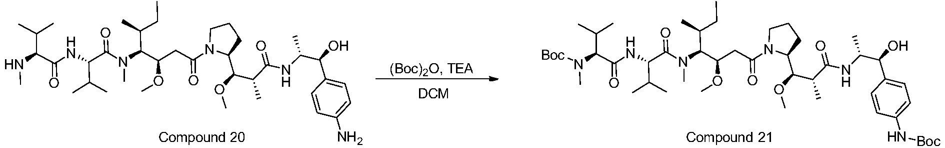

Referring to the synthesis method of Example 5, compound 21 was synthesized using compound 20 (synthesized according to US2017014524A1) as a raw material. LC-MS m/z (ES+): [M+H]⁺: 933.6.

### Example 18 Synthesis of compound 22

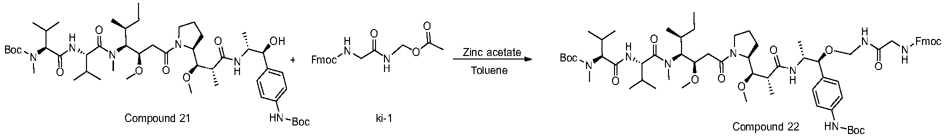

Referring to the synthesis method of Example 6, compound 22 was synthesized using compound 21 and ki-1 as raw materials. LC-MS m/z (ES+): [M+H]⁺: 1241.7.

### Example 19 Synthesis of compound 24

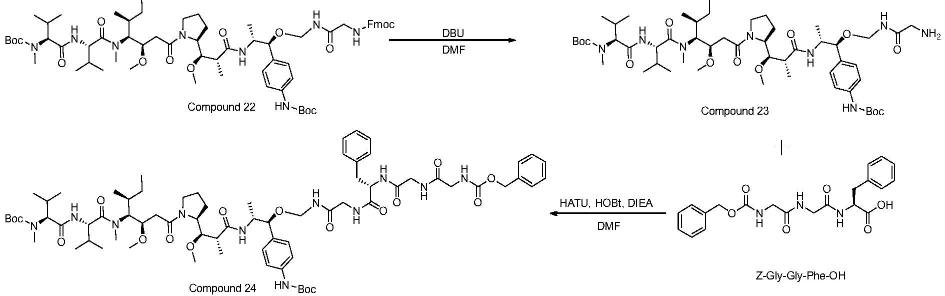

Referring to the synthesis method of Example 7, compound 24 was synthesized using compound 22 as the raw material. LC-MS m/z (ES+): [M+H]⁺: 1414.8.

### Example 20 Synthesis of Compound 26

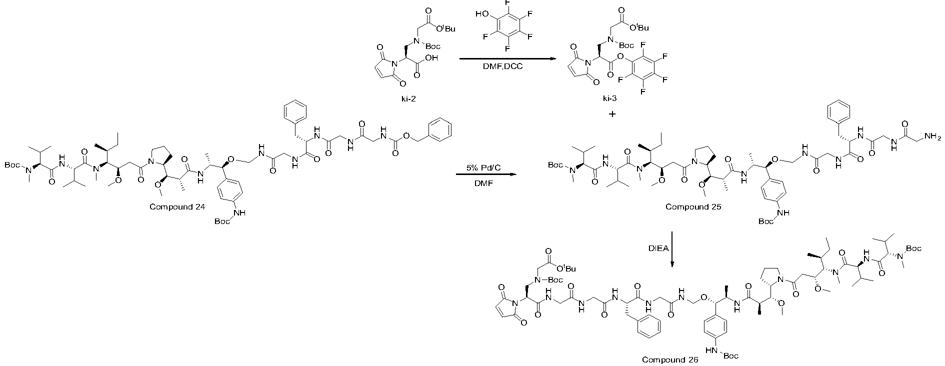

Compound 26 was synthesized from compound 24 with reference to the synthetic method of Example 8. LC-MS m/z (ES+): [M+H]⁺: 1660.9.

### Example 21 Synthesis of Compound 27 (LP-3sa)

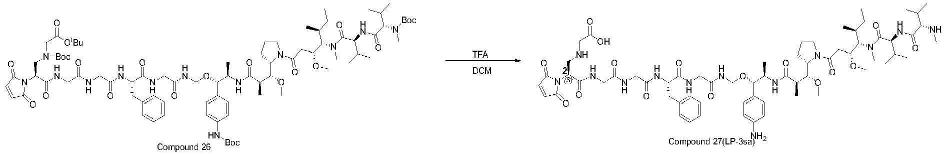

Compound 27 (LP-3sa, whose structure is shown in the structural formula of LP-3s, where the configuration of the chiral carbon at the position-2 is of the S-type) was synthesized from compound 26 with reference to the synthetic method of Example 9. LC-MS m/z (ES⁺):[M+H]⁺ : 1304.7.

### Example 22 Synthesis of compound 29

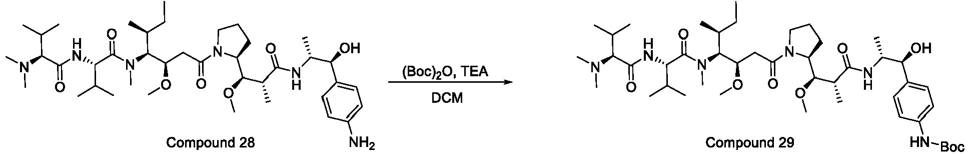

Compound 29 was synthesized from compound 28 (synthesized with reference to patent US2017014524A1) with reference to the synthesis method of Example 5. LC-MSm/z (ES⁺):[M+H]⁺ :847.6.

### Example 23 Synthesis of compound 30

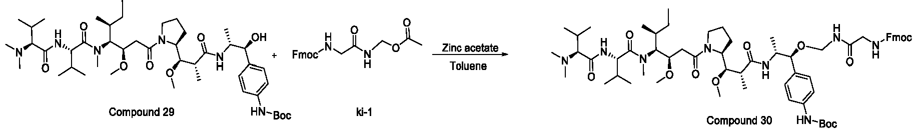

Referring to the synthesis method of Example 6, compound 30 was synthesized using compound 29 and ki-1 as raw materials. LC-MSm/z (ES+): [M+H]⁺: 1155.7.

### Example 24 Synthesis of compound 32

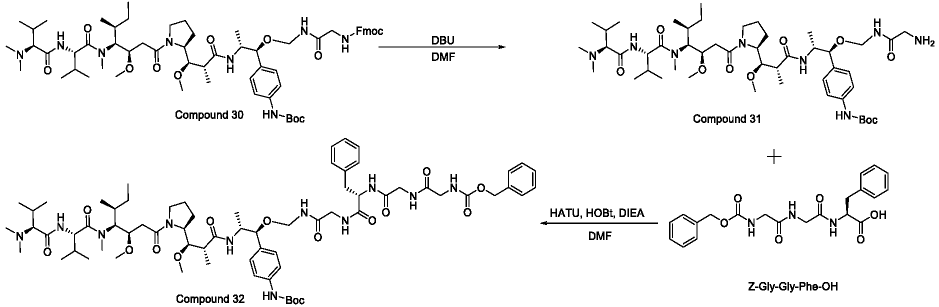

Referring to the synthesis method of Example 7, compound 32 was synthesized using compound 30 as the raw material. LC-MS m/z (ES+): [M+H]⁺: 1328.8.

### Example 25 Synthesis of compound 34

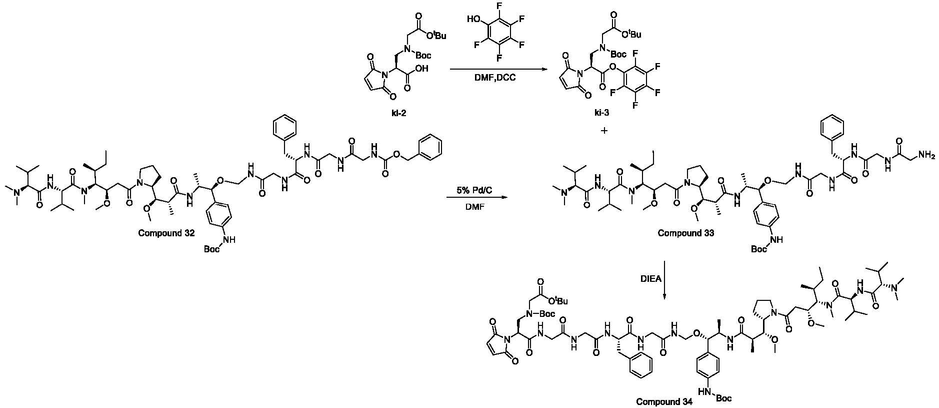

Referring to the synthesis method of Example 8, compound 34 was synthesized using compound 32 as the raw material. LC-MSm/z (ES+): [M+H]⁺: 1574.9.

### Example 26 Synthesis of compound LP-4a

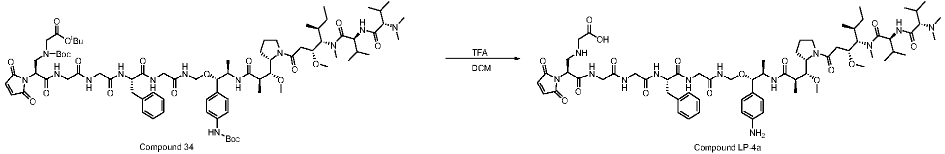

Referring to the synthesis method of Example 9, compound LP-4a was synthesized using compound 34 as the raw material. LC-MS m/z (ES+): [M+H]⁺: 1318.7.

### Example 27 Synthesis of compound 36

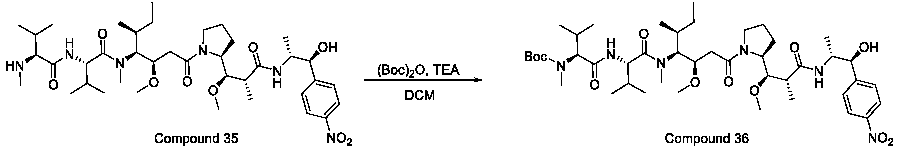

Referring to the synthesis method of Example 5, compound 36 was synthesized using compound 35 (synthesized according to US20210346523A1) as the raw material. LC-MSm/z (ES+): [M+H]⁺: 863.5.

### Example 28 Synthesis of compound 37

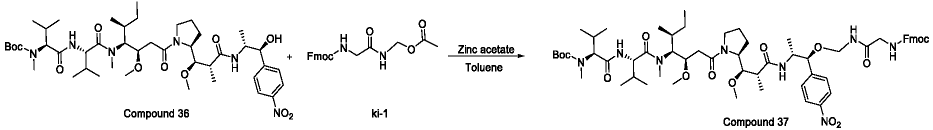

Referring to the synthesis method of Example 6, compound 37 was synthesized using compound 36 and ki-1 as raw materials. LC-MS m/z (ES+): [M+H]⁺: 1171.7.

### Example 29 Synthesis of compound 39

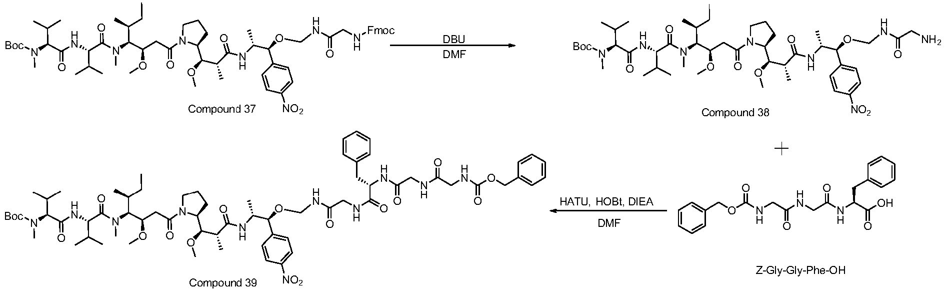

Referring to the synthesis method of Example 7, compound 39 was synthesized using compound 37 as raw material. LC-MS m/z (ES+): [M+H]⁺: 1344.7.

### Example 30 Synthesis of compound 41

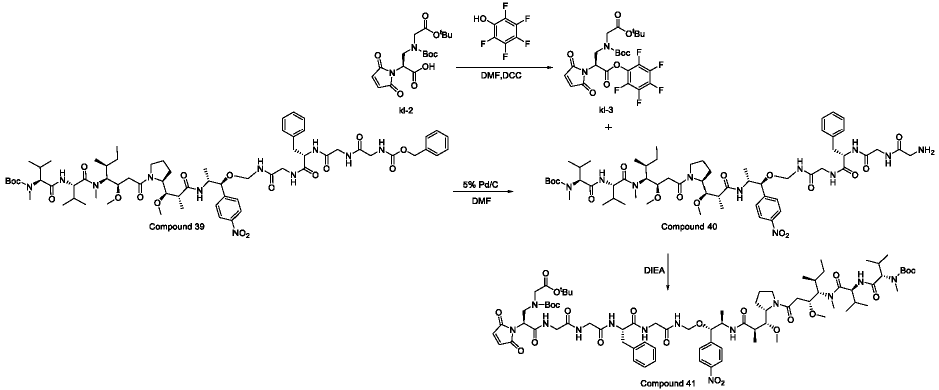

Referring to the synthesis method of Example 8, compound 41 was synthesized using compound 39 as the raw material. LC-MSm/z (ES+): [M+H]⁺: 1590.9.

### Example 31 Synthesis of compound LP-5a

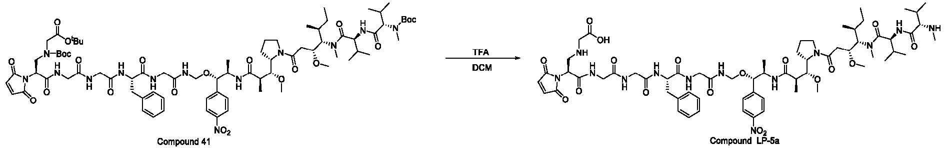

Referring to the synthesis method of Example 9, compound LP-5a was synthesized using compound 41 as the raw material. LC-MS m/z (ES+): [M+H]⁺: 1334.7.

### Example 32 Synthesis of compound 43

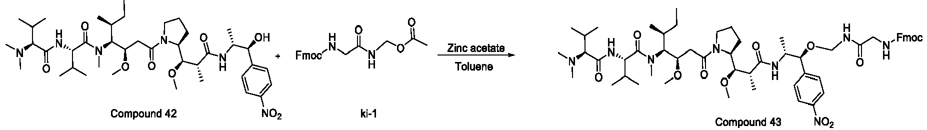

Referring to the synthesis method of Example 6, compound 43 was synthesized using compound 42 (refer to the synthesis of compound 17 in US20210346523A1) and ki-1 as raw materials. LC-MSm/z (ES+): [M+H]⁺: 1085.6.

### Example 33 Synthesis of compound 45

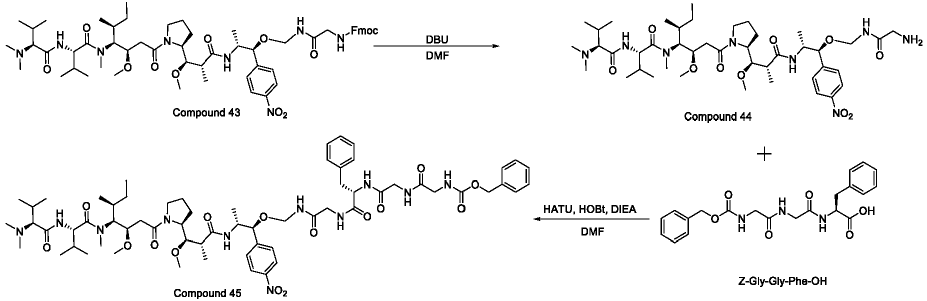

Referring to the synthesis method of Example 7, Compound 45 was synthesized using Compound 43 as the raw material. LC-MS m/z (ES+): [M+H]⁺: 1258.7.

### Example 34 Synthesis of Compound 47

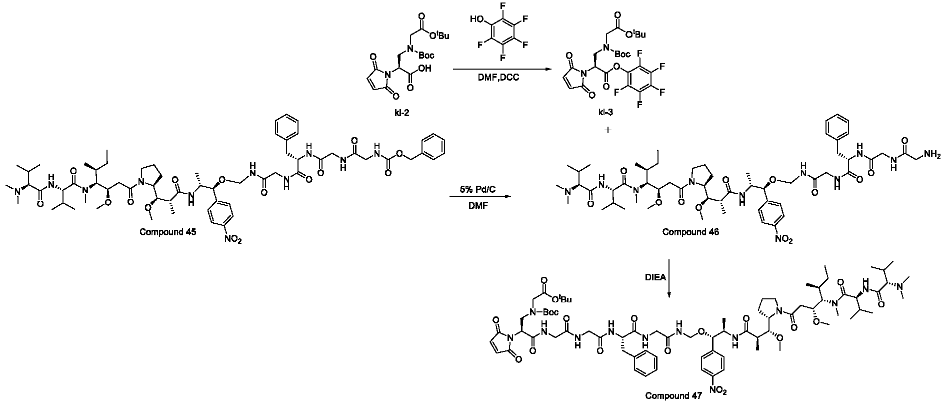

Referring to the synthesis method of Example 8, compound 47 was synthesized using compound 45 as a raw material. LC-MSm/z (ES+): [M+H]⁺: 1504.8.

### Example 35 Synthesis of compound LP-6a

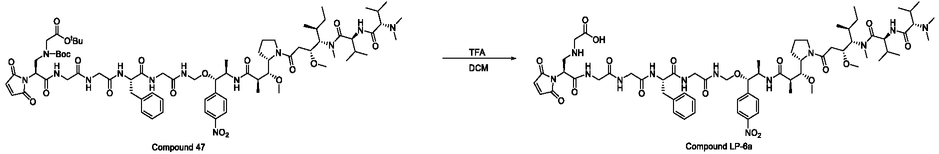

Referring to the synthesis method of Example 9, compound LP-6a was synthesized using compound 47 as the raw material. LC-MS m/z (ES+): [M+H]⁺: 1348.7.

### Example 36 Synthesis of compound 49

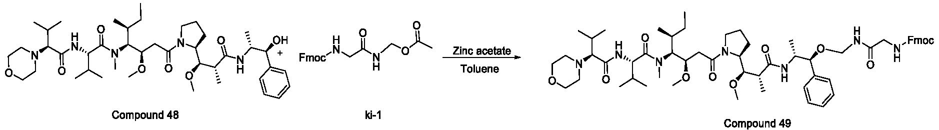

Referring to the synthesis method of Example 6, compound 49 was synthesized using compound 48 (refer to the synthesis of compound 6 in CN106279352) and ki-1 as raw materials. LC-MSm/z (ES+): [M+H]⁺: 1082.6.

### Example 37 Synthesis of compound 51

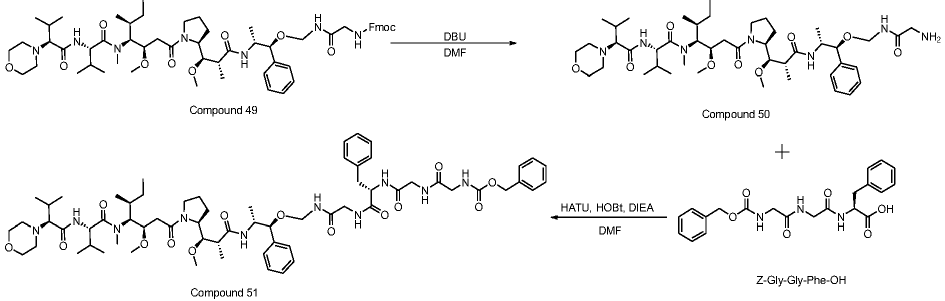

Referring to the synthesis method of Example 7, compound 51 was synthesized using compound 49 as the raw material. LC-MS m/z (ES+): [M+H]⁺: 1255.7.

### Example 38 Synthesis of compound 53

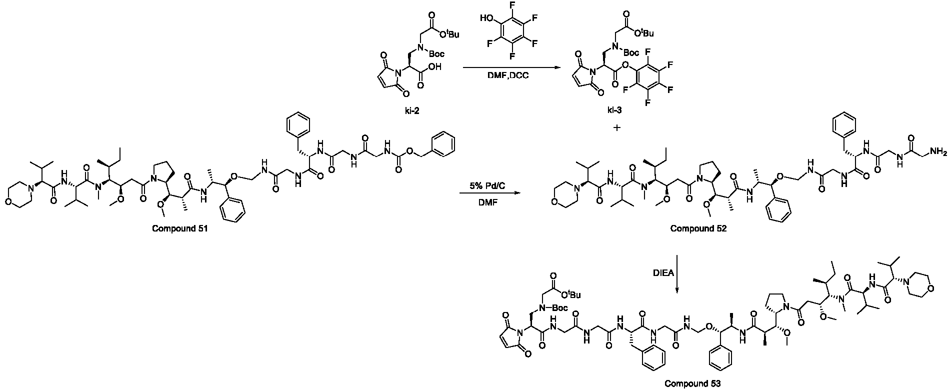

Referring to the synthesis method of Example 8, Compound 53 was synthesized using Compound 51 as the raw material. LC-MSm/z (ES+): [M+H]⁺: 1501.8.

### Example 39 Synthesis of Compound LP-7a

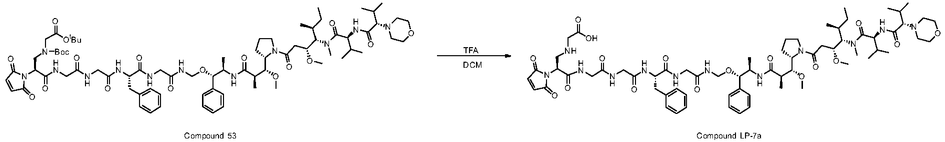

Referring to the synthesis method of Example 9, compound LP-7a was synthesized using compound 53 as the raw material. LC-MS m/z (ES+): [M+H]⁺: 1345.7.

### Example 40 Synthesis of compound 55

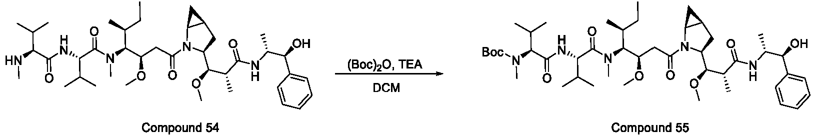

Referring to the synthesis method of Example 5, compound 55 was synthesized using compound 54 (refer to the synthesis of compound 1 in CN113121639A) as the raw material. LC-MSm/z (ES+): [M+H]⁺: 830.6.

### Example 41 Synthesis of compound 56

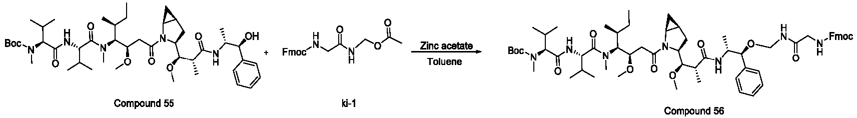

Referring to the synthesis method of Example 6, compound 56 was synthesized using compound 55 and ki-1 as raw materials. LC-MS m/z (ES+): [M+H]⁺: 1138.7.

### Example 42 Synthesis of compound 58

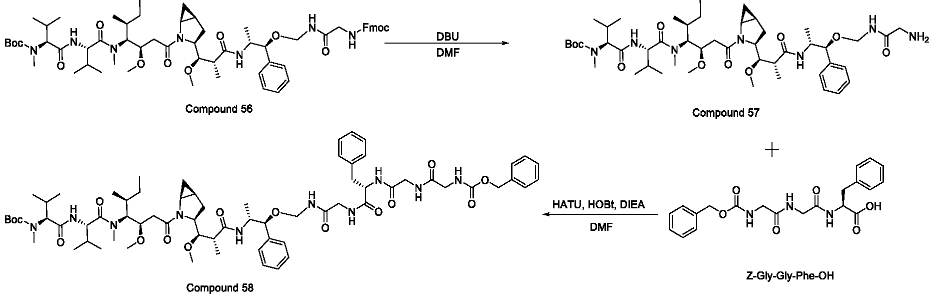

Referring to the synthesis method of Example 7, Compound 58 was synthesized using Compound 56 as the raw material. LC-MS m/z (ES+): [M+H]⁺: 1311.8.

### Example 43 Synthesis of Compound 60

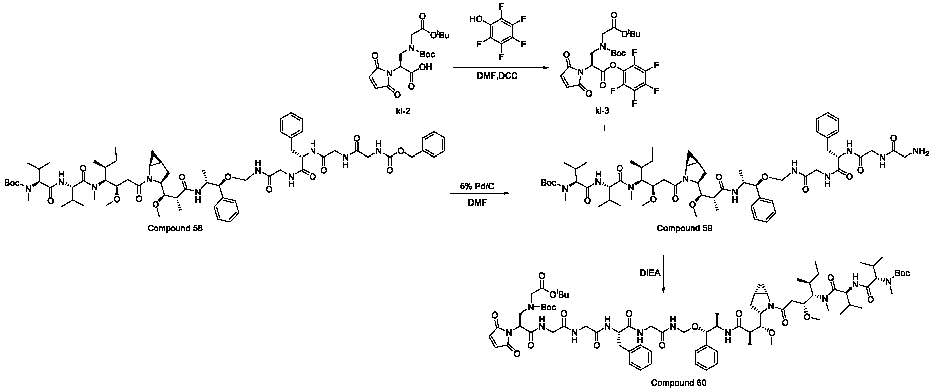

Referring to the synthesis method of Example 8, compound 60 was synthesized using compound 58 as the raw material. LC-MSm/z (ES+): [M+H]⁺: 1557.9.

### Example 44 Synthesis of Compound LP-8a

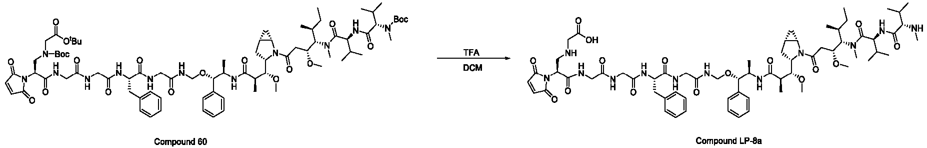

Referring to the synthesis method of Example 9, compound LP-8a was synthesized using compound 60 as the raw material. LC-MS m/z (ES+): [M+H]⁺: 1301.7.

### Example 45 Synthesis of Compound 62

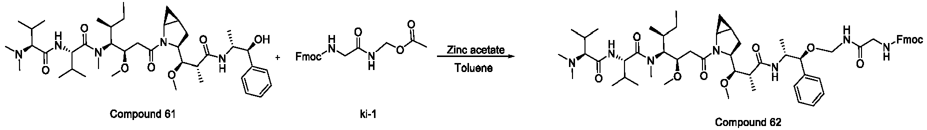

Referring to the synthesis method of Example 6, compound 62 was synthesized using compound 61 (refer to the synthesis of compound 1 in CN113121639A) and ki-1 as raw materials. LC-MSm/z (ES+): [M+H]⁺: 1052.6.

### Example 46 Synthesis of Compound 63

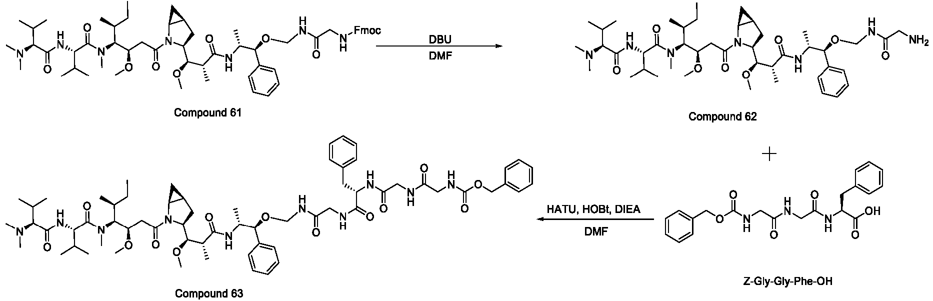

Referring to the synthesis method of Example 7, compound 63 was synthesized using compound 61 as the raw material. LC-MS m/z (ES+): [M+H]⁺: 1225.7.

### Example 47 Synthesis of Compound 65

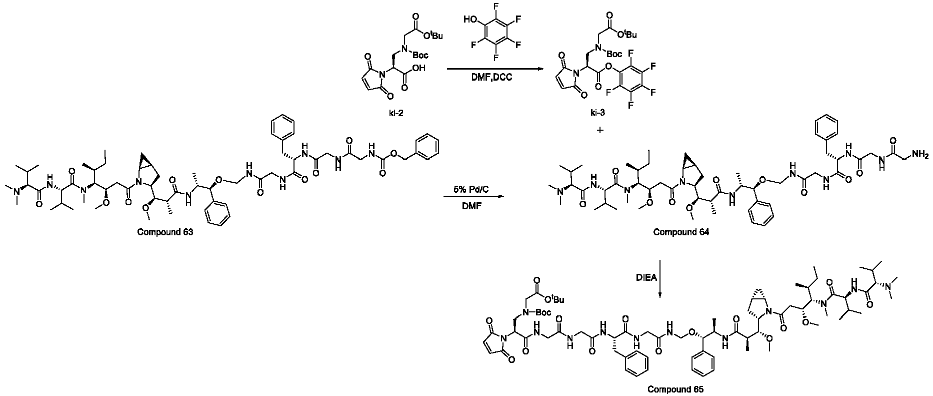

Referring to the synthesis method of Example 8, compound 65 was synthesized using compound 63 as the raw material. LC-MSm/z (ES+): [M+H]⁺: 1471.8.

### Example 48 Synthesis of Compound LP-9a

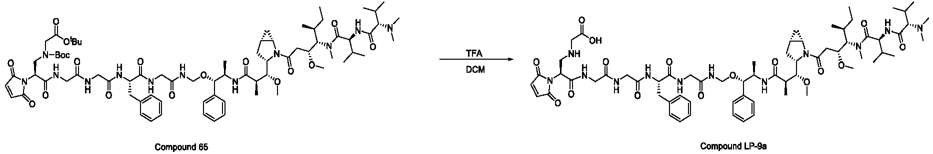

Referring to the synthesis method of Example 9, compound LP-9a was synthesized using compound 65 as the raw material. LC-MS m/z (ES+): [M+H]⁺: 1315.7.

### Example 49 Synthesis of Compound 66

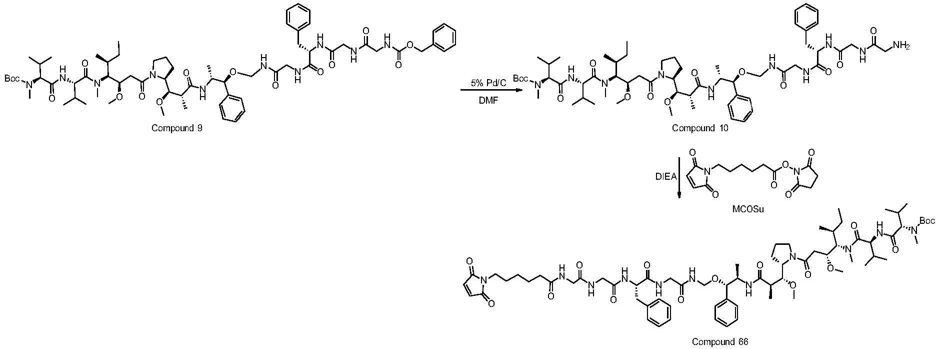

Compound 9 (960 mg, 0.74 mmol, 1.0 eq), 5% Pd/C (960 mg) and DMF (10 mL) were added to a 50 mL round-bottom flask. After H₂ replacement 3 times, the reaction was carried out at room temperature for 1 h. The reaction was monitored by HPLC. The reaction was completed and recorded as reaction solution ①. The reaction solution ① was filtered into a 50 mL single-neck round-bottom flask. MCOSU (274 mg, 0.89 mmol, 1.2 eq) and DIEA (105 mg, 0.81 mmol, 1.1 eq) were added in turn under an ice-water bath. After the addition, the temperature was raised to room temperature for 1 h. The reaction was monitored by HPLC. The reaction solution was purified by reverse phase preparation, and the prepared solution was lyophilized to obtain a white solid (847.5 mg, 84.3%). LC-MSm/z (ES+): [M+H]⁺: 1358.2.

### Example 50 Synthesis of Compound LP-10

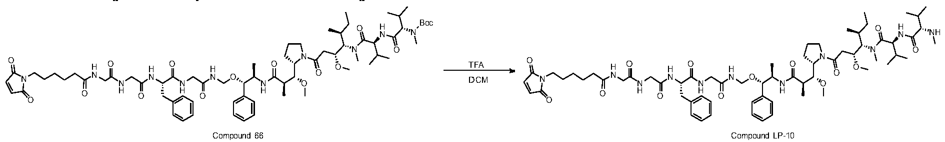

Referring to the synthesis method of Example 9, compound LP-10 was synthesized using compound 66 as the raw material. LC-MS m/z (ES+): [M/2+H]+: 630.0.

### Example 51 Synthesis of Compound LP-11

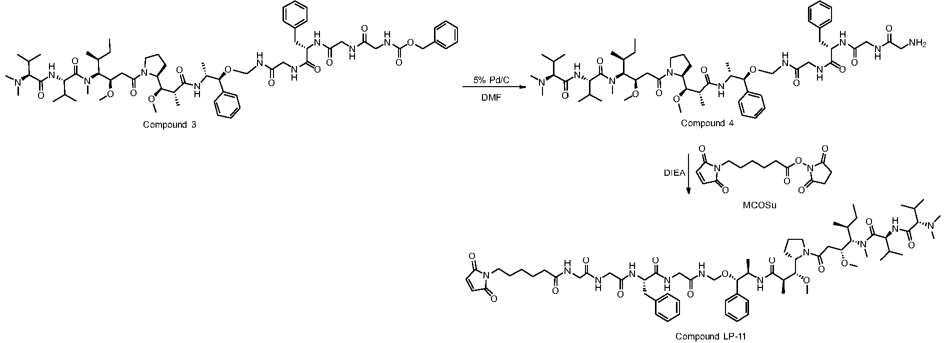

Referring to the synthesis method of Example 49, compound LP-11 was synthesized using compound 3 as the raw material. LC-MS m/z (ES+): [M+H]⁺: 1272.8.

### Example 52 Synthesis of Compound 67

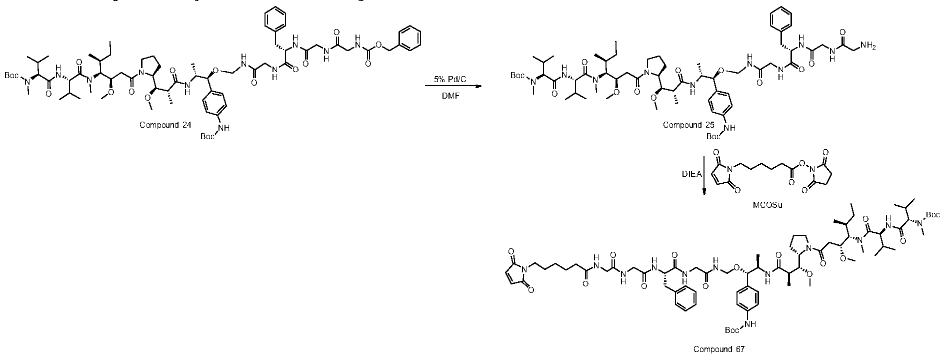

Referring to the synthesis method of Example 49, compound 67 was synthesized using compound 28 as the raw material. LC-MS m/z (ES+): [M+H]⁺: 1473.9.

### Example 53 Synthesis of Compound LP-12

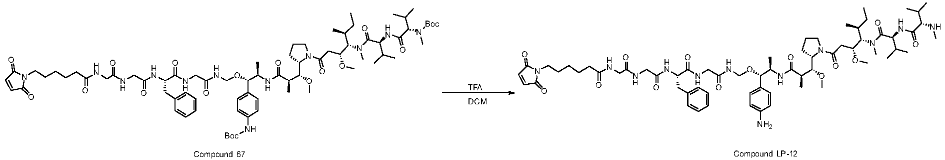

Referring to the synthesis method of Example 9, compound LP-12 was synthesized using compound 67 as the raw material. LC-MS m/z (ES+): [M+H]⁺: 1273.8.

### Example 54 Synthesis of Compound 68

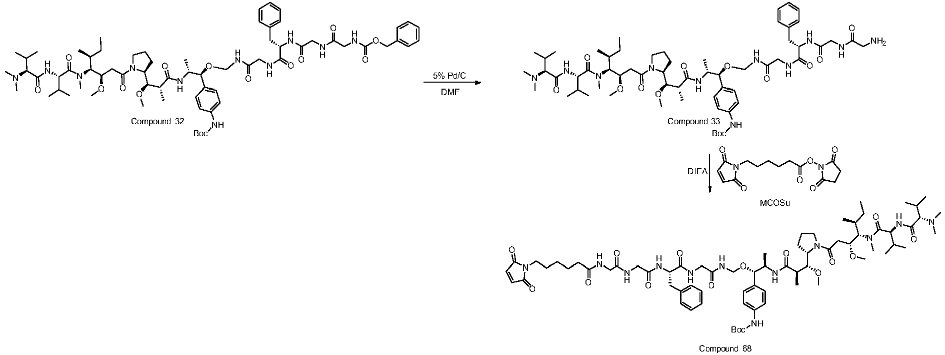

Referring to the synthesis method of Example 49, compound 68 was synthesized using compound 36 as raw material. LC-MS m/z (ES+): [M+H]⁺: 1387.8.

### Example 55 Synthesis of Compound LP-13

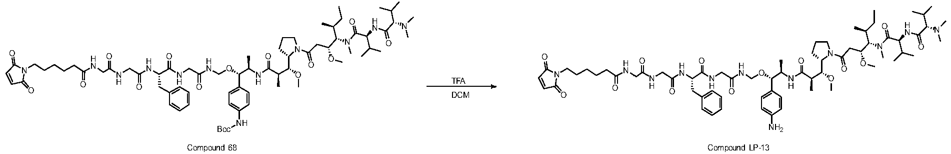

Referring to the synthesis method of Example 9, compound LP-13 was synthesized using compound 68 as the raw material. LC-MS m/z (ES+): [M+H]⁺: 1287.8.

### Example 56 Synthesis of Compound 69

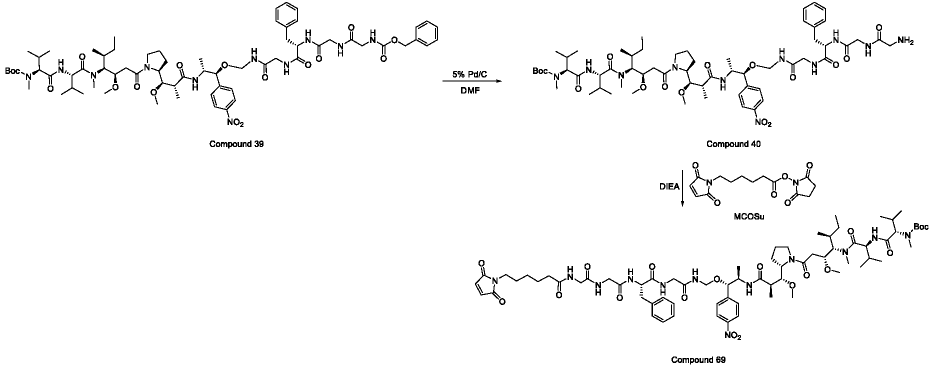

Referring to the synthesis method of Example 49, compound 69 was synthesized using compound 39 as the raw material. LC-MS m/z (ES+): [M+H]⁺: 1403.8.

### Example 57 Synthesis of Compound LP-14

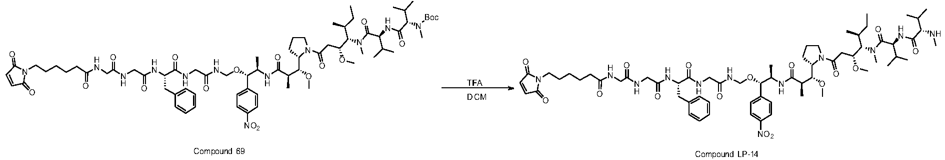

Referring to the synthesis method of Example 9, compound LP-14 was synthesized using compound 69 as the raw material. LC-MS m/z (ES+): [M+H]⁺: 1303.7.

### Example 58 Synthesis of Compound LP-15

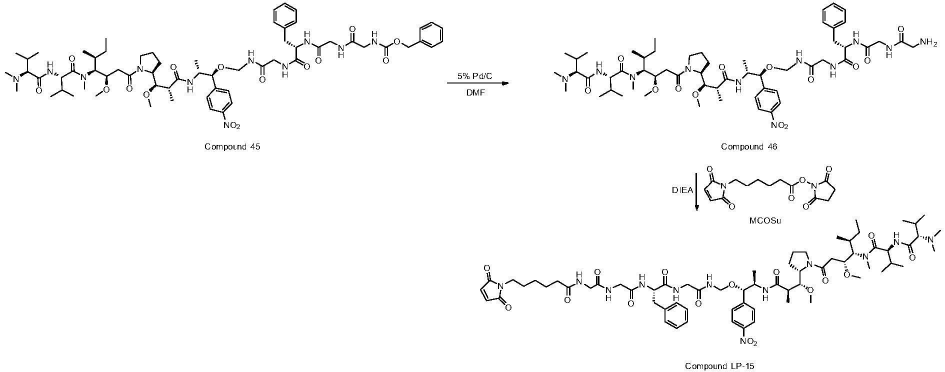

Referring to the synthesis method of Example 49, compound LP-15 was synthesized using compound 45 as the raw material. LC-MSm/z (ES+): [M+H]⁺: 1403.8.

### Example 59 Synthesis of Compound LP-16

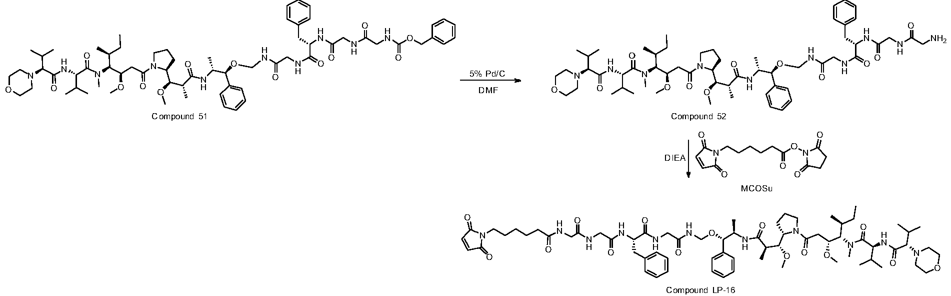

Referring to the synthesis method of Example 49, compound LP-16 was synthesized using compound 51 as the raw material. LC-MS m/z (ES+): [M+H]⁺: 1314.8.

### Example 60 Synthesis of Compound 70

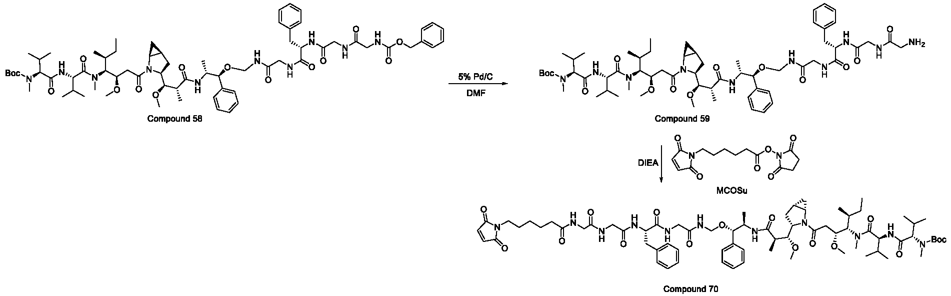

Referring to the synthesis method of Example 49, compound 58 was used as the raw material to synthesize compound 70. LC-MSm/z (ES+): [M+H]⁺: 1370.8.

### Example 61 Synthesis of Compound LP-17

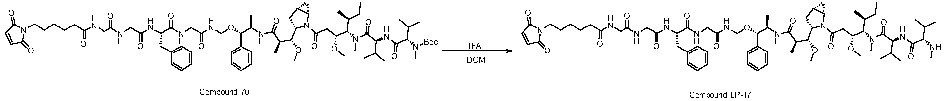

Referring to the synthesis method of Example 9, compound LP-17 was synthesized using compound 70 as the raw material. LC-MS m/z (ES+): [M+H]⁺: 1270.8.

### Example 62 Synthesis of Compound LP-18

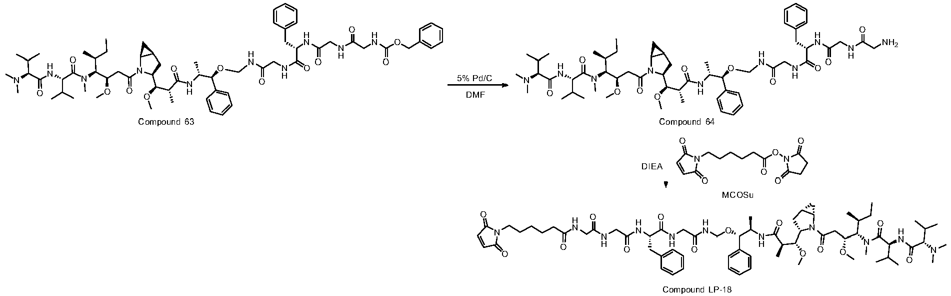

Referring to the synthesis method of Example 49, compound LP-18 was synthesized using compound 63 as the raw material. LC-MS m/z (ES+): [M+H]⁺: 1284.8.

### Example 63 Synthesis of Compound 71

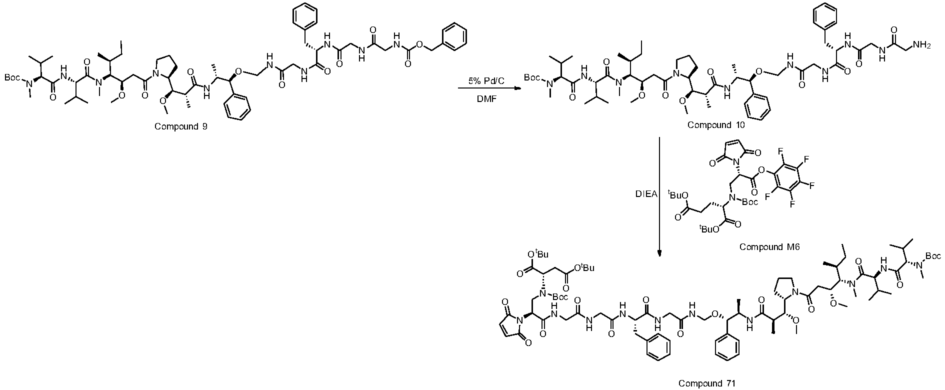

Compound 9 (960 mg, 0.74 mmol, 1.0 eq), 5% Pd/C (960 mg) and DMF (10 mL) were added to a 50 mL round-bottom flask. After H₂ replacement 3 times, the reaction was carried out at room temperature for 1 h. The reaction was monitored by HPLC. The reaction was completed and recorded as reaction solution ①. The reaction solution ① was filtered into a 50 mL single-neck round-bottom flask. Compound M6 (512.5 mg, 0.74 mmol, 1.0 eq, compound M6 refers to the synthesis of compound M6 in CN113827736) and DIEA (105 mg, 0.81 mmol, 1.1 eq) were added in turn under ice-water bath. After the addition, the temperature was raised to room temperature for 1 h. The reaction was monitored by HPLC. The reaction solution was purified by reverse phase preparation, and the prepared solution was lyophilized to obtain a white solid (860 mg, 70%). LC-MSm/z (ES+): [M+H]⁺: 1660.1.

### Example 64 Synthesis of compound LP-19a

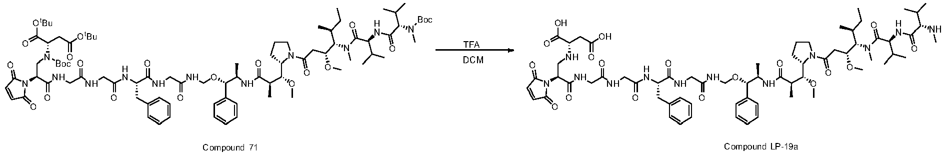

Referring to the synthesis method of Example 9, compound LP-19a was synthesized using compound 71 as the raw material. LC-MS m/z (ES+): [M/2+H]+: 674.4.

### Example 65 Synthesis of Compound 72

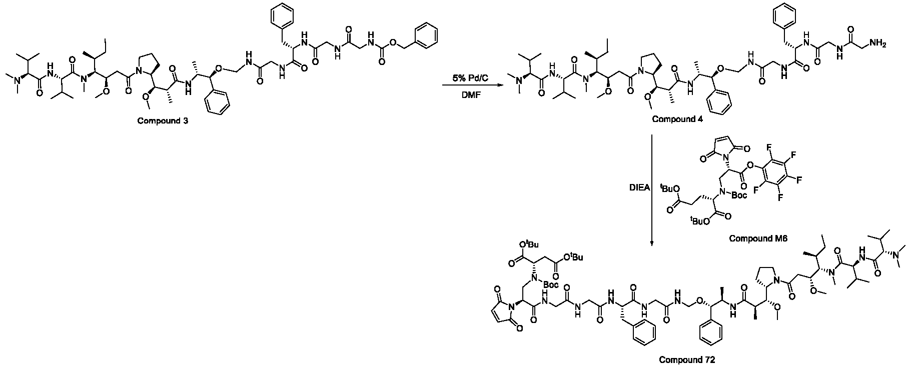

Referring to the synthesis method of Example 63, compound 72 was synthesized using Compound 3 as the raw material. LC-MS m/z (ES+): [M/2+H]+: 783.5.

### Example 66 Synthesis of Compound LP-20a

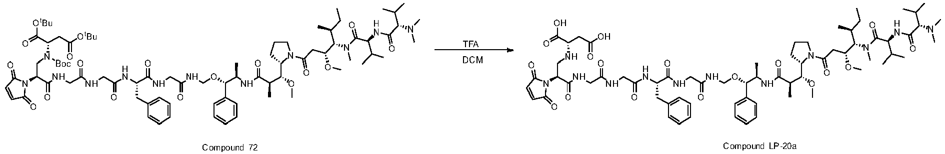

Referring to the synthesis method of Example 9, compound LP-20a was synthesized using compound 72 as the raw material. LC-MS m/z (ES+): [M/2+H]+: 681.4.

### Example 67 Synthesis of Compound 73

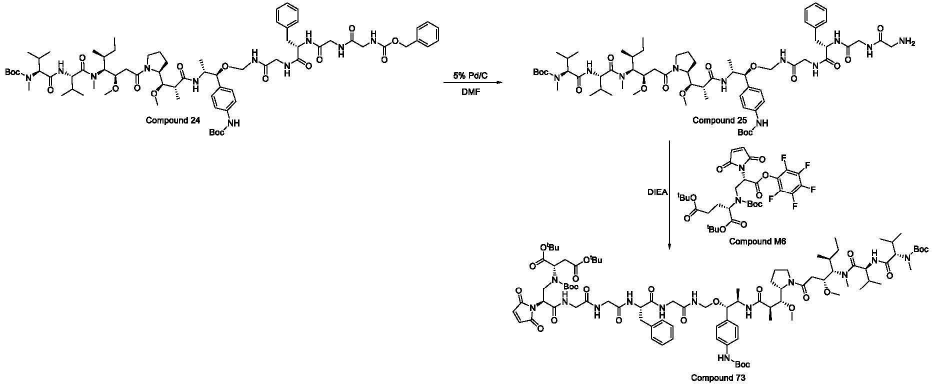

Referring to the synthesis method of Example 63, compound 73 was synthesized using compound 24 as the raw material. LC-MS m/z (ES+): [M/2+H]+: 888.5.

### Example 68 Synthesis of Compound LP-21a

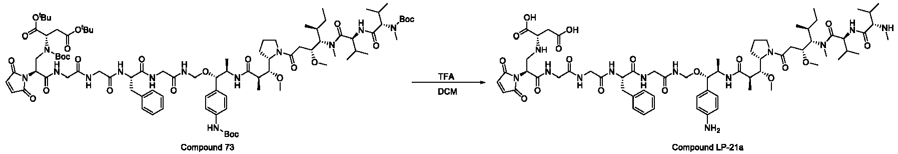

Referring to the synthesis method of Example 9, compound LP-21a was synthesized using compound 73 as the raw material. LC-MS m/z (ES+): [M+H]⁺: 1362.7.

### Example 69 Synthesis of compound 74

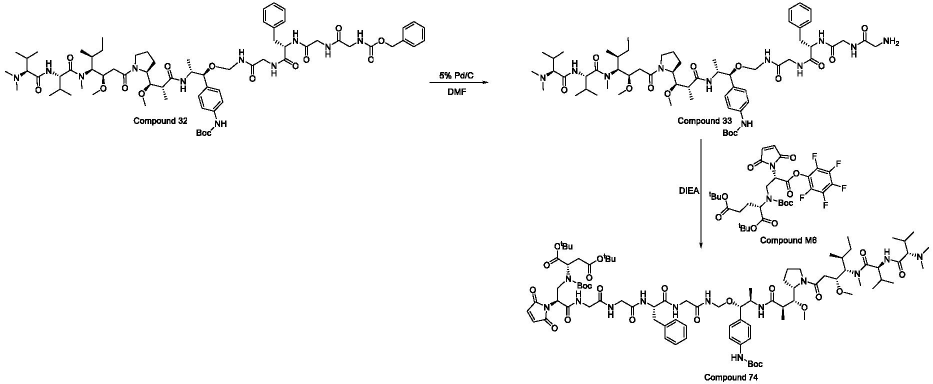

Referring to the synthesis method of Example 63, compound 74 was synthesized using compound 32 as the raw material. LC-MS m/z (ES+): [M/2+H]+: 845.1.

### Example 70 Synthesis of Compound LP-22a

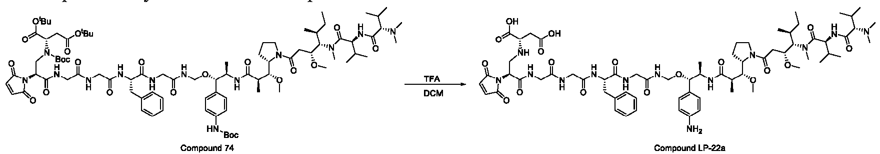

Referring to the synthesis method of Example 9, compound LP-22a was synthesized using compound 74 as the raw material. LC-MS m/z (ES+): [M+H]⁺: 1376.8.

### Example 71 Synthesis of Compound 75

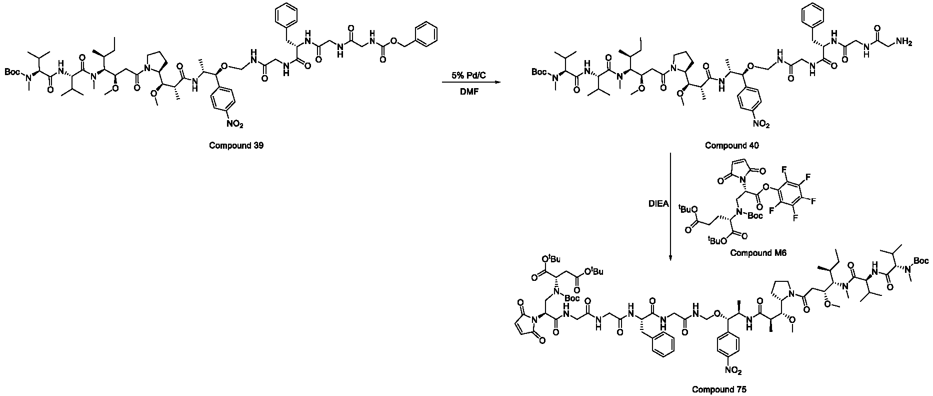

Referring to the synthesis method of Example 63, Compound 75 was synthesized using Compound 39 as the raw material. LC-MS m/z (ES+): [M/2+H]+: 853.1.

### Example 72 Synthesis of Compound LP-23a

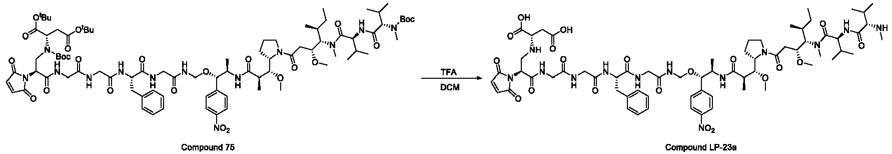

Referring to the synthesis method of Example 9, compound LP-23a was synthesized using compound 75 as the raw material. LC-MS m/z (ES+): [M+H]⁺: 1392.7.

### Example 73 Synthesis of Compound 76

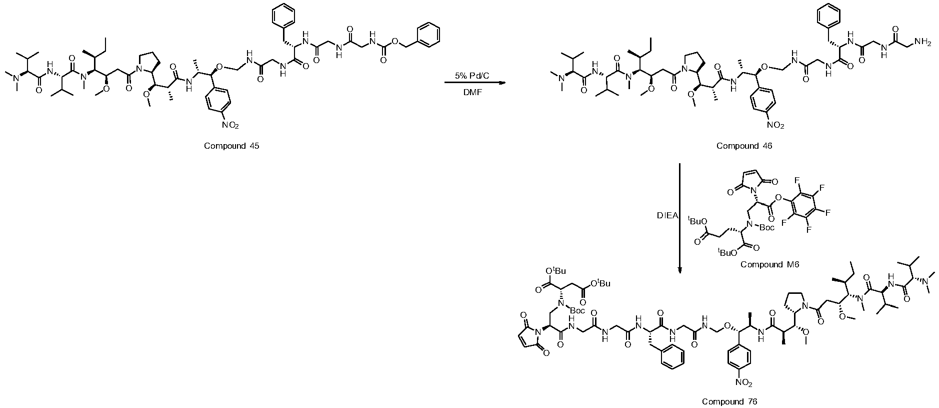

Referring to the synthesis method of Example 63, Compound 76 was synthesized using Compound 45 as the raw material. LC-MS m/z (ES+): [M/2+H]+: 810.1.

### Example 74 Synthesis of Compound LP-24a

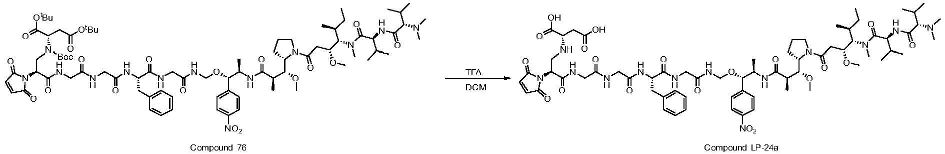

Referring to the synthesis method of Example 9, compound LP-24a was synthesized using compound 76 as the raw material. LC-MS m/z (ES+): [M+H]⁺: 1406.8.

### Example 75 Synthesis of Compound 77

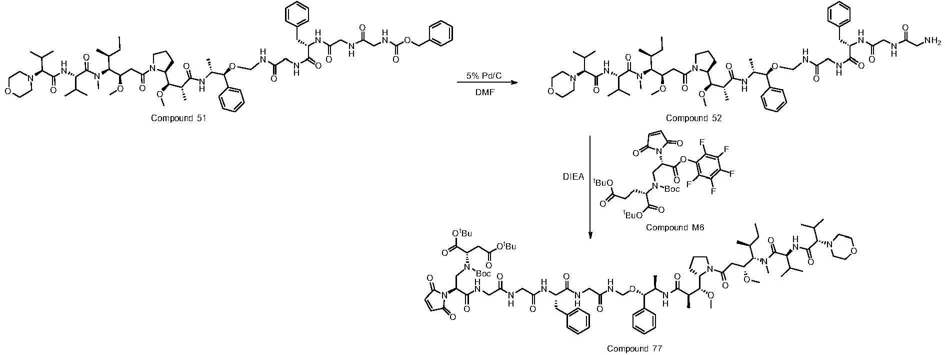

Referring to the synthesis method of Example 63, Compound 77 was synthesized using Compound 51 as the raw material. LC-MS m/z (ES+): [M/2+H]⁺: 808.5.

### Example 76 Synthesis of Compound LP-25a

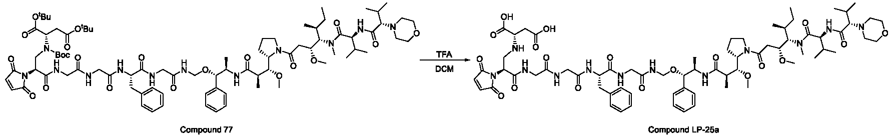

Compound LP-25a was synthesized from compound 77 with reference to the synthetic method of Example 9. LC-MS m/z (ES+): [M+H]⁺: 1403.8.

### Example 77 Synthesis of Compound 78

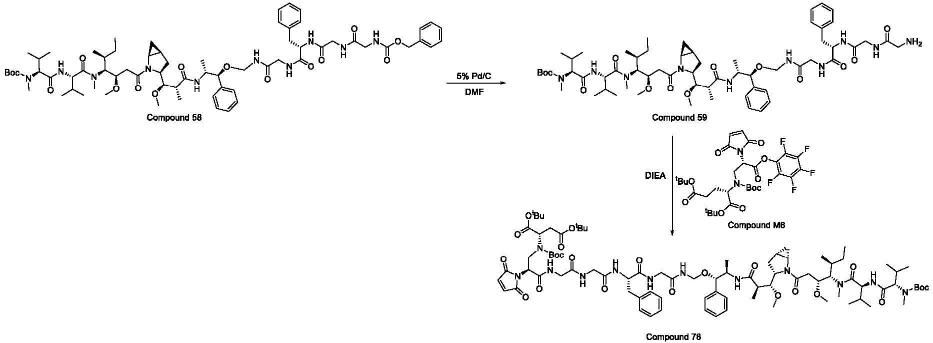

Referring to the synthesis method of Example 63, Compound 78 was synthesized using Compound 58 as the raw material. LC-MS m/z (ES+): [M/2+H]⁺: 836.5.

### Example 78 Synthesis of Compound LP-26a

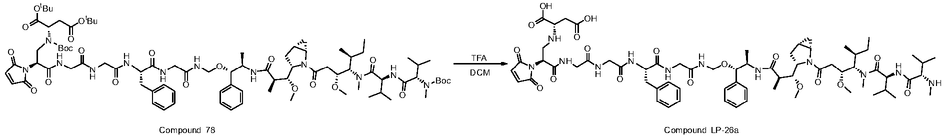

Referring to the synthesis method of Example 9, compound LP-26a was synthesized using compound 78 as the raw material. LC-MS m/z (ES+): [M+H]⁺: 1359.7.

### Example 79 Synthesis of Compound 79

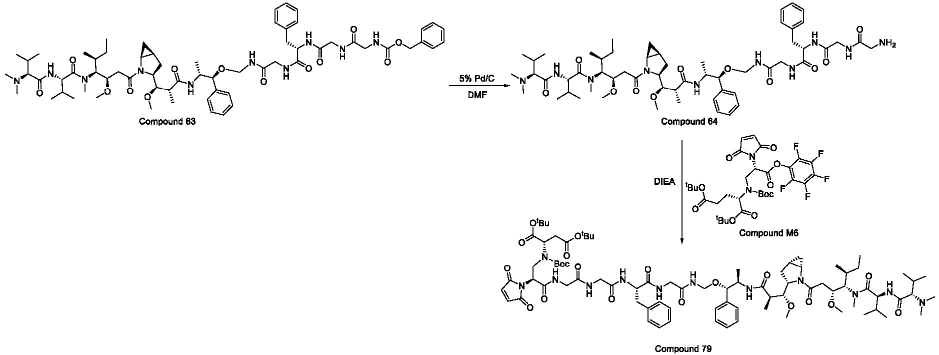

Referring to the synthesis method of Example 63, Compound 79 was synthesized using Compound 63 as the raw material. LC-MS m/z (ES+): [M/2+H]+: 793.5.

### Example 80 Synthesis of Compound LP-27a

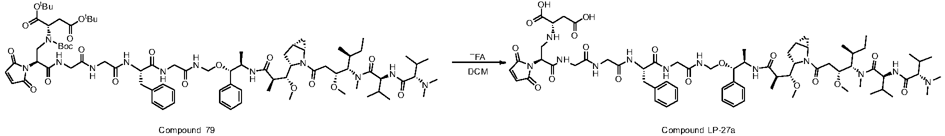

Referring to the synthesis method of Example 9, compound LP-27a was synthesized using compound 79 as the raw material. LC-MS m/z (ES+): [M+H]⁺: 1373.8.

### Example 81 Synthesis of Compound 80

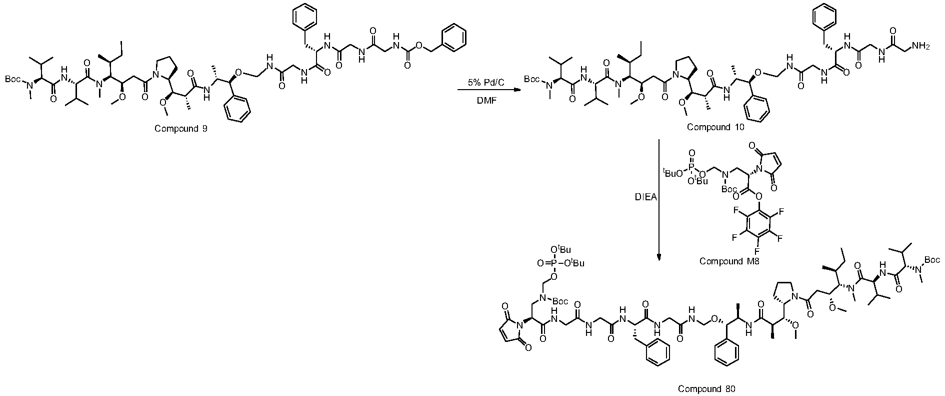

Compound 9 (960 mg, 0.74 mmol, 1.0 eq), 5% Pd/C (960 mg) and DMF (10 mL) were added to a 50 mL round-bottom flask. After H₂ replacement 3 times, the reaction was carried out at room temperature for 1 h. The reaction was monitored by HPLC. The reaction was completed and recorded as reaction solution ①. The reaction solution ① was filtered into a 50 mL single-neck round-bottom flask. Compound M8 (497.7 mg, 0.74 mmol, 1.0 eq, compound M8 refers to the synthesis of compound M8 in CN113827736) and DIEA (105 mg, 0.81 mmol, 1.1 eq) were added in turn under ice-water bath. After the addition, the temperature was raised to room temperature for 1 h. The reaction was monitored by HPLC. The reaction solution was purified by reverse phase preparation, and the prepared solution was lyophilized to obtain a white solid (918 mg, 75%). LC-MSm/z (ES+): [M/2+H]+: 827.5.

### Example 82 Synthesis of Compound LP-28a

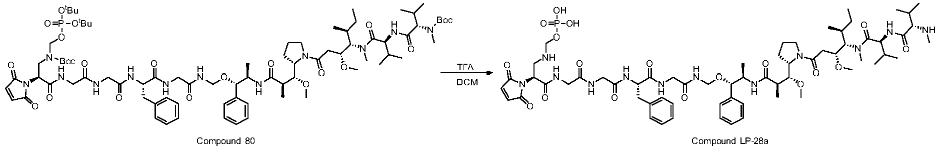

Referring to the synthesis method of Example 9, compound LP-28a was synthesized using compound 80 as the raw material. LC-MS m/z (ES+): [M/2+H]+: 671.4.

### Example 83 Synthesis of Compound 81

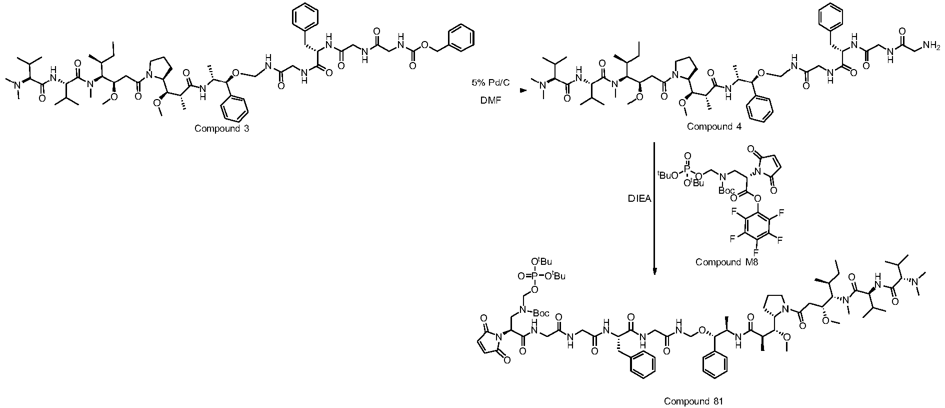

Referring to the synthesis method of Example 81, compound 81 was synthesized using Compound 3 as the raw material. LC-MS m/z (ES+): [M/2+H]+: 784.5.

### Example 84 Synthesis of Compound LP-29a

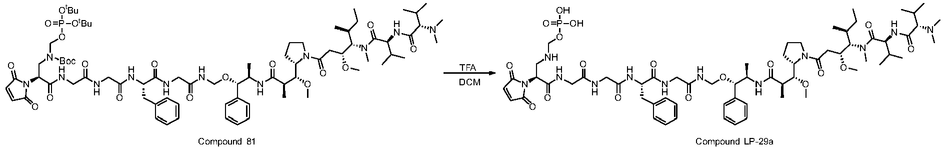

Referring to the synthesis method of Example 9, compound LP-29a was synthesized using compound 81 as the raw material. LC-MS m/z (ES+): [M/2+H]+: 678.4.

### Example 85 Synthesis of Compound 82

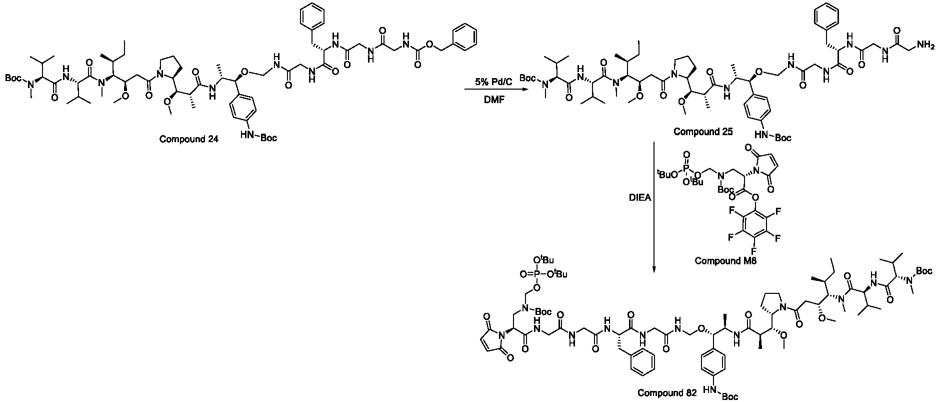

Referring to the synthesis method of Example 81, compound 82 was synthesized using Compound 24 as the raw material. LC-MS m/z (ES+): [M/2+H]+: 885.0.

### Example 86 Synthesis of Compound LP-30a

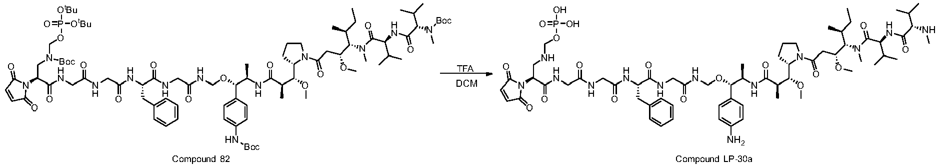

Referring to the synthesis method of Example 9, compound LP-30a was synthesized using compound 82 as the raw material. LC-MS m/z (ES+): [M/2+H]+: 678.9.

### Example 87 Synthesis of Compound 83

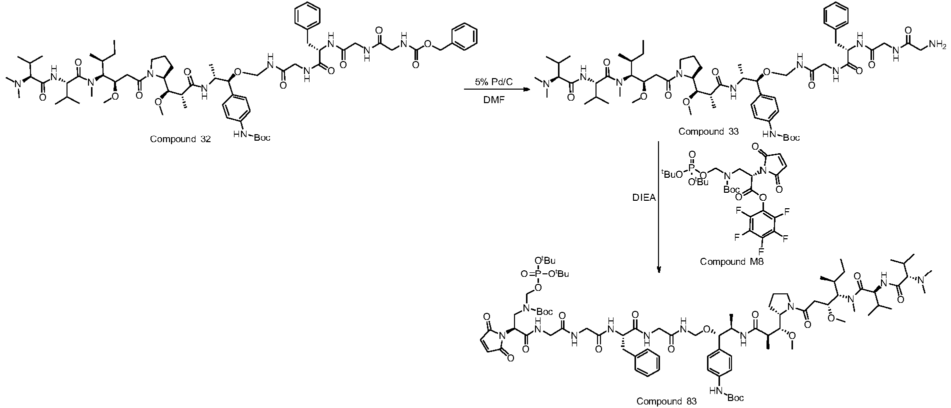

Referring to the synthesis method of Example 81, compound 83 was synthesized using Compound 32 as the raw material. LC-MS m/z (ES+): [M/2+H]+: 842.0.

### Example 88 Synthesis of Compound LP-31a

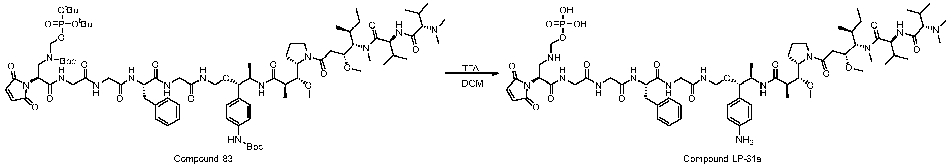

Referring to the synthesis method of Example 9, compound LP-31a was synthesized using compound 83 as the raw material. LC-MS m/z (ES+): [M/2+H]+: 685.9.

### Example 89 Synthesis of Compound 84

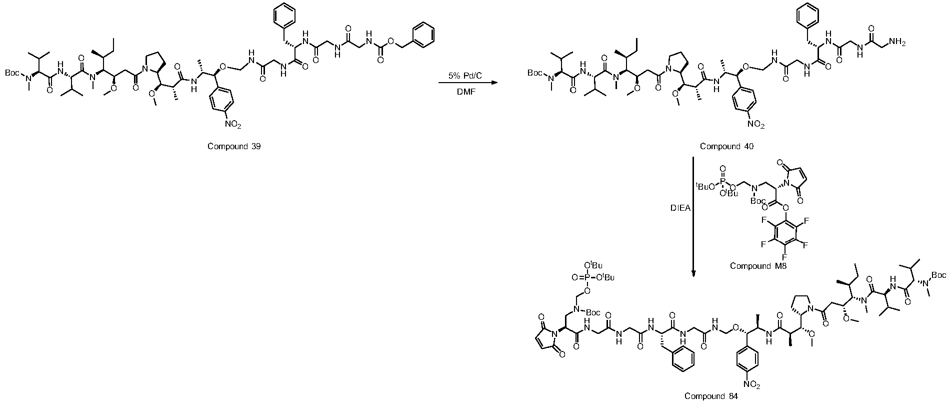

Referring to the synthesis method of Example 81, compound 84 was synthesized using compound 39 as the raw material. LC-MSm/z (ES+): [M/2+H]+: 850.1.

### Example 90 Synthesis of Compound LP-32a

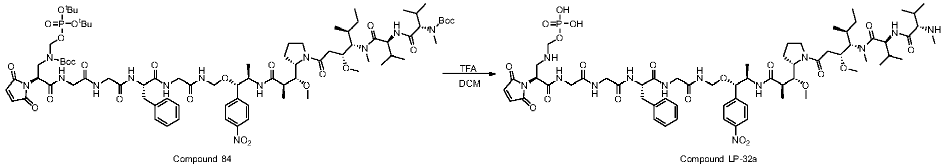

Referring to the synthesis method of Example 9, compound LP-32a was synthesized using compound 84 as the raw material. LC-MS m/z (ES+): [M/2+H]+: 693.9.

### Example 91 Synthesis of Compound 85

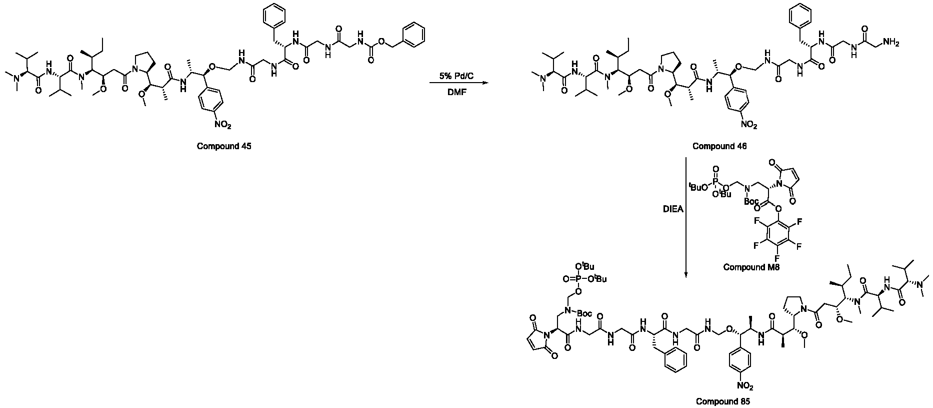

Referring to the synthesis method of Example 81, compound 85 was synthesized using Compound 45 as the raw material. LC-MS m/z (ES+): [M/2+H]+: 806.9.

### Example 92 Synthesis of Compound LP-33a

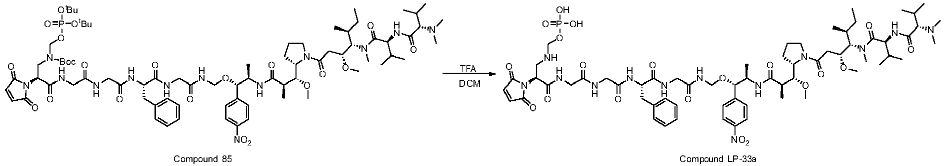

Referring to the synthesis method of Example 9, compound LP-33a was synthesized using compound 85 as the raw material. LC-MS m/z (ES+): [M/2+H]+: 700.9.

### Example 93 Synthesis of Compound 86

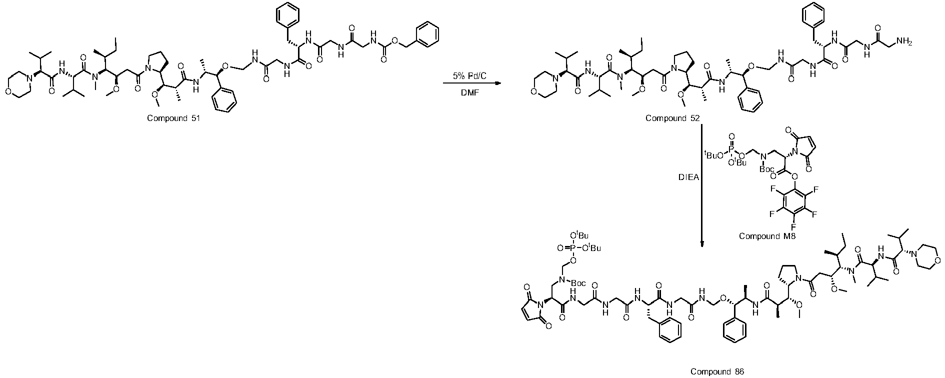

Referring to the synthesis method of Example 81, compound 86 was synthesized using Compound 51 as the raw material. LC-MS m/z (ES+): [M/2+H]+: 805.5.

### Example 94 Synthesis of Compound LP-34a

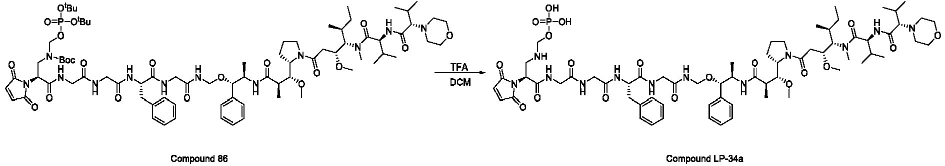

Referring to the synthesis method of Example 9, compound LP-34a was synthesized using compound 86 as the raw material. LC-MS m/z (ES+): [M/2+H]+: 699.4.

### Example 95 Synthesis of Compound 87

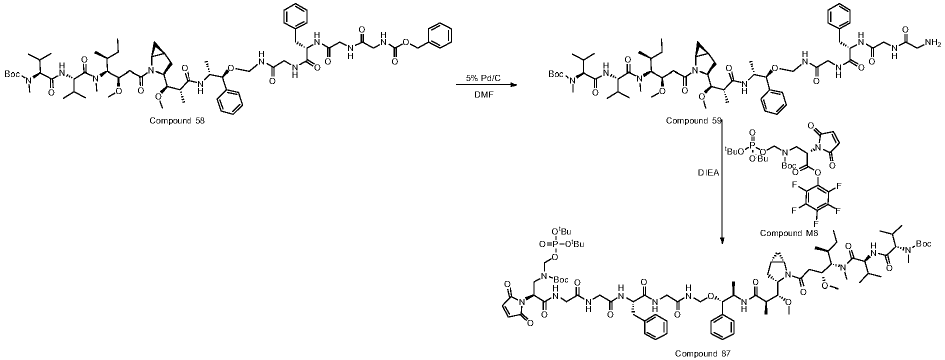

Referring to the synthesis method of Example 81, compound 87 was synthesized using Compound 58 as the raw material. LC-MS m/z (ES+): [M/2+H]+: 833.5.

### Example 96 Synthesis of Compound LP-35a

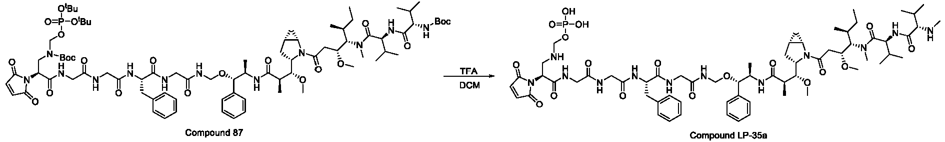

Referring to the synthesis method of Example 9, compound LP-35a was synthesized using compound 87 as the raw material. LC-MS m/z (ES+): [M/2+H]+: 677.4.

### Example 97 Synthesis of Compound 88

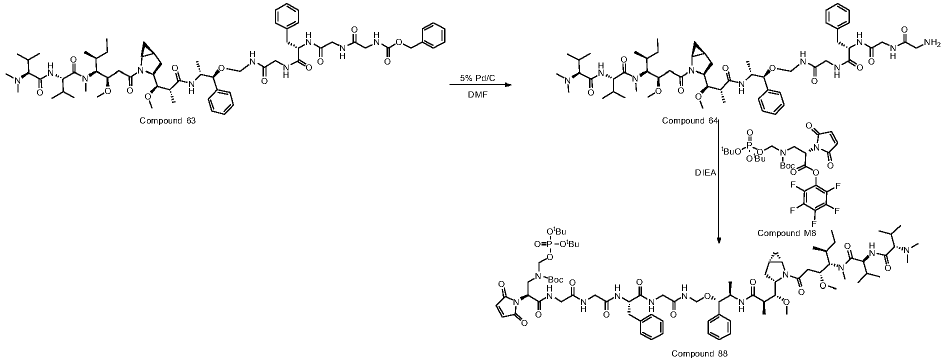

Referring to the synthesis method of Example 81, Compound 88 was synthesized using Compound 63 as the raw material. LC-MSm/z (ES+): [M/2+H]+: 790.5.

### Example 98 Synthesis of Compound LP-36a

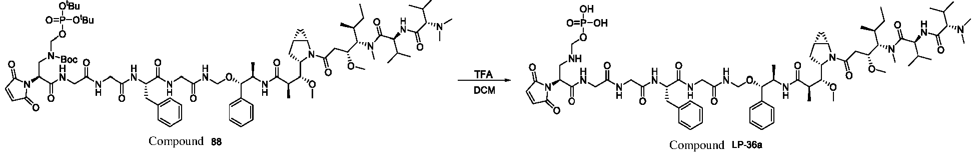

Referring to the synthesis method of Example 9, compound LP-36a was synthesized using compound 88 as a raw material. LC-MS m/z (ES+): [M/2+H]+: 670.4.

### Example 99 Synthesis of Compound 89

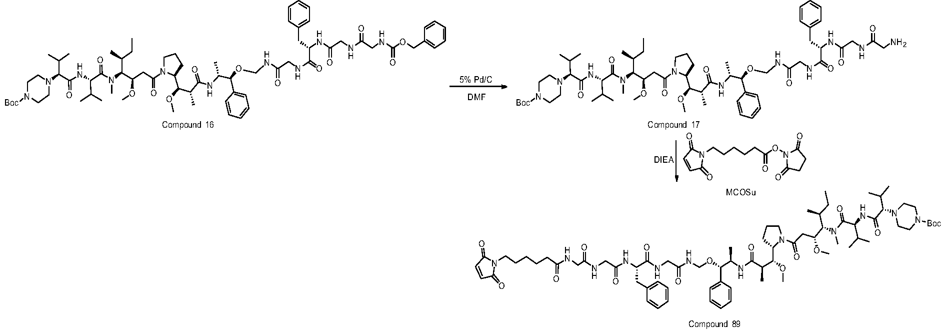

Referring to the synthesis method of Example 49, compound 89 was synthesized using compound 16 as the raw material. LC-MS m/z (ES+): [M/2+H]+: 707.4.

### Example 100 Synthesis of Compound LP-37

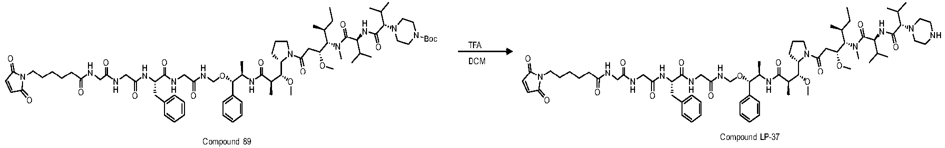

Referring to the synthesis method of Example 9, compound LP-37 was synthesized using compound 89 as the raw material. LC-MS m/z (ES+): [M+H]⁺: 1313.8.

### Example 101 Synthesis of Compound 90

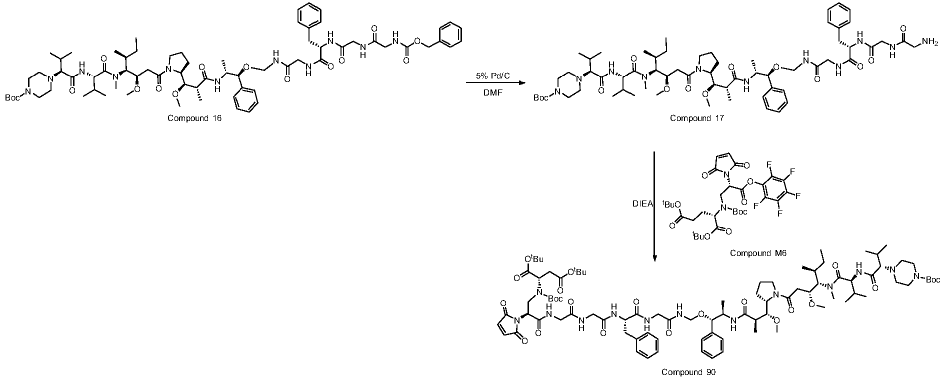

Referring to the synthesis method of Example 63, compound 90 was synthesized using compound 16 as the raw material. LC-MS m/z (ES+): [M/2+H]+: 858.0.

### Example 102 Synthesis of Compound LP-38a

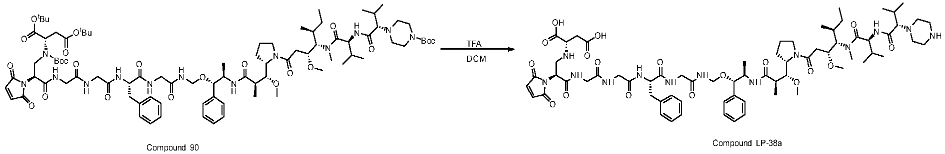

Referring to the synthesis method of Example 9, compound LP-38a was synthesized using compound 90 as the raw material. LC-MS m/z (ES+): [M/2+H]+: 701.9.

### Example 103 Synthesis of Compound 91

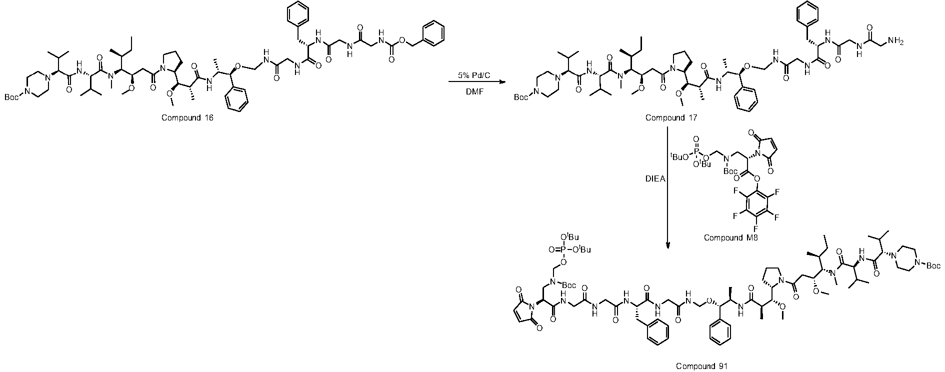

Referring to the synthesis method of Example 81, compound 91 was synthesized using Compound 16 as the raw material. cLC-MS m/z (ES+): [M/2+H]+: 855.0.

### Example 104 Synthesis of Compound LP-39a

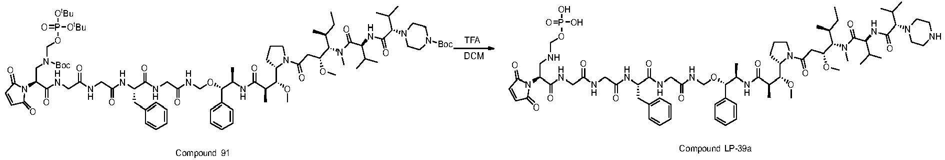

Referring to the synthesis method of Example 9, compound 91 was used as a raw material to synthesize compound LP-39a. LC-MSm/z (ES+): [M/2+H]⁺: 698.9.

### Example 105 Preparation of ADC-1-1a

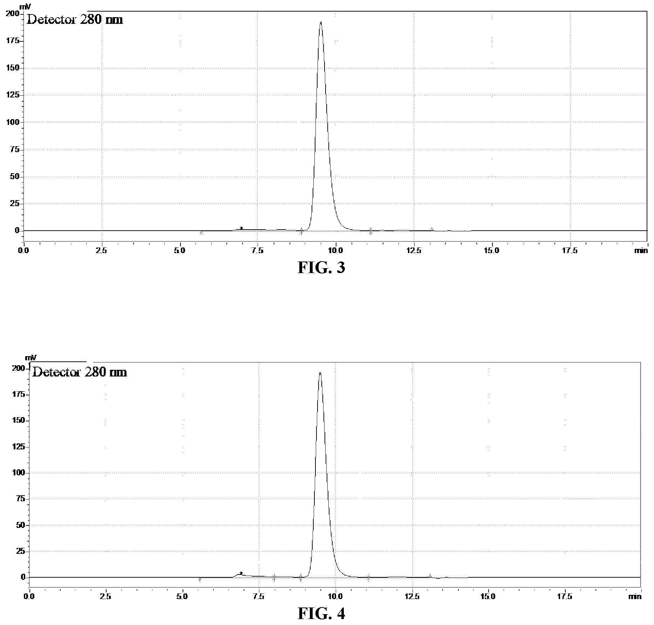

ADC-1-1a was prepared by reacting the corresponding linker-payload (LP-1a, whose structure is shown in the structural formula of LP-1, wherein the configuration of the chiral carbon at position 2 is S-type) with the anti-Trop2 antibody TR000 according to the method of general step A. The sequence information of TR000 is shown in Table 1. The SEC-HPLC detection results are shown in **Figure 3****.**

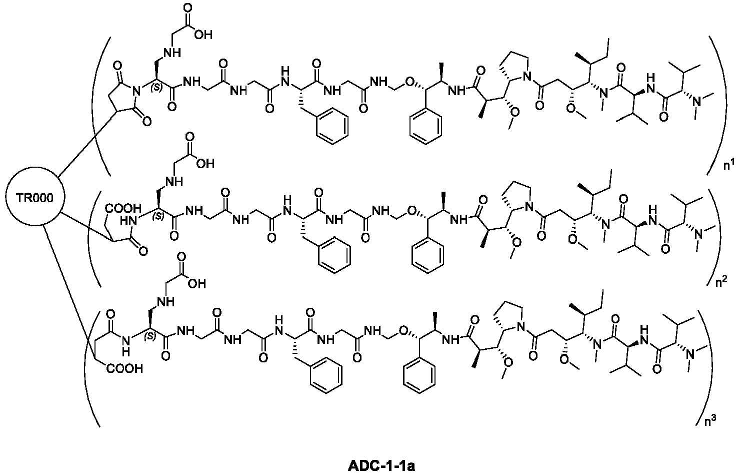

### Example 106 Preparation of ADC-2-1a

ADC-2-1a was prepared by combining the corresponding linker-payload (LP-2a, whose structure is shown in the structural formula of LP-2, wherein the configuration of the chiral carbon at position 2 is S-type) with the anti-Trop2 antibody TR000 according to the method of general step A. The SEC-HPLC test results are shown in Figure 4.

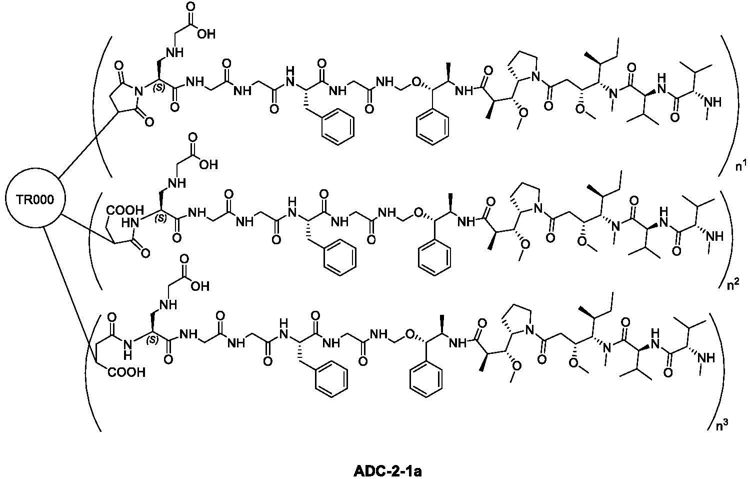

### Example 107 Preparation of Antibody Drug Conjugate ADC-C-1a

ADC-C-1a was prepared by combining compound VcMMAE with anti-Trop2 antibody TR000 according to the method of general step A.

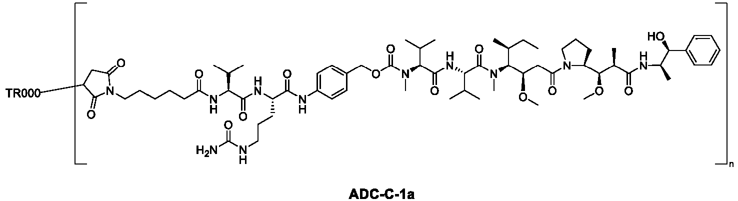

### Example 108 Preparation of ADC-3-1a

ADC-3-1a was prepared by combining the corresponding linker-payload (LP-10) with the anti-Trop2 antibody TR000 according to the method of general step A.

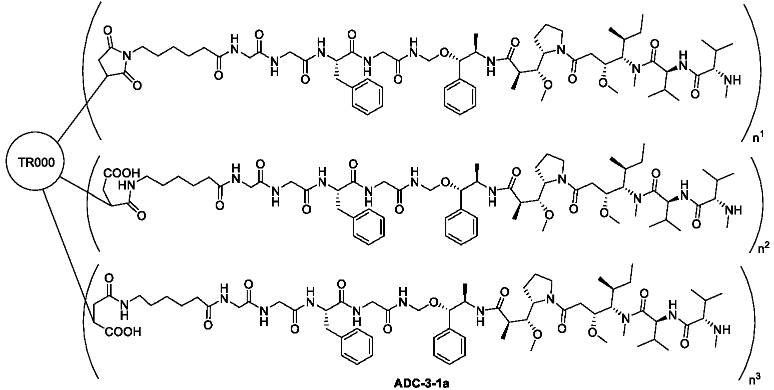

### Example 109 Preparation of ADC-4-1a

ADC-4-1a was prepared by combining the corresponding linker-payload (LP-37) with the anti-Trop2 antibody TR000 according to the method of general step A.

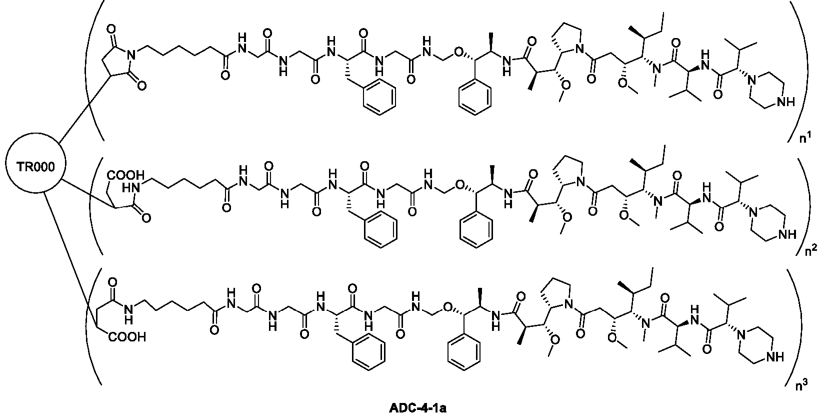

### Example 110 Preparation of ADC-5-1a

ADC-5-1a was prepared by combining the corresponding linker-payload (LP-16) with the anti-Trop2 antibody TR000 according to the method of general step A.

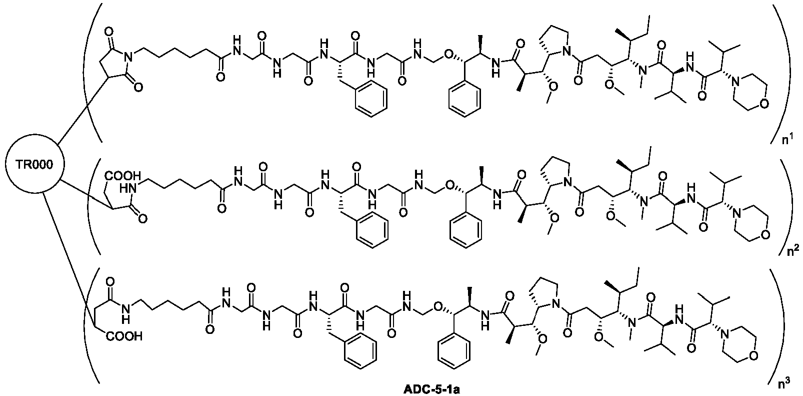

### Example 111 Preparation of ADC-6-1a

ADC-6-1a was prepared by combining the corresponding linker-payload (LP-11) with the anti-Trop2 antibody TR000 according to the method of general step A.

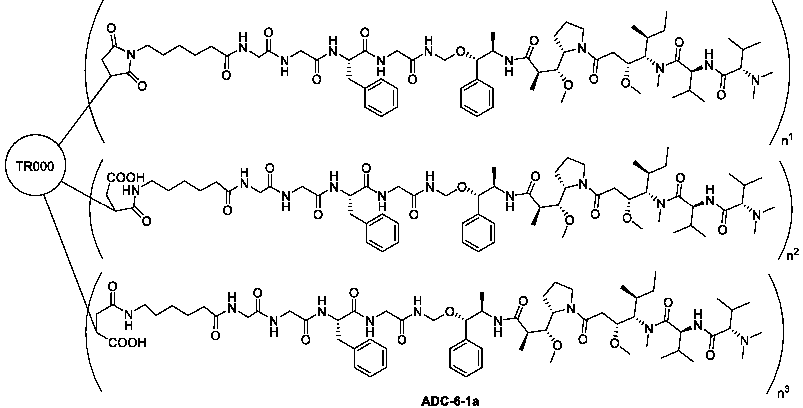

### Example 112 Preparation of ADC-7-1a

ADC-7-1a was prepared by combining the corresponding linker-payload (LP-12) with the anti-Trop2 antibody TR000 according to the method of general step A.

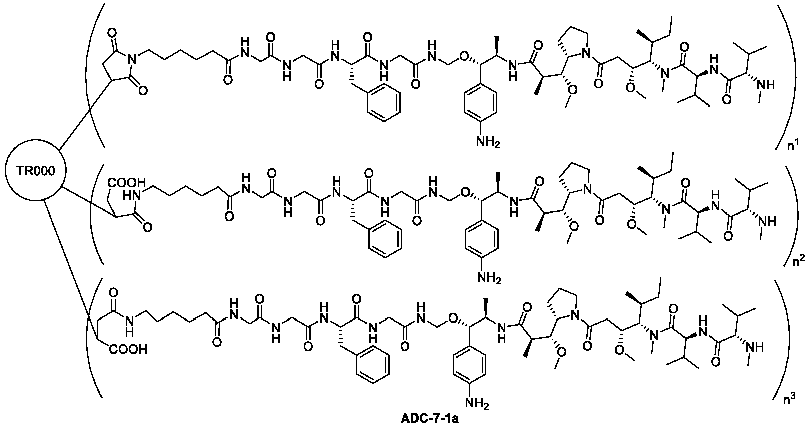

### Example 113 Preparation of ADC-8-1a

ADC-8-1a was prepared by combining the corresponding linker-payload (LP-13) with the anti-Trop2 antibody TR000 according to the method of general step A.

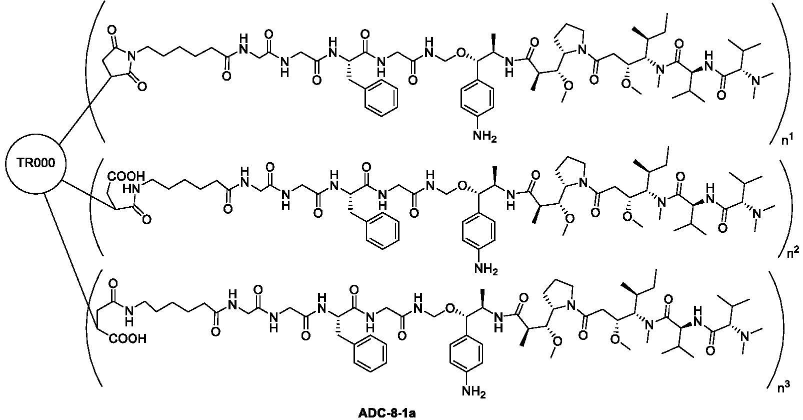

### Example 114 Preparation of ADC-9-1a

ADC-9-1a was prepared by combining the corresponding linker-payload (LP-14) with the anti-Trop2 antibody TR000 according to the method of general step A.

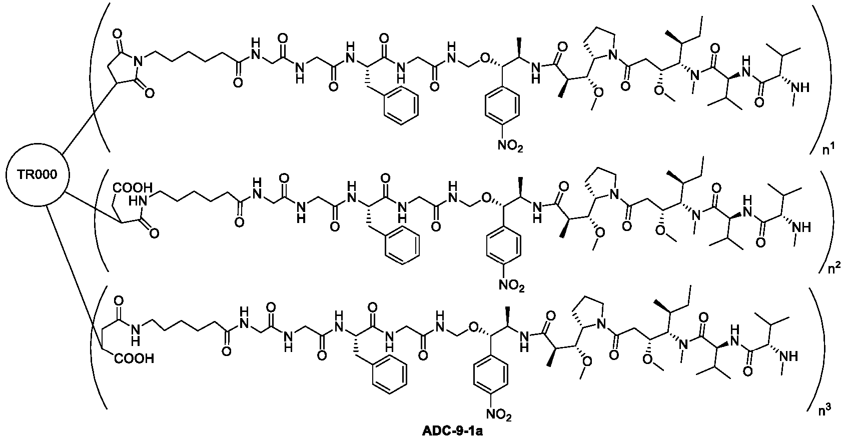

### Example 115 Preparation of ADC-10-1a

ADC-10-1a was prepared by combining the corresponding linker-payload (LP-15) with the anti-Trop2 antibody TR000 according to the method of general step A.

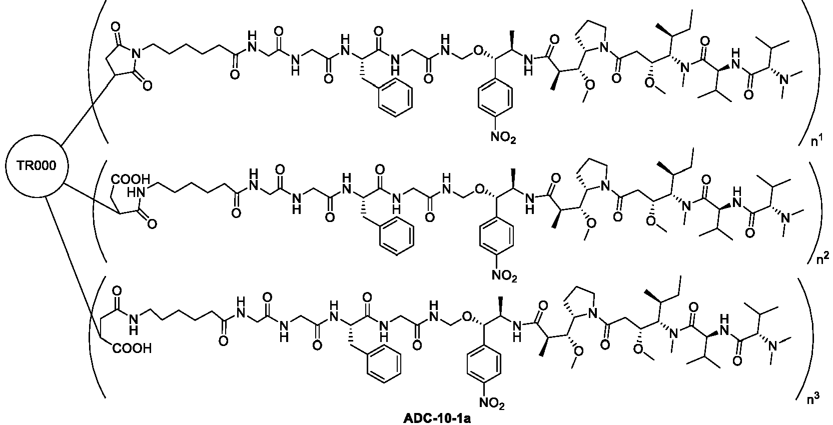

### Example 116 Preparation of ADC-11-1a

ADC-11-1a was prepared by combining the corresponding linker-payload (LP-17) with the anti-Trop2 antibody TR000 according to the method of general step A.

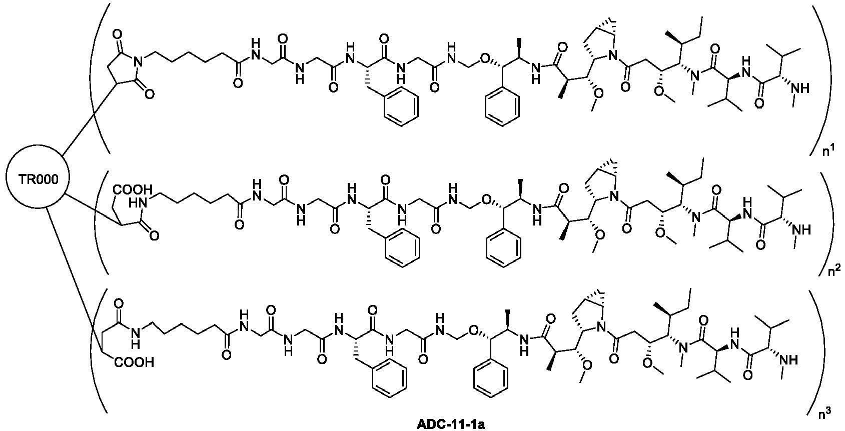

### Example 117 Preparation of ADC-12-1a

ADC-12-1a was prepared by combining the corresponding linker-payload (LP-18) with the anti-Trop2 antibody TR000 according to the method of general step A.

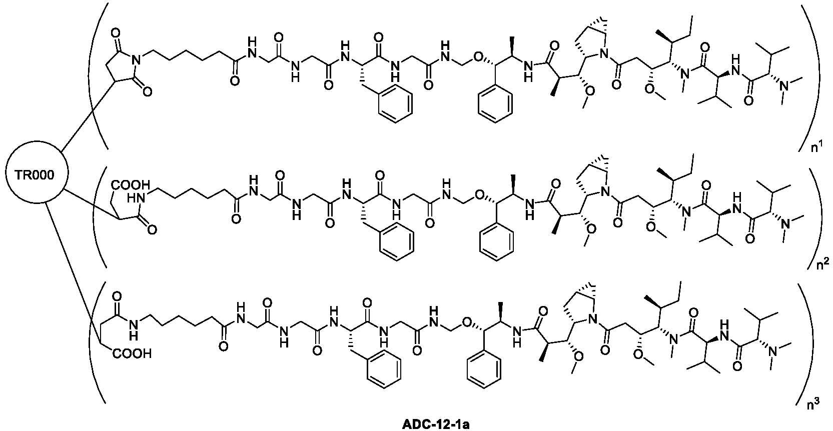

### Example 118 Preparation of ADC-13-1a

ADC-13-1a was prepared by combining the corresponding linker-payload (LP-3a, whose structure is shown in the structural formula of LP-3, wherein the configuration of the chiral carbon at position 2 is S-type) with the anti-Trop2 antibody TR000 according to the method of general step A.

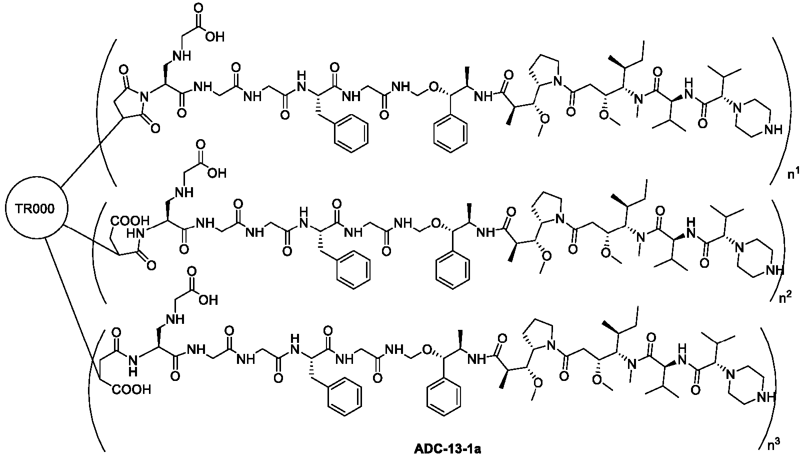

### Example 119 Preparation of ADC-14-1a

ADC-14-1a was prepared by combining the corresponding linker-payload (LP-7a, whose structure is shown in the structural formula of LP-7, wherein the configuration of the chiral carbon at position 2 is S-type) with the anti-Trop2 antibody TR000 according to the method of general step A.

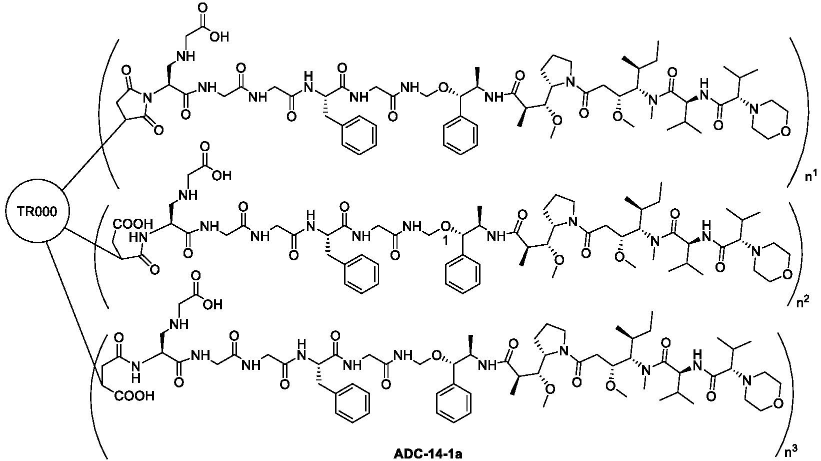

### Example 120 Preparation of ADC-15-1a

ADC-15-1a was prepared by combining the corresponding linker-payload (LP-3sa, whose structure is shown in the structural formula of LP-3s, wherein the configuration of the chiral carbon at position 2 is S-type) with the anti-Trop2 antibody TR000 according to the method of general step A.

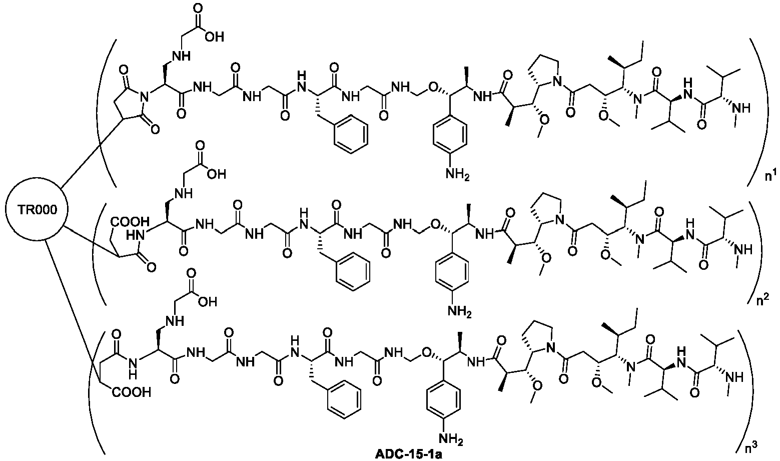

### Example 121 Preparation of ADC-16-1a

ADC-16-1a was prepared by combining the corresponding linker-payload (LP-4a, whose structure is shown in the structural formula of LP-4, wherein the configuration of the chiral carbon at position 2 is S-type) with the anti-Trop2 antibody TR000 according to the method of general step A.

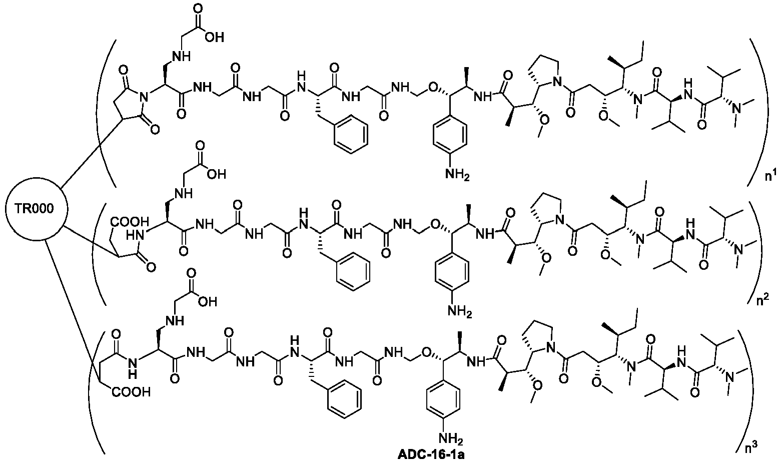

### Example 122 Preparation of ADC-17-1a

ADC-17-1a was prepared by combining the corresponding linker-payload (LP-5a, whose structure is shown in the structural formula of LP-5, wherein the configuration of the chiral carbon at position 2 is S-type) with the anti-Trop2 antibody TR000 according to the method of general step A.

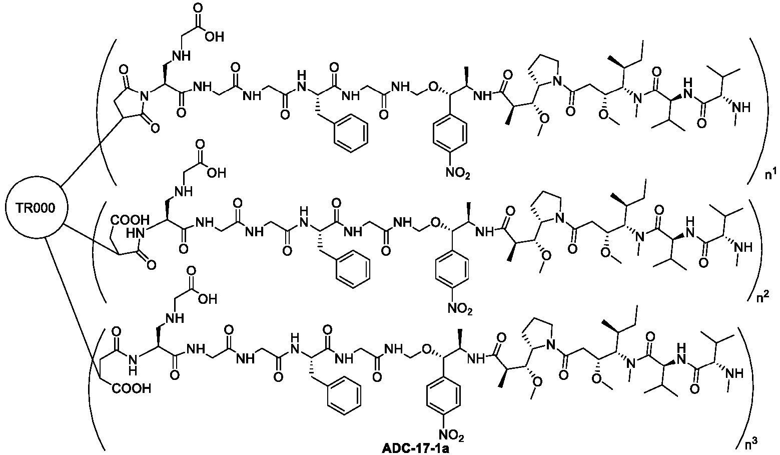

### Example 123 Preparation of ADC-18-1a

ADC-18-1a was prepared by combining the corresponding linker-payload (LP-6a, whose structure is shown in the structural formula of LP-6, wherein the configuration of the chiral carbon at position 2 is S-type) with the anti-Trop2 antibody TR000 according to the method of general step A.

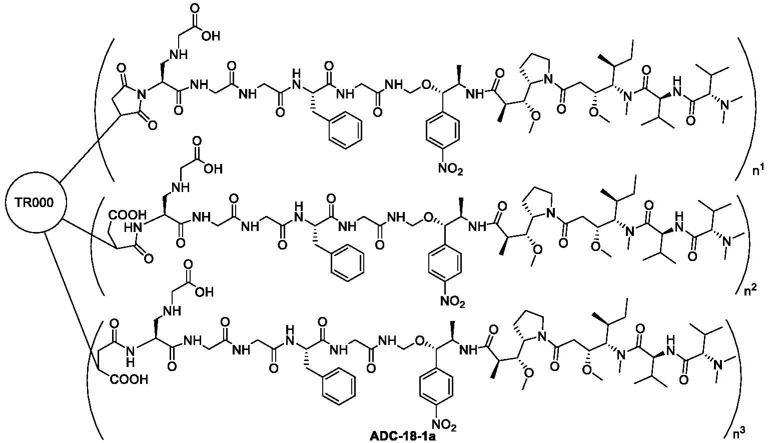

### Example 124 Preparation of ADC-19-1a

ADC-19-1a was prepared by combining the corresponding linker-payload (LP-8a, whose structure is shown in the structural formula of LP-8, wherein the configuration of the chiral carbon at position 2 is S-type) with the anti-Trop2 antibody TR000 according to the method of general step A.

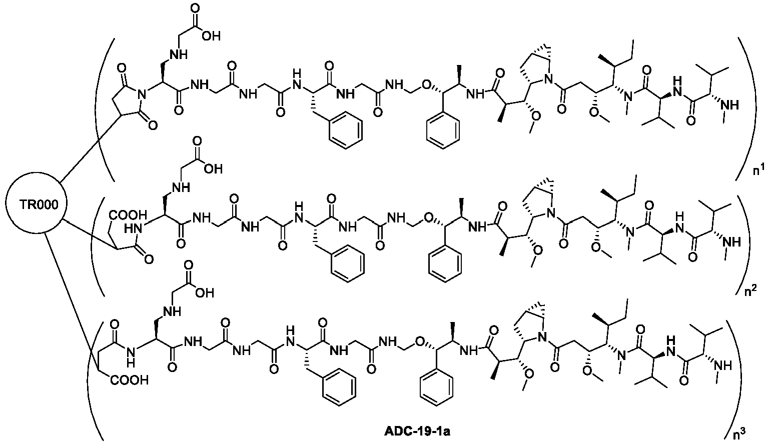

### Example 125 Preparation of ADC-20-1a

ADC-20-1a was prepared by combining the corresponding linker-payload (LP-9a, whose structure is shown in the structural formula of LP-9, wherein the configuration of the chiral carbon at position 2 is S-type) with the anti-Trop2 antibody TR000 according to the method of general step A.

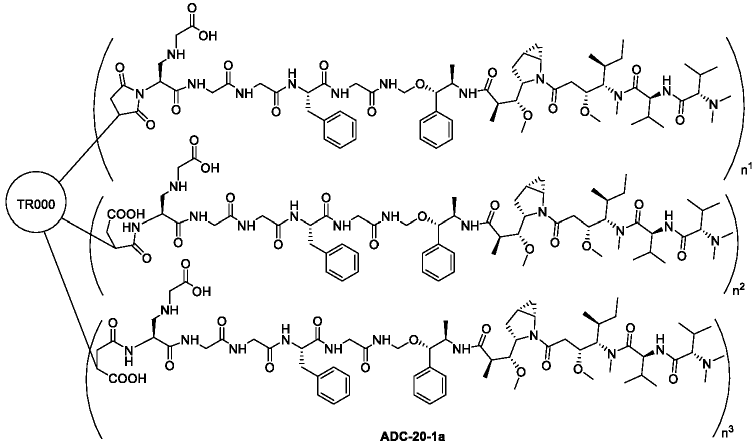

### Example 126 Preparation of ADC-21-1a

ADC-21-1a was prepared by combining the corresponding linker-payload (LP-28a, whose structure is shown in the structural formula of LP-28, wherein the configuration of the chiral carbon at position 2 is S-type) with the anti-Trop2 antibody TR000 according to the method of general step A.

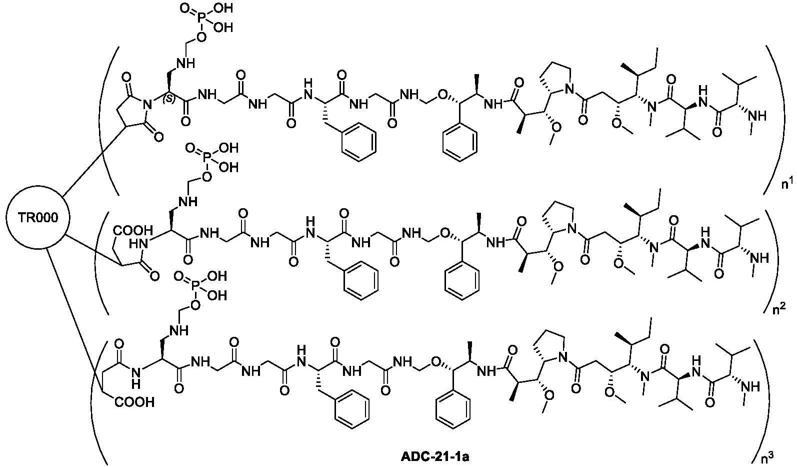

### Example 127 Preparation of ADC-22-1a

ADC-22-1a was prepared by combining the corresponding linker-payload (LP-29a, whose structure is shown in the structural formula of LP-29, wherein the configuration of the chiral carbon at position 2 is S-type) with the anti-Trop2 antibody TR000 according to the method of general step A.

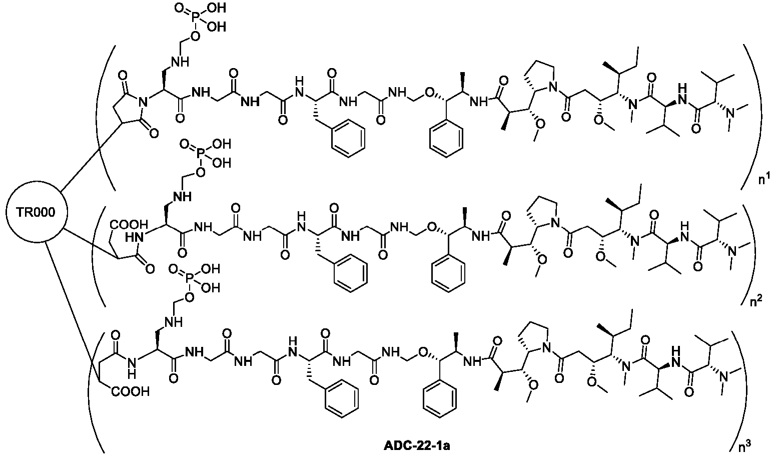

### Example 128 Preparation of ADC-23-1a

ADC-23-1a was prepared by combining the corresponding linker-payload (LP-30a, whose structure is shown in the structural formula of LP-30, wherein the configuration of the chiral carbon at position 2 is S-type) with the anti-Trop2 antibody TR000 according to the method of general step A.

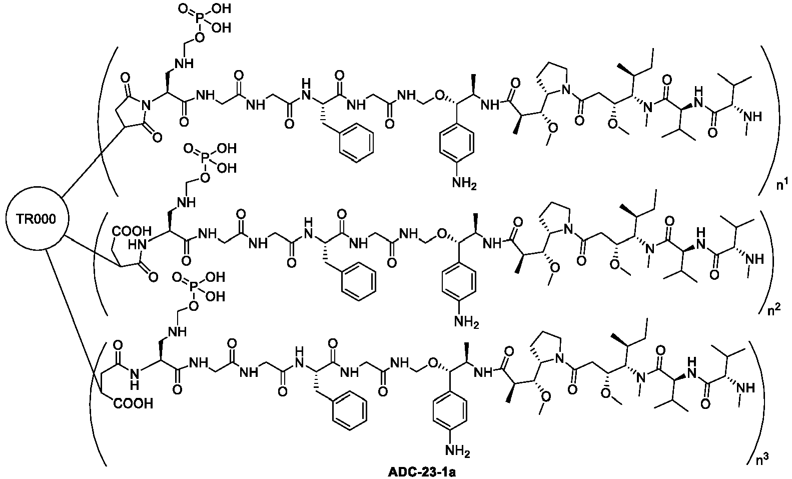

### Example 129 Preparation of ADC-24-1a

ADC-24-1a was prepared by combining the corresponding linker-payload (LP-31a, whose structure is shown in the structural formula ofLP-31, wherein the configuration of the chiral carbon at position 2 is S-type) with the anti-Trop2 antibody TR000 according to the method of general step A.

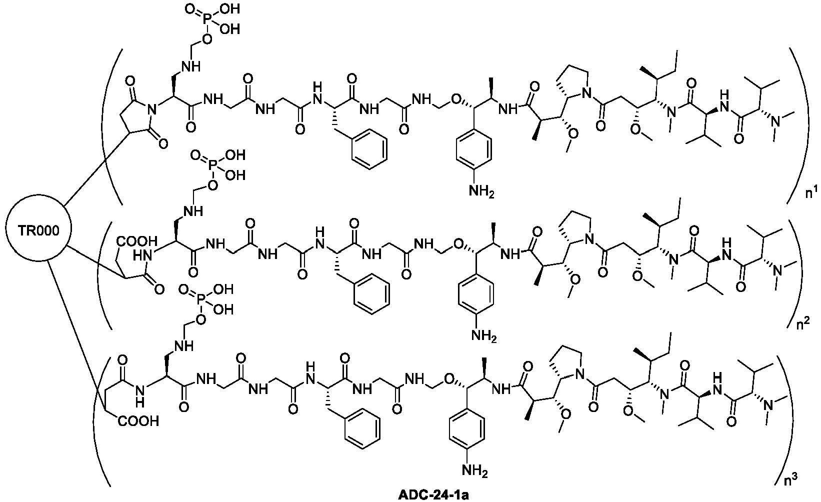

### Example 130 Preparation of ADC-25-1a

ADC-25-1a was prepared by combining the corresponding linker-payload (LP-32a, whose structure is shown in the structural formula of LP-32, wherein the configuration of the chiral carbon at position 2 is S-type) with the anti-Trop2 antibody TR000 according to the method of general step A.

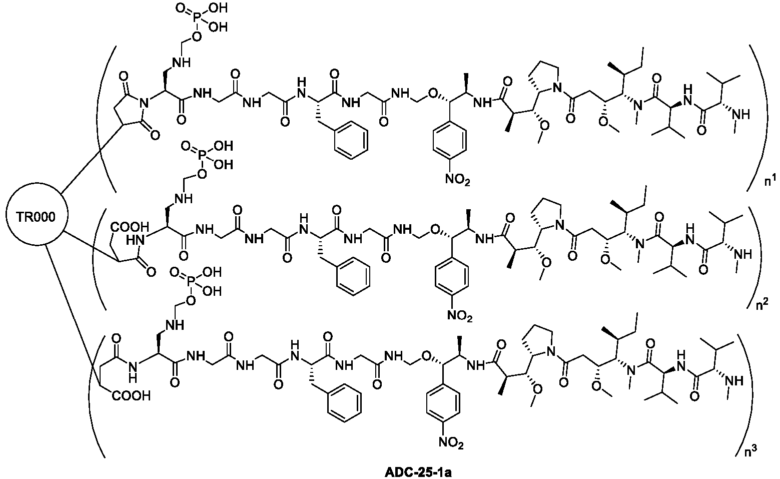

### Example 131 Preparation of ADC-26-1a

ADC-26-1a was prepared by combining the corresponding linker-payload (LP-33a, whose structure is shown in the structural formula of LP-33, wherein the configuration of the chiral carbon at position 2 is S-type) with the anti-Trop2 antibody TR000 according to the method of general step A.

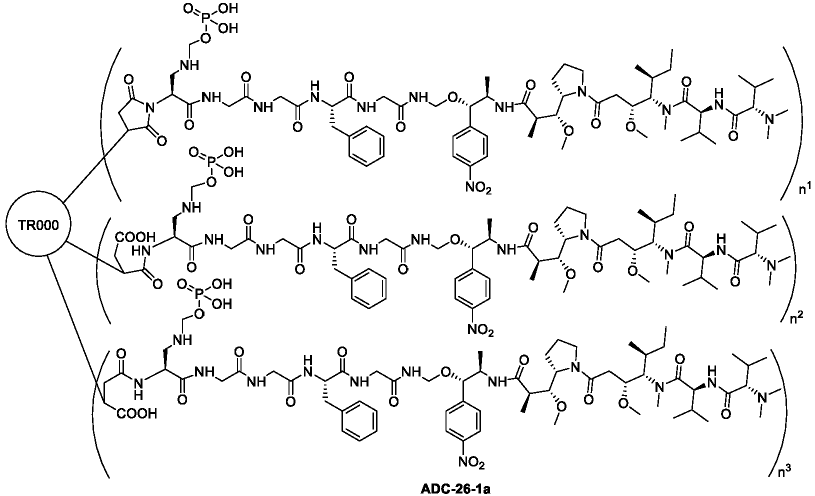

### Example 132 Preparation of ADC-27-1a

ADC-27-1a was prepared by combining the corresponding linker-payload (LP-35a, whose structure is shown in the structural formula of LP-35, wherein the configuration of the chiral carbon at position 2 is S-type) with the anti-Trop2 antibody TR000 according to the method of general step A.

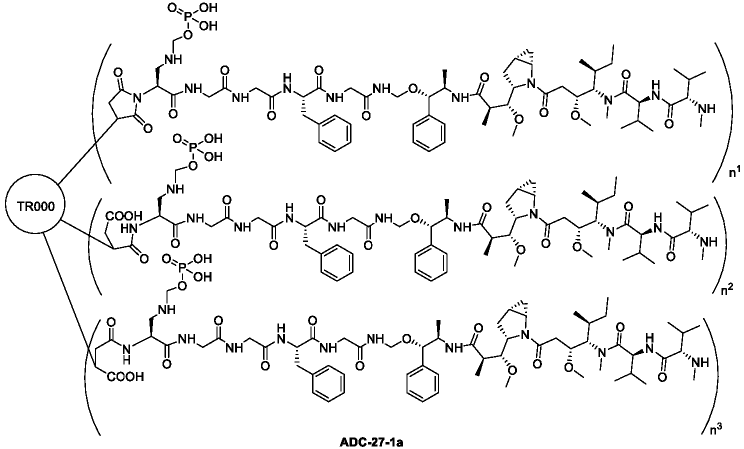

### Example 133 Preparation of ADC-28-1a

ADC-28-1a was prepared by combining the corresponding linker-payload (LP-36a, whose structure is shown in the structural formula of LP-36, wherein the configuration of the chiral carbon at position 2 is S-type) with the anti-Trop2 antibody TR000 according to the method of general step A.

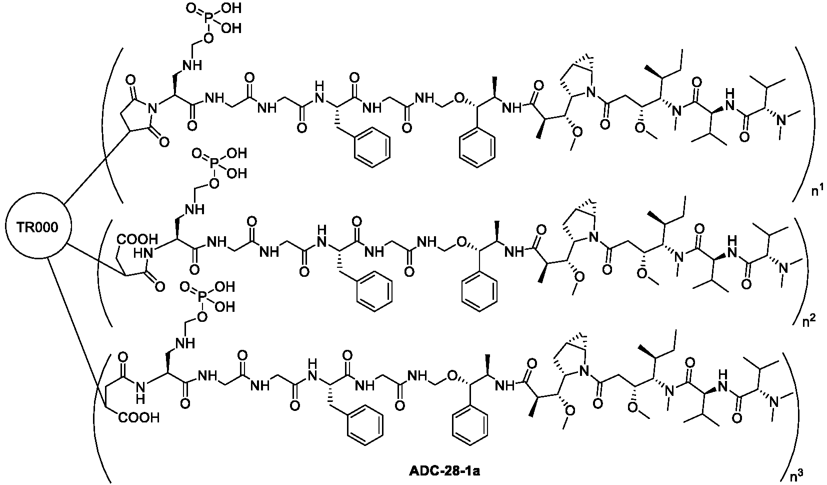

### Example 134 Preparation of ADC-29-1a

ADC-29-1a was prepared by combining the corresponding linker-payload (LP-39a, whose structure is shown in the structural formula of LP-39, wherein the configuration of the chiral carbon at position 2 is S-type) with the anti-Trop2 antibody TR000 according to the method of general step A.

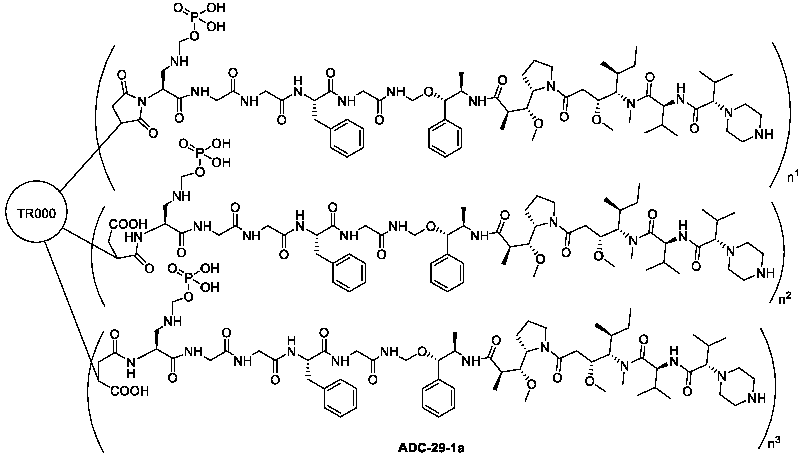

### Example 135 Preparation of ADC-30-1a

ADC-30-1a was prepared by combining the corresponding linker-payload (LP-34a, whose structure is shown in the structural formula of LP-34, wherein the configuration of the chiral carbon at position 2 is S-type) with the anti-Trop2 antibody TR000 according to the method of general step A.

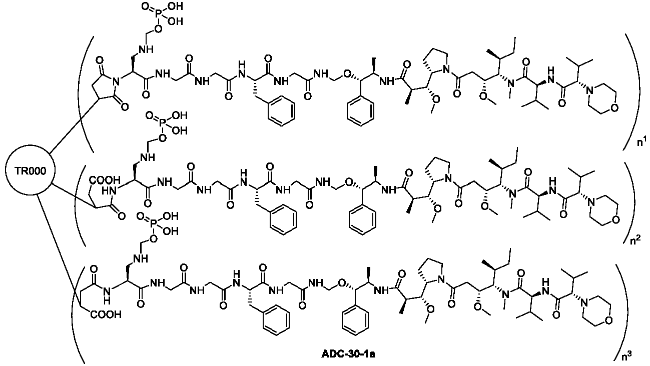

### Example 136 Preparation of ADC-31-1a

ADC-31-1a was prepared by combining the corresponding linker-payload (LP-19a, whose structure is shown in the structural formula of LP-19, wherein the configuration of the chiral carbon at position 2 is S-type) with the anti-Trop2 antibody TR000 according to the method of general step A.

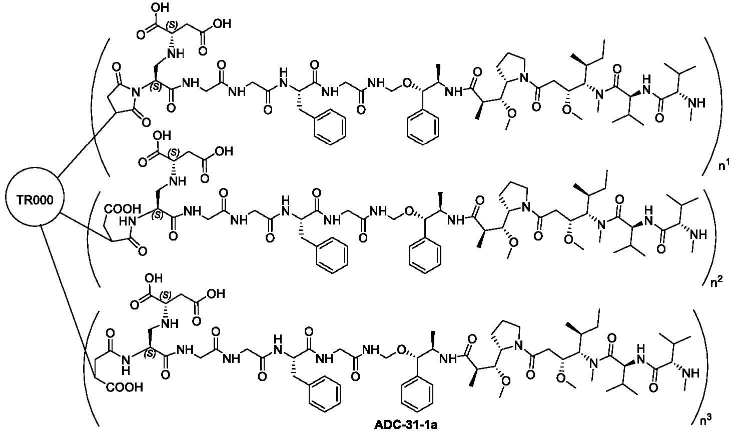

### Example 137 Preparation of ADC-35-1a

ADC-35-1a was prepared by combining the corresponding linker-payload (LP-22a, whose structure is shown in the structural formula of LP-22, wherein the configuration of the chiral carbon at position 2 is S-type) with the anti-Trop2 antibody TR000 according to the method of general step A.

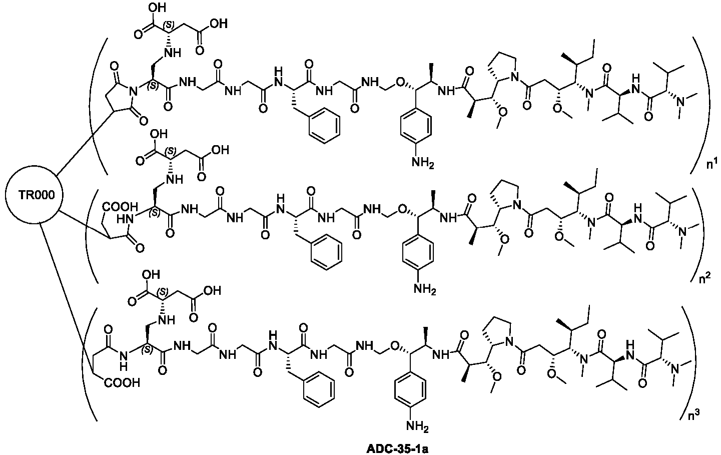

### Example 138 Preparation of ADC-39-1a

ADC-39-1a was prepared by combining the corresponding linker-payload (LP-26a, whose structure is shown in the structural formula of LP-26, wherein the configuration of the chiral carbon at position 2 is S-type) with the anti-Trop2 antibody TR000 according to the method of general step A.

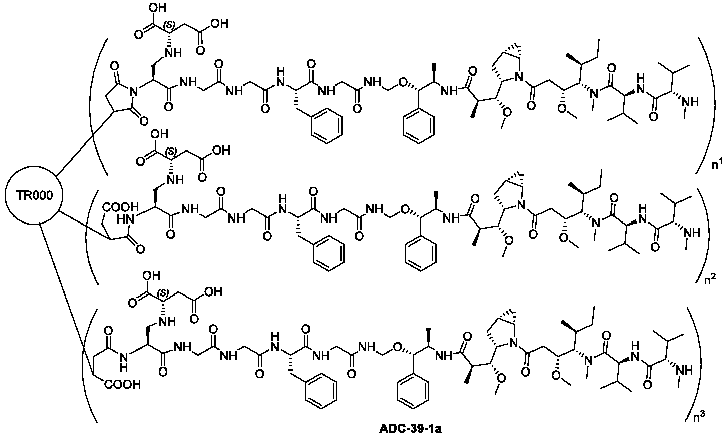

### Example 139 Preparation of ADC-45-1a

ADC-45-1a was prepared by combining the corresponding linker-payload (LP-20a, whose structure is shown in the structural formula of LP-20, wherein the configuration of the chiral carbon at position 2 is S-type) with the anti-Trop2 antibody TR000 according to the method of general step A.

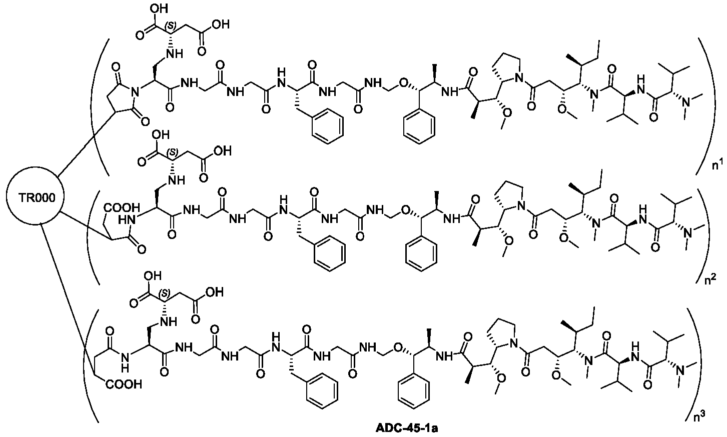

### Example 140 Preparation of ADC-48-1a

ADC-48-1a was prepared by combining the corresponding linker-payload (LP-27a, whose structure is shown in the structural formula of LP-27, wherein the configuration of the chiral carbon at position 2 is S-type) with the anti-Trop2 antibody TR000 according to the method of general step A.

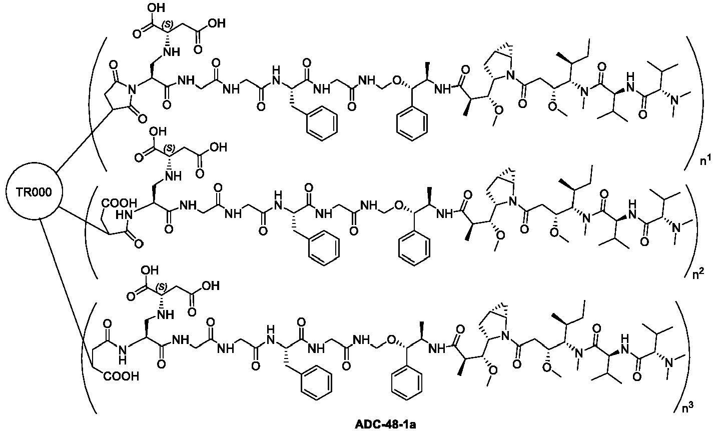

### Example 141 Preparation of ADC-1-2a

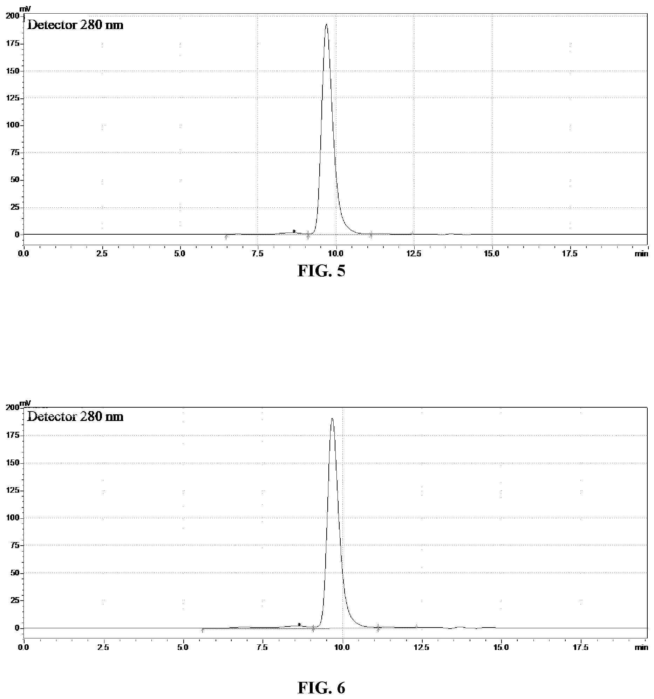

ADC-1-2a was prepared by reacting the corresponding linker-payload (LP-1a, whose structure is shown in the structural formula of LP-1, wherein the configuration of the chiral carbon at position 2 is S-type) with the anti-Trop2 antibody TR005 according to the method of general step B. The sequence information of TR005 is shown in Table 1. The SEC-HPLC test results are shown in Figure 5.

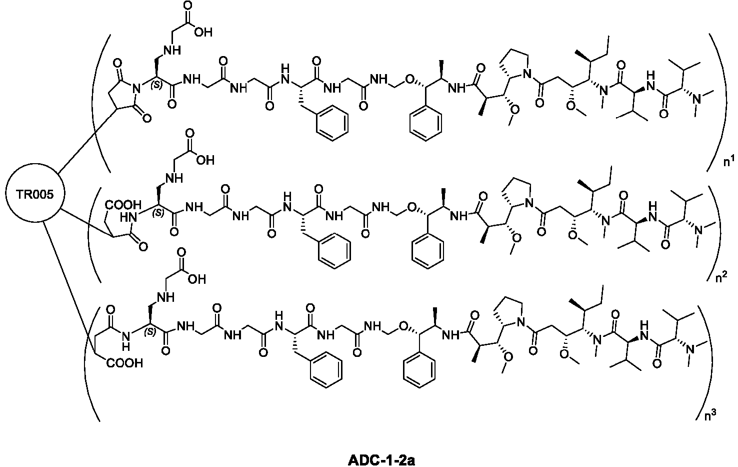

### Example 142 Preparation of ADC-2-2a

ADC-2-2a was prepared by combining the corresponding linker-payload (LP-2a, whose structure is shown in the structural formula of LP-2, wherein the configuration of the chiral carbon at position 2 is S-type) with the anti-Trop2 antibody TR005 according to the method of general step B. The SEC-HPLC test results are shown in Figure 6.

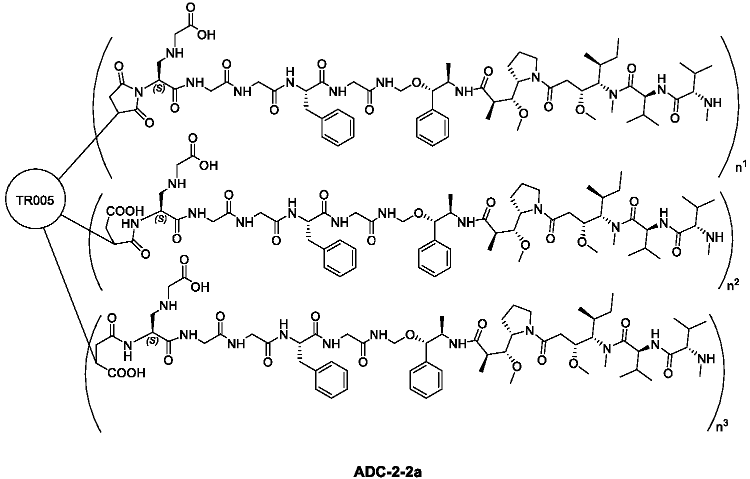

### Example 143 Preparation of ADC-C-2a

ADC-C-2a was prepared by combining compound VcMMAE with anti-Trop2 antibody TR005 according to the method of general step B.

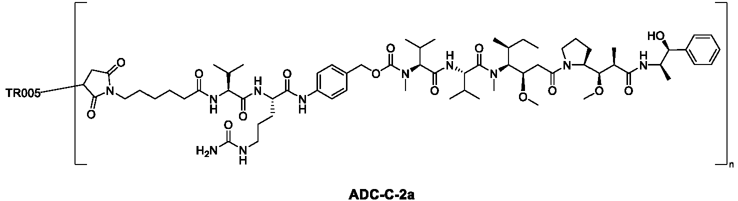

### Example 144 Preparation of ADC-3-2a

ADC-3-2a was prepared by combining the corresponding linker-payload (LP-10) with the anti-Trop2 antibody TR005 according to the method of general step B.

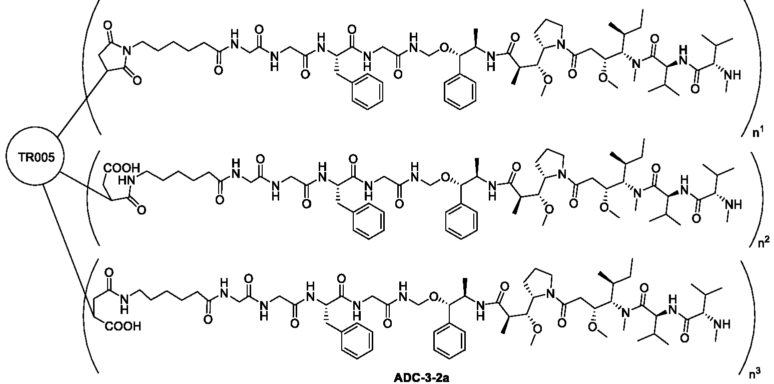

### Example 145 Preparation of ADC-4-2a

ADC-4-2a was prepared by combining the corresponding linker-payload (LP-37) with the anti-Trop2 antibody TR005 according to the method of general step B.

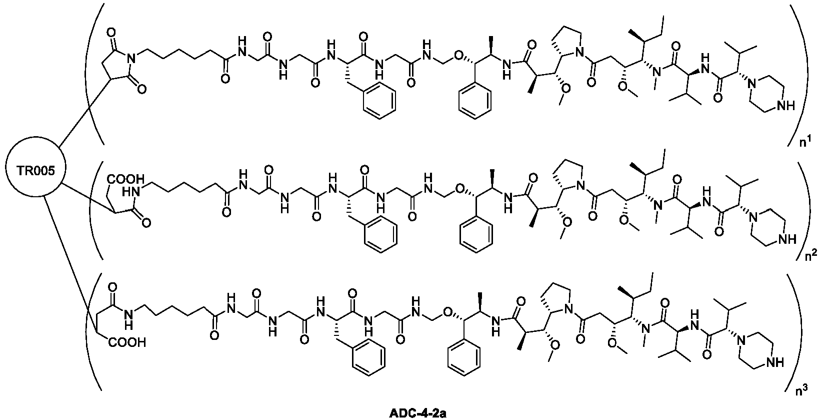

### Example 146 Preparation of ADC-5-2a

ADC-5-2a was prepared by combining the corresponding linker-payload (LP-16) with the anti-Trop2 antibody TR005 according to the method of general step B.

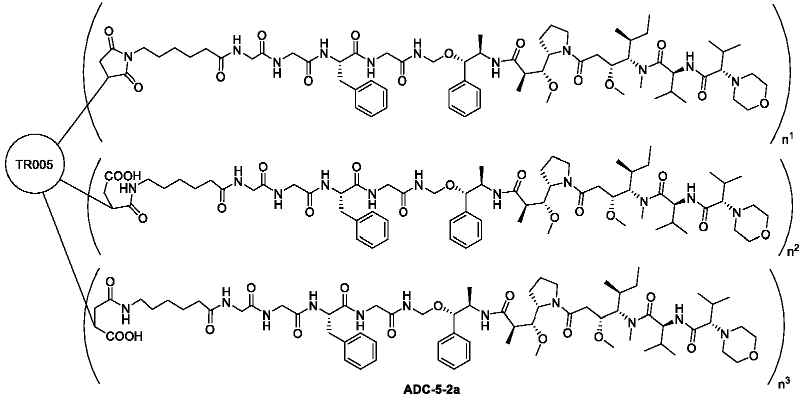

### Example 147 Preparation of ADC-6-2a

ADC-6-2a was prepared by combining the corresponding linker-payload (LP-11) with the anti-Trop2 antibody TR005 according to the method of general step B.

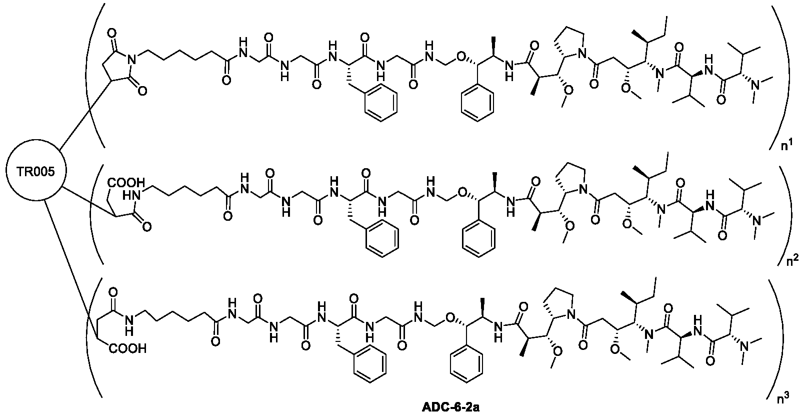

### Example 148 Preparation of ADC-7-2a

ADC-7-2a was prepared by combining the corresponding linker-payload (LP-12) with the anti-Trop2 antibody TR005 according to the method of general step B.

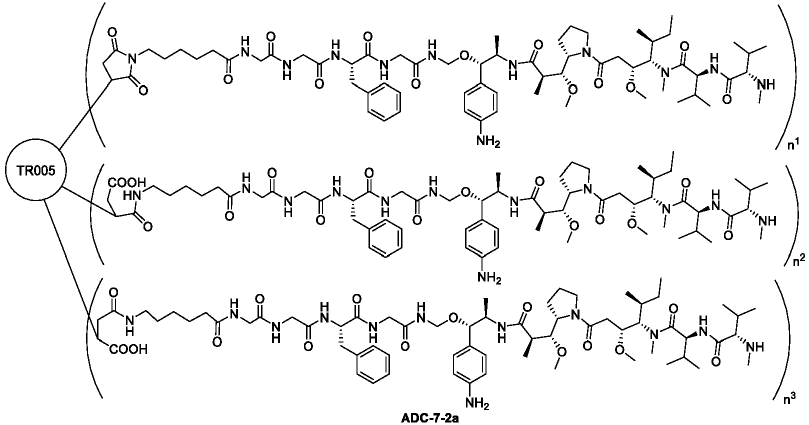

### Example 149 Preparation of ADC-8-2a

ADC-8-2a was prepared by combining the corresponding linker-payload (LP-13) with the anti-Trop2 antibody TR005 according to the method of general step B.

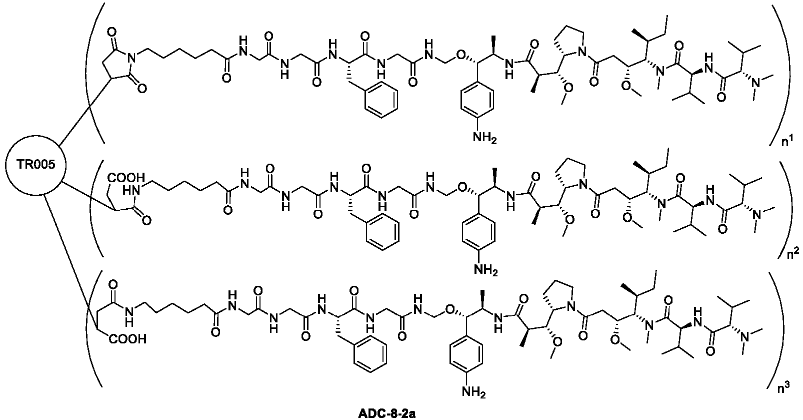

### Example 150 Preparation of ADC-9-2a

ADC-9-2a was prepared by combining the corresponding linker-payload (LP-14) with the anti-Trop2 antibody TR005 according to the method of general step B.

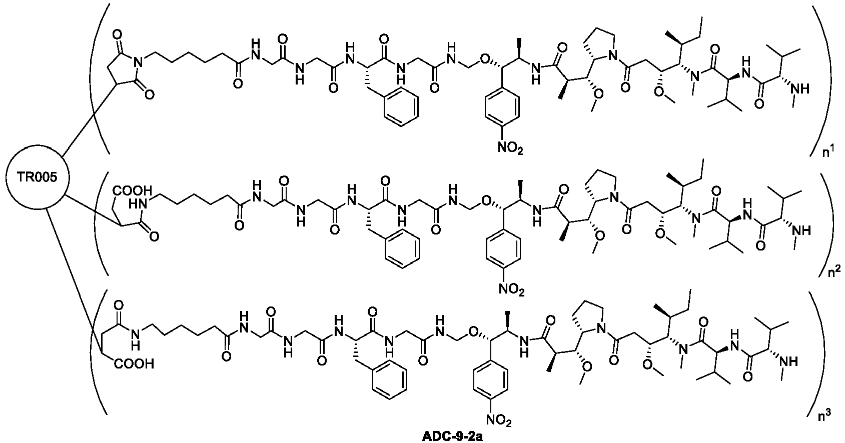

### Example 151 Preparation of ADC-10-2a

ADC-10-2a was prepared by combining the corresponding linker-payload (LP-15) with the anti-Trop2 antibody TR005 according to the method of general step B.

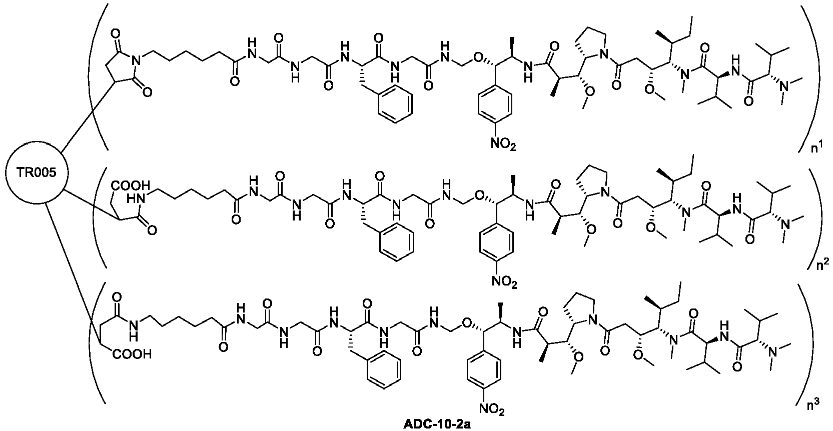

### Example 152 Preparation of ADC-11-2a

ADC-11-2a was prepared by combining the corresponding linker-payload (LP-17) with the anti-Trop2 antibody TR005 according to the method of general step B.

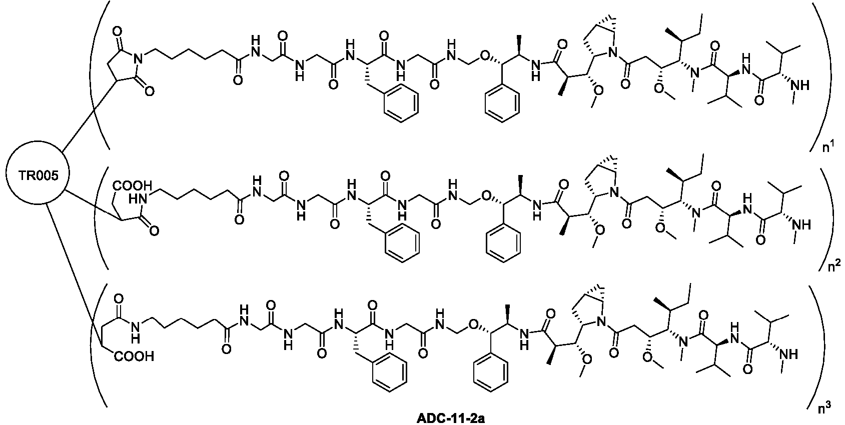

### Example 153 Preparation of ADC-12-2a

ADC-12-2a was prepared by combining the corresponding linker-payload (LP-18) with the anti-Trop2 antibody TR005 according to the method of general step B.

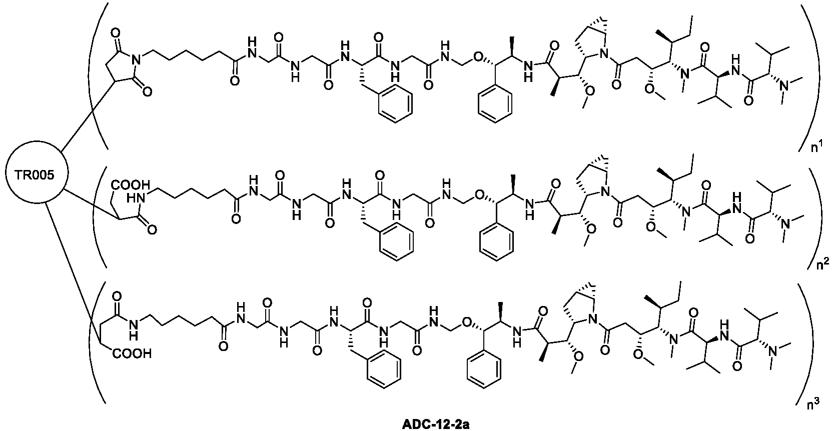

### Example 154 Preparation of ADC-13-2a

ADC-13-2a was prepared by combining the corresponding linker-payload (LP-3a, whose structure is shown in the structural formula of LP-3, wherein the configuration of the chiral carbon at position 2 is S-type) with the anti-Trop2 antibody TR005 according to the method of general step B.

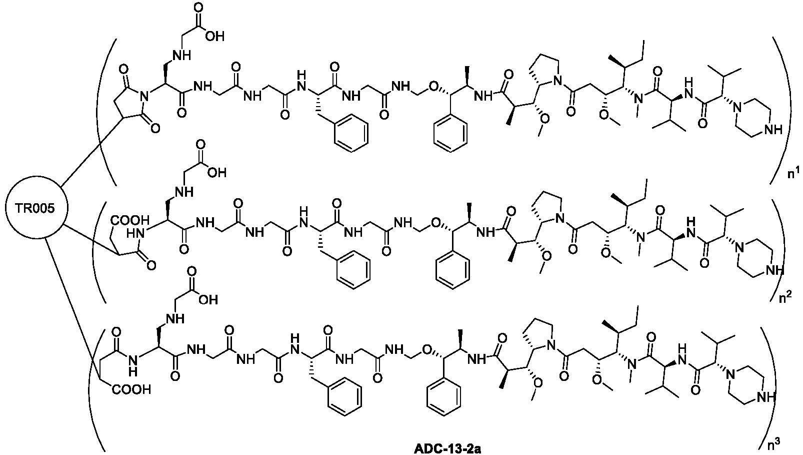

### Example 155 Preparation of ADC-14-2a

ADC-14-2a was prepared by combining the corresponding linker-payload (LP-7a, whose structure is shown in the structural formula of LP-7, wherein the configuration of the chiral carbon at position 2 is S-type) with the anti-Trop2 antibody TR005 according to the method of general step B.

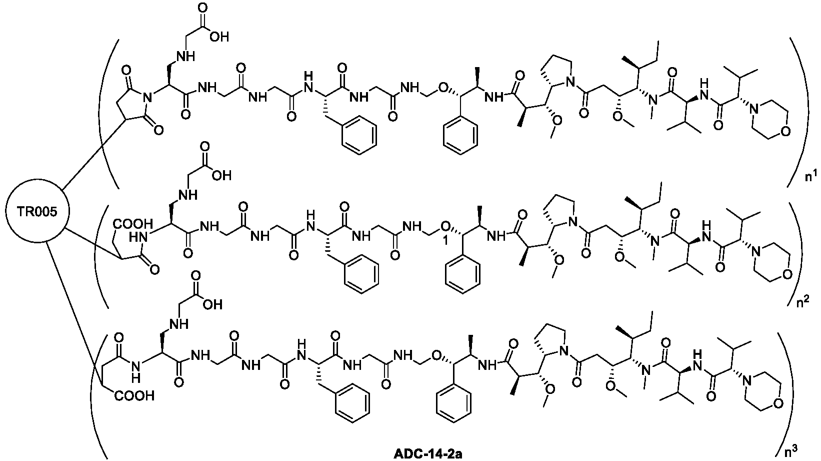

### Example 156 Preparation of ADC-15-2a

ADC-15-2a was prepared by reacting the corresponding linker-payload (LP-3sa, whose structure is shown in the structural formula of LP-3s, wherein the configuration of the chiral carbon at position 2 is S-type) with the anti-Trop2 antibody TR005 according to the method of general step B.

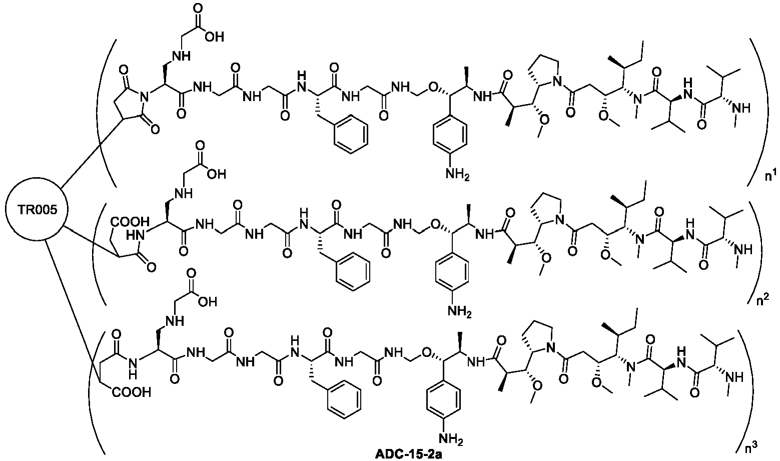

### Example 157 Preparation of ADC-16-2a

ADC-16-2a was prepared by combining the corresponding linker-payload (LP-4a, whose structure is shown in the structural formula of LP-4, wherein the configuration of the chiral carbon at position 2 is S-type) with the anti-Trop2 antibody TR005 according to the method of general step B.

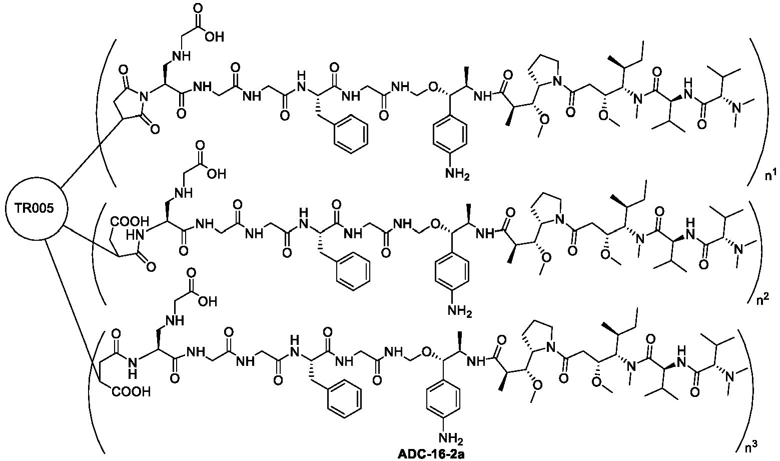

### Example 158 Preparation of ADC-17-2a

ADC-17-2a was prepared by reacting the corresponding linker-payload (LP-5a, whose structure is shown in the structural formula of LP-5, wherein the configuration of the chiral carbon at position 2 is S-type) with the anti-Trop2 antibody TR005 according to the method of general step B.

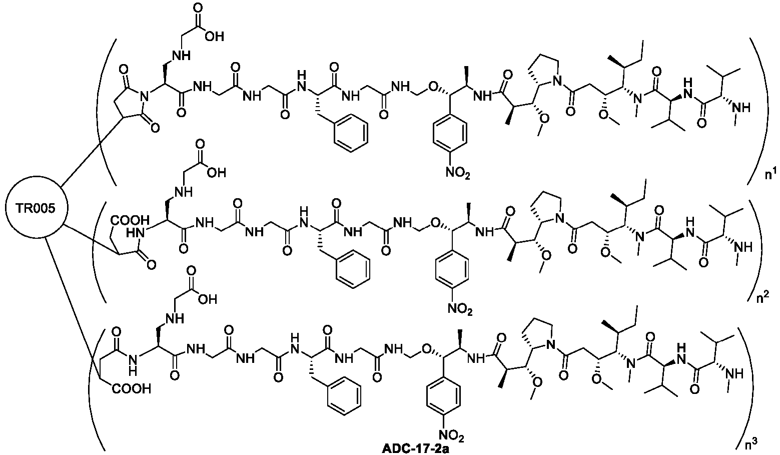

### Example 159 Preparation of ADC-18-2a

ADC-18-2a was prepared by combining the corresponding linker-payload (LP-6a, whose structure is shown in the structural formula of LP-6, wherein the configuration of the chiral carbon at position 2 is S-type) with the anti-Trop2 antibody TR005 according to the method of general step B.

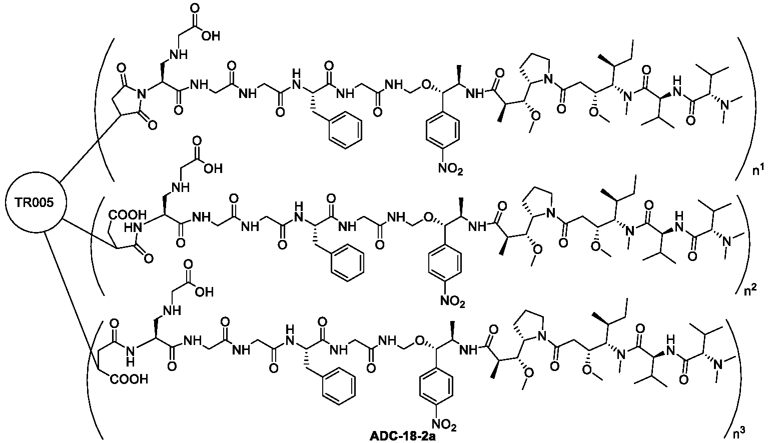

### Example 160 Preparation of ADC-19-2a

ADC-19-2a was prepared by combining the corresponding linker-payload (LP-8a, whose structure is shown in the structural formula of LP-8, wherein the configuration of the chiral carbon at position 2 is S-type) with the anti-Trop2 antibody TR005 according to the method of general step B.

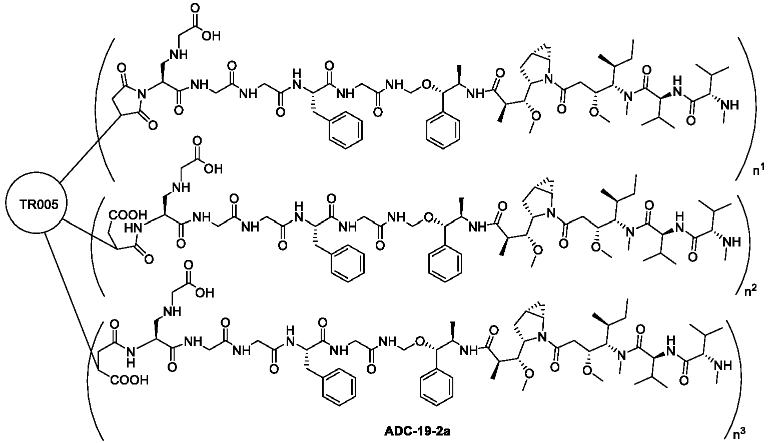

### Example 161 Preparation of ADC-20-2a

ADC-20-2a was prepared by combining the corresponding linker-payload (LP-9a, whose structure is shown in the structural formula of LP-9, wherein the configuration of the chiral carbon at position 2 is S-type) with the anti-Trop2 antibody TR005 according to the method of general step B.

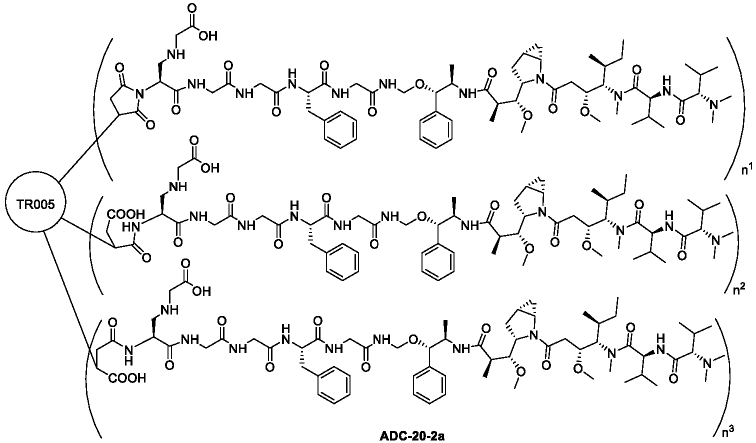

### Example 162 Preparation of ADC-21-2a

ADC-21-2a was prepared by reacting the corresponding linker-payload (LP-28a, whose structure is shown in the structural formula of LP-28, wherein the configuration of the chiral carbon at position 2 is S-type) with the anti-Trop2 antibody TR005 according to the method of general step B.

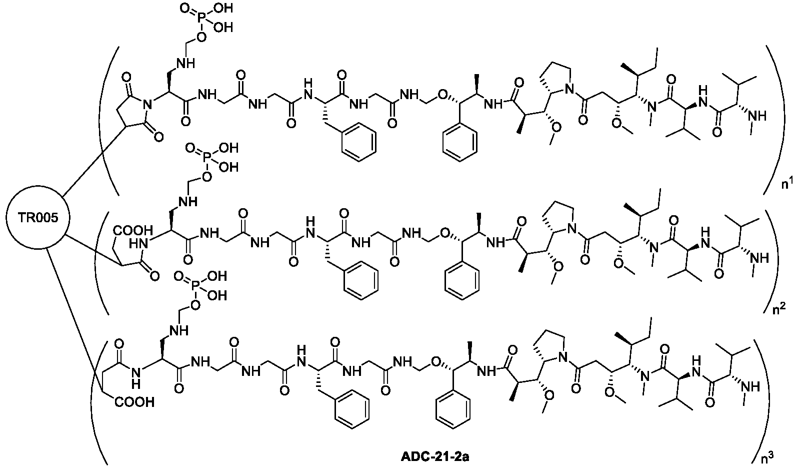

### Example 163 Preparation of ADC-22-2a

ADC-22-2a was prepared by reacting the corresponding linker-payload (LP-29a, whose structure is shown in the structural formula of LP-29, wherein the configuration of the chiral carbon at position 2 is S-type) with the anti-Trop2 antibody TR005 according to the method of general step B.

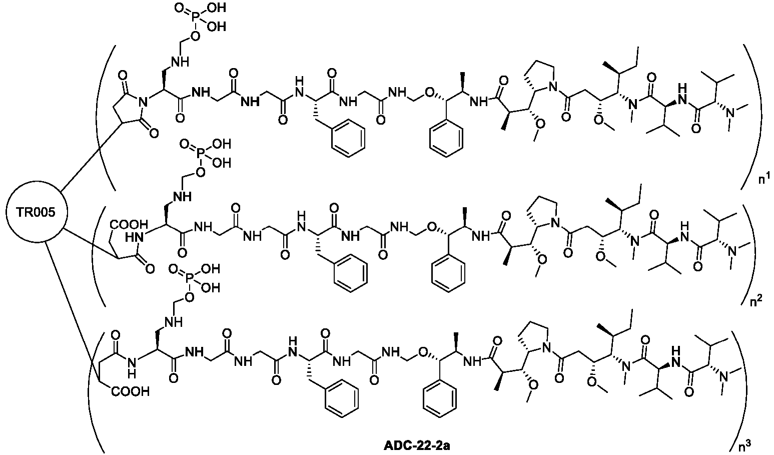

### Example 164 Preparation of ADC-23-2a

ADC-23-2a was prepared by reacting the corresponding linker-payload (LP-30a, whose structure is shown in the structural formula of LP-30, wherein the configuration of the chiral carbon at position 2 is S-type) with the anti-Trop2 antibody TR005 according to the method of general step B.

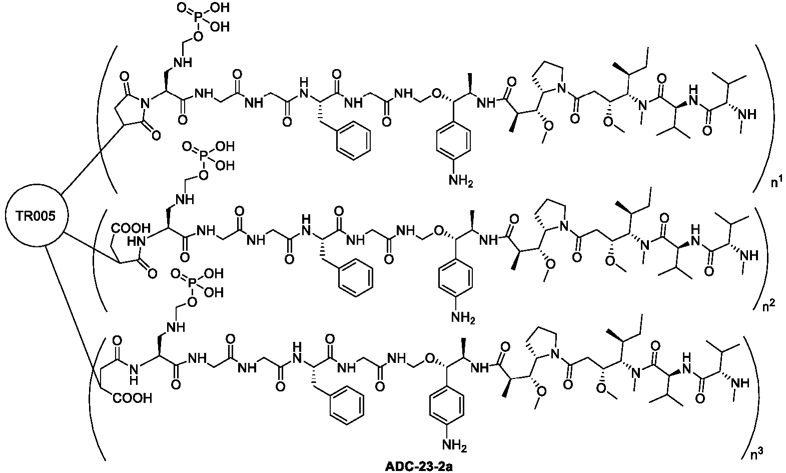

### Example 165 Preparation of ADC-24-2a

ADC-24-2a was prepared by combining the corresponding linker-payload (LP-31a, whose structure is shown in the structural formula ofLP-31, wherein the configuration of the chiral carbon at position 2 is S-type) with the anti-Trop2 antibody TR005 according to the method of general step B.

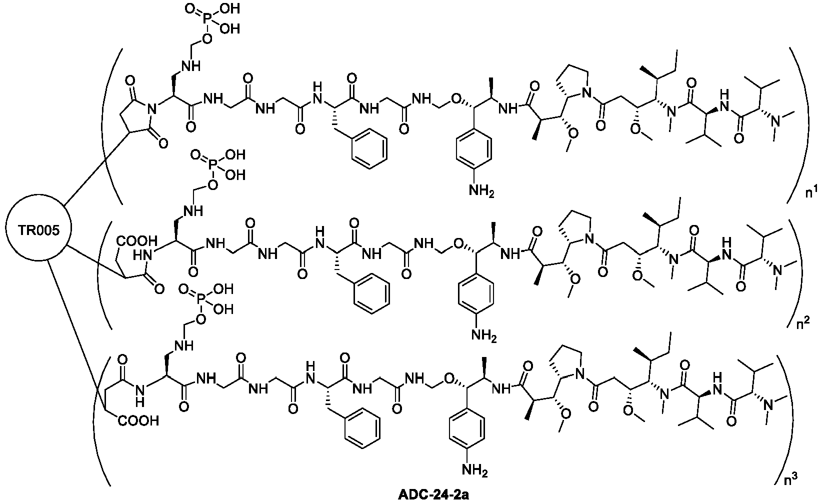

### Example 166 Preparation of ADC-25-2a

ADC-25-2a was prepared by combining the corresponding linker-payload (LP-32a, whose structure is shown in the structural formula of LP-32, wherein the configuration of the chiral carbon at position 2 is S-type) with the anti-Trop2 antibody TR005 according to the method of general step B.

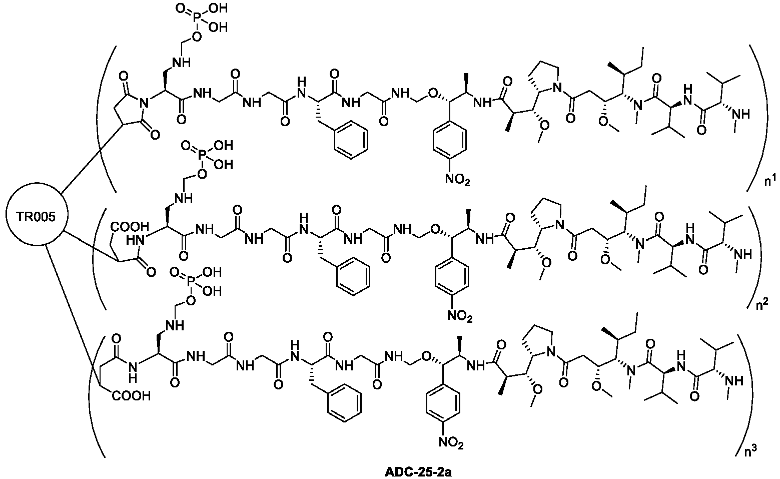

### Example 167 Preparation of ADC-26-2a

ADC-26-2a was prepared by combining the corresponding linker-payload (LP-33a, whose structure is shown in the structural formula of LP-33, wherein the configuration of the chiral carbon at position 2 is S-type) with the anti-Trop2 antibody TR005 according to the method of general step B.

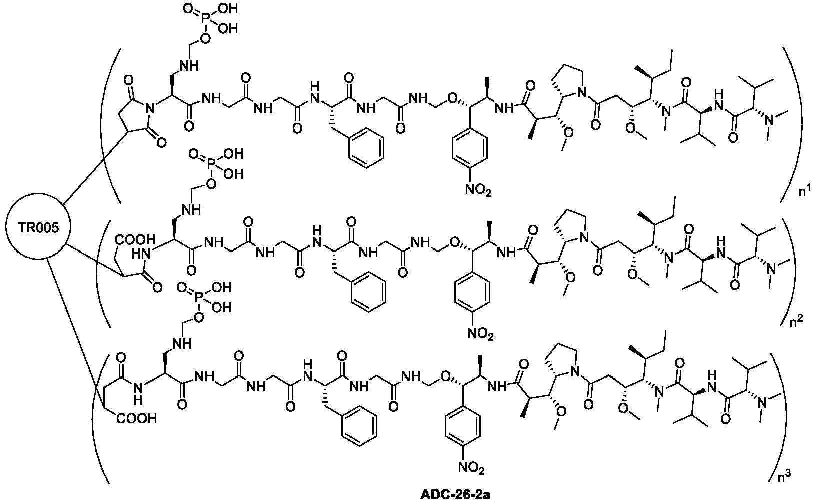

### Example 168 Preparation of ADC-27-2a

ADC-27-2a was prepared by combining the corresponding linker-payload (LP-35a, whose structure is shown in the structural formula of LP-35, wherein the configuration of the chiral carbon at position 2 is S-type) with the anti-Trop2 antibody TR005 according to the method of general step B.

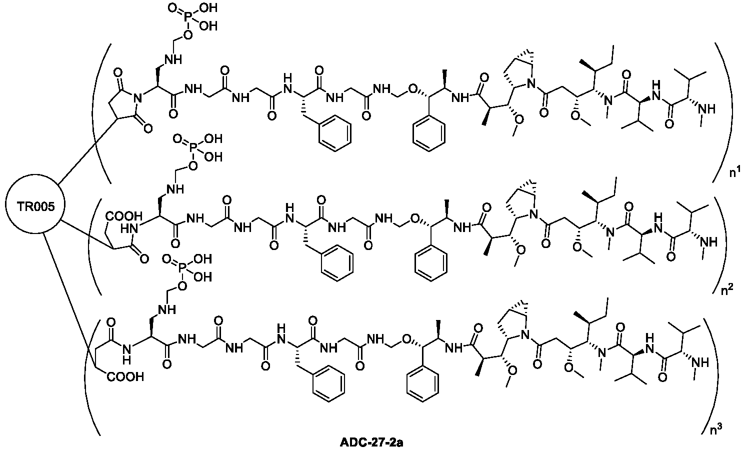

### Example 169 Preparation of ADC-28-2a

ADC-28-2a was prepared by combining the corresponding linker-payload (LP-36a, whose structure is shown in the structural formula of LP-36, wherein the configuration of the chiral carbon at position 2 is S-type) with the anti-Trop2 antibody TR005 according to the method of general step B.

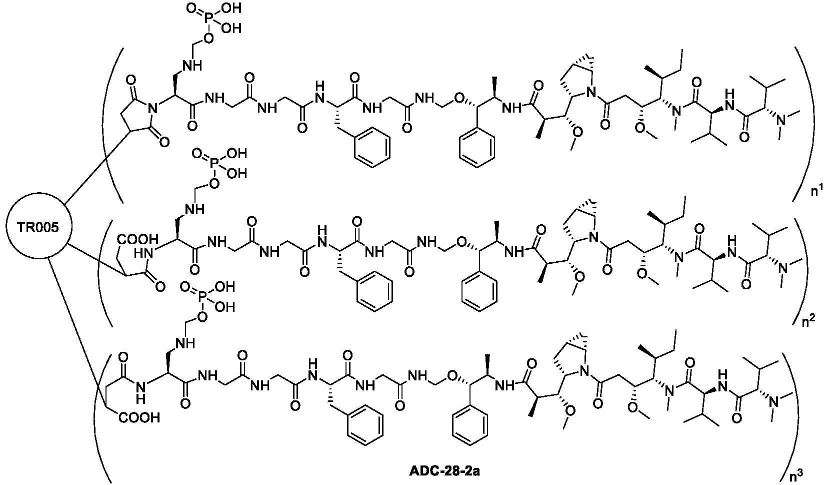

### Example 170 Preparation of ADC-29-2a

ADC-29-2a was prepared by reacting the corresponding linker-payload (LP-39a, whose structure is shown in the structural formula of LP-39, wherein the configuration of the chiral carbon at position 2 is S-type) with the anti-Trop2 antibody TR005 according to the method of general step B.

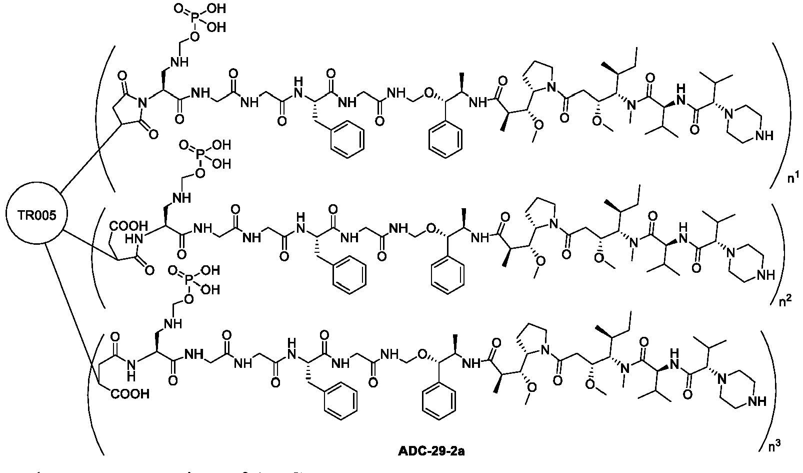

### Example 171 Preparation of ADC-30-2a

ADC-30-2a was prepared by combining the corresponding linker-payload (LP-34a, whose structure is shown in the structural formula of LP-34, wherein the configuration of the chiral carbon at position 2 is S-type) with the anti-Trop2 antibody TR005 according to the method of general step B.

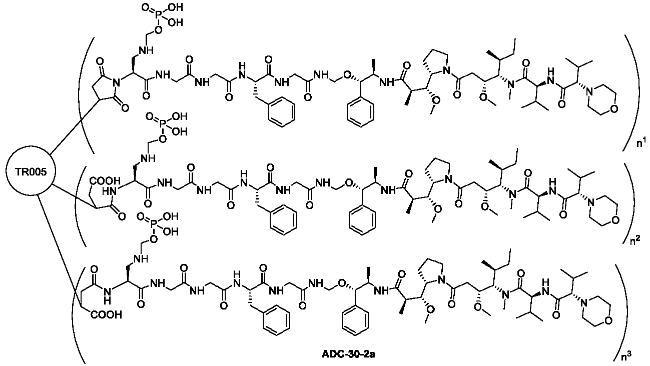

### Example 172 Preparation of ADC-31-2a

ADC-31-2a was prepared by combining the corresponding linker-payload (LP-19a, whose structure is shown in the structural formula of LP-19, wherein the configuration of the chiral carbon at position 2 is S-type) with the anti-Trop2 antibody TR005 according to the method of general step B.

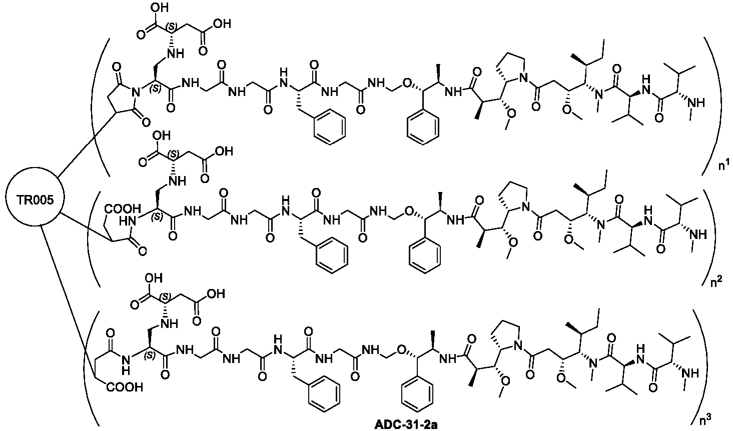

### Example 173 Preparation of ADC-35-2a

ADC-35-2a was prepared by combining the corresponding linker-payload (LP-22a, whose structure is shown in the structural formula of LP-22, wherein the configuration of the chiral carbon at position 2 is S-type) with the anti-Trop2 antibody TR005 according to the method of general step B.

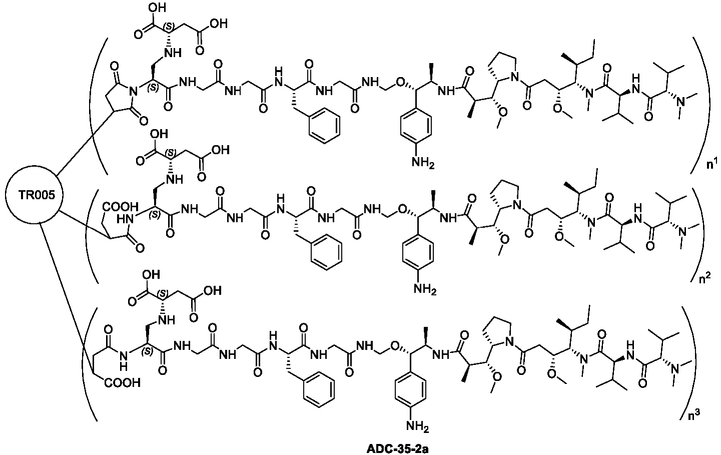

### Example 174 Preparation of ADC-39-2a

ADC-39-2a was prepared by combining the corresponding linker-payload (LP-26a, whose structure is shown in the structural formula of LP-26, wherein the configuration of the chiral carbon at position 2 is S-type) with the anti-Trop2 antibody TR005 according to the method of general step B.

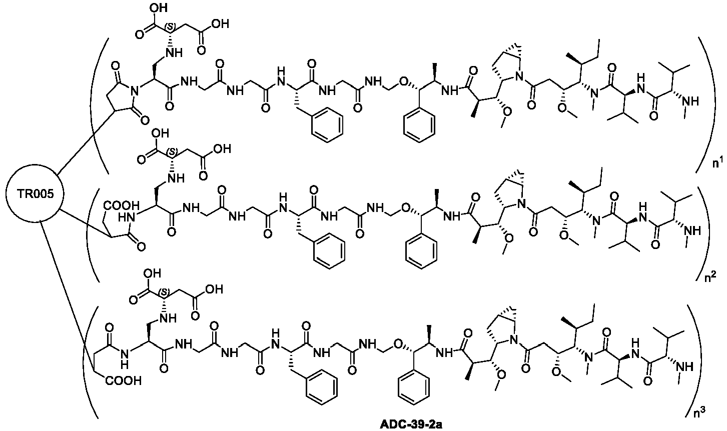

### Example 175 Preparation of ADC-45-2a

ADC-45-2a was prepared by combining the corresponding linker-payload (LP-20a, whose structure is shown in the structural formula of LP-20, wherein the configuration of the chiral carbon at position 2 is S-type) with the anti-Trop2 antibody TR005 according to the method of general step B.

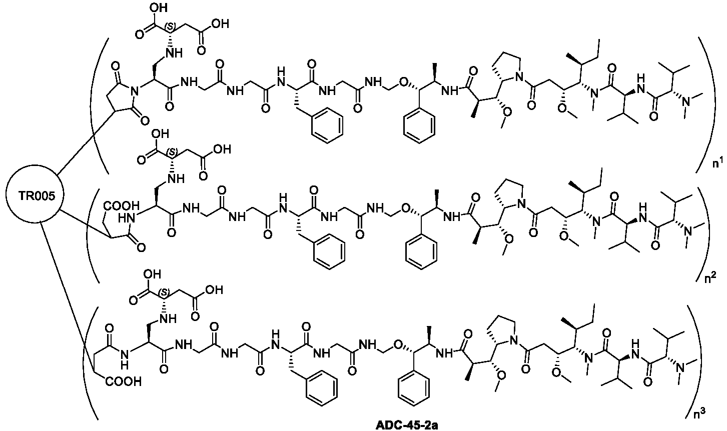

### Example 176 Preparation of ADC-48-2a

ADC-48-2a was prepared by combining the corresponding linker-payload (LP-27a, whose structure is shown in the structural formula of LP-27, wherein the configuration of the chiral carbon at position 2 is S-type) with the anti-Trop2 antibody TR005 according to the method of general step B.

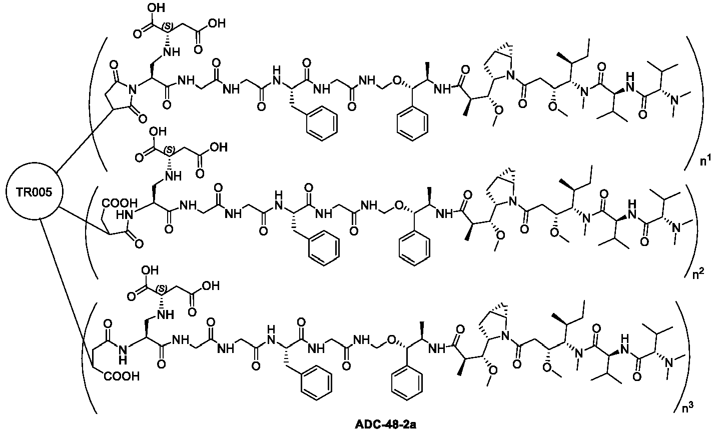

### Example 177 Preparation of ADC-1-3a

ADC-1-3a was prepared by reacting the corresponding linker-payload (LP-1a, whose structure is shown in the structural formula of LP-1, wherein the configuration of the chiral carbon at position 2 is S-type) with the anti-Trop2 antibody hu7F 11 according to the method of general step A. The sequence information of hu7F 11 is shown in Table 2.

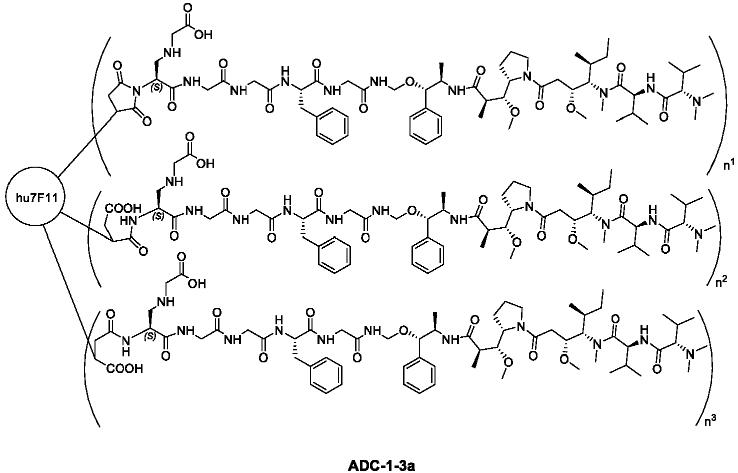

### Example 178 Preparation of ADC-2-3a

ADC-2-3a was prepared by combining the corresponding linker-payload (LP-2a, whose structure is shown in the structural formula of LP-2, wherein the configuration of the chiral carbon at position 2 is S-type) with the anti-Trop2 antibody hu7F 11 according to the method of general step A.

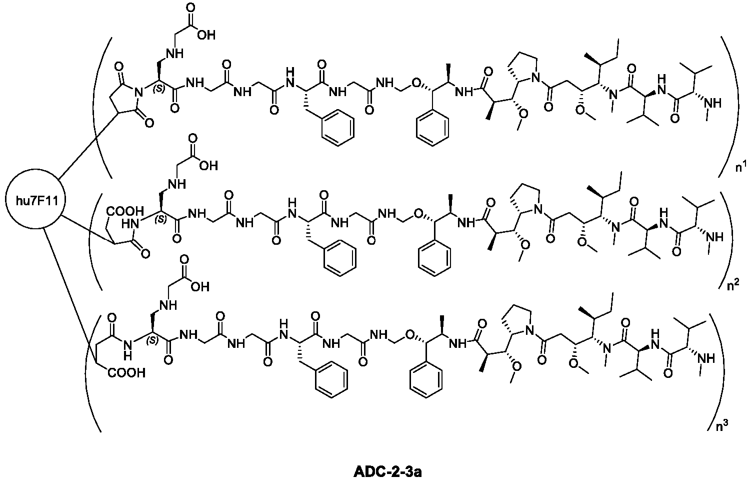

### Example 179 Preparation of ADC-C-3a

ADC-C-3a was prepared by combining compound VcMMAE with anti-Trop2 antibody hu7F 11 according to the method of general step A.

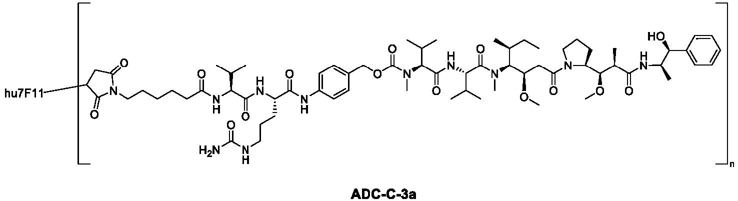

### Example 180 Preparation of ADC-3-3a

ADC-3-3a was prepared by combining the corresponding linker-payload (LP-10) with the anti-Trop2 antibody hu7F 11 according to the method of general step A.

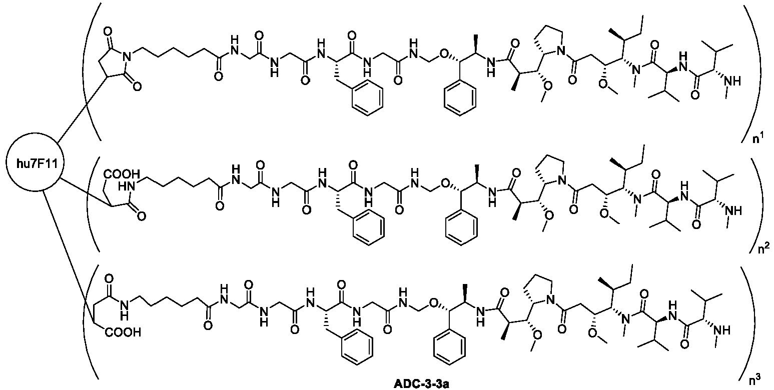

### Example 181 Preparation of ADC-4-3a

ADC-4-3a was prepared by combining the corresponding linker-payload (LP-37) with the anti-Trop2 antibody hu7F 11 according to the method of general step A.

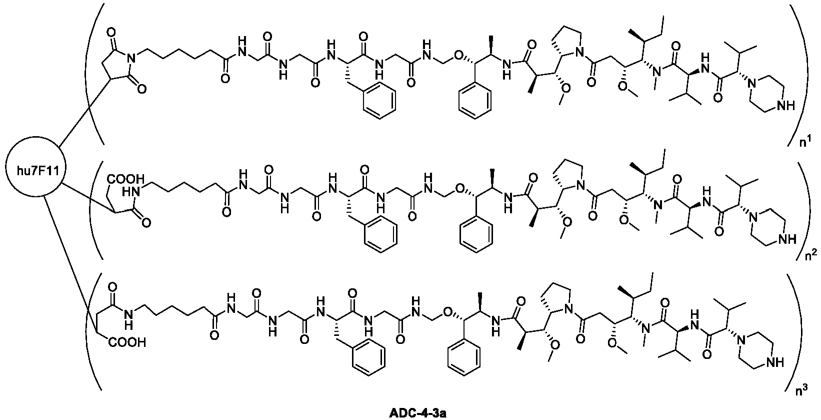

### Example 182 Preparation of ADC-5-3a

ADC-5-3a was prepared by combining the corresponding linker-payload (LP-16) with the anti-Trop2 antibody hu7F 11 according to the method of general step A.

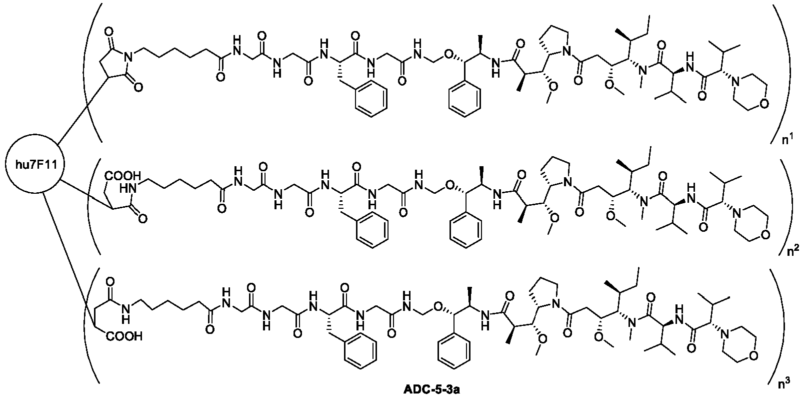

### Example 183 Preparation of ADC-6-3a

ADC-6-3a was prepared by combining the corresponding linker-payload (LP-11) with the anti-Trop2 antibody hu7F 11 according to the method of general step A.

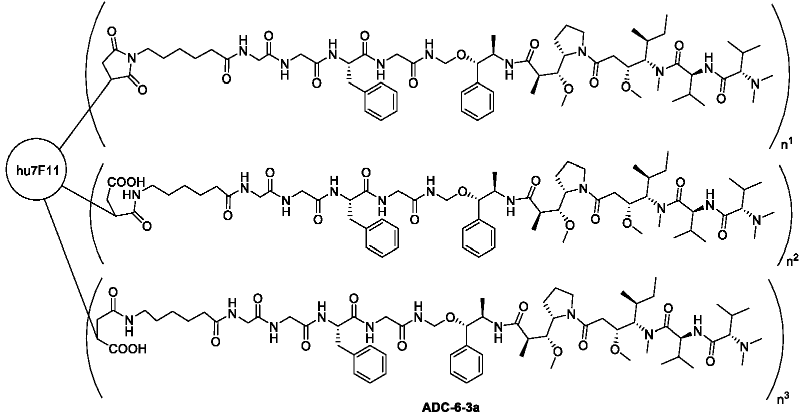

### Example 184 Preparation of ADC-7-3a

ADC-7-3a was prepared by combining the corresponding linker-payload (LP-12) with the anti-Trop2 antibody hu7F 11 according to the method of general step A.

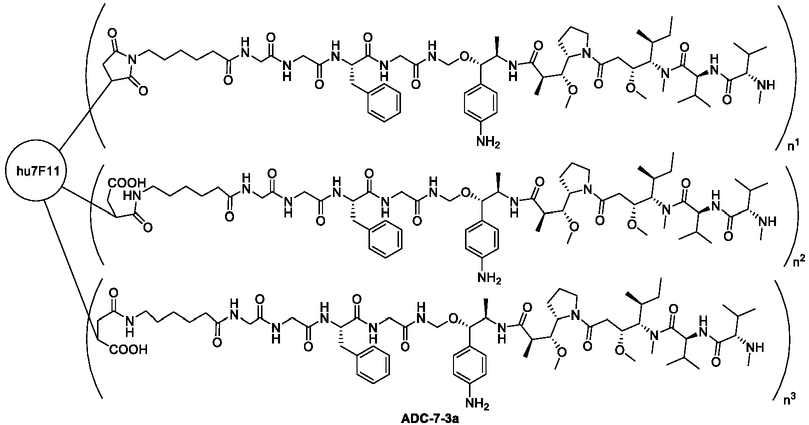

### Example 185 Preparation of ADC-8-3a

ADC-8-3a was prepared by combining the corresponding linker-payload (LP-13) with the anti-Trop2 antibody hu7F 11 according to the method of general step A.

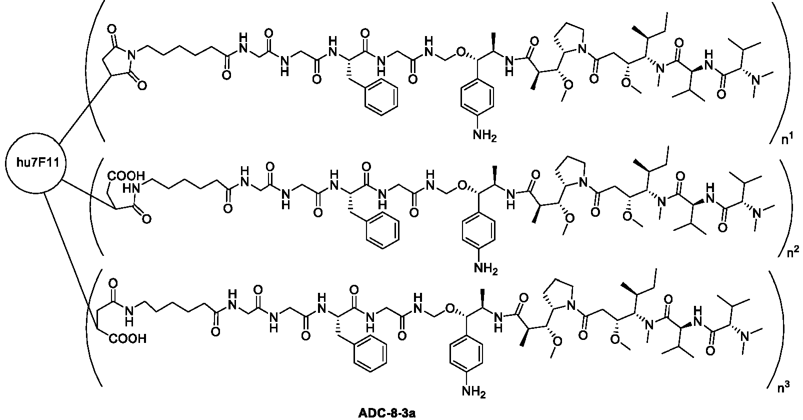

### Example 186 Preparation of ADC-9-3a

ADC-9-3a was prepared by combining the corresponding linker-payload (LP-14) with the anti-Trop2 antibody hu7F 11 according to the method of general step A.

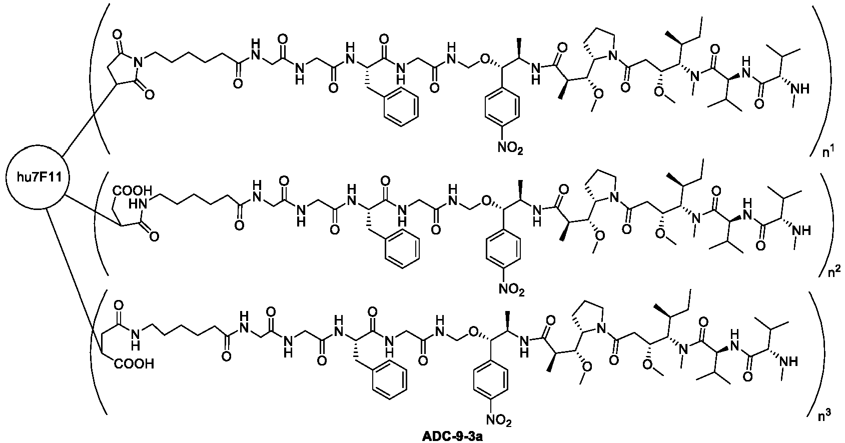

### Example 187 Preparation of ADC-10-3a

ADC-10-3a was prepared by combining the corresponding linker-payload (LP-15) with the anti-Trop2 antibody hu7F 11 according to the method of general step A.

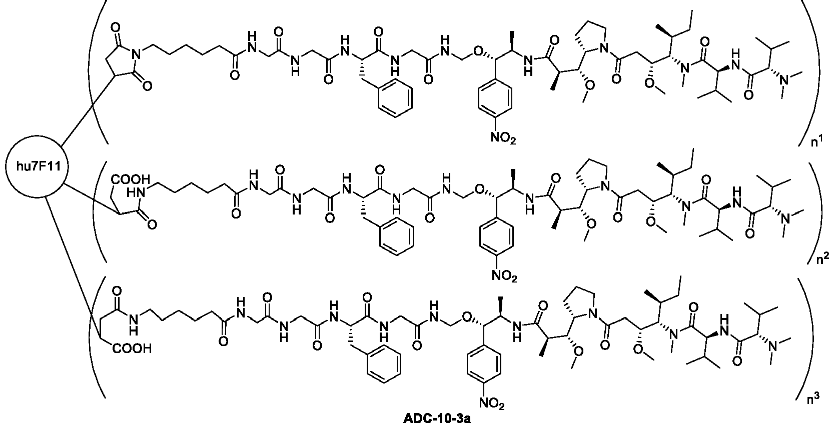

### Example 188 Preparation of ADC-11-3a

ADC-11-3a was prepared by combining the corresponding linker-payload (LP-17) with the anti-Trop2 antibody hu7F 11 according to the method of general step A.

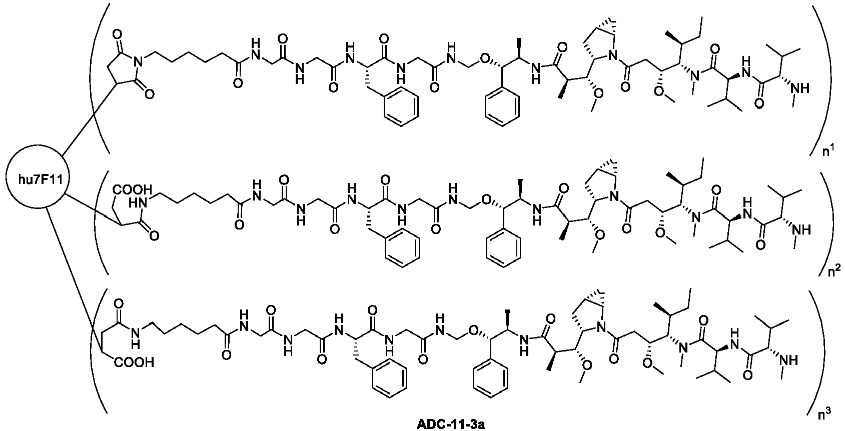

### Example 189 Preparation of ADC-12-3a

ADC-12-3a was prepared by combining the corresponding linker-payload (LP-18) with the anti-Trop2 antibody hu7F 11 according to the method of general step A.

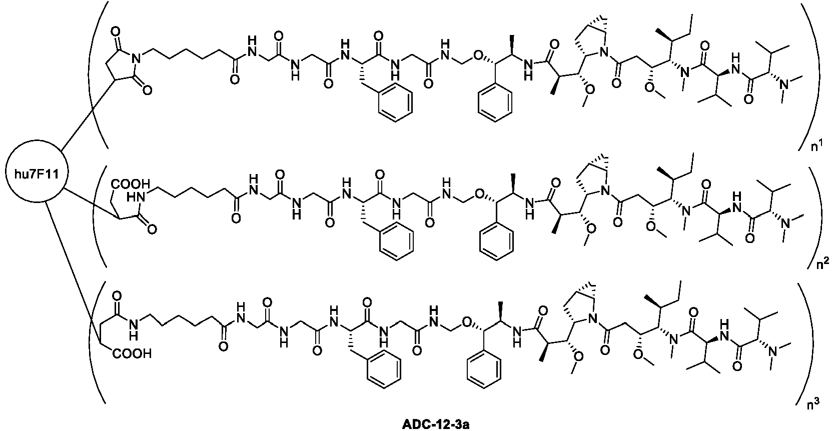

### Example 190 Preparation of ADC-13-3a

ADC-13-3a was prepared by combining the corresponding linker-payload (LP-3a, whose structure is shown in the structural formula of LP-3, wherein the configuration of the chiral carbon at position 2 is S-type) with the anti-Trop2 antibody hu7F 11 according to the method of general step A.

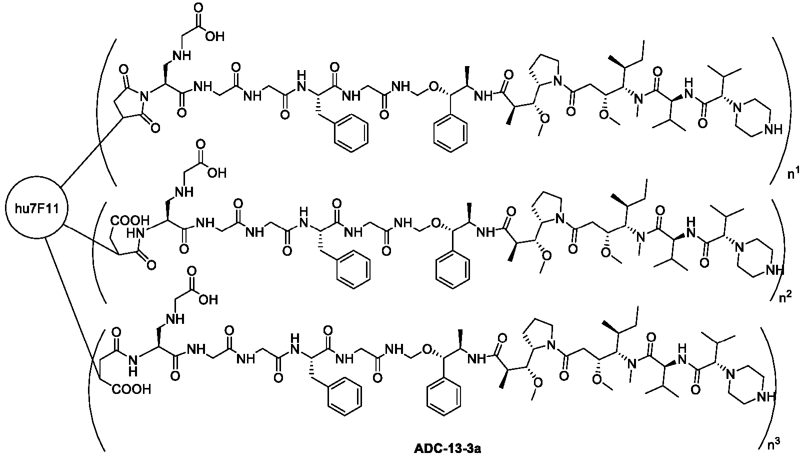

### Example 191 Preparation of ADC-14-3a

ADC-14-3a was prepared by combining the corresponding linker-payload (LP-7a, whose structure is shown in the structural formula of LP-7, wherein the configuration of the chiral carbon at position 2 is S-type) with the anti-Trop2 antibody hu7F 11 according to the method of general step A.

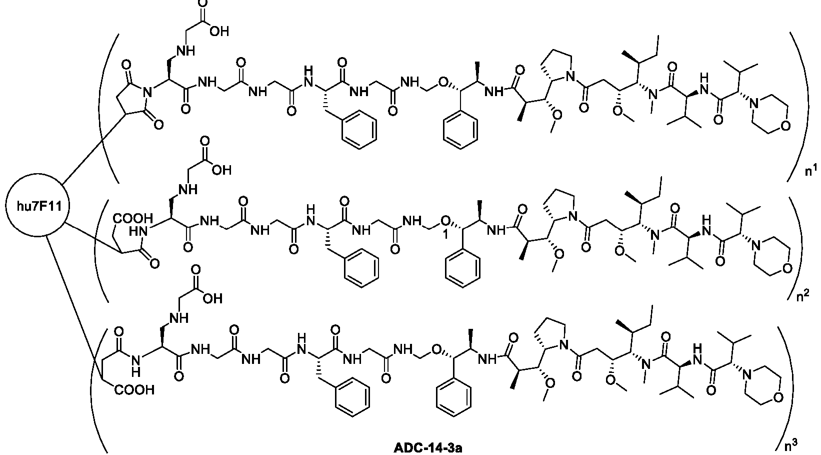

### Example 192 Preparation of Antibody Drug Conjugate ADC-15-3a

ADC-15-3a was prepared by reacting the corresponding linker-payload (LP-3sa, whose structure is shown in the structural formula of LP-3s, wherein the configuration of the chiral carbon at position 2 is S-type) with the anti-Trop2 antibody hu7F 11 according to the method of general step A.

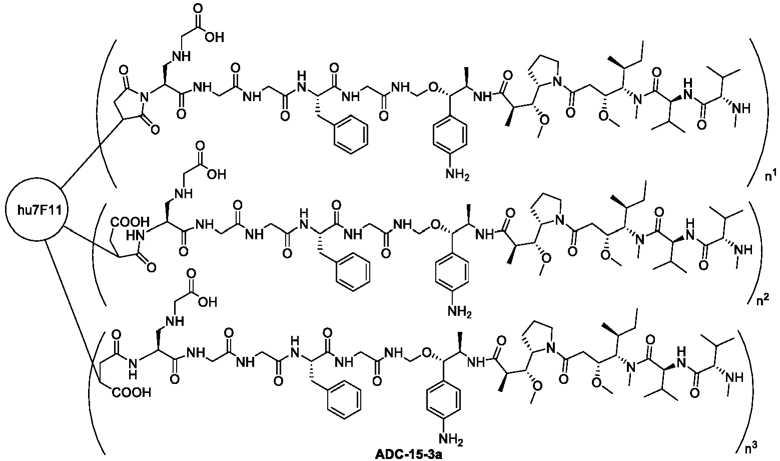

### Example 193 Preparation of ADC-16-3a

ADC-16-3a was prepared by combining the corresponding linker-payload (LP-4a, whose structure is shown in the structural formula of LP-4, wherein the configuration of the chiral carbon at position 2 is S-type) with the anti-Trop2 antibody hu7F 11 according to the method of general step A.

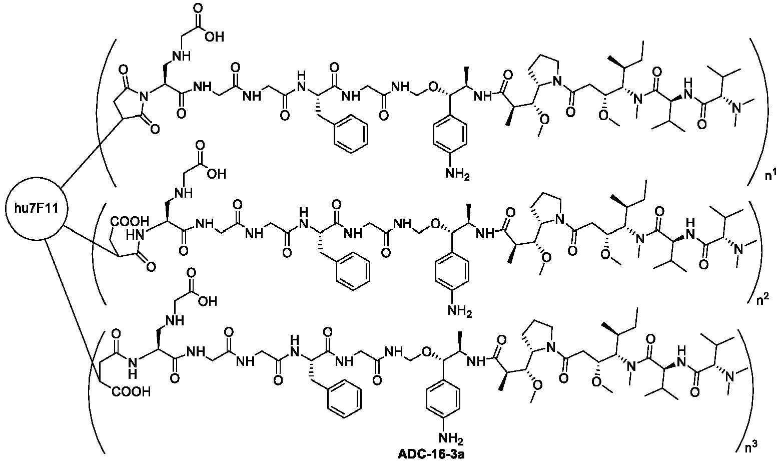

### Example 194 Preparation of ADC-17-3a

ADC-17-3a was prepared by reacting the corresponding linker-payload (LP-5a, whose structure is shown in the structural formula of LP-5, wherein the configuration of the chiral carbon at position 2 is S-type) with the anti-Trop2 antibody hu7F 11 according to the method of general step A.

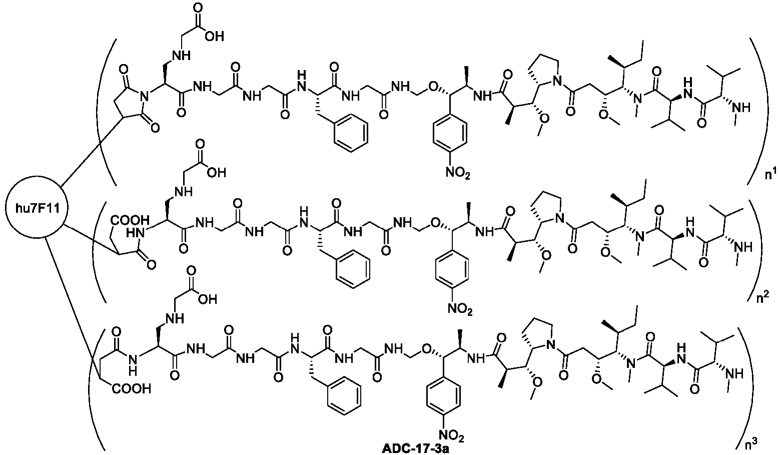

### Example 195 Preparation of ADC-18-3a

ADC-18-3a was prepared by combining the corresponding linker-payload (LP-6a, whose structure is shown in the structural formula of LP-6, wherein the configuration of the chiral carbon at position 2 is S-type) with the anti-Trop2 antibody hu7F 11 according to the method of general step A.

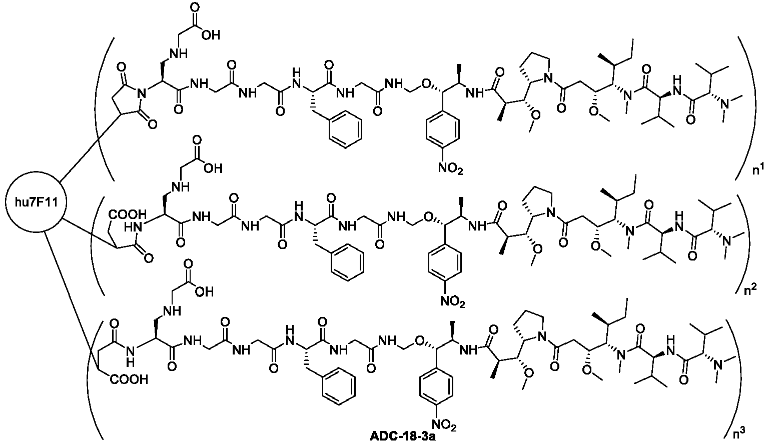

### Example 196 Preparation of ADC-19-3a

ADC-19-3a was prepared by combining the corresponding linker-payload (LP-8a, whose structure is shown in the structural formula of LP-8, wherein the configuration of the chiral carbon at position 2 is S-type) with the anti-Trop2 antibody hu7F 11 according to the method of general step A.

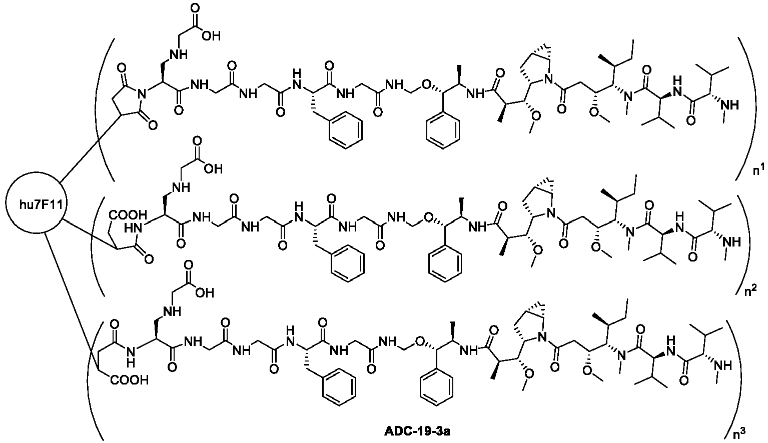

### Example 197 Preparation of ADC-20-3a

ADC-20-3a was prepared by combining the corresponding linker-payload (LP-9a, whose structure is shown in the structural formula of LP-9, wherein the configuration of the chiral carbon at position 2 is S-type) with the anti-Trop2 antibody hu7F 11 according to the method of general step A.

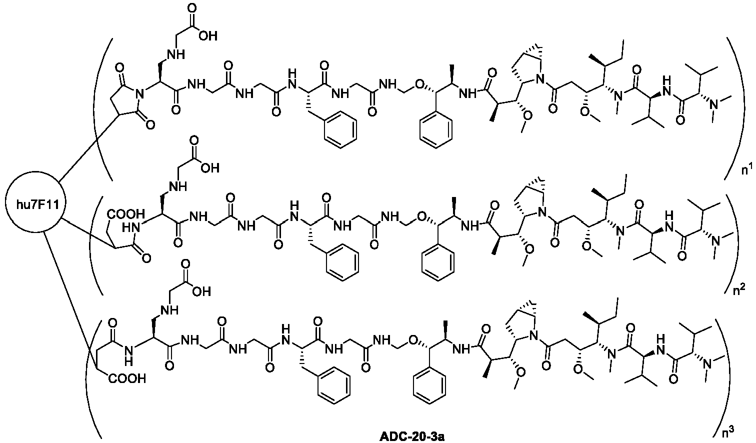

### Example 198 Preparation of ADC-21-3a

ADC-21-3a was prepared by reacting the corresponding linker-payload (LP-28a, whose structure is shown in the structural formula of LP-28, wherein the configuration of the chiral carbon at position 2 is S-type) with the anti-Trop2 antibody hu7F 11 according to the method of general step A.

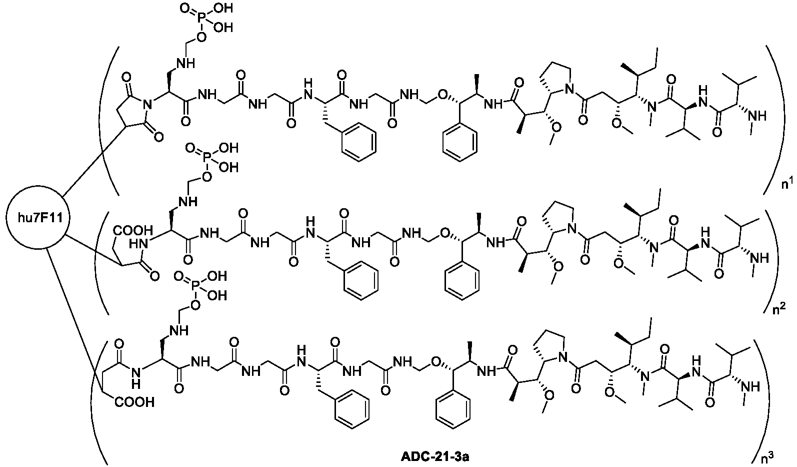

### Example 199 Preparation of antibody drug conjugate ADC-22-3a

ADC-22-3a was prepared by reacting the corresponding linker-payload (LP-29a, whose structure is shown in the structural formula of LP-29, wherein the configuration of the chiral carbon at position 2 is S-type) with the anti-Trop2 antibody hu7F 11 according to the method of general step A.

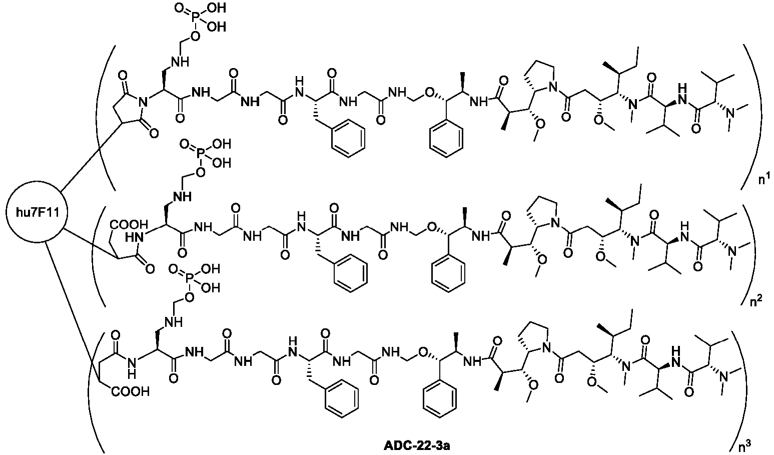

### Example 200 Preparation of ADC-23-3a

ADC-23-3a was prepared by reacting the corresponding linker-payload (LP-30a, whose structure is shown in the structural formula of LP-30, wherein the configuration of the chiral carbon at position 2 is S-type) with the anti-Trop2 antibody hu7F 11 according to the method of general step A.

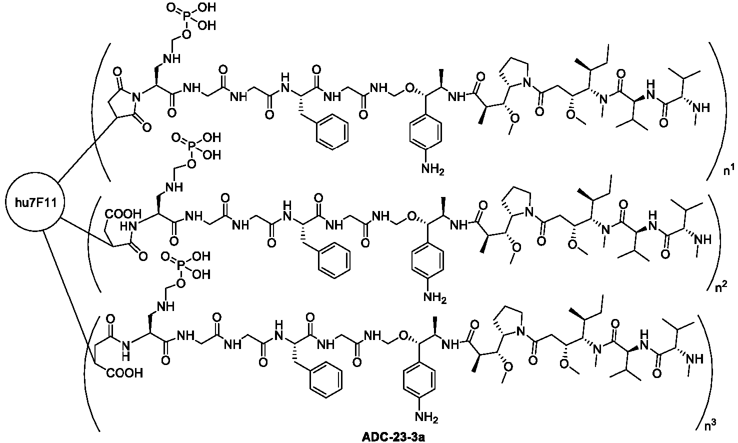

### Example 201 Preparation of ADC-24-3a

ADC-24-3a was prepared by combining the corresponding linker-payload (LP-31a, whose structure is shown in the structural formula ofLP-31, wherein the configuration of the chiral carbon at position 2 is S-type) with the anti-Trop2 antibody hu7F11 according to the method of general step A.

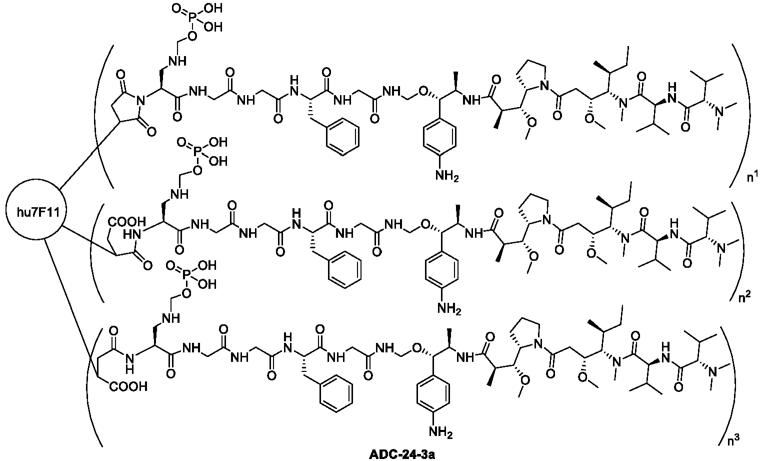

### Example 202 Preparation of ADC-25-3a

ADC-25-3a was prepared by combining the corresponding linker-payload (LP-32a, whose structure is shown in the structural formula of LP-32, wherein the configuration of the chiral carbon at position 2 is S-type) with the anti-Trop2 antibody hu7F 11 according to the method of general step A.

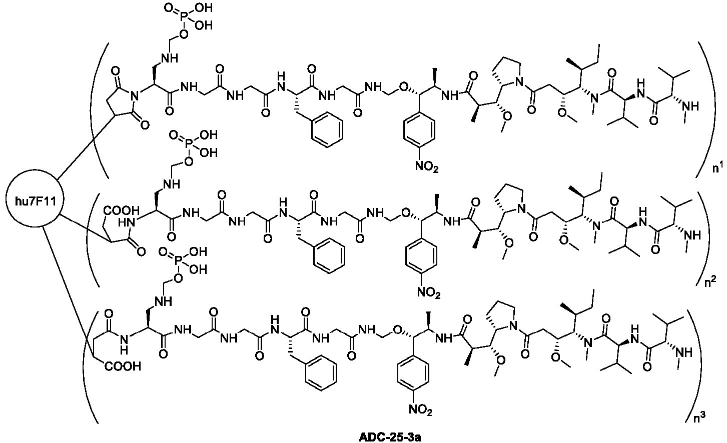

### Example 203 Preparation of ADC-26-3a

ADC-26-3a was prepared by combining the corresponding linker-payload (LP-33a, whose structure is shown in the structural formula of LP-33, wherein the configuration of the chiral carbon at position 2 is S-type) with the anti-Trop2 antibody hu7F 11 according to the method of general step A.

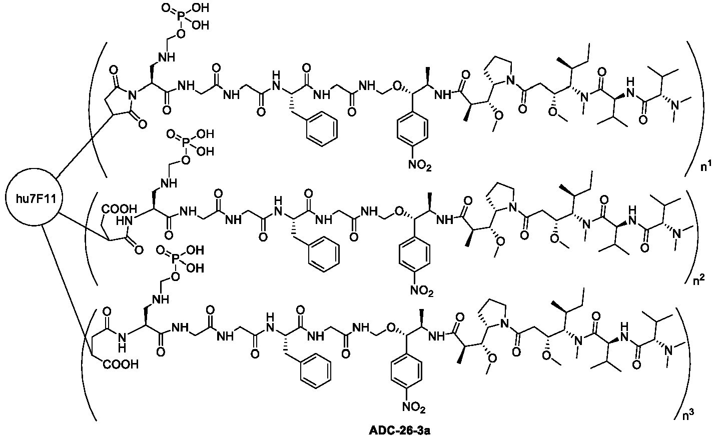

### Example 204 Preparation of ADC-27-3a

ADC-27-3a was prepared by combining the corresponding linker-payload (LP-35a, whose structure is shown in the structural formula of LP-35, wherein the configuration of the chiral carbon at position 2 is S-type) with the anti-Trop2 antibody hu7F 11 according to the method of general step A.

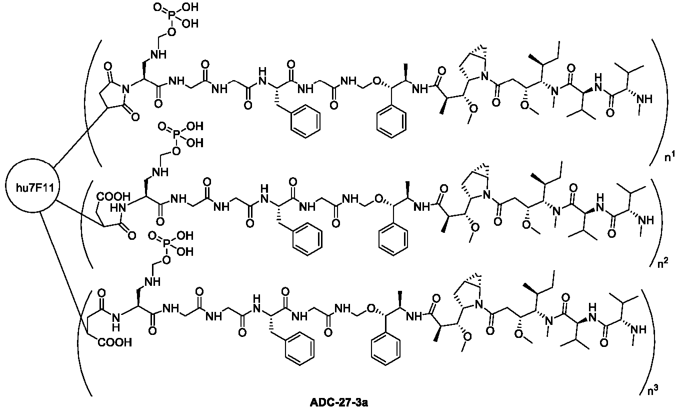

### Example 205 Preparation of ADC-28-3a

ADC-28-3a was prepared by combining the corresponding linker-payload (LP-36a, whose structure is shown in the structural formula of LP-36, wherein the configuration of the chiral carbon at position 2 is S-type) with the anti-Trop2 antibody hu7F 11 according to the method of general step A.

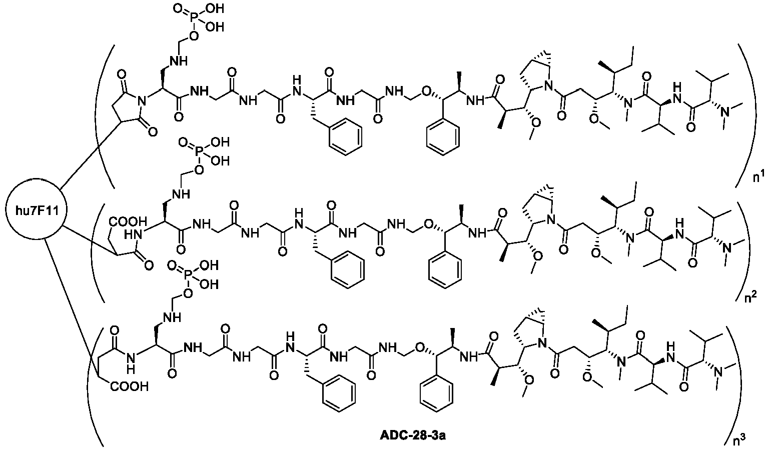

### Example 206 Preparation of ADC-29-3a

ADC-29-3a was prepared by reacting the corresponding linker-payload (LP-39a, whose structure is shown in the structural formula of LP-39, wherein the configuration of the chiral carbon at position 2 is S-type) with the anti-Trop2 antibody hu7F 11 according to the method of general step A.

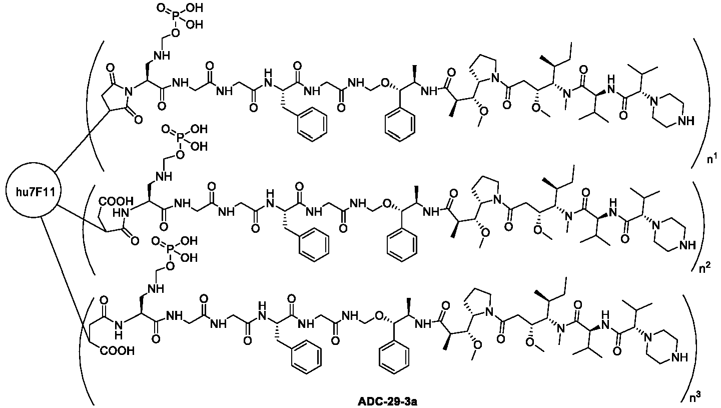

### Example 207 Preparation of ADC-30-3a

ADC-30-3a was prepared by combining the corresponding linker-payload (LP-34a, whose structure is shown in the structural formula of LP-34, wherein the configuration of the chiral carbon at position 2 is S-type) with the anti-Trop2 antibody hu7F 11 according to the method of general step A.

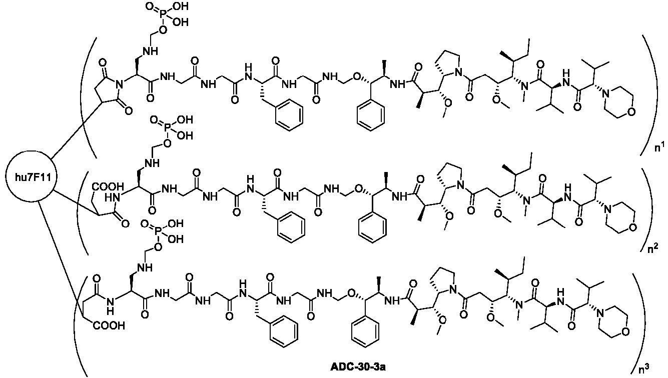

### Example 208 Preparation of antibody drug conjugate ADC-31-3a

ADC-31-3a was prepared by combining the corresponding linker-payload (LP-19a, whose structure is shown in the structural formula of LP-19, wherein the configuration of the chiral carbon at position 2 is S-type) with the anti-Trop2 antibody hu7F11 according to the method of general step A.

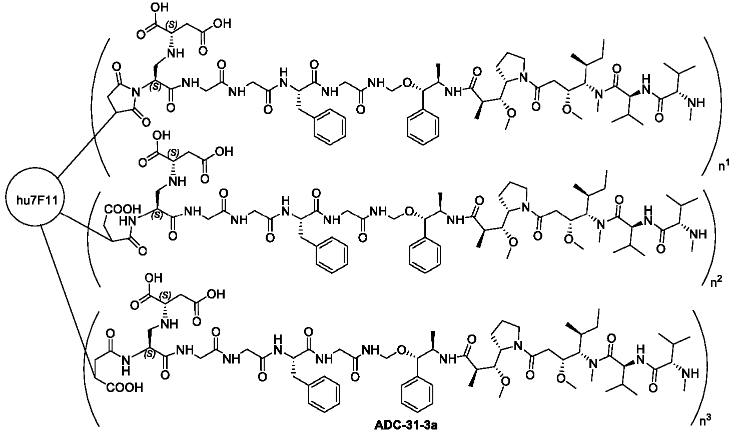

### Example 209 Preparation of ADC-35-3a

ADC-35-3a was prepared by combining the corresponding linker-payload (LP-22a, whose structure is shown in the structural formula of LP-22, wherein the configuration of the chiral carbon at position 2 is S-type) with the anti-Trop2 antibody hu7F 11 according to the method of general step A.

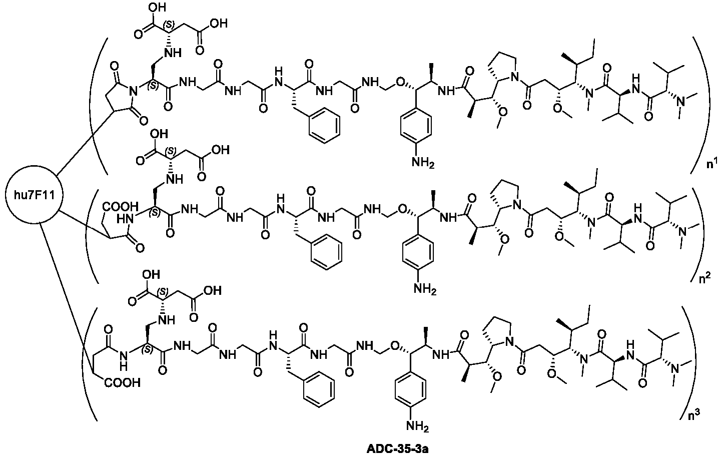

### Example 210 Preparation of ADC-39-3a

ADC-39-3a was prepared by combining the corresponding linker-payload (LP-26a, whose structure is shown in the structural formula of LP-26, wherein the configuration of the chiral carbon at position 2 is S-type) with the anti-Trop2 antibody hu7F 11 according to the method of general step A.

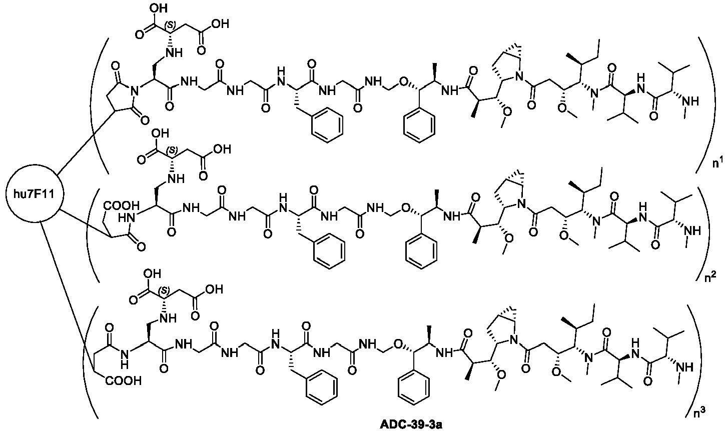

### Example 211 Preparation of ADC-45-3a

ADC-45-3a was prepared by reacting the corresponding linker-payload (LP-20a, whose structure is shown in the structural formula of LP-20, wherein the configuration of the chiral carbon at position 2 is S-type) with the anti-Trop2 antibody hu7F 11 according to the method of general step A.

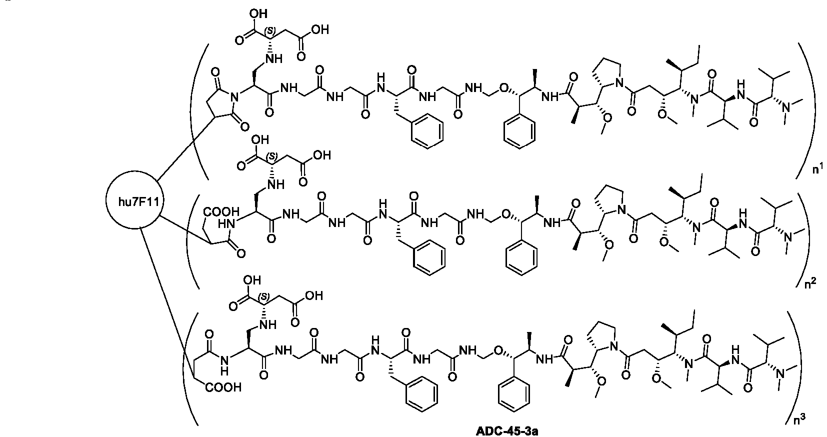

### Example 212 Preparation of ADC-48-3a

ADC-48-3a was prepared by combining the corresponding linker-payload (LP-27a, whose structure is shown in the structural formula of LP-27, wherein the configuration of the chiral carbon at position 2 is S-type) with the anti-Trop2 antibody hu7F 11 according to the method of general step A.

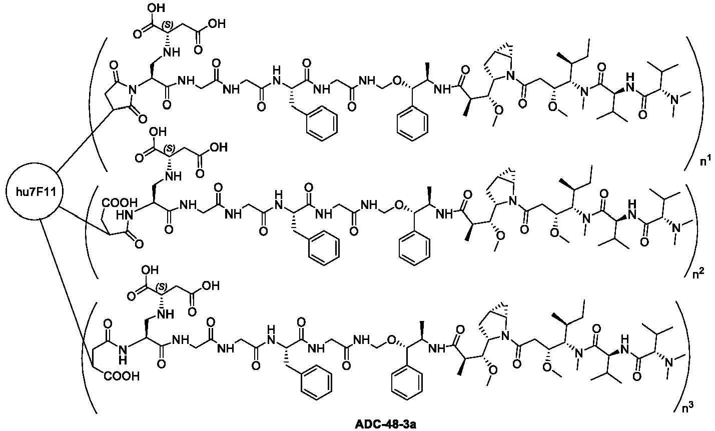

### Example 213 Detection of DAR of ADC by reverse phase high performance liquid chromatography (RP-HPLC)

The average DAR (Drug to Antibody Ratio) of the corresponding ADCs was determined by reverse phase high performance liquid chromatography in general step C as shown in Table 2 below. For ADC-C-1a, ADC-C-2a, and ADC-C-3a, their DAR = n. For the remaining ADCs, DAR = n1 + n2 + n3.

**Table 2**

| Antibodies | Linker-Payload Name | ADC Name | DAR |
|---|---|---|---|
| TR000 | LP-1a | ADC-1-1a | 7.59 |
| | LP-2a | ADC-2-1a | 7.31 |
| | VcMMAE | ADC-C-1a | 7.34 |
| | LP-10 | ADC-3-1a | 7.38 |
| | LP-11 | ADC-6-1a | 7.42 |
| | LP-17 | ADC-11-1a | 7.33 |
| | LP-18 | ADC-12-1a | 7.35 |
| | LP-8a | ADC-19-1a | 7.44 |
| | LP-9a | ADC-20-1a | 7.48 |
| | LP-28a | ADC-21-1a | 7.39 |
| | LP-29a | ADC-22-1a | 7.41 |
| | LP-35a | ADC-27-1a | 7.43 |
| | LP-36a | ADC-28-1a | 7.45 |
| | LP-21a | ADC-31-1a | 7.39 |
| | LP-22a | ADC-35-1a | 7.48 |
| | LP-26a | ADC-39-1a | 7.36 |
| | LP-20a | ADC-45-1a | 7.44 |
| TR005 | LP-1a | ADC-1-2a | 1.79 |
| | LP-2a | ADC-2-2a | 1.81 |
| | VcMMAE | ADC-C-2a | 1.85 |
| | LP-10 | ADC-3-2a | 1.83 |
| | LP-11 | ADC-6-2a | 1.77 |
| | LP-17 | ADC-11-2a | 1.78 |
| | LP-18 | ADC-12-2a | 1.80 |
| | LP-8a | ADC-19-2a | 1.81 |
| | LP-9a | ADC-20-2a | 1.84 |
| | LP-28a | ADC-21-2a | 1.79 |
| | LP-29a | ADC-22-2a | 1.81 |
| | LP-35a | ADC-27-2a | 1.78 |
| | LP-36a | ADC-28-2a | 1.85 |
| | LP-21a | ADC-31-2a | 1.82 |
| | LP-22a | ADC-35-2a | 1.84 |
| | LP-26a | ADC-39-2a | 1.79 |
| | LP-20a | ADC-45-2a | 1.82 |
| hu7F 11 | LP-1a | ADC-1-3a | 7.58 |
| | LP-2a | ADC-2-3a | 7.32 |
| | VcMMAE | ADC-C-3a | 7.33 |
| | LP-10 | ADC-3-3a | 7.36 |
| | LP-11 | ADC-6-3a | 7.44 |
| | LP-17 | ADC-11-3a | 7.37 |
| | LP-18 | ADC-12-3a | 7.39 |
| | LP-8a | ADC-19-3a | 7.43 |
| | LP-9a | ADC-20-3a | 7.46 |
| | LP-28a | ADC-21-3a | 7.41 |
| | LP-29a | ADC-22-3a | 7.44 |
| | LP-35a | ADC-27-3a | 7.42 |
| | LP-36a | ADC-28-3a | 7.48 |
| | LP-21a | ADC-31-3a | 7.42 |
| | LP-22a | ADC-35-3a | 7.49 |
| | LP-26a | ADC-39-3a | 7.38 |
| | LP-20a | ADC-45-3a | 7.47 |

### Example 214 SEC detection of ADC monomer rate

The aggregation degree of each ADC can be obtained through general step D. The SEC-HPLC peak diagram is shown in Figures 3 to 6. The data is summarized in the following table. The monomer rates of ADC-1-1a, ADC-2-1a, ADC-1-2a and ADC-2-2a are all greater than 95%, and the aggregation and degradation degrees are low.

**Table 3**

| Sample Name | Mono Rate (100%) | Aggregation Rate (100%) | Degradation Rate (100%) |
|---|---|---|---|
| ADC-1-1a | 97.51 | 1.94 | 0.55 |
| ADC-2-1a | 96.71 | 2.64 | 0.65 |
| ADC-1-2a | 98.04 | 1.61 | 0.35 |
| ADC-2-2a | 97.65 | 2.02 | 0.33 |

### Example 215 Hydrophobic Interaction Chromatography (HIC) Assay

According to the general procedure A, after completely reducing the 4 interchain disulfide bonds of the IgG1 antibody, the ADCs (ADC-1-1a and ADC-2-1a) with acid-stable linkers introduced via hydroxyl groups of payloads and ADC-C-1a with conventional linker-payload (MC-VC-PAB-MMAE) were further separated and purified by hydrophobic interaction chromatography (HIC). Subsequently, according to the general procedure E, the ADCs were analyzed by hydrophobic interaction chromatography (HIC).

ADCs with greater hydrophobicity or higher DAR (drug-to-antibody ratio) exhibit a later retention time. The results are shown in the following table. The ADCs with acid-stable linkers introduced via hydroxyl groups of payloads, ADC-1-1a and ADC-2-1a, exhibited shorter retention times in HIC, while ADC-C-1a with the conventional MC-VC-PAB linker introduced via amino group of payload, showed the longest retention time. These results indicate that ADC-1-1a and ADC-2-1a, with acid-stable linkers introduced via hydroxyl groups, possess better hydrophilicity.

**Table 4**

| Sample Name | Retention time (minutes) |
|---|---|
| ADC-1-1a | 11.52 |
| ADC-2-1a | 10.51 |
| ADC-C-1a | 15.268 |
| Naked Antibody | 5.77 |

### Example 216 In vitro plasma stability

The plasma stability of ADC was studied according to general step F. The results are shown in the following table. The experimental results show that the ADCs with an acidic stable linker introduced via the hydroxyl group of the payload have almost no drug loss during plasma incubation, while the DAR of the classic MC-linker-ADC (ADC-C-1a) is significantly reduced after 72 hours of incubation. The experimental results demonstrate that the introduction of an acidic stable linker at the hydroxyl group of the payload can significantly enhance the plasma stability of the ADCs.

**Table 5**

| Target Coupling | Day 0 DAR | Day 3 DAR | Day 7 DAR |
|---|---|---|---|
| ADC-1-1a | 7.69 | 7.61 | 7.47 |

| | | | |
|---|---|---|---|
| ADC-2-1a | 7.51 | 7.42 | 7.35 |
| ADC-C-1a | 7.50 | 6.76 | 5.96 |

### Example 217 Analysis of ADC hydrolysis

The molecular weights of the heavy and light chains of ADC-2-1a were determined by ultra-performance liquid chromatography coupled with high-resolution mass spectrometry (UPLC-MS). The hydrolysis profile of ADC-2-1a was characterized based on the changes in the molecular weights of the heavy and light chains.

Sample preparation: Take 100 µg of ADC-2-1a, add 2 µL of 1M DTT, mix well, and briefly centrifuge (10-20 seconds). Then, the mixture is incubated at room temperature for 20 minutes to obtain the reduced ADC.
Instrument: WatesAcquity UPLC H-Class, Wates Xevo G2-XS QTof with UNIFI;
Column: Sepax RP-1000, 4.6*100mm, 5µm 1000 Å;
Column Temperature: 60°C;
Mobile Phase: A:Water (0.05% TFA); B: Acetonitrile (0.05% TFA);
Detection wavelength: 280 nm;

The sample running method is edited as follows:

**Table 6**

| Time (min) | Flow rates (mL/min) | Volume percent of Mobile phase A (%) | Volume percent of Mobile phase B (%) |
|---|---|---|---|
| 0 | 0.3 | 66 | 34 |
| 5 | 0.3 | 66 | 34 |
| 30 | 0.3 | 56 | 44 |
| 30.1 | 0.3 | 5 | 95 |
| 35 | 0.3 | 5 | 95 |
| 35.1 | 0.3 | 66 | 34 |
| 40 | 0.3 | 66 | 34 |

| | | | |
|---|---|---|---|
| Injection volume: 1 mg/mL, 10 µL, i.e. 20µg; Mass spectrometry scanning settings: low mass end (m/z): 500; high mass end (m/z): 4000; Detection range: 1000-3500Da; Output range: 20000-120000Da. | | | |

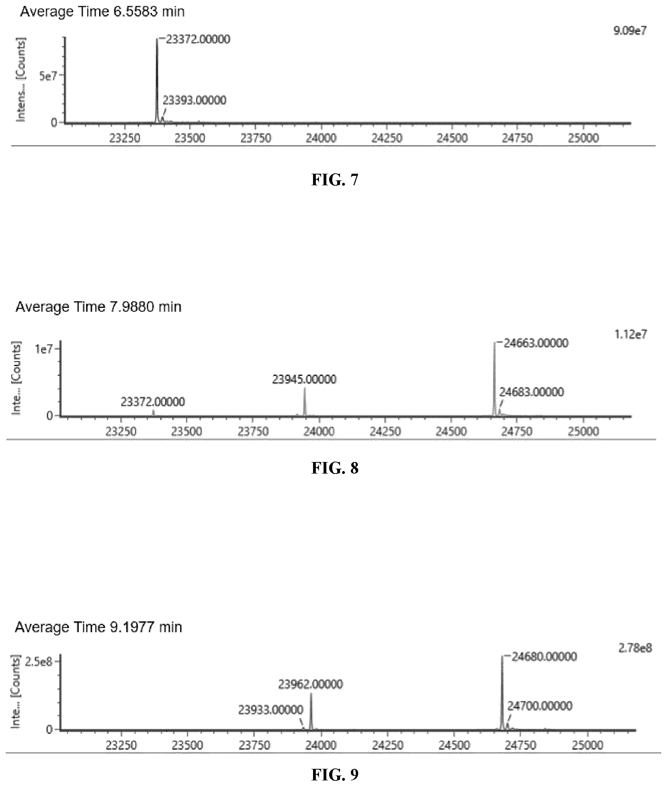

**Figure 7** shows the mass spectrometry detection results of the light chain of the reduced antibody, and **Figure 8** and **Figure 9** show the mass spectrometry detection results of the light chain of ADC-2-1a after reduction. It is known that the molecular weight of the antibody TR000 light chain (LC) is 23372.7721Da. Since the molecular weight of payload (compound 13) is 1289.5400Da, the molecular weight of its ring-opening hydrolysis product is 1307.5550Da. Therefore, when one antibody is connected to 8 payloads, one light chain is coupled with one payload, and its theoretical molecular weight is 24662.3121Da under non-hydrolyzed conditions. As shown in Figures 8 and 9, the molecular weights of the light chains of ADC detected include 24663.0000Da and 24680.0000Da, of which 24680.0000Da differs from the theoretical molecular weight by 17.6879Da. When the light chain of ADC is hydrolyzed and one maleimide group is hydrolyzed, the theoretical molecular weight is 24680.3271Da. Compared to the measured value, the relative deviation falls within the instrument's error range, suggesting that ADC-2-1a is partially hydrolyzed, resulting in an increase in molecular weight by approximately 18 Da. This indicates that the maleimide groups in the ADC exist in both ring-opened and ring-closed forms.

### Example 218 In vitro cytotoxicity assay of ADCs

DMEM medium and RMPI1640 medium were purchased from Source Biotechnology, and fetal bovine serum was purchased from ExCellBio. Human skin squamous cell carcinoma cell line A431 (Trop2 positive expression cells), human primary pancreatic adenocarcinoma BxPC-3 (Trop2 positive expression cells), human gastric cancer cell line NCI-N87 (Trop2 positive expression cells) and human colon cancer cell line SW620 (negative control cells) were purchased from ATCC (American type culture collection) and cultured using the method recommended in the literature. The test ADCs used were ADC-1-1a, ADC-2-1a, ADC-C-1a, ADC-1-2a, ADC-2-2a, and ADC-C-2a. Cell Titer 96^{®} AQueous One Solution Cell Proliferation Assay was purchased from Promega.

Human skin squamous cell carcinoma cell line A431, human primary pancreatic adenocarcinoma BxPC-3, human gastric cancer cell line NCI-N87 and human colon cancer cell line SW620 in the logarithmic growth phase were selected. A certain number of tumor cell lines were inoculated in a 96-well plate, then the cells were treated with gradient dilutions of the test antibody and the corresponding ADCs for 5 days. Finally, the cell viability was detected by MTS, and the inhibitory effect of the test antibody and ADCs on the tumor cell line was evaluated by calculating IC₅₀. The initial concentration of the ADCs was 500 nM, the dilution multiple was 7 times, a total of 8 concentration points, and the treatment lasted for 5 days. The cell survival rate was calculated as follows: ((OD value of the experimental group - OD value of the blank group)/(OD value of the control group - OD value of the blank group)) × 100%, and then the half-maximal inhibitory concentration (IC₅₀) was calculated using GraphPad Prism.

**Table 7 In vitro cytotoxicity of ADCs (120h)**

| ADCs | IC₅₀ (nM) | | | |
|---|---|---|---|---|
| | A431 | BxPC-3 | NCI-N87 | SW620 |
| ADC-1-1a | 0.01 | 0.02 | 0.36 | 15.59 |
| ADC-2-1a | 0.005 | 0.02 | 0.35 | 30.88 |
| ADC-C-1a | 0.02 | 0.04 | 0.63 | 50.58 |
| ADC-1-2a | 0.05 | 0.25 | 2.6 | 295.77 |
| ADC-2-2a | 0.11 | 0.46 | 4.7 | 314.03 |
| ADC-C-2a | 0.32 | 0.87 | 4.9 | 598.24 |

| | | | | |
|---|---|---|---|---|
| P<0.05, the difference is statistically significant. | | | | |

The results of cytotoxicity showed that the ADCs described in this invention demonstrated significant anti-tumor activity across multiple Trop2-positive tumor cell lines, indicating great substantial potential for clinical application.

### Example 218 In vivo antitumor activity studies of ADCs

In this invention, an A431 tumor-bearing mouse model was established to evaluate the *in vivo* antitumor activity of ADCs. 3×10⁶ A431 cells were injected subcutaneously into the right flanks of BALB/c nude mice aged 4 to 6 weeks. When the volume of subcutaneous xenografts reached 140 to 150 mm³, the mice were divided into groups, with 5 mice per group. On days 0, 7, 14, and 21, the groups were administered either a blank control (buffer solution) or ADCs (ADC-1-1a, ADC-2-1a, ADC-C-1a, ADC-1-2a, ADC-2-2a, ADC-C-2a), all at a dose of 2 mg/kg via tail vein injection.

The tumor volume measurement data is displayed as the average tumor volume at the time of measurement, and the changes in the weight of the mice are recorded at the same time to observe the initial toxicity of the ADCs *in vivo.* The results are shown in the following Table. As indicated in the table, among ADCs using the same payload, the high DAR ADCs (ADC-1-1a, ADC-2-1a, and ADC-C-1a) exhibit stronger tumor suppression effects compared to their low DAR counterparts (ADC-1-2a, ADC-2-2a, and ADC-C-2a), and ADC-2-1a exhibits the strongest tumor suppression effect. When the DAR is essentially the same, the ADCs with acid-stable linkers introduced via hydroxyl group of payload (ADC-1-1a and ADC-2-1a) show stronger tumor suppression effects than ADC-C-1a with the MC-VC-PAB linker. On the other hand, no significant changes in body weight were observed in any of the treatment groups, indicating that ADC-1-1a, ADC-2-1a, ADC-C-1a, ADC-1-2a, ADC-2-2a, and ADC-C-2a exhibit no obvious toxicity.

The above results show that the ADCs with an acid-stable linkers introduced via the hydroxyl group of payload exhibit superior *in vivo* antitumor activity than the ADCs using the MC-VC-PAB linker, achieving equivalent tumor suppression effects at lower doses. Since the ADCs of this invention can achieve excellent antitumor effects at a lower dosage and possess improved plasma stability, they also demonstrate reduced hematotoxicity and neurotoxicity.

**Table 8**

| Group | Mean Tumor Volume (mm³) | | | | | | | | |
|---|---|---|---|---|---|---|---|---|---|
| | D0 | D3 | D7 | D10 | D14 | D17 | D21 | D24 | D28 |
| Control | 182.57 | 354.82 | 547.04 | 649.84 | 781.09 | 854.94 | 1012.95 | 1129.45 | 1320.67 |
| ADC-1-1a | 180.22 | 352.21 | 442.54 | 414.76 | 337.97 | 287.14 | 241.00 | 216.74 | 228.47 |
| ADC-2-1a | 180.15 | 344.89 | 343.26 | 358.64 | 375.86 | 336.41 | 280.08 | 226.42 | 232.17 |
| ADC-C-1a | 183.07 | 359.96 | 531.32 | 641.13 | 707.77 | 839.34 | 1027.59 | 1196.59 | 1556.72 |
| ADC-1-2a | 183.78 | 286.15 | 390.57 | 471.77 | 516.78 | 589.15 | 595.04 | 599.58 | 779.54 |
| ADC-2-2a | 182.57 | 391.54 | 596.61 | 716.69 | 775.49 | 811.97 | 951.41 | 929.08 | 1262.70 |
| ADC-C-2a | 182.16 | 336.26 | 570.83 | 632.82 | 911.55 | 1007.99 | 1287.42 | 1399.82 | 1915.84 |

**Table 9**

| Group | Average Body Weight of Mice (g) | | | | | | | | |
|---|---|---|---|---|---|---|---|---|---|
| | D0 | D3 | D7 | D10 | D14 | D17 | D21 | D24 | D28 |
| Control | 22.17 | 22.63 | 23.20 | 23.51 | 23.96 | 24.75 | 25.29 | 25.56 | 25.97 |
| ADC-1-1a | 21.97 | 22.13 | 22.59 | 22.72 | 23.61 | 23.70 | 24.18 | 24.49 | 24.66 |
| ADC-2-1a | 21.74 | 22.58 | 22.52 | 23.12 | 23.50 | 23.65 | 23.87 | 24.18 | 24.52 |
| ADC-C-1a | 20.87 | 21.72 | 21.86 | 22.60 | 23.02 | 23.56 | 23.94 | 24.60 | 24.84 |
| ADC-1-2a | 21.98 | 22.00 | 22.10 | 22.87 | 23.61 | 23.55 | 23.91 | 24.03 | 24.03 |
| ADC-2-2a | 21.29 | 21.63 | 22.23 | 22.57 | 23.34 | 23.97 | 23.94 | 24.48 | 24.50 |
| ADC-C-2a | 21.72 | 22.02 | 22.25 | 23.13 | 23.69 | 24.29 | 24.56 | 25.34 | 25.61 |

It is understood that the above specific description of the application is only used to illustrate the application and is not limited to the technical solutions described in the embodiments of the application, and the person of ordinary skill in the art should be able to understand that modifications or equivalent replacements can still be made to the application to achieve the same technical effect; the above modifications or equivalent replacements are within the scope of protection of the application.

## Claims

1. A ligand-drug conjugate as shown in general formula I or a pharmaceutically acceptable salt or solvate thereof, 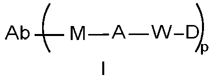 wherein:
Ab is a ligand unit selected from an antibody, an antibody fragment, a targeting protein or an Fc fusion protein;
M is a linker unit connected to Ab;
A is a peptide residue consisting of 2 to 7 amino acids, wherein, optionally, each amino acids is independently substituted by one or more substituents selected from: deuterium, halogens, hydroxyl group, cyano group, amino group, nitro group, alkyl group, substituted alkyl group, alkoxy group, cycloalkyl group, and substituted cycloalkyl group;
W represents an amino methylene oxide structural unit as shown in formula (i): 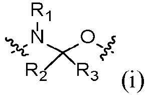 wherein:
the wavy line on the left represents the connection site between the nitrogen atom and A in formula (i), and the wavy line on the right represents the connection site between the oxygen atom in formula (i) and drug D, and the oxygen atom is a common group between D and W;
R₁, R₂ and R₃ are each independently selected from hydrogen, deuterium, alkyl and substituted alkyl;
p is an integer or decimal from 1 to 20; and
D is auristatin having a structure shown in formula D, or isomer, mesomorph, racemate, enantiomer or mixture thereof, or pharmaceutically acceptable salt thereof, 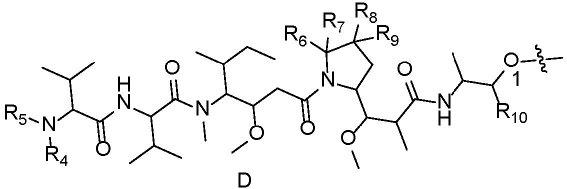 wherein:
R₄, R₅ are each independently selected from hydrogen, deuterium, alkyl, and deuterated alkyl,
or R₄, R₅ are linked to form the following structure: -(CR₁₁R₁₂)ₙ-B-(CR₁₃R₁₄)ₘ-, wherein R₁₁, R₁₂, R₁₃, and R₁₄ are selected from hydrogen, deuterium, alkyl and deuterated alkyl; B is selected from O, NR₁₅, and CR₁₆R₁₇, wherein R₁₅, R₁₆, and R₁₇ are selected from hydrogen, deuterium, and alkyl; n and m are each independently an integer from 0-8; the nitrogen atom bonded with R₄ and R₅ forms a ring with -(CR₁₁R₁₂)ₙ-B-(CR(₁₃₎R₁₄₎ₘ-;
R₆, R₇, R₈, R₉ are each independently hydrogen, deuterium, halogen, an azide group, alkyl, or NR₁₈R₁₉, or any two of R₆, R₇, R₈, R₉ form a cycloalkyl group together with the atoms to which they are bonded, and the remaining two group are each independently hydrogen, halogen, an azide group, alkyl, and NR₁₈R₁₉, wherein R₁₈, R₁₉ are each hydrogen or alkyl;
R₁₀ is aryl, or heteroaryl, said aryl or heteroaryl being optionally substituted with one or more substituents, said substituents being selected from: hydrogen, a halogen, alkyl, an alkoxy group, an amino group, a nitro group;
the wavy line in formula D represents the connection site between the oxygen atom at position 1 in the structure of D and W, and the oxygen atom is a common group between D and W.

2. The ligand-drug conjugate according to Claim 1 or a pharmaceutically acceptable salt or solvate thereof, wherein Ab is an antibody, an antibody fragment or a protein, wherein the antibody is a murine antibody, a rabbit antibody, a phage display antibody, a yeast display antibody, a chimeric antibody, a humanized antibody, a fully human antibody, an antibody fragment, a bispecific antibody or a multispecific antibody.

3. The ligand-drug conjugate according to Claim 1 or 2, or a pharmaceutically acceptable salt or solvate thereof, wherein the antibody is a monoclonal antibody comprising anti-EGFRvIII antibody, anti-PD-1 antibody, anti-PD-L1 antibody, anti-DLL-3 antibody, anti-PSMA antibody, anti-CD70 antibody, anti-MUC16 antibody, anti-ENPP3 antibody, anti-TDGF1 antibody, anti-ETBR antibody, anti-MSLN antibody, anti-TIM-1 antibody, anti-LRRC15 antibody, anti-LIV-1 antibody, anti-CanAg/AFP antibody, anti-claudin 18.2 antibody, anti-Mesothelin antibody, anti-HER2 (ErbB2) antibody, anti-EGFR antibody, anti-c MET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3 (ErbB3) antibody, anti-MUC1/CD227 antibody, anti-AXL antibody, anti-CD166 antibody, anti-B7-H3 (CD276) antibody, anti-PTK7/CCK4 antibody, anti-PRLR antibody, anti-EFNA4 antibody, anti-5T4 antibody, anti-NOTCH3 antibody, anti-Nectin 4 antibody, anti-Trop2 antibody, anti-CD142 antibody, anti-CA6 antibody, anti-GPR20 antibody, anti-CD174 antibody, anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody, anti-CD33 antibody, anti-CD56 antibody, anti-CD74 antibody, anti-CD22 antibody, anti-CD30 antibody, anti-CD37 antibody, anti-CD47 antibody, anti-CD138 antibody, anti-CD352 antibody, anti-CD25 antibody, or anti-CD123 antibody.

4. The ligand-drug conjugate or a pharmaceutically acceptable salt or solvate thereof according to any one of Claims 1 to 3, wherein,
R₄ and R₅ are each independently selected from hydrogen, a C₁-C₄ alkyl group,
or R₄ and R₅ are connected to form the following structure: -(CH₂)₂-B-(CH₂)₂-, B is selected from O, NH, and the nitrogen atom bonded to R₄ and R₅ forms a ring with -(CH₂)₂-B-(CH₂)₂-.

5. The ligand-drug conjugate or a pharmaceutically acceptable salt or solvate thereof according to any one of Claims 1 to 4,
wherein R₆, R₇, R₈, R₉ in formula D are each hydrogen; or, one of R₆, R₇, R₈, R₉ in formula D is selected from halogen, azido, and amino, and the remaining three are each hydrogen; or, any two group among R₆, R₇, R₈, R₉ in formula D together with the atoms to which they are bonded form a cyclopropyl group, and the remaining two group are each independently hydrogen.

6. The ligand-drug conjugate or a pharmaceutically acceptable salt or solvate thereof according to any one of Claims 1 to 5,
wherein R₁₀ in formula D is a phenyl group, and optionally, the phenyl group is substituted by one or more of the substituents; preferably, the substituents are selected from amino and nitro;
preferably, the drug unit D is selected from the following compounds or the isomers, mesomers, racemates, enantiomers or mixtures thereof, or pharmaceutically acceptable salts thereof:

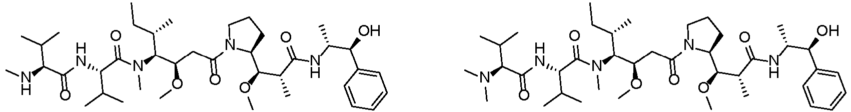

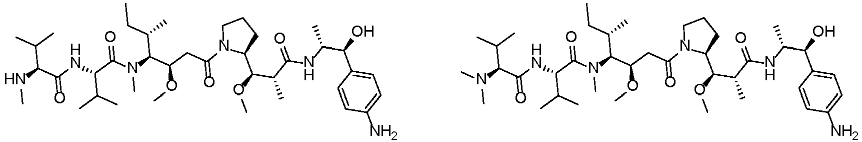

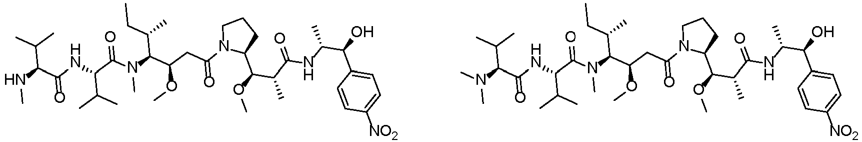

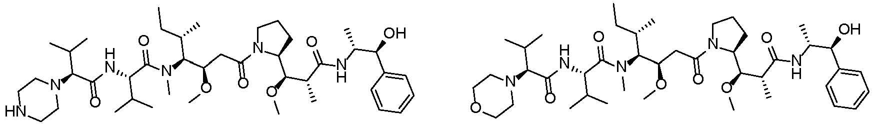

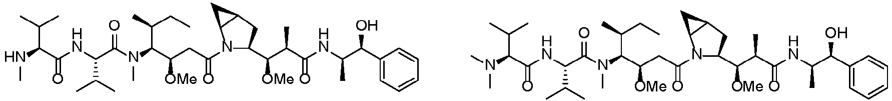

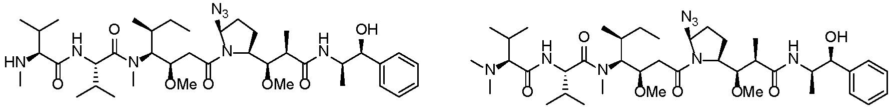

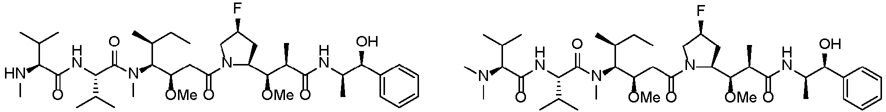

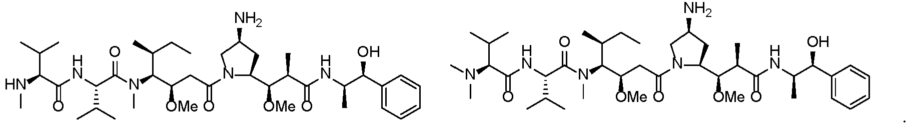

7. The ligand-drug conjugate according to any one of Claims 1 to 6 or a pharmaceutically acceptable salt or solvate thereof, wherein the ligand-drug conjugate has a structure shown in formula Ia 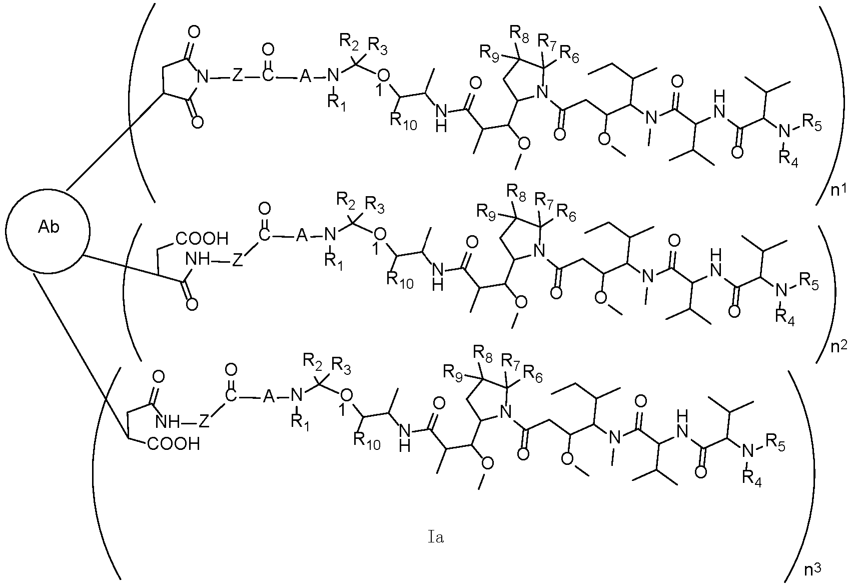 wherein:
Z is selected from -C₁-C10 alkylene-, -C₃-C8 carbocyclic-, -arylidene-, -C₁-C10 alkylene-arylidene-, -arylidene-C₁-C₁₀-alkylene-, -C₁-C₁₀ alkylene-(C₃-C₈ carbocycle)-, -(C₃-C₈ carbocycle)-C₁-C₁₀ alkylene-, -3-8 membered heterocycle-, -C₁-C₁₀ alkylene-(3-8 membered heterocycle)-, -(3-8 membered heterocycle)-C₁-C₁₀ alkylene-, -(CH₂CH₂O)ᵣ-, -(CH₂CH₂O)ᵣ-CH₂- or 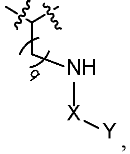 wherein X is selected from -C₁-C10 alkylene-, -C₃-C₈ carbocyclic-, -arylidene-, - C₁-C₁₀ alkylene-arylidene-, , -arylidene-C₁-C₁₀-alkylene--C₁-C₁₀ alkylene-(C₃-C₈ carbocyclylidene)-, -(C₃-C₈ carbocycle)-C₁-C₁₀-alkylene-, -3-8 meta-heterocyclic-, -C₁-C₁₀ alkylene-(3-8 meta-heterocyclic)-, -(3-8 meta-heterocyclic)-C₁-C₁₀ alkylene-, -(CH₂CH₂O)ᵣ-, - (CH₂CH₂O)ᵣ-CH₂; Y is a hydrophilic structure selected from carboxyl, phosphoric acid, polyphosphoric acid, phosphorous acid, sulfonic acid, sulfinic acid or polyethylene glycol (PEG); the heterocycles each independently contain 1 to 3 atoms selected from N, O and S; the - C₁-C₁₀ alkylene-, -C₃-C₈ carbocycle- and heterocycles are each independently substituted by one or more substituents selected from deuterium, halogens, hydroxyl group, cyano group, nitro group, amino group, alkyl group, heteroalkyl group, substituted alkyl group, alkoxy group, carboxyl group or cycloalkyl group; 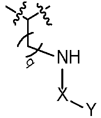 the left wavy line represents the connection site to N on maleimide, and the right wavy line represents the connection site to the carbonyl group; r is an integer from 1 to 10; q is an integer from 1 to 8;
n¹, n², and n³ are independently selected from integers or decimals between 0 and 20, n¹, n², and n³ are not simultaneously 0, and n¹ + n² + n³≤20.

8. The ligand-drug conjugate of any one of Claims 1-7 or a pharmaceutically acceptable salt or solvate thereof,
wherein A is a polypeptide residue formed by 2 to 7 amino acids selected from phenylalanine (F), glycine (G), valine (V), lysine (K), alanine (A), citrulline, serine (S), glutamic acid (E), and aspartic acid (D);
preferably, A is a peptide residue formed by 2 to 4 amino acids selected from phenylalanine and glycine;
preferably, A is a tetrapeptide residue consisting of glycine-glycine-phenylalanine-glycine.

9. The ligand-drug conjugate of Claim 7 or 8 or a pharmaceutically acceptable salt or solvate thereof,
Z is selected from -C₁-C₁₀ alkylene-, such as -C₄-C₆ alkylene-, such as -C₅ alkylene-;
preferably, Z is 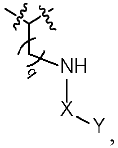 wherein q is selected from an integer between 1 and 8.

10. The ligand-drug conjugate of any one of Claims 7-9 or a pharmaceutically acceptable salt or solvate thereof,
R₁, R₂ and R₃ are each independently selected from hydrogen, deuterium, alkyl, a haloalkyl group, deuteroalkyl and a hydroxyalkyl group;
preferably, R₁, R₂ and R₃ are both hydrogen atoms or deuterium;
preferably, R₁, R₂ and R₃ are simultaneously hydrogen atoms.

11. The ligand-drug conjugate of any one of Claims 1-10 or a pharmaceutically acceptable salt or solvate thereof, said ligand-drug conjugate having a structure as shown in formula Ib:

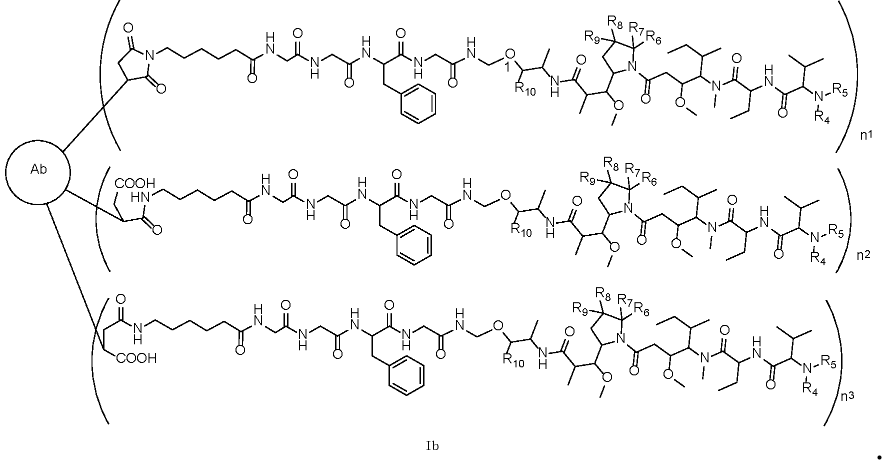

12. The ligand-drug conjugate of any one of Claims 1-10 or a pharmaceutically acceptable salt or solvate thereof, said ligand-drug conjugate having a structure as shown in Formula Ic 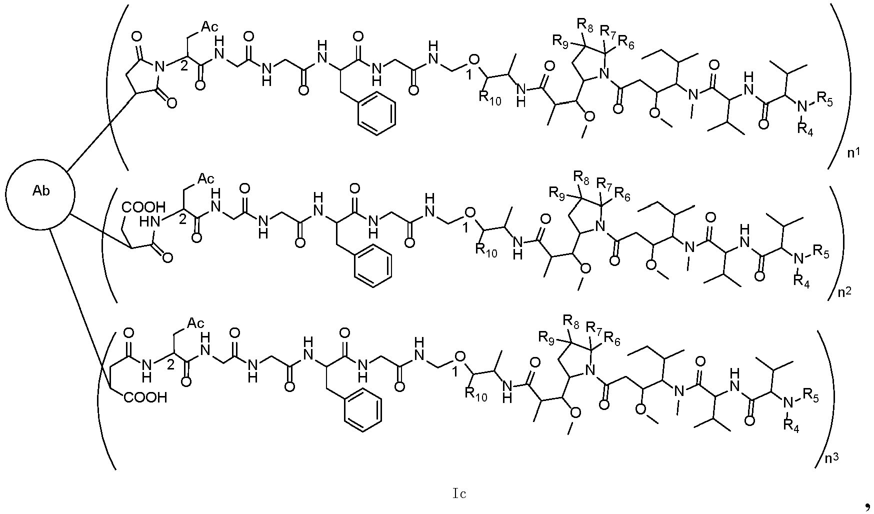
wherein Ac is a hydrophilic structural unit having the structure as shown in formula c: 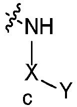
Ac is linked, via an amino group, to 2-position methylene carbon as labeled in formula Ie; X, Y are as defined in Claim 5;
preferably, said Ac is selected from glycine, (D/L) alanine, (D/L) leucine, (D/L) isoleucine, (D/L) valine, (D/L) phenylalanine, (D/L) proline, (D/L) tryptophan, (D/L) serine, (D/L) ) tyrosine, (D/Lcysteine, (D/L) cystine, (D/L) arginine, (D/L) histidine, (D/L) (D/L) methionine,asparagine, (D/L) glutamine, (D/L) threonine, (D/L) aspartic acid, (D/L) glutamic acid, natural or non-natural amino acid derivatives, or the following structures:

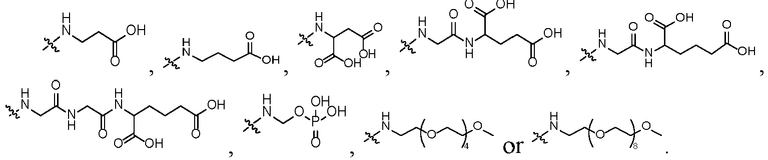

13. The ligand-drug conjugate of any one of Claims 1-12 or a pharmaceutically acceptable salt or solvate thereof, said ligand-drug conjugate compound being selected from the following structures: 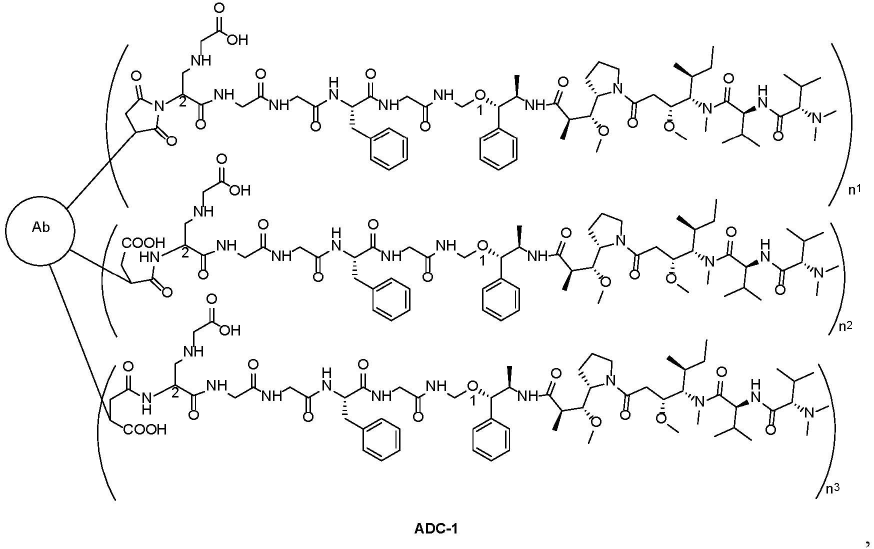 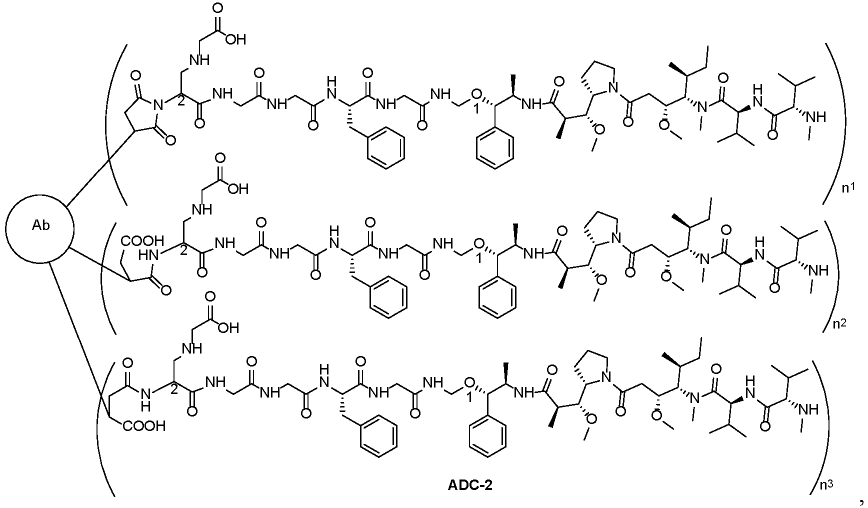 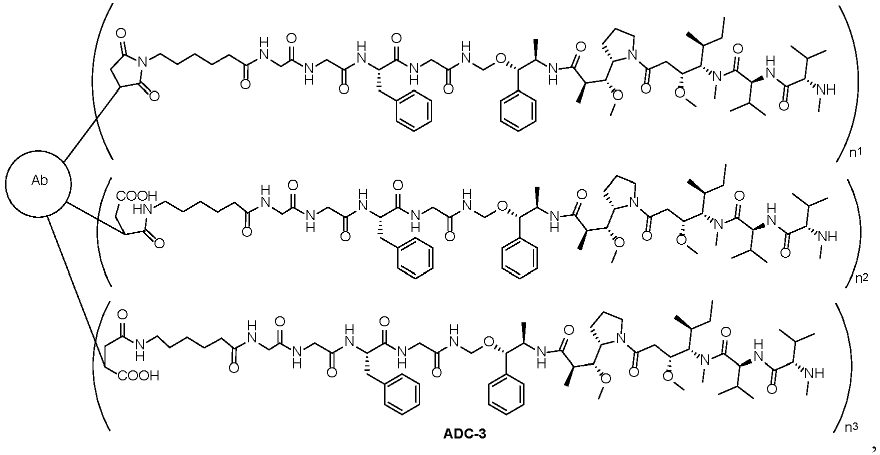 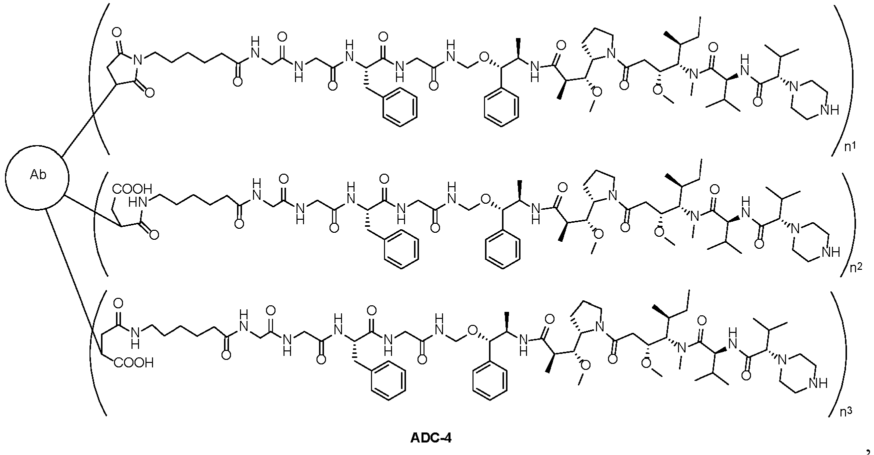 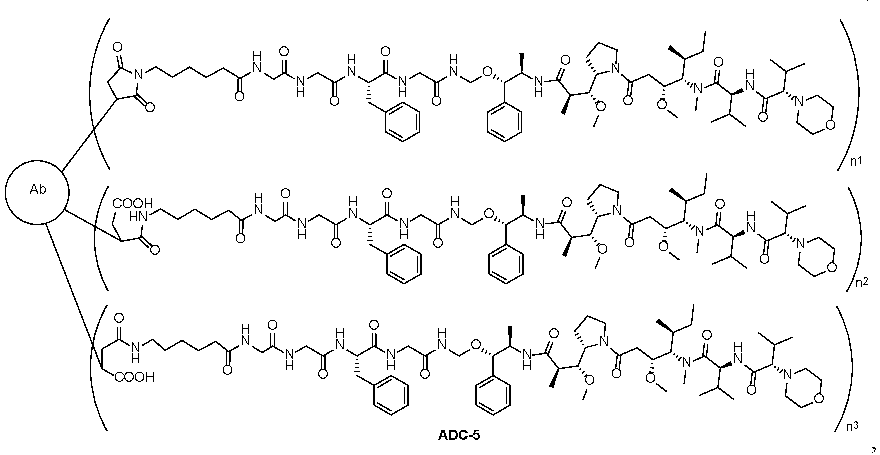 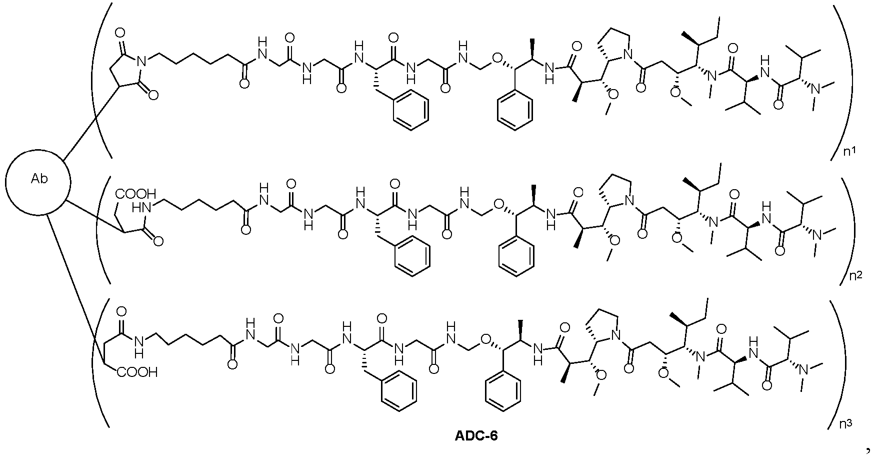 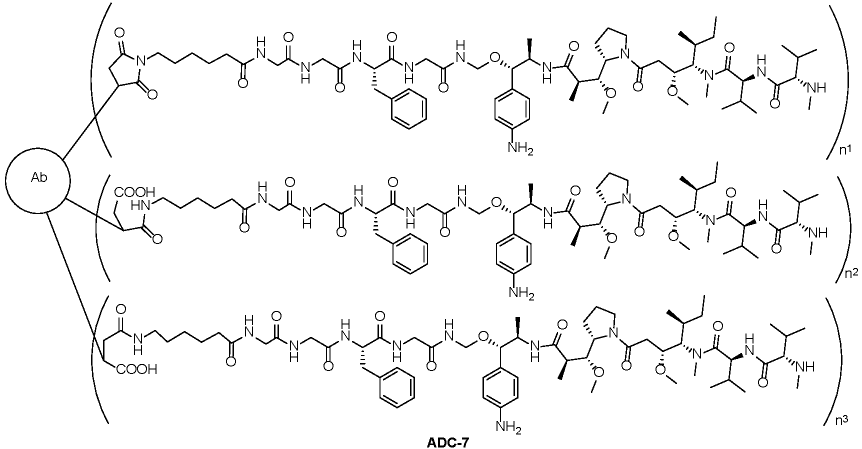 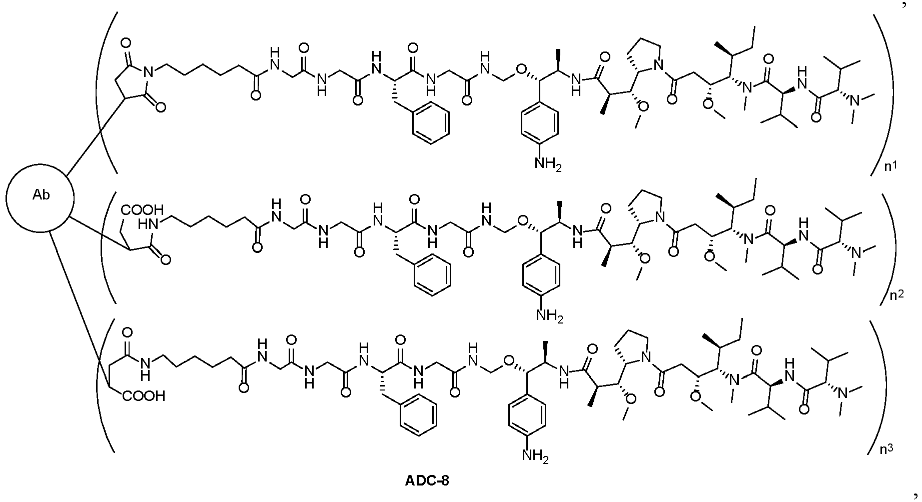 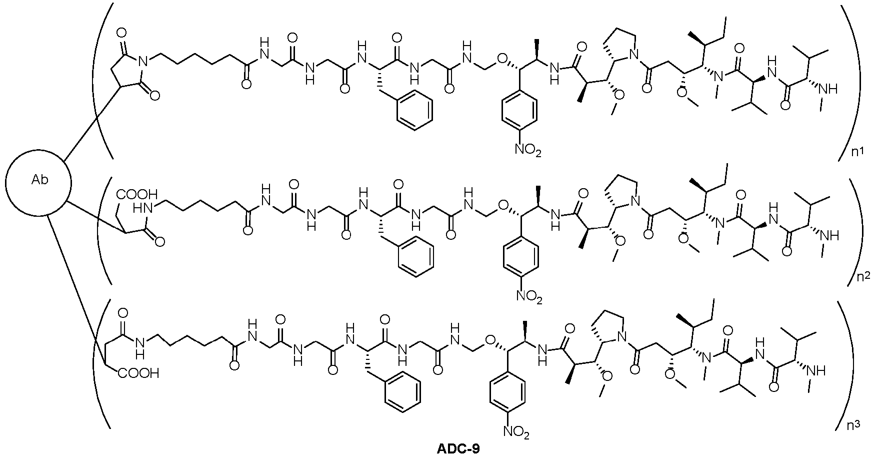 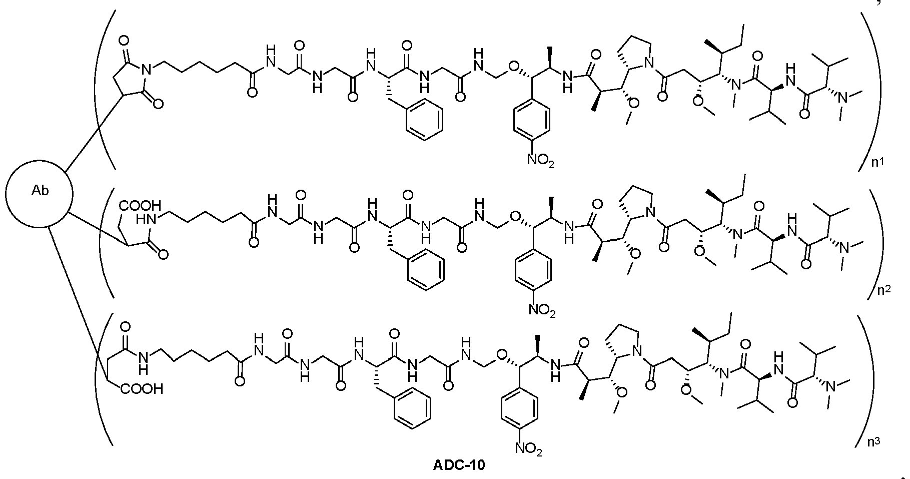 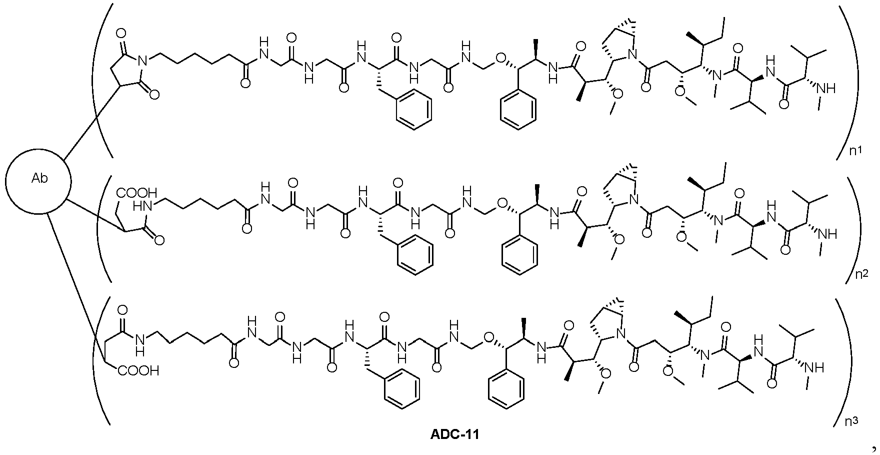 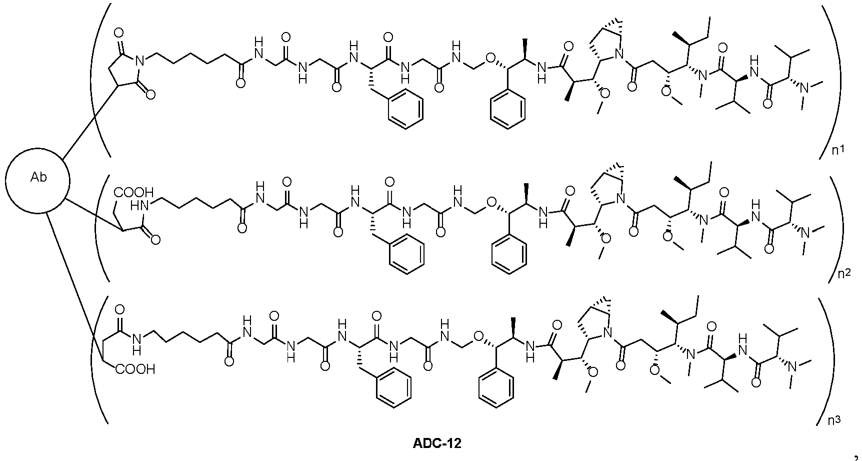 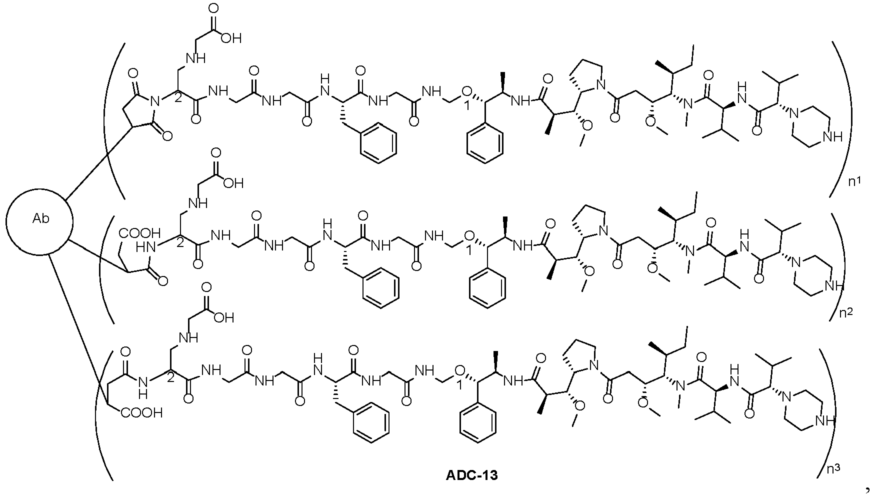 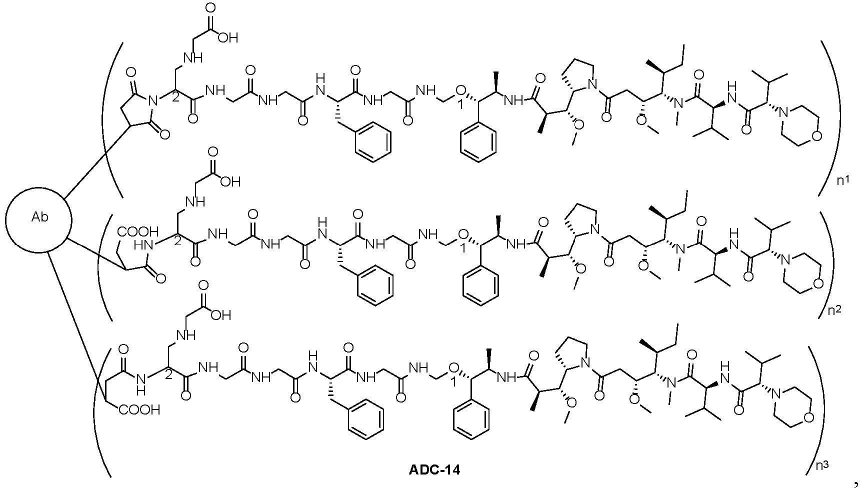 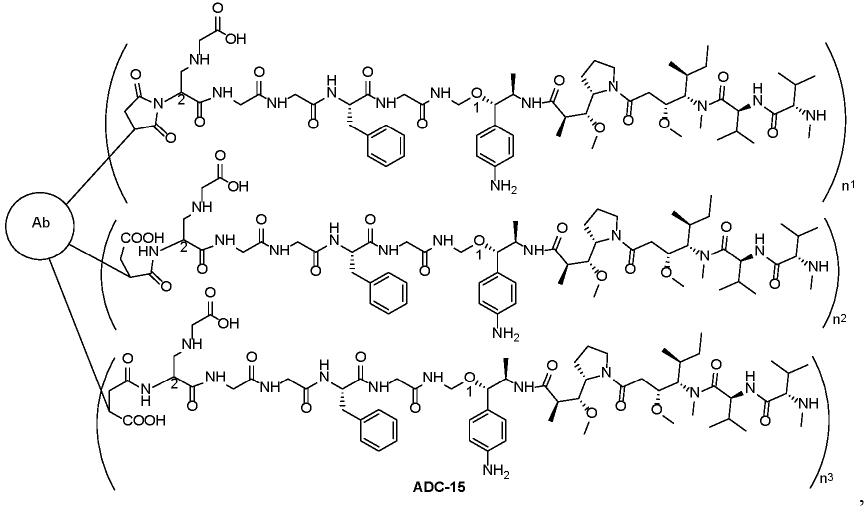 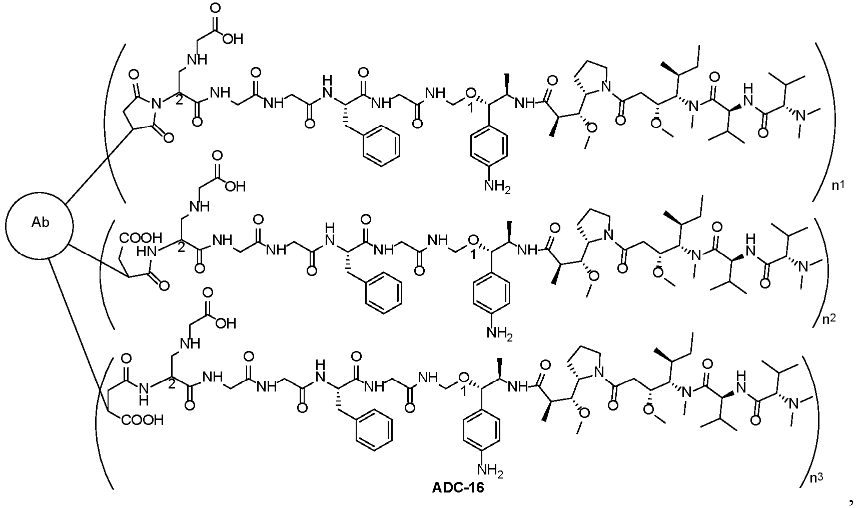 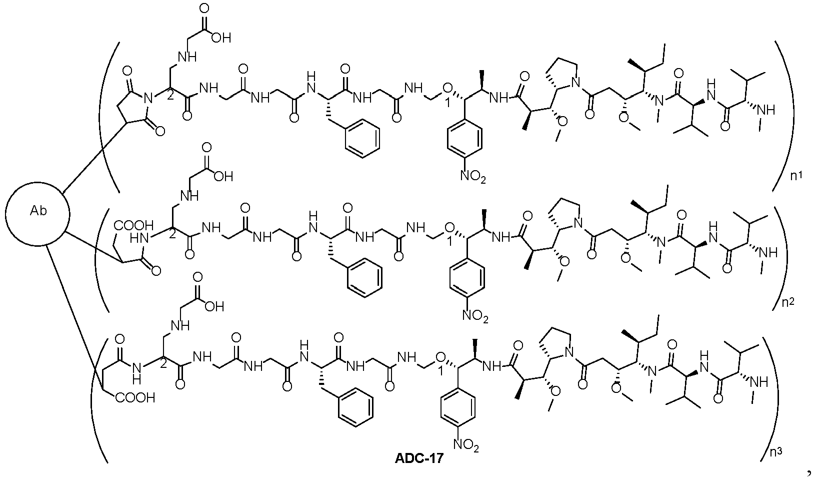 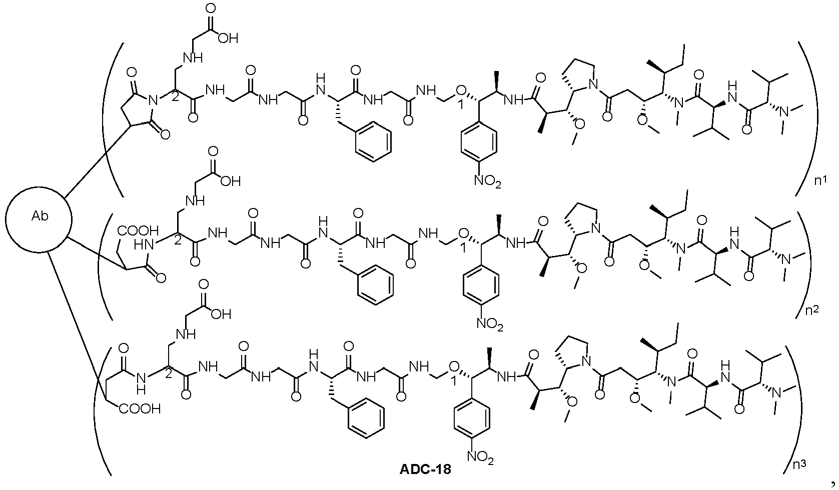 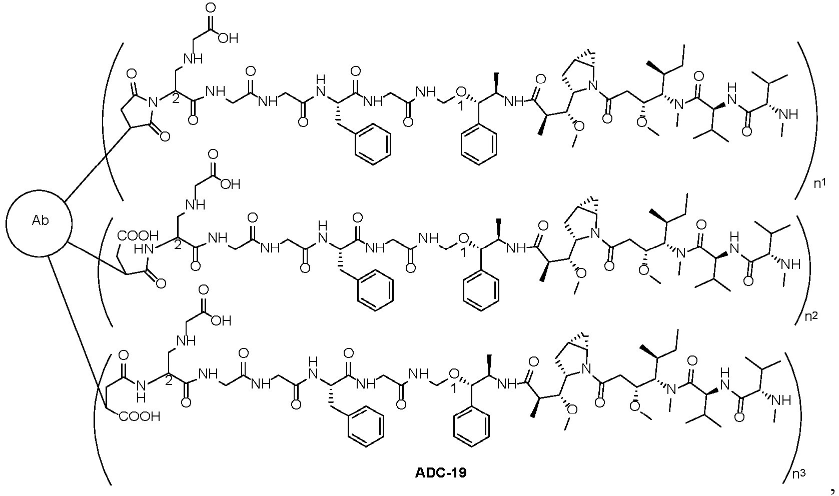 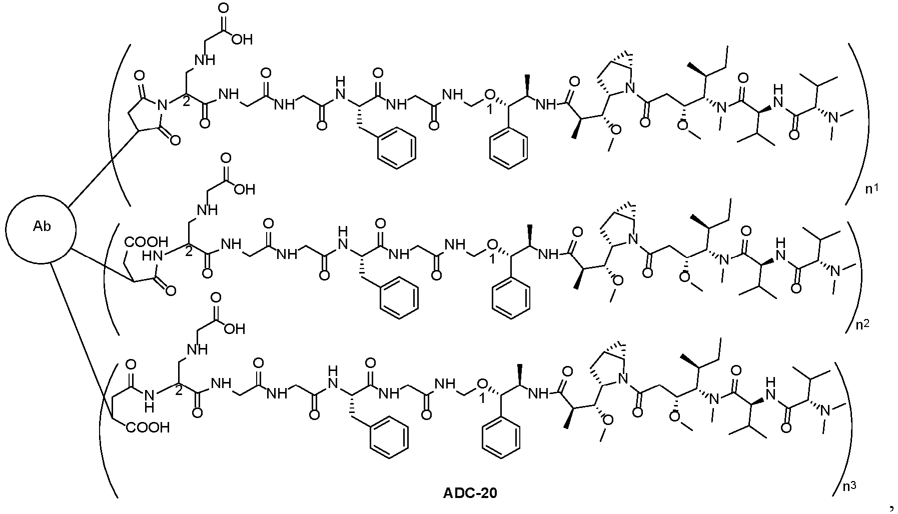 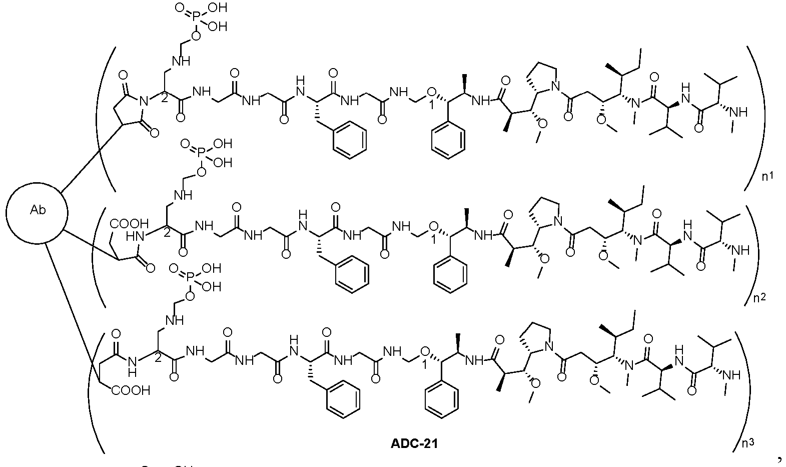 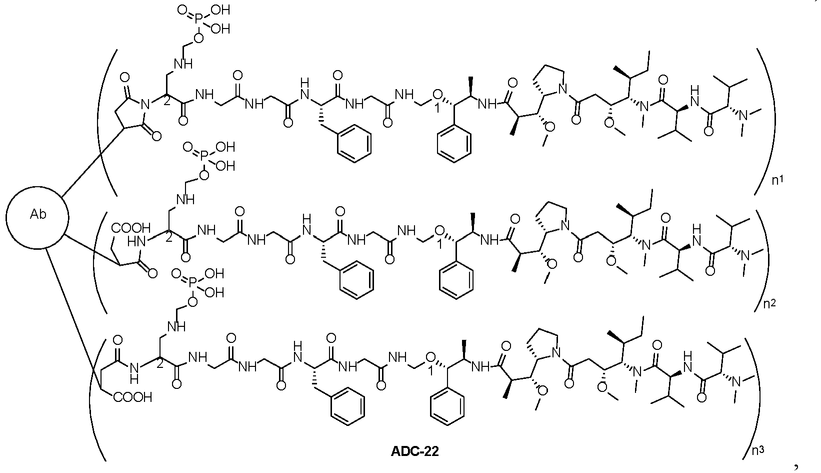 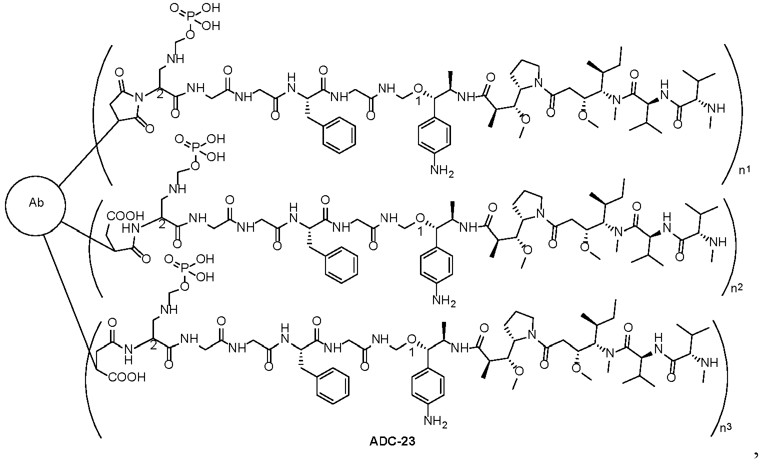 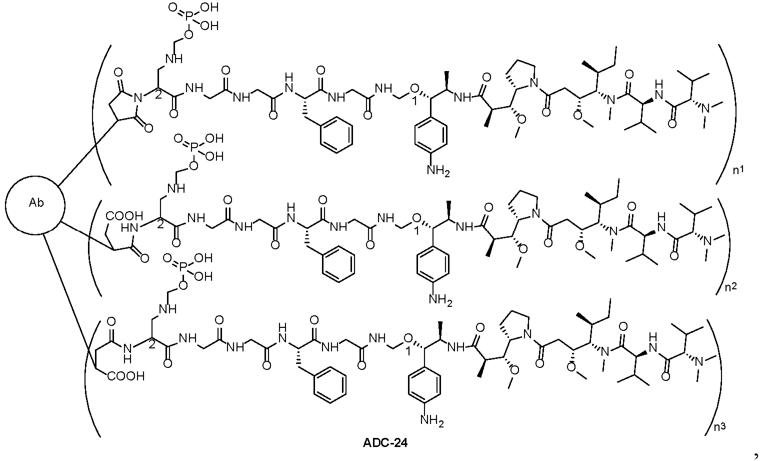 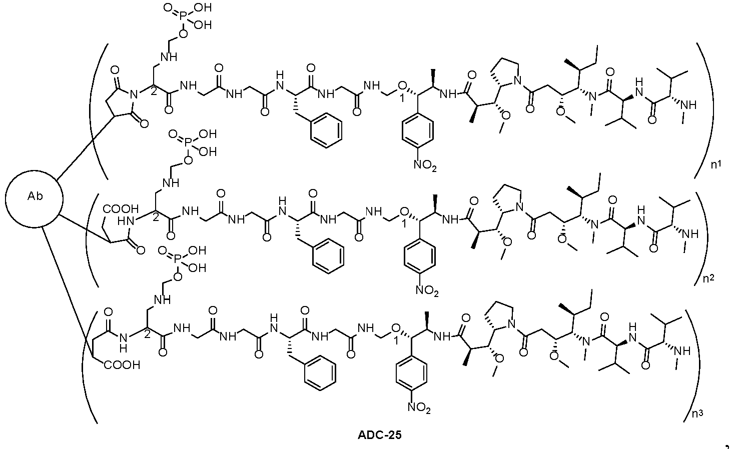 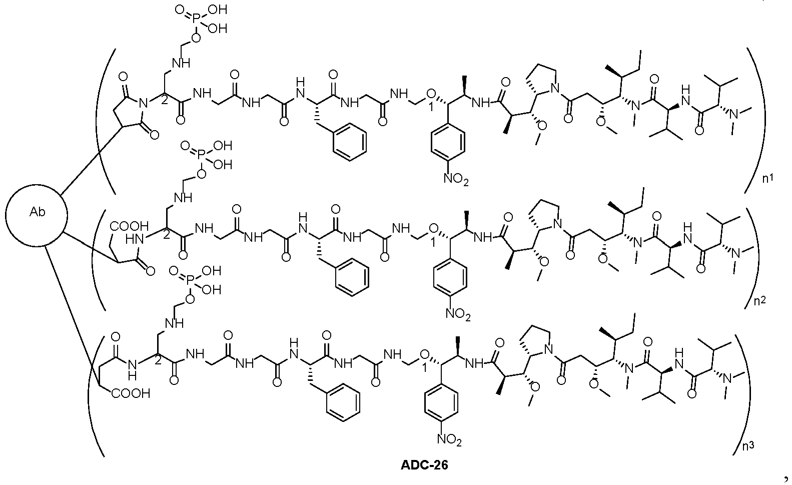 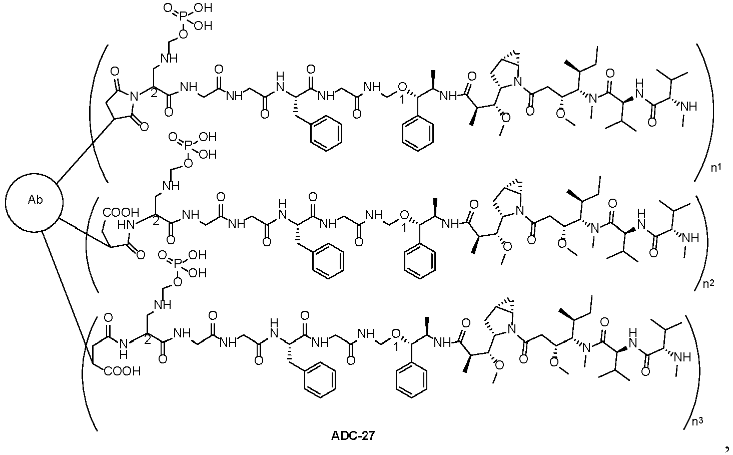 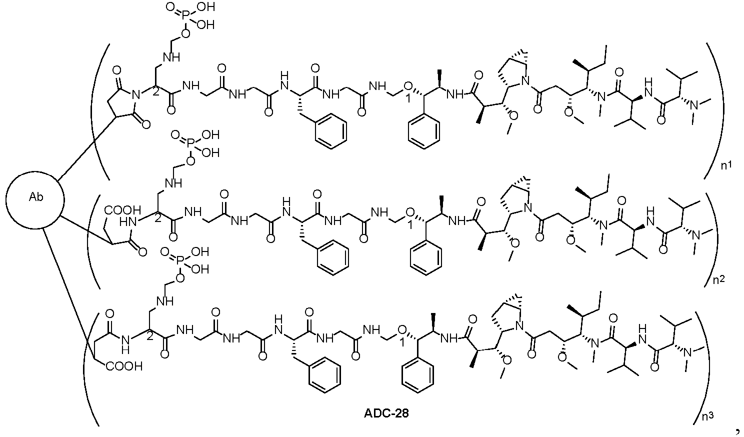 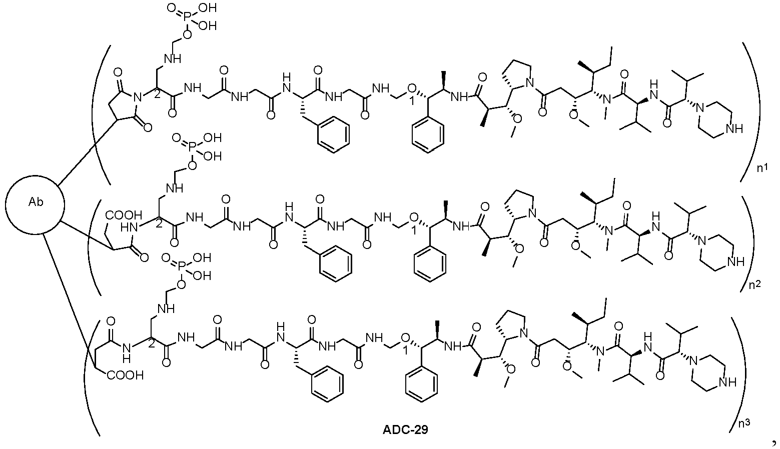 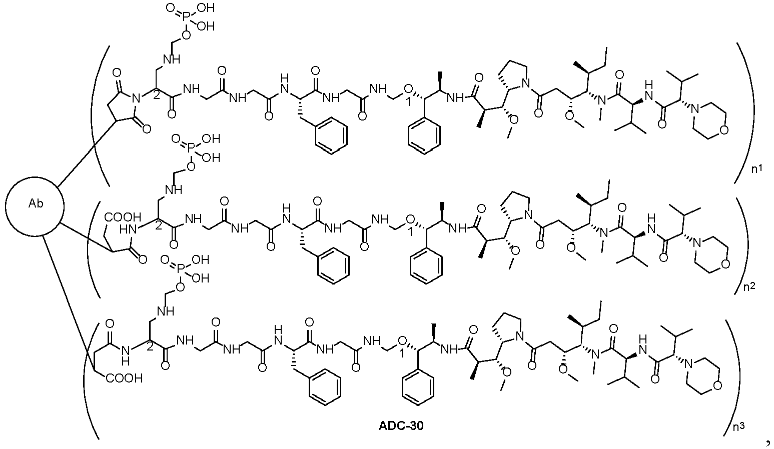 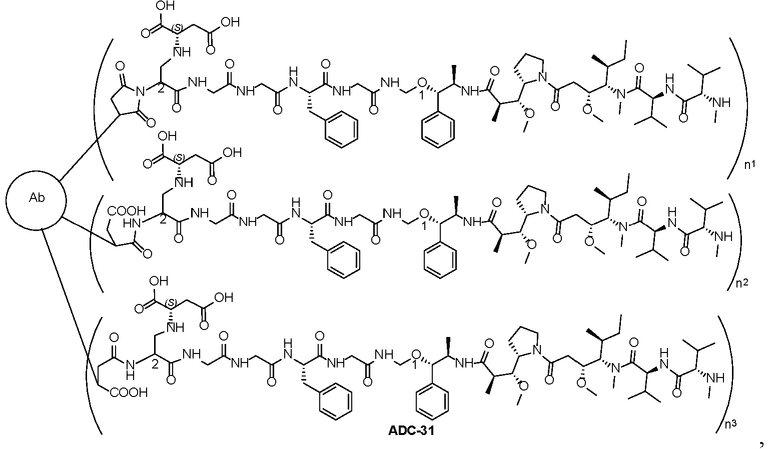 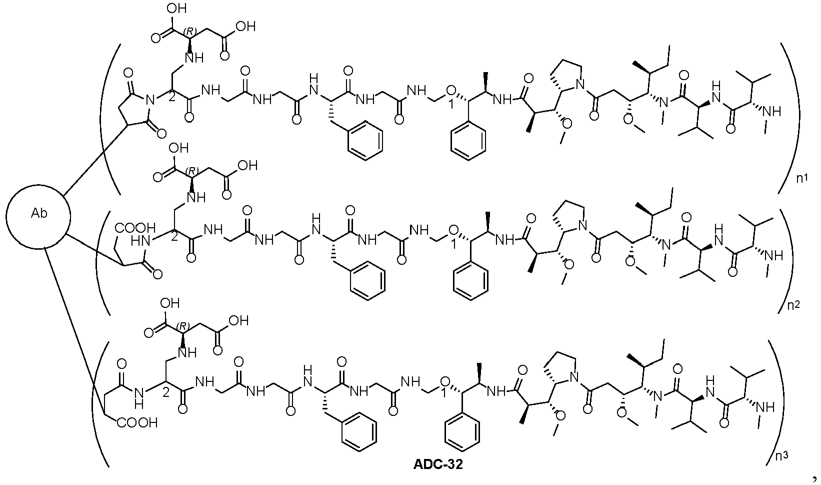 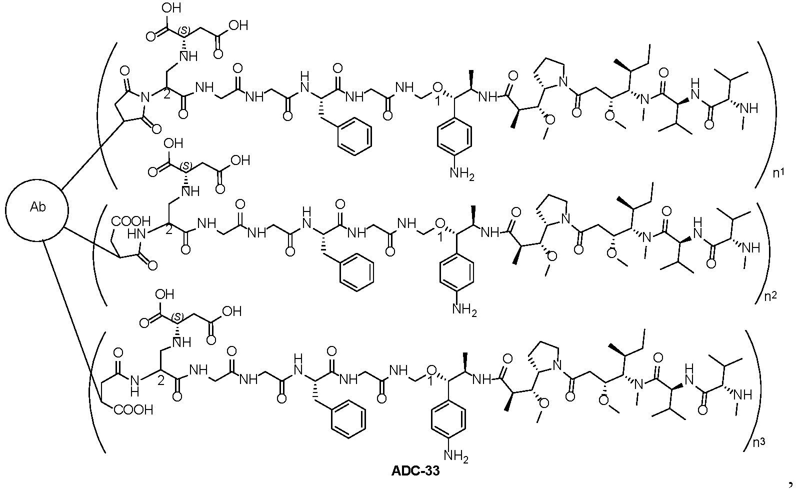 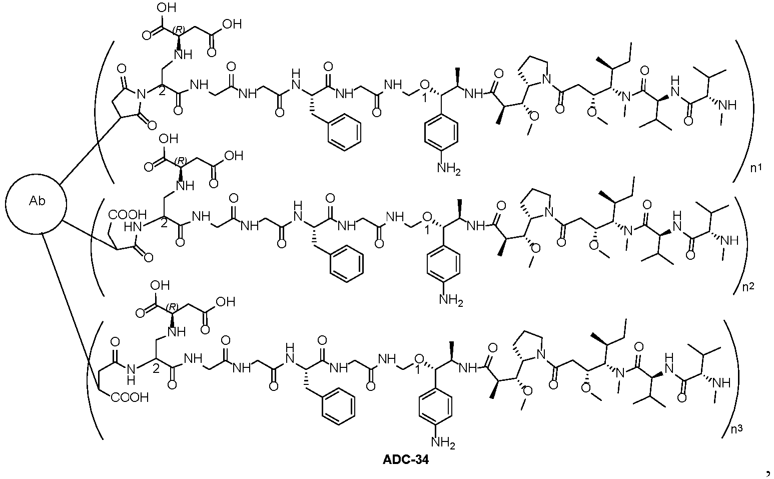 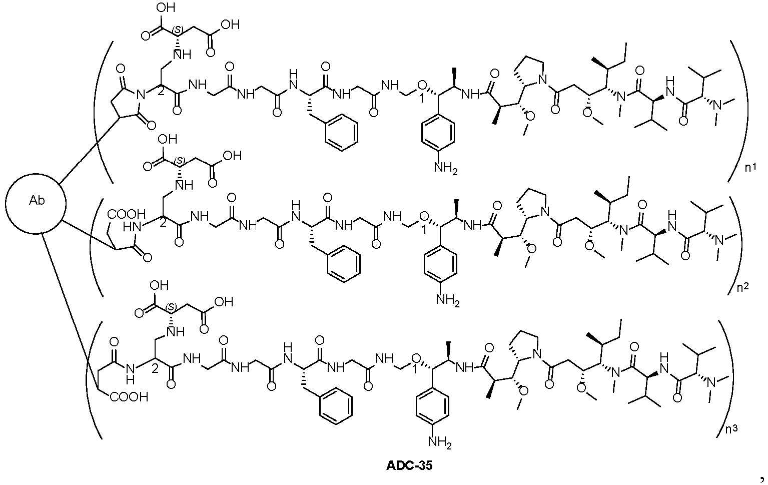 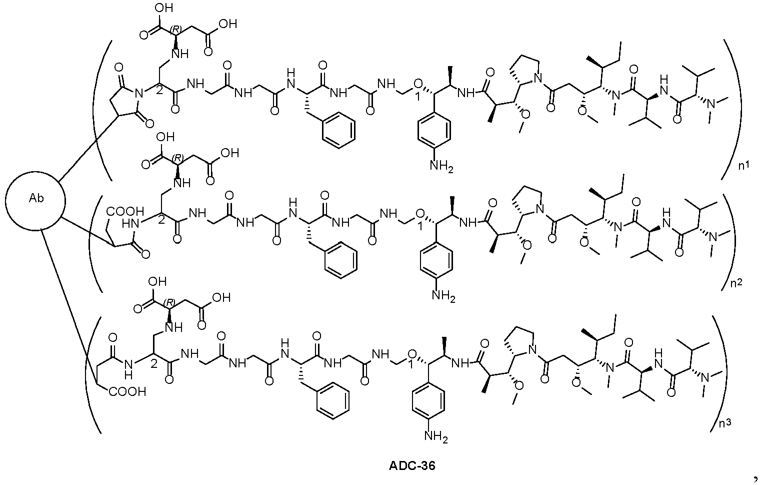 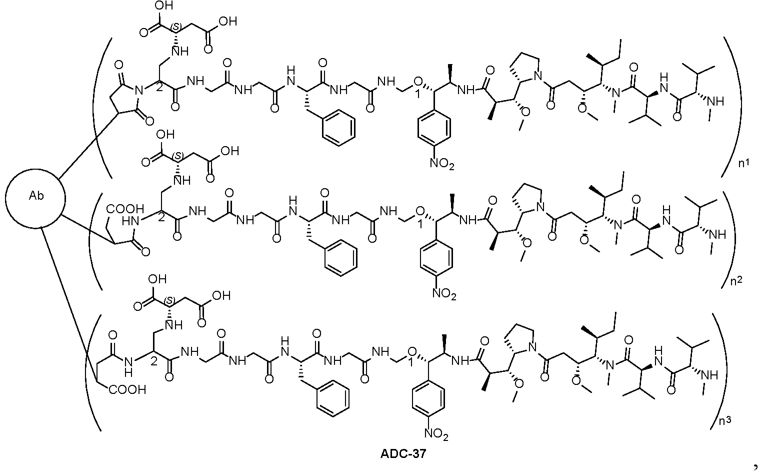 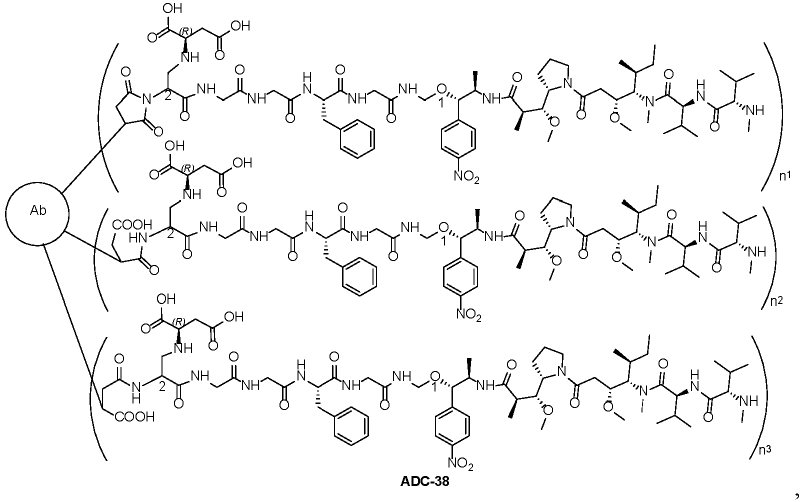 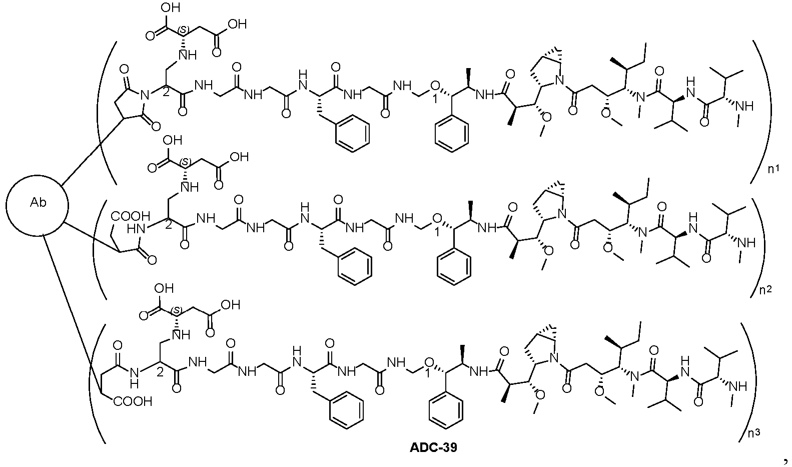 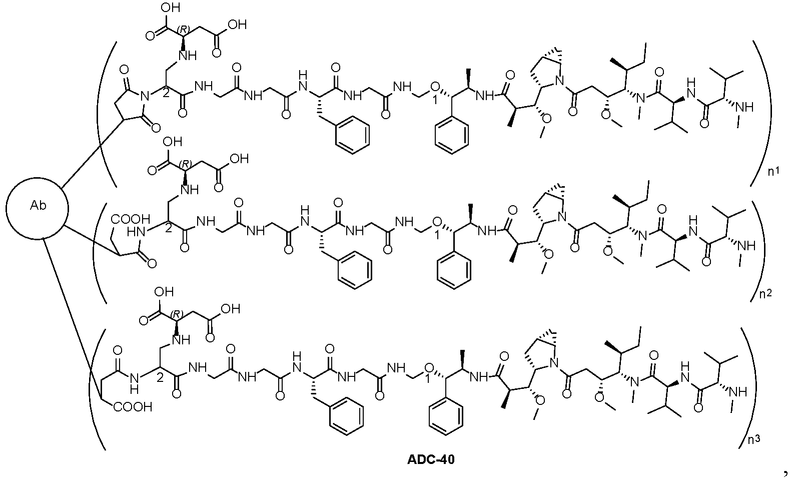 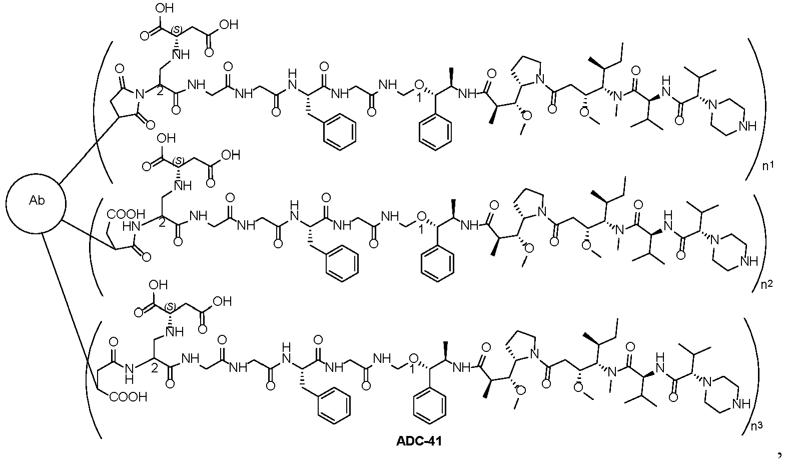 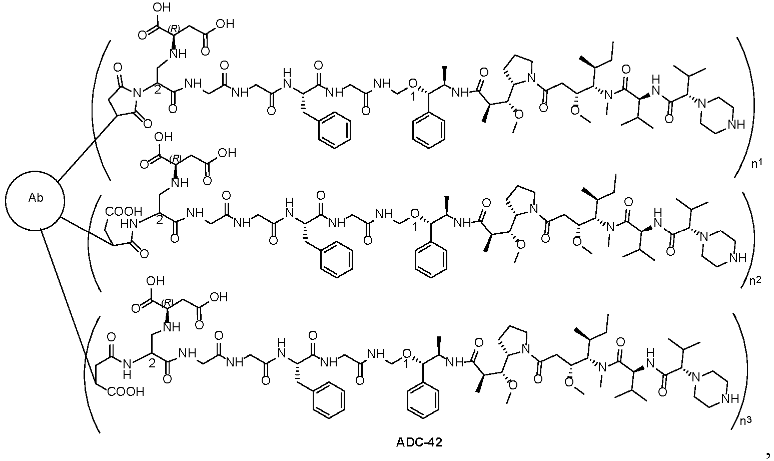 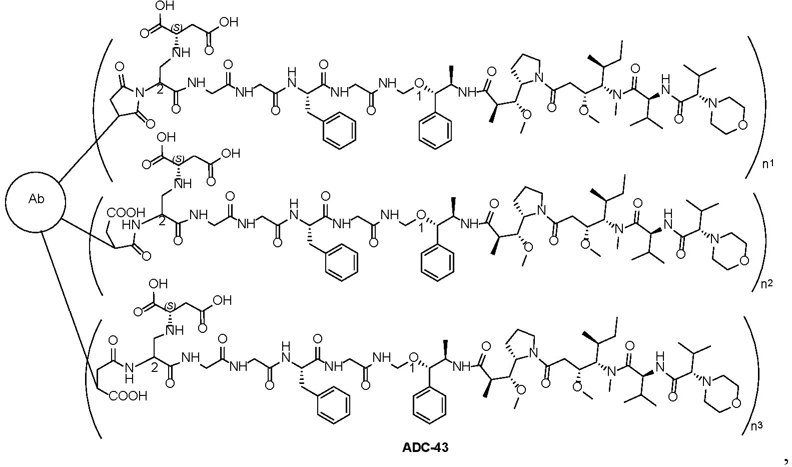 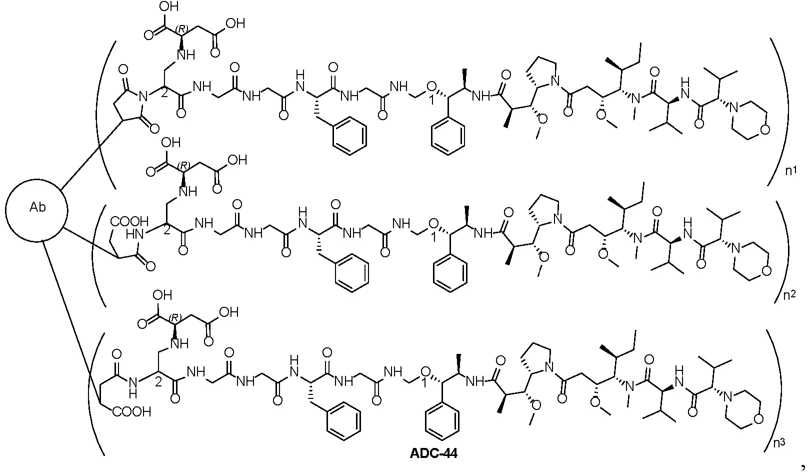 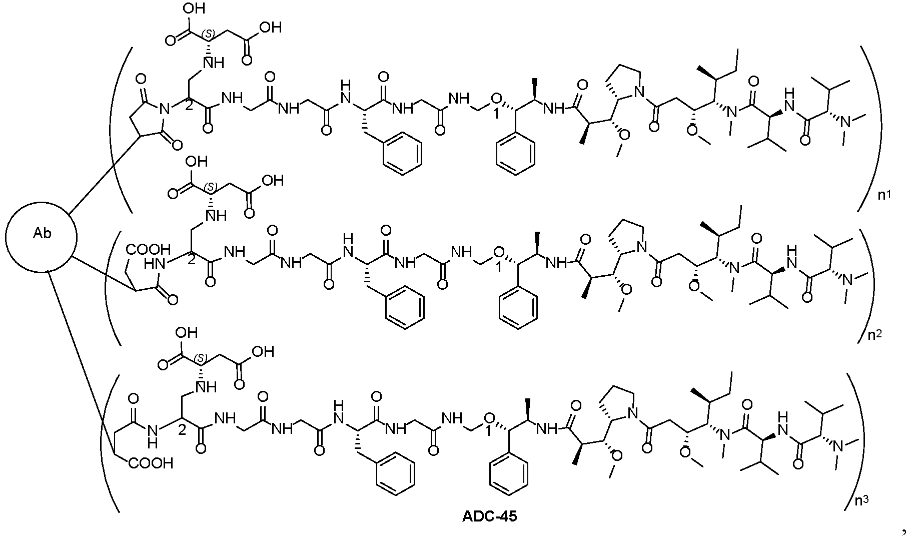 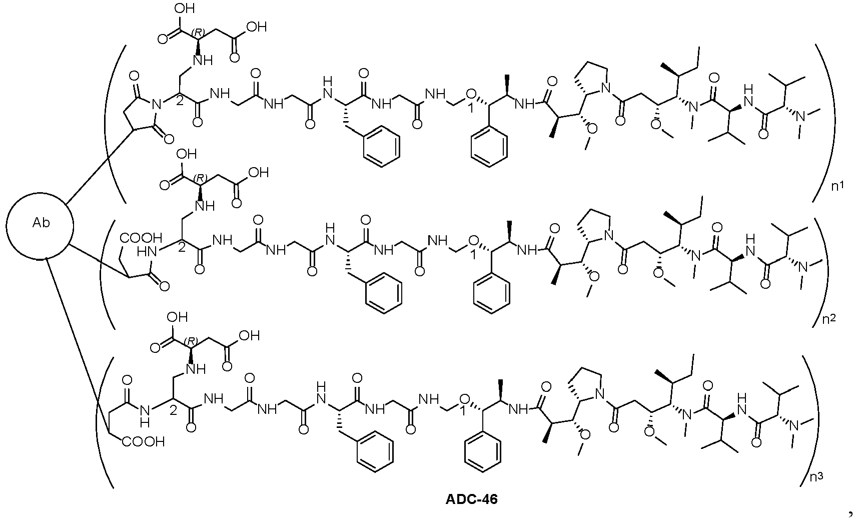 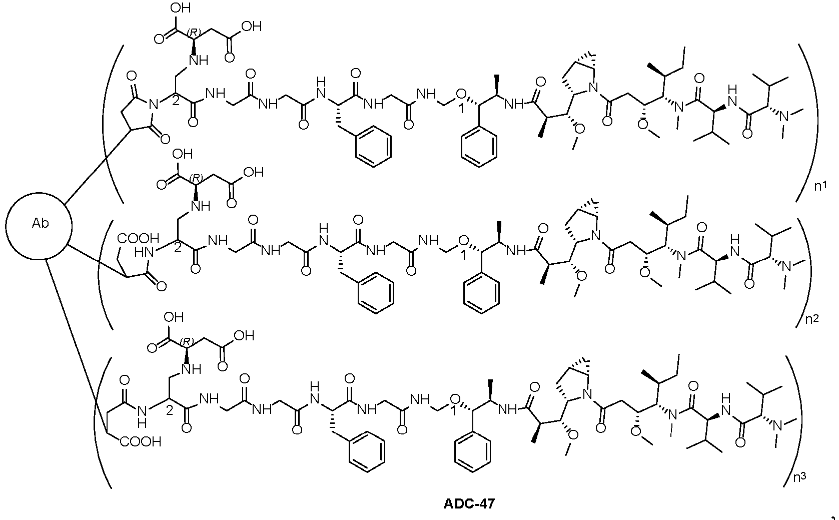 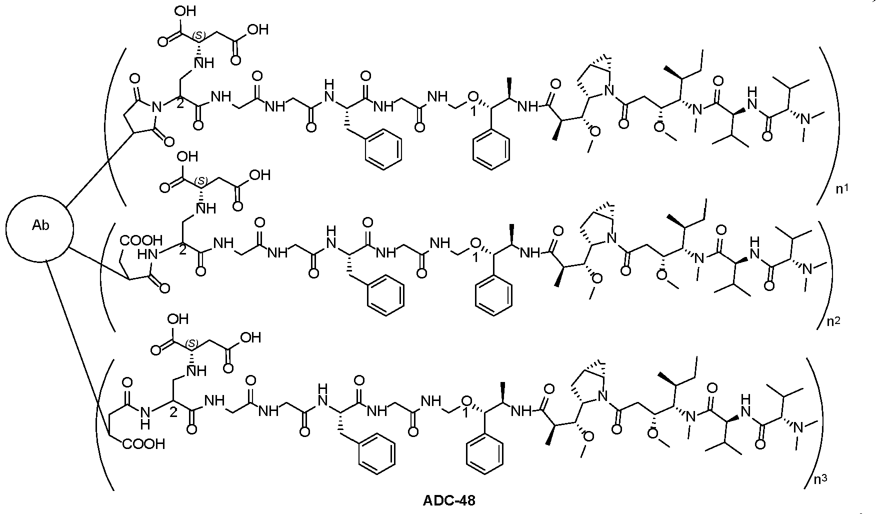 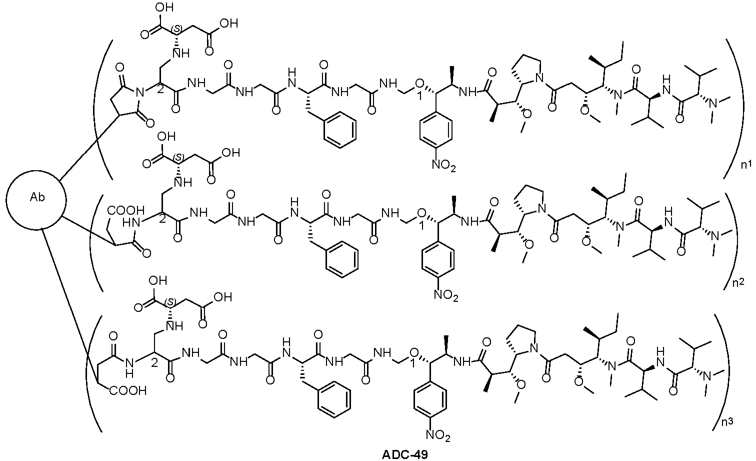 or 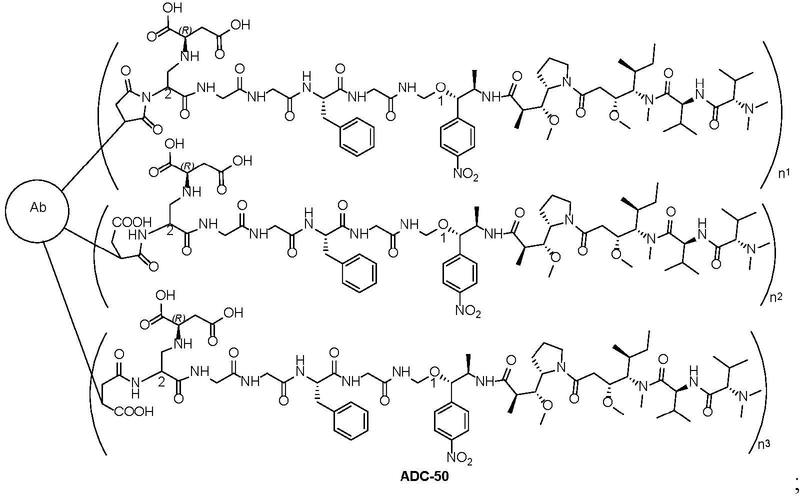 wherein the configuration of the chiral carbon at the 2-position is R- or S-.

14. The ligand-drug conjugate of any one of Claims 1-13 or a pharmaceutically acceptable salt or solvent compound thereof, said antibody being an anti-Trop2 antibody;
preferably, said antibody comprises a light chain and a heavy chain, said light chain comprising CDR-L1, CDR-L2 and CDR-L3 each having an amino acid sequence of SEQ ID NO: 1, SEQ ID NO: 2, SEQ ID NO: 3, respectively;
preferably, said heavy chain comprises CDR-H1, CDR-H2, CDR-H3 each having an amino acid sequence of SEQ ID NO: 4, SEQ ID NO: 5, SEQ ID NO: 6, respectively.

15. The ligand-drug conjugate of Claim 14 or a pharmaceutically acceptable salt or solvate thereof, wherein said light chain comprises a light chain variable region having an amino acid sequence of SEQ ID NO: 7;
preferably, said light chain further comprises a light chain constant region having an amino acid sequence of SEQ ID NO: 8
preferably, the amino acid sequence of said light chain is SEQ ID NO: 9.

16. The ligand-drug conjugate of Claim 14 or a pharmaceutically acceptable salt or solvate thereof, said light chain comprising a light chain variable region having an amino acid sequence of SEQ ID NO: 10;
preferably, said light chain further comprises a light chain constant region having an amino acid sequence of SEQ ID NO: 11
preferably, the amino acid sequence of said light chain is SEQ ID NO: 12.

17. The ligand-drug conjugate of Claim 15 or 16 or a pharmaceutically acceptable salt or solvate thereof, said heavy chain comprising a heavy chain variable region having an amino acid sequence of SEQ ID NO: 13;
preferably, said heavy chain further comprises a heavy chain constant region having an amino acid sequence of SEQ ID NO: 14
preferably, the amino acid sequence of said heavy chain is SEQ ID NO: 15.

18. The ligand-drug conjugate of any one of Claims 1-13 or a pharmaceutically acceptable salt or solvent compound thereof, said antibody being an anti-Trop2 antibody; said antibody comprising a light chain and a heavy chain, said light chain comprising CDR-L1, CDR-L2, and CDR-L3 each having an amino acid sequence as shown in SEQ ID NO: 31, SEQ ID NO: 32, , SEQ ID NO: 33, respectively;
preferably, said heavy chain comprises CDR-H1, CDR-H2, CDR-H3 each having an amino acid sequences as shown in SEQ ID NO:34, SEQ ID NO:35, , SEQ ID NO:36, respectively;
preferably, said light chain comprises a light chain variable region having amino acid sequence of SEQ ID NO: 37;
preferably, said light chain further comprises a light chain constant region amino acid sequence of SEQ ID NO: 38;
preferably, said light chain comprises an amino acid sequence of SEQ ID NO: 39; preferably, said heavy chain comprises a heavy chain variable region having amino acid sequence of SEQ ID NO: 40;
preferably, said heavy chain further comprises a heavy chain constant region having amino acid sequence of SEQ ID NO: 41;
preferably, said heavy chain comprises an amino acid sequence of SEQ ID NO: 42.

19. A linker-payload as shown formula II, or isomers, mesomers, racemates, enantiomers or mixtures thereof, or pharmaceutically acceptable salts or solvates thereof, 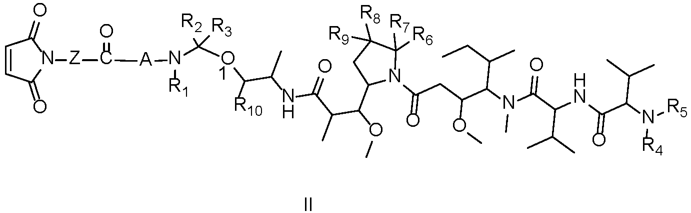 wherein:
Z, A, R₁, R₂, R₃, R₄, R₅, R₁₁, R₁₂, R₁₃, R₁₄, B, R₁₅, R₁₆, R₁₇, n, m, R₆, R₇, R₈, R₉, R₁₈, R₁₉, and R₁₀ are as defined in any one of Claims 1-10.

20. The linker-payload of Claim 19, or isomers, mesomers, racemates, enantiomers or mixtures thereof, or pharmaceutically acceptable salts or solvates thereof,
said linker-payload having the structure shown in formula IIa

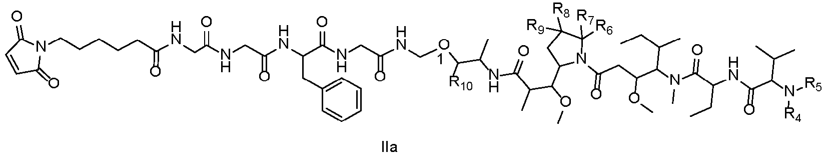

21. The linker-payload of Claim 20, or isomers, mesomers, racemates, enantiomers or mixtures thereof, or pharmaceutically acceptable salts or solvates thereof,
said linker-payload having the structure shown in formula IIb: 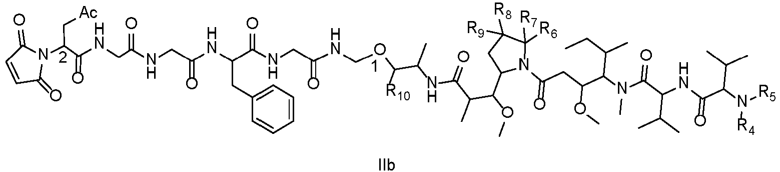 wherein Ac is a hydrophilic structural unit having the structure shown in formula c: 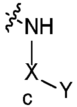 wherein X, Y are as defined in Claim 5, and Ac is linked, via -NH-, to 2-position methylene carbon as labeled in formula IIb.

22. The linker-payload of any one of Claims 19-21, or isomers, mesomers, racemates, enantiomers or mixtures thereof, or pharmaceutically acceptable salts or solvates thereof, said linker-payload being selected from the following structures: 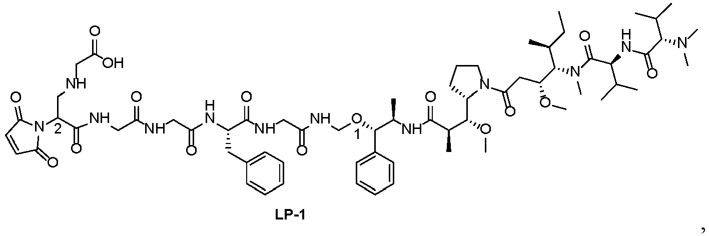 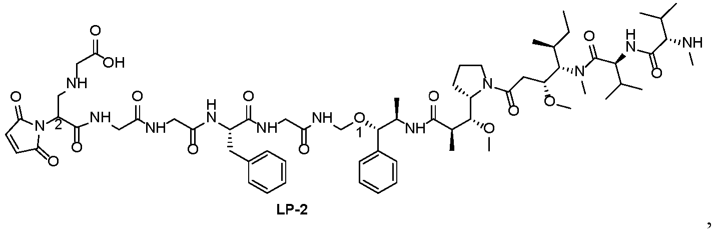 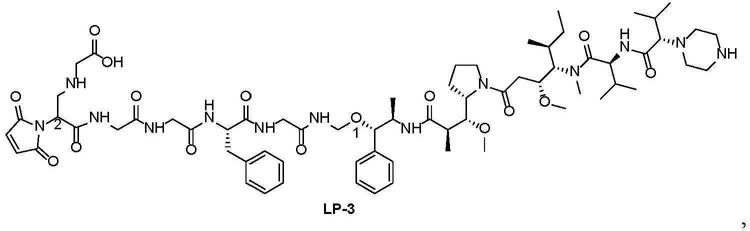 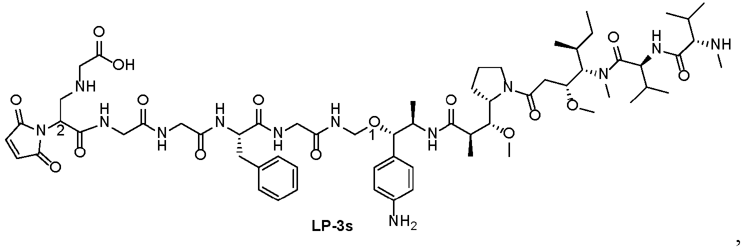 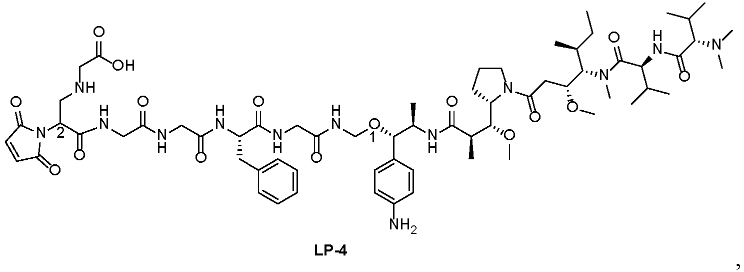 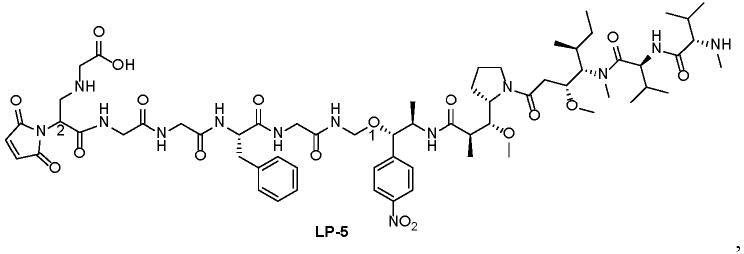 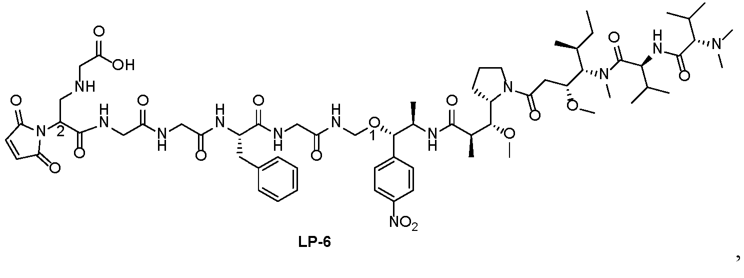 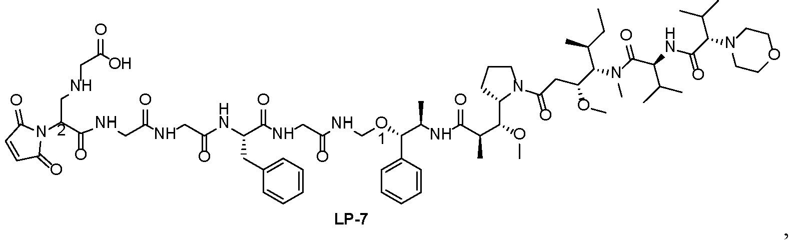 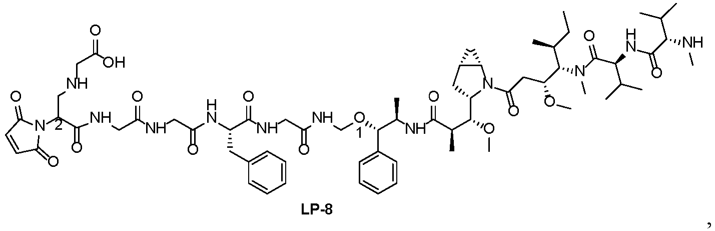 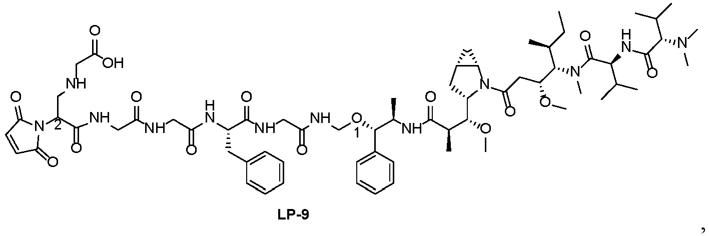 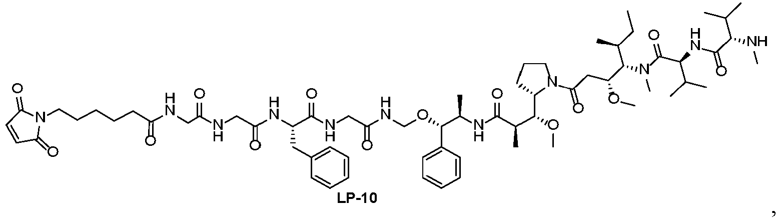 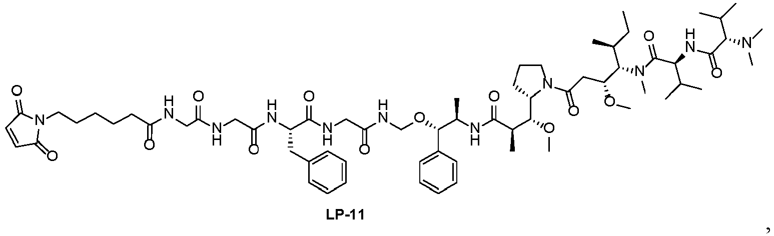 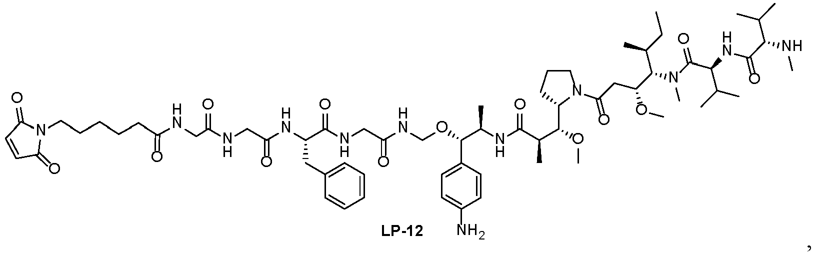 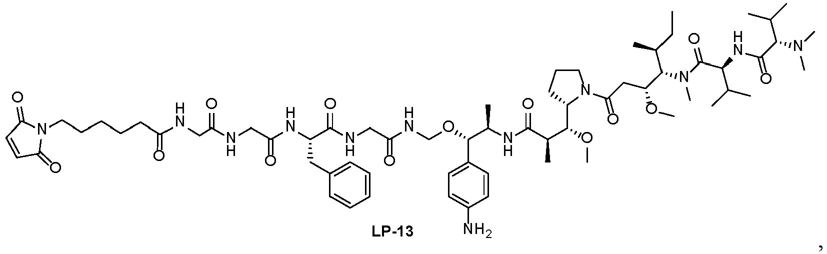 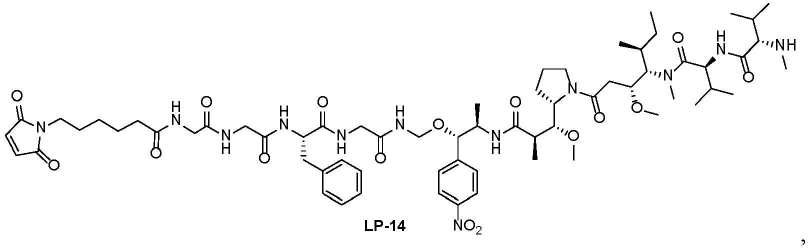 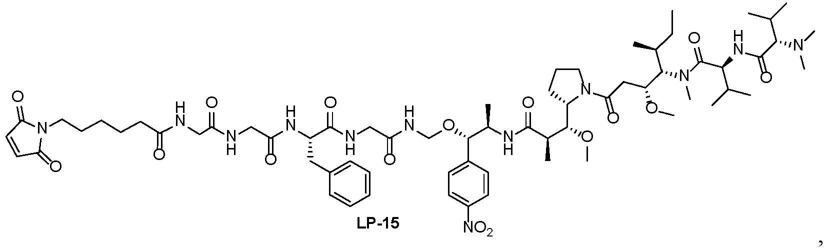 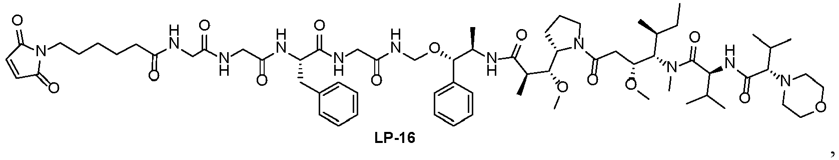 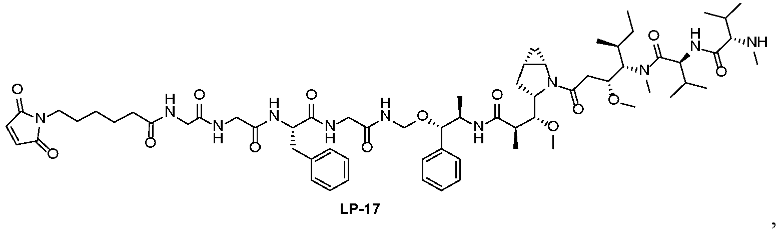 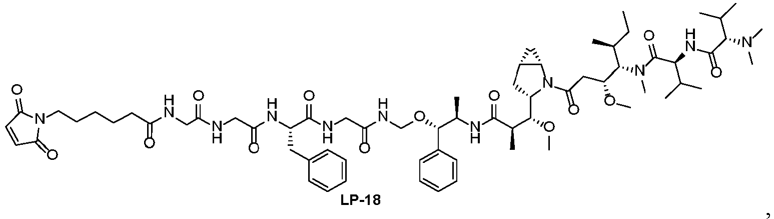 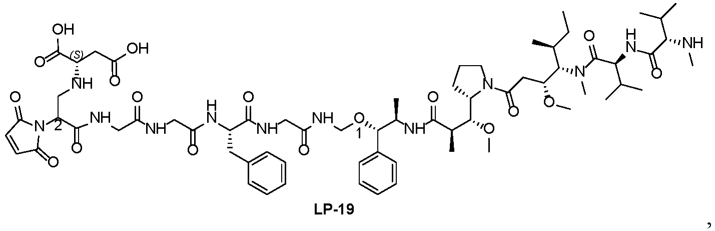 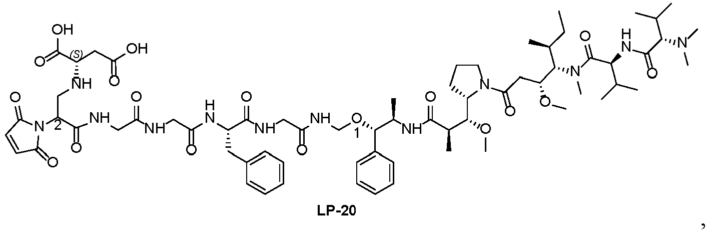 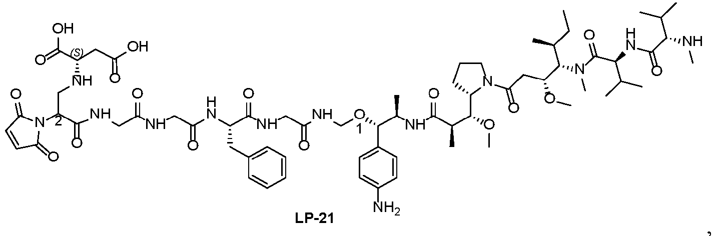 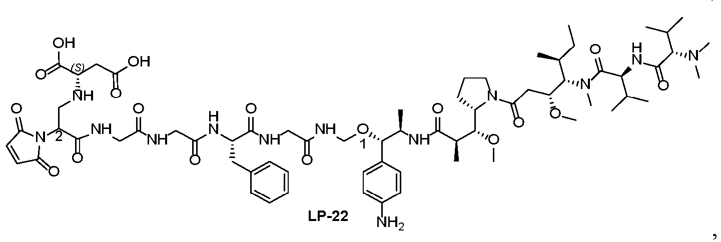 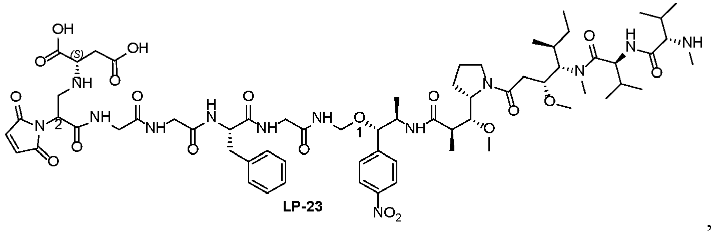 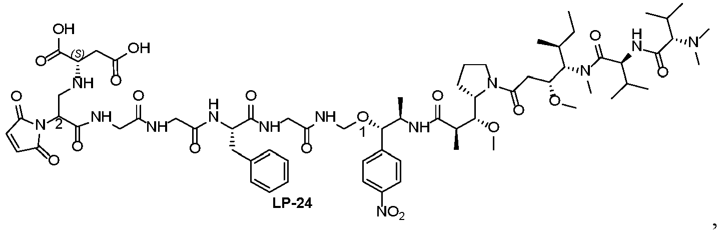 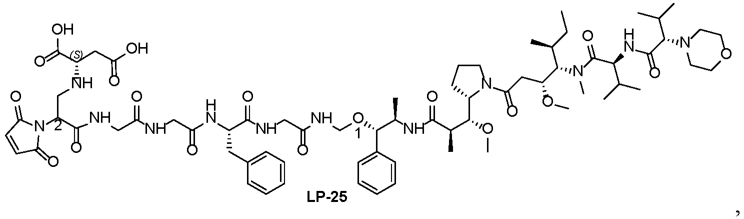 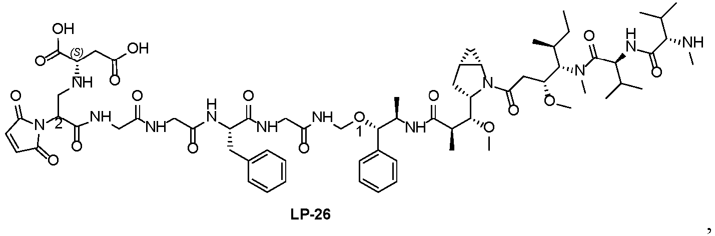 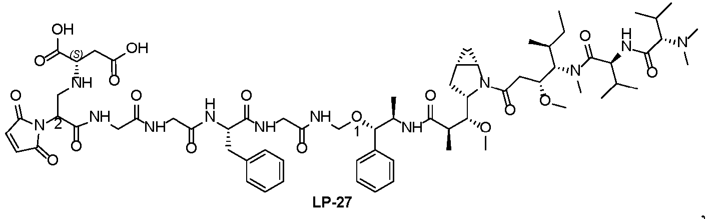 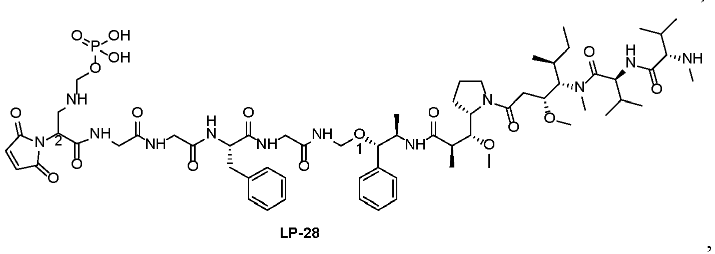 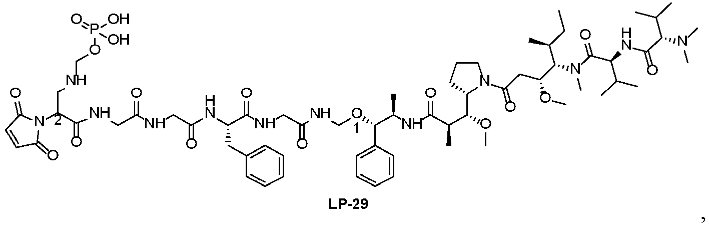 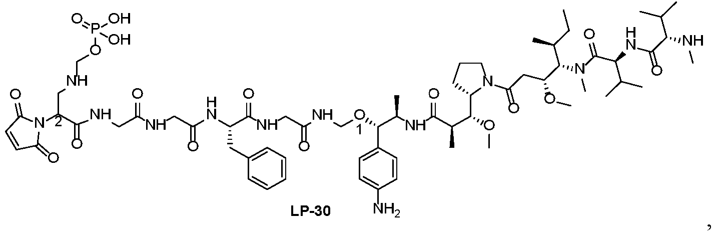 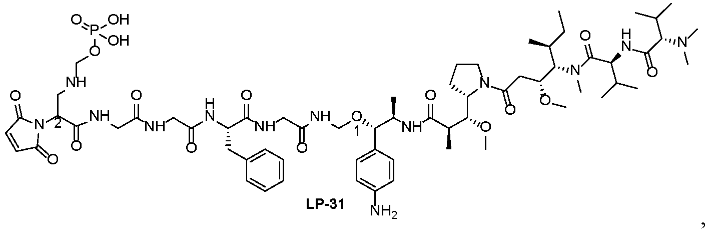 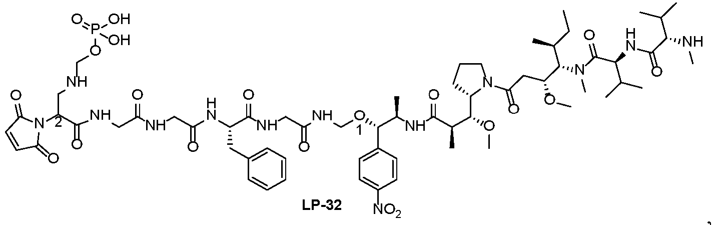 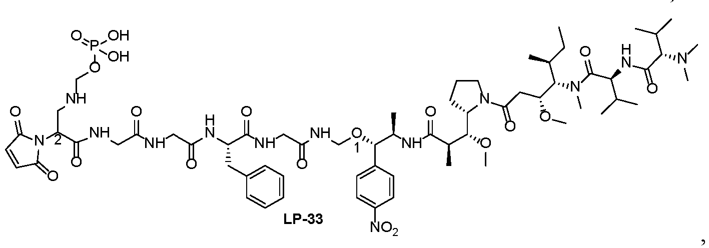 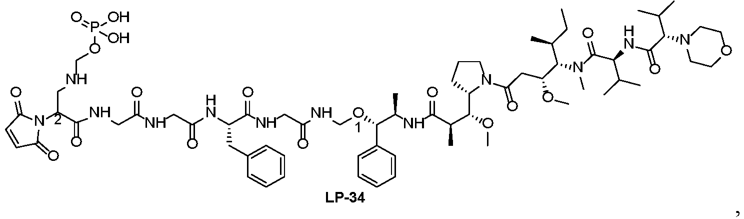 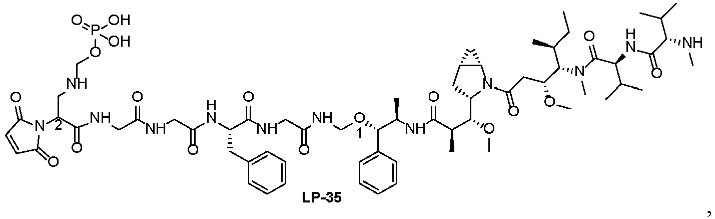 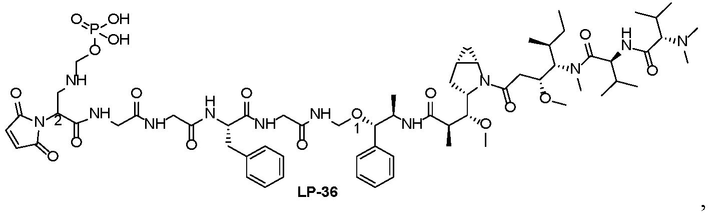 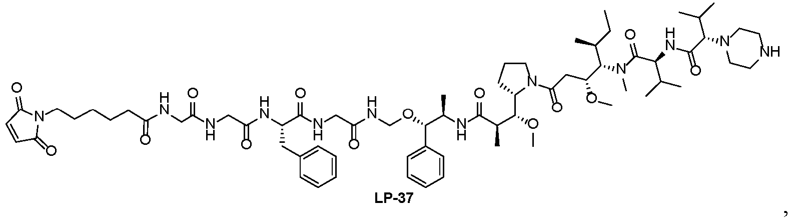 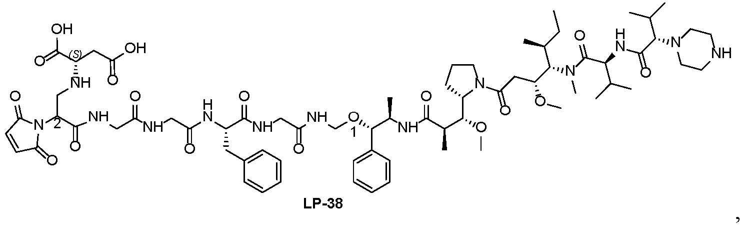 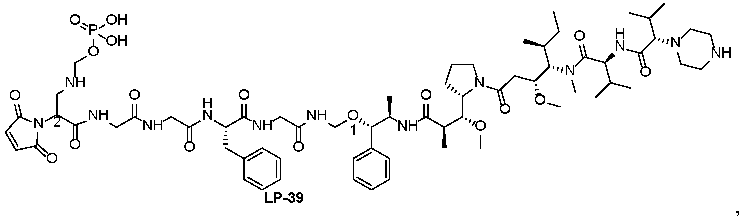 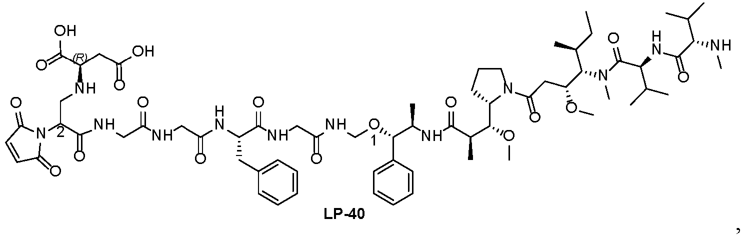 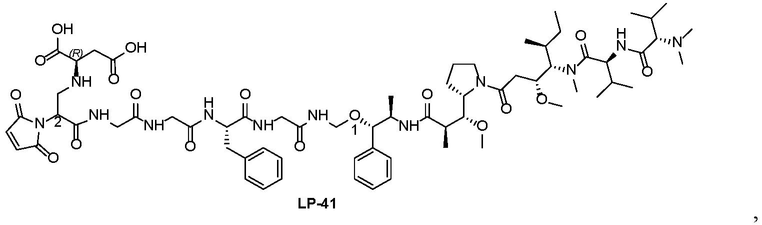 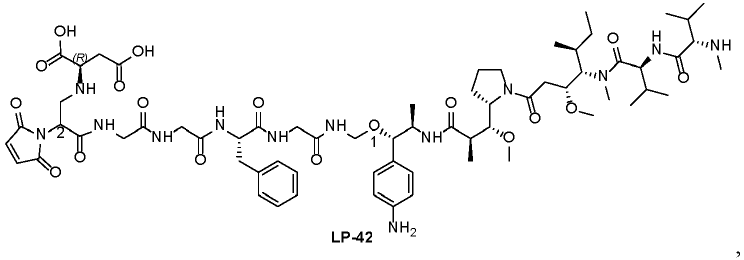 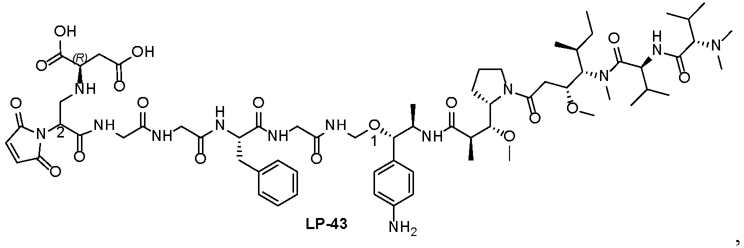 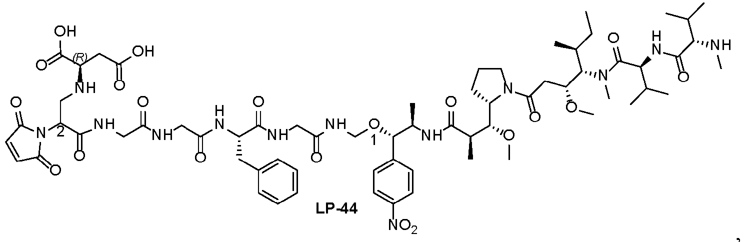 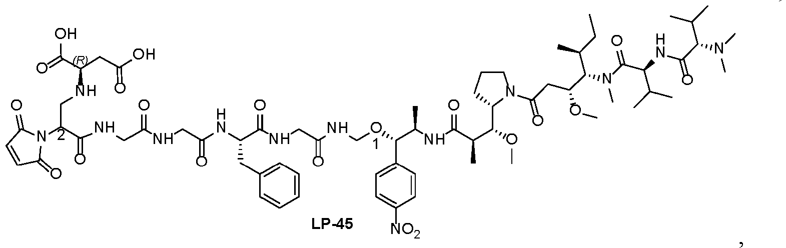 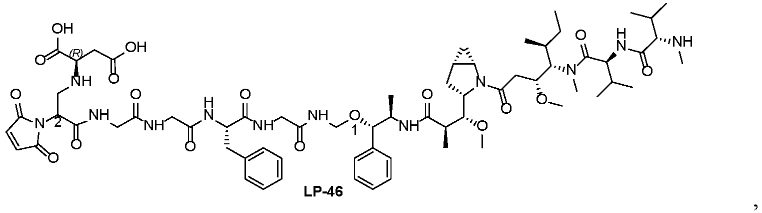 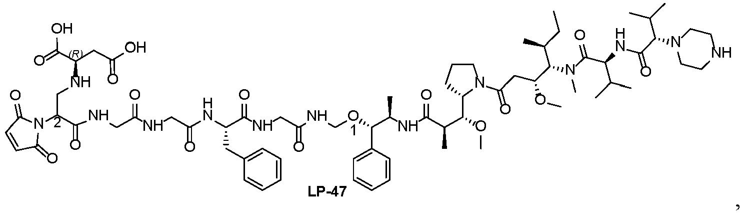 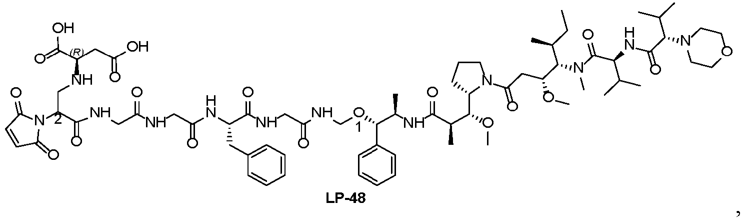 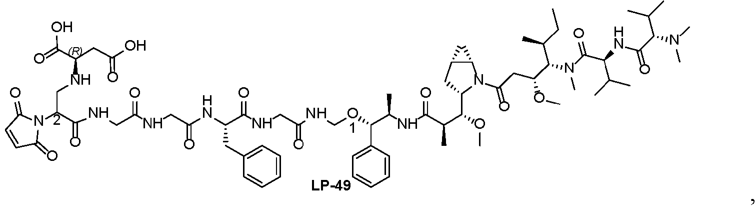 or 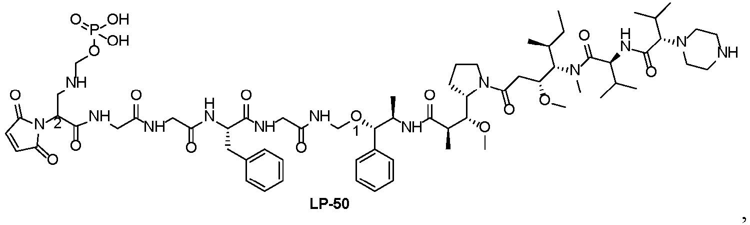 wherein the configuration of the chiral carbon at the 2-position is R- or S-.

23. Use of the ligand-drug conjugate or pharmaceutically acceptable salts or solvates thereof of any one of Claims 1-18, or the linker-payload or isomers, mesomers, racemates, enantiomers or mixtures thereof, or pharmaceutically acceptable salts or solvates thereof of any one of Claims 19-22, for the preparation of a drug, said drug is used for the treatment or prevention of tumors;
preferably, said tumors are selected from solid or non-solid tumors, such as breast cancer, ovarian cancer, cervical cancer, uterine cancer, prostate cancer, renal cancer, urothelial cancer, bladder cancer, hepatocellular carcinoma, gastric cancer, endometrial cancer, salivary gland cancer, esophageal cancer, lung cancer, colon cancer, rectal cancer, colorectal cancer, bone cancer, skin cancer, thyroid cancer, pancreatic cancer, melanoma, glioma, neuroblastoma, glioblastoma multiforme cell tumors, sarcomas, lymphomas, and leukemias.

24. A pharmaceutical composition comprising an effective amount of the ligand-drug conjugate or a pharmaceutically acceptable salt or solvate thereof of any one of Claims 1-18, or the linker-payload or isomers, mesomers, racemates, enantiomers or mixtures thereof, or pharmaceutically acceptable salts or solvates thereof of any one of Claims 19-22, and pharmaceutically acceptable carriers, diluents or excipients.

25. Use of the linker-payload or isomers, mesomers, racemates, enantiomers or mixtures thereof, or pharmaceutically acceptable salts or solvates thereof of Claims 19-22, for the preparation of a ligand-drug conjugate or pharmaceutically acceptable salts or solvates thereof,
preferably, said ligand-drug conjugate is selected from the ligand-drug conjugate of any one of Claims 1-18.
